(19) Europäisches Patentamt
European Patent Office
Office européen des brevets

(11) EP 4 306 558 A1

(12) EUROPEAN PATENT APPLICATION
published in accordance with Art. 153(4) EPC

(43) Date of publication:
**17.01.2024 Bulletin 2024/03**

(21) Application number: **21930420.1**

(22) Date of filing: **24.12.2021**

(51) International Patent Classification (IPC):
***C08F 212/14*** *(2006.01)* ***C08F 220/38*** *(2006.01)*
***A61B 5/25*** *(2021.01)* ***A61B 5/266*** *(2021.01)*

(52) Cooperative Patent Classification (CPC):
**A61B 5/25; A61B 5/259; A61B 5/263; A61B 5/266; A61B 5/268; C08F 212/14; C08F 220/38;**
Y02E 60/50

(86) International application number:
**PCT/JP2021/048074**

(87) International publication number:
**WO 2022/190575 (15.09.2022 Gazette 2022/37)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.03.2021 JP 2021040742**

(71) Applicant: **SHIN-ETSU CHEMICAL CO., LTD.**
**Tokyo 1000005 (JP)**

(72) Inventors:
- **HATAKEYAMA, Jun**
  **Joetsu-shi, Niigata 942-8601 (JP)**
- **IWABUCHI, Motoaki**
  **Joetsu-shi, Niigata 942-8601 (JP)**
- **IKEDA, Joe**
  **Tokyo 100-0005 (JP)**
- **WATANABE, Osamu**
  **Joetsu-shi, Niigata 942-8601 (JP)**
- **NONAKA, Shiori**
  **Joetsu-shi, Niigata 942-8601 (JP)**
- **HASEGAWA, Koji**
  **Tokyo 100-0005 (JP)**

(74) Representative: **Sonnenhauser, Thomas Martin**
**Wuesthoff & Wuesthoff**
**Patentanwälte PartG mbB**
**Schweigerstraße 2**
**81541 München (DE)**

# (54) BIOELECTRODE, PRODUCTION METHOD FOR BIOELECTRODE, AND MEASUREMENT METHOD FOR BIOSIGNALS

(57) The present invention is a bio-electrode including a porous, stretchy base material, an adhesive, conductive film containing silicon, and a conducting path. The conductive film is formed on one surface of the porous, stretchy base material. The conducting path is connected to the conductive film, and penetrates the stretchy base material to be exposed on the opposite side. This enables provision of the bio-electrode that has high sensitivity to a bio-signal and excellent biocompatibility, that is lightweight, that can be produced at low cost, that is free of itching, red spots, and rash of the skin and comfortable even when being wet, dried, or attached to the skin for a long period of time, without a significant decrease in sensitivity to the bio-signal, a production method for the bio-electrode, and a measurement method for a bio-signa

[FIG. 1]

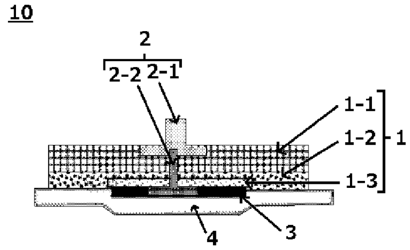


EP 4 306 558 A1

## Description

### TECHNICAL FIELD

The present invention relates to: a bio-electrode that is in contact with a skin of a living body, and that is capable of detecting a body state such as a heart rate using an electric signal from the skin; a production method for the bio-electrode; and a measurement method for a bio-signal.

### BACKGROUND ART

A recent growing popularity of Internet of Things (IoT) has accelerated the development of a wearable device. Representative examples of the wearable device include a watch and eye glasses that allow for Internet access. Even in the fields of medicine and sports, a wearable device for constantly monitoring the user's physical state is increasingly demanded, and is a growing field in the future. Especially, the worldwide epidemic of the new coronavirus (COVID-19) has caused a heavy medical burden, and there is a crying need for home care for people who are not infected by the virus and acceleration of the home care.

In the field of medicine, for example, sold is a wearable device that monitors the state of an organ of the body by sensing a small current, like electrocardiographic measurement for sensing the motion of the heart using an electric signal. In the electrocardiographic measurement, electrodes to which a conductive paste is applied are put on the body for measurement, which is only single-time measurement for a short period of time. In contrast, the development of the medical wearable device as described above aims at developing a device that constantly monitors a health state for consecutive several weeks. Thus, a bio-electrode used in a medical wearable device is required to be capable of acquiring a bio-signal even if used for a long period of time in daily life with showering, bathing, sweating, and the like, be free of itching and skin allergy, and have comfortableness. In addition to these requirements, the bio-electrode is also required to be light and thin to such an extent as to provide no feeling of wearing, and be able to be produced at low cost with high productivity.

The electrocardiographic measurement has become possible by a wristwatch device represented by an Apple Watch and contactless sensing with radar. However, an electrocardiograph of a type of which bio-electrodes are attached to the body at several locations is still required for high-accuracy electrocardiographic measurement for medical purposes.

As the medical wearable device, there is a type adhering to the body and a type incorporated into clothing. As the type adhering to the body, widely used is a bio-electrode that is made of the above-mentioned conductive paste material, that uses a hydrophilic gel containing water and electrolytes, and that is described in, for example, Patent Literature 1. The hydrophilic gel contains, as the electrolytes, sodium, potassium, and calcium in a hydrophilic polymer to retain water, and converts a change in concentration of ions from the skin to an electric signal by a reduction reaction of silver chloride that is in contact with the hydrophilic gel. In contrast, as the type incorporated into clothing, proposed is a method using a cloth in which a conductive polymer such as poly-3,4-ethylenedioxythiophene-polystyrenesulfonate (PEDOT-PSS) or a silver paste is incorporated into fibers for an electrode (Patent Literature 2).

In a case where the above-mentioned hydrophilic gel that contains water and electrolytes is used, however, there are an issue that conductivity is lost when the hydrophilic gel runs dry, and an issue that a gel (conductive gel) expands when the hydrophilic gel comes in contact with water by showering or bathing and comes off the skin. To address this, an attempt to attach a protective film for the gel to increase water resistance or the like has been made (Patent Literature 3). When a water-resistant base material is used as a base material that is in contact with the gel to increase water resistance, there are various issues such as an issue that the skin is prevented from breathing and itching occurs when the wearable device has been attached for a long period of time, an issue that an increase in the number of sheets of gel and the number of base materials increases a film thickness of the gels and the base materials in combination, and thereby increases a feeling of strangeness when the wearable device is put on the skin, and the wearable device easily comes off due to friction against clothing. As a result, the wearable device is difficult to be worn for a long period of time.

Meanwhile, also in a case where the conductive polymer such as the PEDOT-PSS is used, there are an issue of risk of causing skin allergy due to high acidity of the conductive polymer, an issue that a feeling of discomfort increases due to wearing of clothing containing the conductive polymer for many consecutive days, an issue that measurement during bathing cannot be performed because of necessity for undressing during bathing, and an issue that the conductive polymer peels away from fibers during laundry.

A dry bio-electrode using conductive silicone has been proposed (Patent Literature 4). In this dry bio-electrode, ion conductivity is improved by addition of a silicone compound, in which alkali metal salts of sulfonic acid defined as a surface-active agent and a cleaning agent is pendent, to a silicone rubber that is intrinsically insulating, and a bio-signal is detected.

The bio-electrode described in the above-mentioned Patent Literature 4 does not have adhesiveness. To

acquire the bio-signal accurately even when the body is moving, the bio-electrode needs to be constantly in contact with the skin. Thus, the bio-electrode needs to have the adhesiveness. Among the conductive gels described above, there are some gels without the adhesiveness. Although there is a case where the adhesiveness is ensured by an adhesive layer that is attached to the periphery of the conductive gel, the conductive layer itself needs to have the adhesiveness to obtain a bio-signal more stably.

A bio-electrode material in which an ionic polymer is mixed with a silicone adhesive has been proposed (Patent Literatures 5 and 6). In each bio-electrode material, an ionic polymer that has high ion conductivity and that does not penetrate the skin is combined with a silicone adhesive that is less allergic to the skin, that has high water repellency, and that provides an effect of preventing occurrence of itching and redness of the skin after the bio-electrode material is peeled off, whereby bio-signals can be stably obtained without coming-off of the bio-electrode material even if the bio-electrode material has been attached for a long period of time including daily bathing and exercise. However, the bio-electrode material has been required to improve comfortableness at the time of being attached and worn for a longer period of time.

CITATION LIST

PATENT LITERATURE

Patent Document 1: WO 2013/039151 A1
Patent Document 2: JP 2015-100673 A
Patent Document 3: JP 2000-271100 A
Patent Document 4: JP 2018-515279 A
Patent Document 5: JP 2018-126496 A
Patent Document 6: JP 2018-130533 A

SUMMARY OF INVENTION

TECHNICAL PROBLEM

The present invention has been made in view of the above-described circumstances. An object of the present invention is to provide: a bio-electrode that has high sensitivity to a bio-signal and excellent bio-compatibility, that is lightweight, that can be produced at low cost, that is free of itching, red spots, rash, and the like of the skin and comfortable even when being wet, dried, or attached to the skin for a long period of time, without a significant decrease in sensitivity to the bio-signal; a production method for the bio-electrode; and a measurement method for a bio-signal.

SOLUTION TO PROBLEM

To achieve the object, the present invention provides a bio-electrode comprising:

a porous, stretchy base material;
an adhesive, conductive film containing silicon; and
a conducting path,
wherein the conductive film is formed on one surface of the porous, stretchy base material,
the conducting path is connected to the conductive film, and penetrates the stretchy base material to be exposed on the opposite side, or is exposed on a side surface of the stretchy base material.

Such a bio-electrode has high sensitivity to a bio-signal and excellent bio-compatibility, that is lightweight, that can be produced at low cost, that is free of itching, red spots, rash, and the like of the skin, and comfortable even when being wet, dried, or attached to the skin for a long period of time, without a significant decrease in sensitivity to the bio-signal.

In the present invention, the porous, stretchy base material is preferably a non-woven fabric or a membrane film.

Such a stretchy base material can implement the bio-electrode that allows the skin to breathe even when being attached to the skin for a long period of time.

In the present invention, the adhesive conductive film containing the silicon preferably contains an ionic material (A) selected from the group consisting of salts of ammonium, lithium, sodium, potassium, and silver formed with any of fluorosulfonic acid, fluorosulfonimide, and N-carbonylfluorosulfonic amide.

Such an adhesive conductive film does not swell by absorption of water even in a state of being constantly and closely attached to the skin and immersed in water, keeps adherence for a long period of time, and is capable of stably obtaining an electric signal.

In this case, the above-mentioned ionic material (A) preferably has a partial structure shown by the following general formulae (1)-1 to (1)-4,

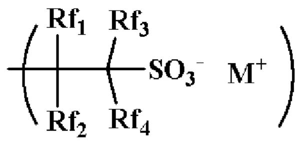

(1)-1

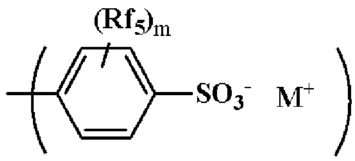

(1)-2

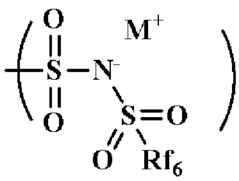

(1)-3

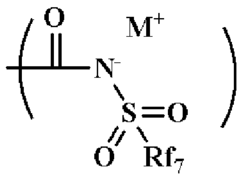

(1)-4

wherein $Rf_1$ and $Rf_2$ each represent a hydrogen atom, a fluorine atom, an oxygen atom, a methyl group, or a trifluoromethyl group, provided that when $Rf_1$ and $Rf_2$ represent an oxygen atom, the single oxygen atom represented by $Rf_1$ and $Rf_2$ bonds to a single carbon atom to form a carbonyl group; $Rf_3$ and $Rf_4$ each represent a hydrogen atom, a fluorine atom, or a trifluoromethyl group, provided that at least one of $Rf_1$ to $Rf_4$ is a fluorine atom or a trifluoromethyl group; $Rf_5$, $Rf_6$, and $Rf_7$ each represent a fluorine atom, a trifluoromethyl group, or a linear or branched alkyl group having 1 to 4 carbon atoms, and have at least one fluorine atom; $M^+$ represents an ion selected from the group consisting of an ammonium ion, a sodium ion, a potassium ion, and a silver ion; and, "m" represents an integer of 1 to 4.

The ionic material (A) having such a partial structure can implement the bio-electrode having more excellent conductivity and bio-compatibility.

The above-mentioned ionic material (A) comprises at least one ionic polymer selected from the group consisting of repeating units A1 to A7 shown by the following general formula (2),

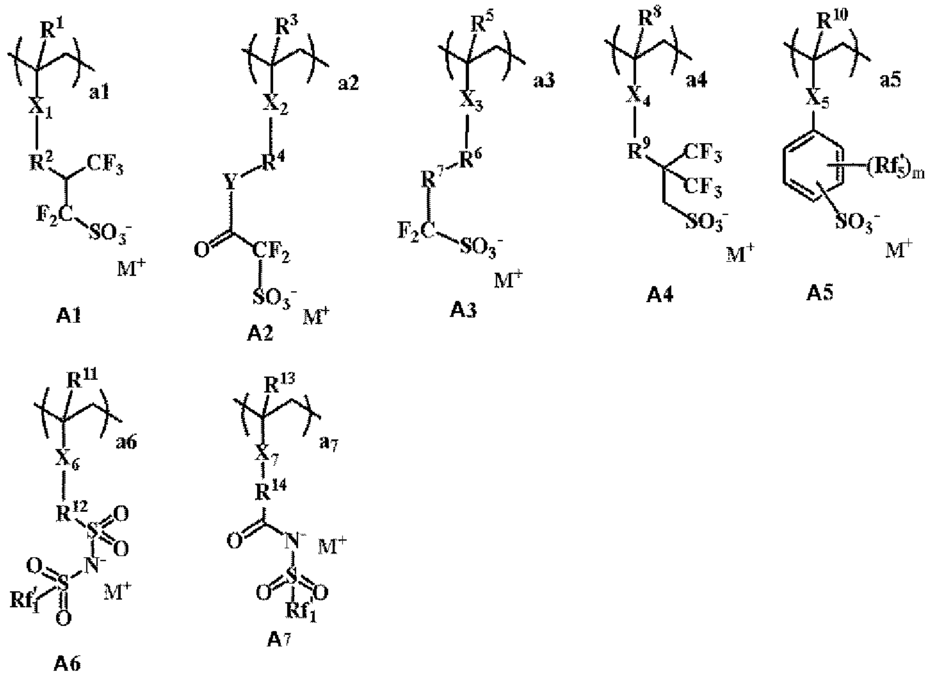

A1 A2 A3 A4 A5 A6 A7

(2)

wherein $R^1$, $R^3$, $R^5$, $R^8$, $R^{10}$, $R^{11}$, and $R^{13}$ each independently represent a hydrogen atom or a methyl group; $R^2$, $R^4$, $R^6$, $R^9$, $R^{12}$, and $R^{14}$ each independently represent a single bond, or a linear, branched, or cyclic hydrocarbon group having 1 to 13 carbon atoms, the hydrocarbon group optionally having either or both of an ester group and an ether group; $R^7$ represents a linear or branched alkylene group having 1 to 4 carbon atoms, and one or two hydrogen atoms in $R^7$ are optionally substituted with a fluorine atom; $X_1$, $X_2$, $X_3$, $X_4$, $X_6$ and $X_7$ each independently represent any of a single bond, a phenylene group, a naphthylene group, an ether group, an ester group, and an amide group; $X_5$ represents any of a single bond, an ether group, and an ester group; Y represents any of an oxygen atom and a $-NR^{19}-$ group; $R^{19}$ represents any of a hydrogen atom, a linear, branched, or cyclic alkyl group having 2 to 12 carbon atoms, and a phenyl group, and optionally have one or more selected from the group consisting of an ether group, a carbonyl group, an ester group, and an amide group; Y optionally forms a ring together with $R^4$; $Rf_1$' and $Rf_5$' each represent a fluorine atom, a trifluoromethyl group, or a linear or branched alkyl group having 1 to 4 carbon atoms, and have at least one fluorine atom; "m" represents an integer of 1 to 4; a1, a2, a3, a4, a5, a6, and a7 are $0 \leq a1 \leq 1.0$, $0 \leq a2 \leq 1.0$, $0 \leq a3 \leq 1.0$, $0 \leq a4 \leq 1.0$, $0 \leq a5 \leq 1.0$, $0 \leq a6 \leq 1.0$, $0 \leq a7 \leq 1.0$, and $0 < a1+a2+a3+a4+a5+a6+a7 \leq 1.0$; $M^+$ represents an ion selected from the group consisting of an ammonium ion, a sodium ion, a potassium ion, and a silver ion.

The ionic material (A) having such a structure can implement the bio-electrode having furthermore excellent conductivity and bio-compatibility.

In the present invention, the above-described ionic material (A) preferably contains an ammonium ion shown by the following general formula (3) as an ammonium ion for forming the ammonium salts,

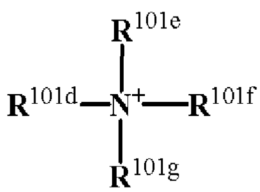

(3)

wherein $R^{101d}$, $R^{101e}$, $R^{101f}$, and $R^{101g}$ each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, and optionally have one or more selected from the group consisting of an ether group, a carbonyl group, an ester group, a hydroxy group, an amino group, a nitro group, a sulfonyl group, a sulfinyl group, a halogen atom, and a sulfur atom; and $R^{101d}$ and $R^{101e}$, or $R^{101d}$, $R^{101e}$, and $R^{101f}$, are optionally bonded to each other together with a nitrogen atom bonded therewith to form a ring in which $R^{101d}$ and $R^{101e}$, or $R^{101d}$, $R^{101e}$, and $R^{101f}$, represent an alkylene group having 3 to 10 carbon atoms, or to form a heteroaromatic ring having the nitrogen atom in the general formula (3) within the ring.

The bio-electrode, which includes the ionic material (A) containing such an ammonium ion, can have further excellent conductivity and bio-compatibility.

The bio-electrode preferably contains, in addition to the above-mentioned component (A), an adhesive resin as a component (B) that is different from the component (A), which is one or more selected from the group consisting of a silicone resin, a (meth) acrylate resin, and a urethane resin.

Such a resin (B) is compatibilized with the ionic material (A) (salts) and prevents elution of salts, retains a conductivity improver such as a metal powder, a carbon powder, a silicon powder, a lithium titanate powder, contains silicone for imparting water repellency, and is capable of manifesting adhesiveness.

At this time, the component (B) preferably contains a diorganosiloxane having an alkenyl group and an organohydrogenpolysiloxane having a SiH group.

Such a resin (B) is compatibilized with the ionic material (A) and is capable of preventing elution of salts.

At this time, the component (B) preferably further contains a silicone resin having an $SiO_2$ unit and an $R_xSiO_{(4-x)/2}$ unit, wherein R represents a substituted or unsubstituted monovalent hydrocarbon group having 1 to 10 carbon atoms, and "x" represents a number in a range from 2.5 to 3.5.

Even when such a resin (B) is used, the resin (B) is compatibilized with the ionic material (A) and is capable of preventing elution of salts.

In the present invention, the bio-electrode preferably further contains a component (C), which is one or more selected from the group consisting of a carbon powder, a metal powder, a silicon powder, and a lithium titanate powder.

Since the carbon powder and the metal powder each function as a conductivity improver and are capable of imparting more excellent conductivity and the silicon powder and the lithium titanate powder are capable of further increasing ion reception sensitivity, it is possible to implement a more preferable bio-electrode.

At this time, the carbon powder is preferably one or both of carbon black and carbon nanotube.

The bio-electrode containing such a carbon powder is capable of providing higher conductivity.

In the present invention, the conducting path preferably includes one or more selected from the group consisting of gold, silver, silver chloride, platinum, aluminum, magnesium, tin, tungsten, iron, copper, nickel, stainless steel, chromium, titanium, carbon, and a conductive polymer.

In the bio-electrode according to the present invention, such a conducting path can be preferably used.

In the present invention, the conducting path preferably has a snap shape.

Such a conducting path can be easily connected to a device for receiving a bio-signal and processing the bio-signal.

In the present invention, a film thickness of the stretchy base material and the conductive film in combination is preferably 1 mm or less.

With such a film thickness, the bio-electrode is formed in a thin film, and is comfortably worn with a less feeling of wearing.

At this time, the total film thickness of the stretchy base material and the conductive film in combination is preferably 500 $\mu$m or less.

With such a film thickness, the bio-electrode is more comfortably worn.

In the present invention, the stretchy base material preferably includes an adhesive layer.

Such a stretchy base material is capable of increasing an adhesive force of the bio-electrode.

In the present invention, a top of the conductive film is preferably covered with a release liner.

Such a bio-electrode can be preferably used.

At this time, the release liner is selected from the group consisting of a fluorine resin, polyethylene, polypropylene, cyclic polyolefin, polyethylene terephthalate, polybutylene terephthalate, polyethylene naphthalate, polybutylene naphthalate, polyvinyl chloride, polymethylmethacrylate, polyvinyl acetate, polystyrene, polymethylpentene, polyimide, polyphenylene sulfide, polysulfone, polyethersulfone, polyetherketone, polyamide-imide, polyetherimide, polyacetal, polycarbonate, polyphenylene ether, polyacrylonitrile, cellophane, and paper, to which, excluding the fluorine resin, a fluorine-based peeling agent or a silicone/fluorine-based peeling agent is preferably applied.

The release liner used in the bio-electrode according to the present invention as described above is easily peeled off the conductive film.

The present invention provides a production method for the bio-electrode, the method comprising:

preparing a conducting path that penetrates a porous, stretchy base material, or preparing a conducting path on the stretchy base material so that the conducting path is exposed on a side surface of the stretchy base material and forming an adhesive, conductive film comprising silicon so as to be connected to one side of the conducting path, the one side being attached to a skin.

According to such a production method, it is possible to easily produce, at low cost, a bio-electrode that has high sensitivity to a bio-signal and excellent bio-compatibility, that is lightweight, that is free of itching, red spots, rash, and the like of the skin, and comfortable even when being wet, dried, or attached to the skin for a long period of time, without a significant decrease in sensitivity to the bio-signal.

At this time, it is possible to form the adhesive, conductive film containing silicon on one side of the conducting path that penetrates the porous, stretchy base material, or the conducting path that is on the stretchy base material and exposed on a side surface of the stretchy base material, the one side being attached to a skin, by transferring the formed, adhesive, conductive film comprising silicon onto a release linear or by directly printing the formed, adhesive, conductive film comprising silicon onto the conducting path.

According to such a production method, it is possible to produce the bio-electrode of the present invention more easily.

At this time, it is preferable to apply an adhesive, conductive film material containing silicon onto a release linear, and curing the adhesive, conductive film material.

According to such a production method, it is possible to produce the bio-electrode of the present invention more easily.

The present invention provides a measurement method for a bio-signal comprising:

attaching the bio-electrode to a skin; and
measuring the bio-signal after bathing or showering, or during bathing or showering.

The bio-electrode according to the present invention has water repellency, and thereby enables execution of such a measurement method for a bio-signal.

ADVANTAGEOUS EFFECTS OF INVENTION

As described above, the bio-electrode, the production method for the bio-electrode, and the measurement method for the bio-signal according to the present invention enable provision of the bio-electrode that has high sensitivity to the bio-signal and excellent bio-compatibility, that is lightweight, that can be produced at low cost, that is free of itching, red spots, rash, and the like of the skin, and comfortable even when being wet, dried, or attached to the skin for a long period of time, without a significant decrease in sensitivity to the bio-signal, the production method for the bio-electrode, the measurement method for the bio-signal.

BRIEF DESCRIPTION OF DRAWINGS

FIG. 1 is a schematic cross-sectional illustration showing a bio-electrode according to the present invention, and showing that upper and lower snaps are arranged at the center of a conductive film;
FIG. 2 is a schematic cross-sectional illustration showing an example of a component configuration before the bio-electrode according to the present invention is assembled;
FIG. 3 is a schematic bird's-eye illustration showing an example of a component configuration before the bio-electrode according to the present invention is assembled;
FIG. 4 is a schematic cross-sectional illustration showing an example of a production method for the bio-electrode according to the present invention;
FIG. 5 is a schematic cross-sectional illustration showing another example of the production method for the bio-electrode according to the present invention;
FIG. 6 is a schematic cross-sectional illustration showing still another example of the production method for the bio-electrode according to the present invention;
FIG. 7 is a schematic cross-sectional illustration showing still another example of the production method for the bio-electrode according to the present invention;
FIG. 8 is a schematic cross-sectional illustration showing the bio-electrode in a lead line whose terminal is connected to a female connector;
FIG. 9 is a schematic cross-sectional illustration showing the bio-electrode in a lead line whose terminal is connected to a male connector;
FIG. 10 is a photograph of the bio-electrode produced according to an embodiment of the present invention;
FIG. 11 is a schematic cross-sectional illustration showing a thickness of each component of the bio-electrode produced according to the embodiment of the present invention;
FIG. 12 is a schematic cross-sectional illustration showing a thickness of a portion in which a porous film and conductive film of the bio-electrode produced according to the embodiment of the present invention overlap with each other;
FIG. 13 is a schematic cross-sectional illustration showing a diameter of each component of the bio-electrode produced according to the embodiment of the present invention;

FIG. 14 is a schematic cross-sectional illustration showing the bio-electrode according to the present invention, and showing that the upper and lower snaps are arranged outside the conductive film;

FIG. 15 is a schematic cross-sectional illustration showing an example of a component configuration before the bio-electrode according to the present invention is assembled, and the upper and lower snaps are arranged outside the conductive film;

FIG. 16 is a schematic bird's-eye illustration showing an example of a component configuration before the bio-electrode according to the present invention is assembled, and showing that the upper and lower snaps are arranged outside the conductive film;

FIG. 17 is a schematic cross-sectional illustration showing the bio-electrode according to the present invention, and a surface of the upper snap has a flat shape;

FIG. 18 is a schematic cross-sectional illustration showing the bio-electrode according to the present invention, and showing that the upper and lower snaps are arranged outside a silicone conductive film and the lower snap is covered with the silicon conductive film;

FIG. 19 is a schematic cross-sectional illustration showing an example of a component configuration before the bio-electrode according to the present invention is assembled, and showing that the upper and lower snaps are arranged outside the silicone conductive film and the lower snap is covered with the silicon conductive film;

FIG. 20 is a schematic bird's-eye illustration showing an example of a component configuration before the bio-electrode according to the present invention is assembled, and showing that the upper and lower snaps are arranged outside the silicone conductive film and the lower snap is covered with the silicone conductive film;

FIG. 21 is a photograph of the bio-electrode produced according to the embodiment of the present invention, and showing that the upper and lower snaps are arranged outside the silicone conductive film, and the lower snap is covered with the silicone conductive film;

FIG. 22 is a schematic cross-sectional illustration showing the bio-electrode according to the present invention, and showing that the upper and lower snaps are arranged outside the conductive film, penetrate a non-woven fabric, and are located on the opposite side of the conductive film;

FIG. 23 is a photograph of the bio-electrode that is produced according to the embodiment of the present invention and that is shown in FIG. 22;

FIG. 24 is a schematic cross-sectional illustration showing the bio-electrode according to the present invention, and showing a form where the upper and lower snaps of the bio-electrode shown in FIG. 22 are not attached;

FIG. 25 is a photograph of the bio-electrode that is produced according to the embodiment of the present invention and that is shown in FIG. 24;

FIG. 26 is a photograph showing that a conductive wiring line and a clip are attached to the bio-electrode that is produced according to the embodiment of the present invention and that is shown in FIG. 24;

FIG. 27 is a schematic cross-sectional illustration showing the bio-electrode according to the present invention in a form in which the conductive wiring line and the film in the bio-electrode shown in FIG. 24 are replaced with conductive fibers;

FIG. 28 is a schematic illustration showing that the conductive film, the conductive wiring line, and the film in the bio-electrode shown in FIG. 14 are perforated;

FIG. 29 is a schematic illustration showing that the conductive film, the conductive wiring line, the film, and the non-woven fabric in the bio-electrode shown in FIG. 14 are perforated;

FIG. 30 is a schematic illustration showing that the conductive film, the conductive wiring line, the film, the non-woven fabric in a location excluding an adhesive layer, and the adhesive layer in the bio-electrode shown in FIG. 14 are perforated;

FIG. 31 is a schematic cross-sectional illustration showing the bio-electrode according to the present invention in a form in which the conductive wiring line in contact with the conductive film does not penetrate the non-woven fabric;

FIG. 32 is a schematic cross-sectional illustration showing the bio-electrode according to the present invention in a form in which the conductive wiring line and the film shown in FIG. 31 are replaced with conductive fibers;

FIG. 33 is a photograph showing that a sheet in which a top and bottom of the conductive film is sandwiched by release liners, respectively;

FIG. 34 is an illustration showing locations where electrodes and a grounding terminal are attached to the human body when a bio-signal is measured in an example of the present invention;

FIG. 35 shows one electrocardiographic waveform that is obtained by using the bio-electrode according to the example of the present invention.

As described above, there has been a demand for development of a lightweight, thin-film bio-electrode that has high sensitivity, high conductivity for detecting a low noise bio-signal, and excellent bio-compatibility, that is lightweight, that can be produced at low cost, that is capable of measuring the bio-signal even when being wet or dried, and that is free of skin disorders and itching even when being attached to the skin for a long period of time, a production

method for the bio-electrode, and a measurement method for a bio-signal.

Sodium, potassium, and calcium ions are emitted from the skin surface in conjunction with a heartbeat. The bio-electrode needs to covert an increase/decrease of ions emitted from the skin into an electric signal. Thus, a material that has excellent ion conductivity for transmitting the increase/decrease of ions is required. A potential of the skin surface also fluctuates in conjunction with the heartbeat. This fluctuation of the potential is subtle, and electron conductivity for transmitting a small current to a device is also required.

A hydrophilic gel containing sodium chloride or potassium chloride has high ion conductivity and high electron conductivity, but loses conductivity when dried. In addition, elution of the sodium chloride or the potassium chloride to the outside of the bio-electrode by bathing or showering also decreases conductivity.

A bio-electrode using a metal such as gold and silver detects only a small current and has low ion conductivity, and thus has lower sensitivity as the bio-electrode. Carbon also has electron conductivity similarly to the metal, but has electron conductivity that is lower than that of the metal and sensitivity, as the bio-electrode, that is lower than that of the metal.

A conductive polymer represented by poly-3,4-ethylenedioxythiophene-polystyrenesulfonate (PEDOT-PSS) has both the electron conductivity and the ion conductivity, but the ion conductivity is low because of its low polarizability.

Slats of fluorosulfonic acid, fluorosulfonimide, and N-carbonylfluorosulfonic amide have high polarizability and high ion conductivity. Combining these salts with carbon or the like enables manifestation of both high ion conductivity and high electron conductivity.

The bio-electrode needs to have adherence to stably obtain a bio-signal by attaching the bio-electrode to the skin. Meanwhile, a residue on the skin after the bio-electrode is attached to the skin for a long period of time and peeled off could cause a rash or skin disorders.

Examples of causes for the rash, the skin disorders, itching, or the like include prevention of the skin from breathing. In a medical adhesive plaster that is assumed to be attached for a long period of time of one week or more, efforts have been made to form a base material of the adhesive plaster as a thin film and use a breathing non-woven fabric to allow the skin to breathe as much as possible, and emboss the surface to have concavo-convex shapes to facilitate sweat release.

As for a bio-electrode in which a conventional gel electrode without containing silicon is attached to a non-woven fabric in which a metal snap penetrates its center portion, at the time of bathing in a state where the bio-electrode is attached to the skin, the gel swells because water easily passes through the non-woven fabric and a volume of the bio-electrode expands, and the bio-electrode peels away from the skin. When a waterproof cover is attached to the bio-electrode to prevent penetration of water as described in the above-mentioned Patent Literature 3, the bio-electrode increases in thickness and does not provide a comfortable feeling of wearing.

If a waterproof conductive film could be combined with the thin-film, non-woven fabric, the conductivity would not be decreased by bathing. Furthermore, if the conductive film had adhesiveness, the bio-electrode could stably detect the bio-signal.

Examples of the waterproof conductive film can include a conductive film containing silicone. Furthermore, examples of an adhesive containing silicone can include a silicone adhesive, a urethane adhesive, and an acrylic adhesive. Among these adhesives, the silicone adhesive is the most preferable in terms of high water repellency, low occurrence of a rash on the skin, and allowing the skin to breathe.

Silicone is an insulating material as a basic property. Thus, in a case where the adhesive containing silicone is used as a base, a material for improving conductivity is required. While examples of a conductivity improver can include a metal powder and a carbon powder, not only a small potential is generated from the skin, but also ions such as sodium, potassium, and calcium are emitted from the skin, as described above. Hence, the material needs to have properties of high sensitivity to the increase/decrease of ions and high ion conductivity.

To improve ion conductivity of an ion battery, addition of an ionic liquid has been examined. When acidity of acid forming neutralized salts is high, ions are strongly polarized, and the ion conductivity improves. This is why lithium salts of bis (trifluoromethanesulfonyl) imide acid or tris (trifluoromethanesulfonyl) methide acid show high ion conductivity as a lithium-ion battery. Meanwhile, the salts have an issue of being more irritating to the living body as acid strength becomes greater in an acid state before becoming neutralized salts. That is, the ion conductivity and the property of being irritating to the living body have a trade-off relationship. However, the salts to be applied to the bio-electrode need to be highly ion conductive and less irritating to the living body at the same time.

An ionic compound has a characteristic that as the molecular weight becomes higher, the permeability to the skin becomes lower, and it is less irritating to the skin. Proposed are bio-electrodes, to each of which an ion polymer containing salts of ammonium, lithium, sodium, and potassium formed with any of fluorosulfonic acid, which is described in JP 2018-099504 A, fluorosulfonimide, which is described in JP 2018-126496 A, and N-carbonylfluorosulfonic amide, which is described in JP 2018-130533 A, or salts of silver, which is described in JP 2019-180467 A, is added.

It is an adhesive material containing silicone to which the ion polymer is added. With such an adhesive material, each of these bio-electrodes is closely attached to the skin on a constant basis, does not swell by absorption of water

even in a state of being immersed in water, keeps adhesiveness for a long period of time, and is capable of stably obtaining an electric signal. In these bio-electrodes, however, a living body contact layer is formed on a non-porous conductive base material by using a composition containing the above-mentioned ion polymer.

As a result of earnest examination of the above-mentioned issues, the inventors of the present invention have found out a bio-electrode having the following configuration, a production method for the bio-electrode, and a measurement method for a bio-signal, and have completed the present invention.

That is, the present invention relates to a bio-electrode that includes a porous, stretchy base material, an adhesive, conductive film containing silicon, and a conducting path. The conductive film is formed on one surface of the porous, stretchy base material. The conducting path is connected to the conductive film, and penetrates the stretchy base material to be exposed on the opposite side, or is exposed on a side surface of the stretchy base material.

The bio-electrode according to the present invention has water repellency, and thereby allows for daily showering and bathing. The bio-electrode allows for not only measurement of a bio-signal after bathing or showering, but also measurement during bathing or showering.

The bio-electrode allows for not only electrocardiographic measurement, but also measurement using an electromyogram, measurement of brain waves, and measurement of a breathing rate. The bio-electrode allows for not only measurement of a signal emitted from the skin, but also provision of an electric signal to the skin to transmit a signal to muscles and control brain waves. For example, the bio-electrode is expected to be used for purposes for applying a stimulus to muscles during swimming to increase performance or reduce fatigue, and for enhancing relaxation during bathing.

To configure the bio-electrode with a high sensitivity, not only high ion conductivity, but also high electron conductivity is required. To increase electron conductivity, it is effective to add a metal powder or a carbon powder in addition to the ion polymer.

The above-mentioned ion polymer can be used for formation of the above-mentioned adhesive, conductive film containing silicon. In a case where silicone is contained in the ion polymer, another silicone is not necessarily contained.

The present invention will be described in detail below, but is not limited to the description.

Bio-electrode

The bio-electrode according to the present invention includes the porous, stretchy base material, the adhesive, conductive film containing silicon, and the conducting path. The conductive film is formed on one surface of the porous, stretchy base material. The conducting path is connected to the conductive film, and penetrates the stretchy base material to be exposed on the opposite side, or is exposed on the side surface of the stretchy base material. In addition, the bio-electrode according to the present invention includes the adhesive, conductive film containing silicone. The adhesive, conductive film serves as the living body contact layer formed on the conducting path that penetrates the porous film or on the conducting path on the porous film.

The bio-electrode is now briefly described using the drawings. Regarding an example of a bio-electrode 10 according to the present invention, FIG. 1 shows a cross section of the bio-electrode 10. The bio-electrode 10 includes a porous, stretchy base material 1, a conducting path 2, a conductive film 3, and a release liner 4. The porous, stretchy base material 1 includes a non-woven fabric 1-1, an adhesive layer 1-2, and a reinforcing film 1-3. The conducting path 2 includes an upper snap 2-1 and a lower snap 2-2.

Composition of Conductive Film

Each component forming the conductive film of the bio-electrode according to the present invention is described below in a more detailed manner.

Ionic Material (Salts) (A)

Salts blended as the ionic material (A) (conductive material) serving as a material for forming the conductive film used for the bio-electrode according to the present invention can contain, as the ionic material (A), a polymer having ionic repeating units selected from the group consisting of salts of ammonium, lithium, sodium, potassium, and silver formed with any of fluorosulfonic acid, fluorosulfonimide, and N-carbonylfluorosulfonic amide.

The ionic material (A) selected from the group consisting of salts of ammonium, sodium, potassium, and silver formed with any of fluorosulfonic acid, fluorosulfonimide, and N-carbonylfluorosulfonic amide can have a partial structure shown by the following general formulae (1)-1 to (1)-4.

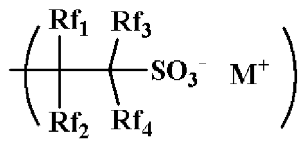

(1)-1

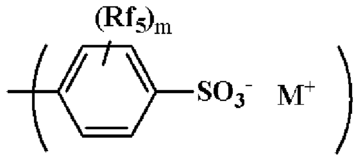

(1)-2

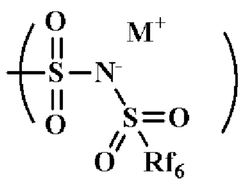

(1)-3

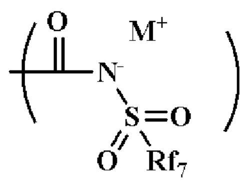

(1)-4

(In the general formula (1)-1, $Rf_1$ and $Rf_2$ each represent a hydrogen atom, a fluorine atom, an oxygen atom, a methyl group, or a trifluoromethyl group. When $Rf_1$ and $Rf_2$ represent an oxygen atom, the single oxygen atom represented by $Rf_1$ and $Rf_2$ bonds to a single carbon atom to form a carbonyl group. $Rf_3$ and $Rf_4$ each represent a hydrogen atom, a fluorine atom, or a trifluoromethyl group. At least one of $Rf_1$ to $Rf_4$ is a fluorine atom or a trifluoromethyl group. In the general formulae (1)-2, (1) -3, and (1)-4, $Rf_5$, $Rf_6$, and $Rf_7$ each represent a fluorine atom, a trifluoromethyl group, or a linear or branched alkyl group having 1 to 4 carbon atoms, and have at least one fluorine atom. In the general formulae (1)-1 to (1)-4, $M^+$ represents an ion selected from the group consisting of an ammonium ion, a sodium ion, a potassium ion, and a silver ion. In the general formula (1)-2, "m" represents an integer of 1 to 4.)

The ionic polymer preferably has at least one repeating unit selected from the group consisting of salts of ammonium, sodium, potassium, and silver formed with any of fluorosulfonic acid shown by the above-mentioned general formulae (1)-1 and (1)-2, fluorosulfonimide shown by the general formula (1)-3, and N-carbonylfluorosulfonic amide shown by the general formula (1)-4, which are at least one selected from the repeating units A1 to A7 shown by the following general formula (2).

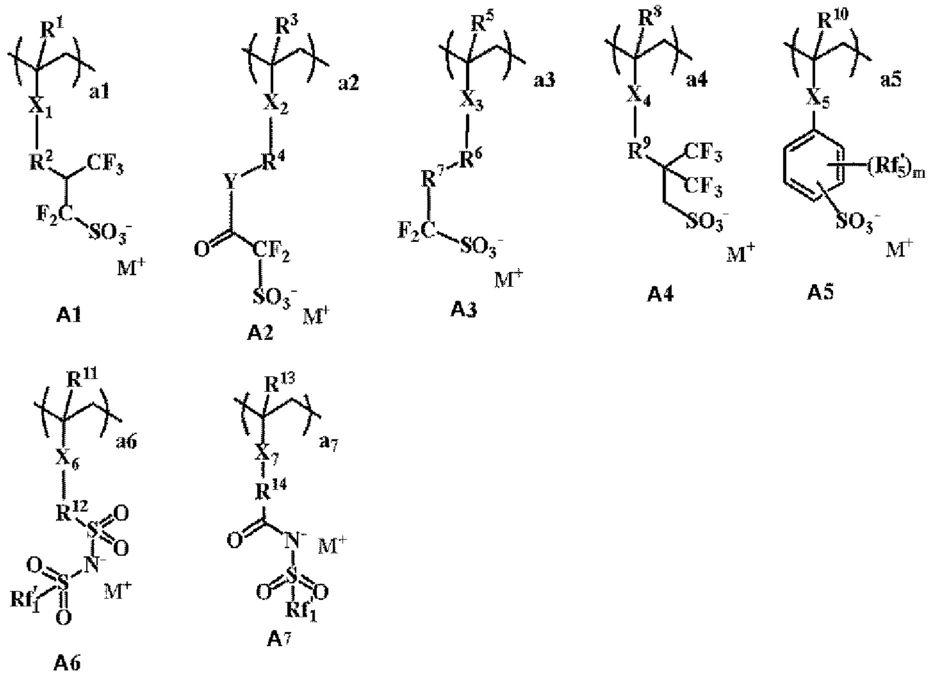

A1 A2 A3 A4 A5 A6 A7

(2)

(In the general formula (2), $R^1$, $R^3$, $R^5$, $R^8$, $R^{10}$, $R^{11}$, and $R^{13}$ each independently represent a hydrogen atom or a methyl group. $R^2$, $R^4$, $R^6$, $R^9$, $R^{12}$, and $R^{14}$ each independently represent a single bond, or a linear, branched, or cyclic hydrocarbon group having 1 to 13 carbon atoms. The hydrocarbon group optionally has either or both of an ester group and an ether group. $R^7$ represents a linear or branched alkylene group having 1 to 4 carbon atoms, and one or two hydrogen atoms in $R^7$ are optionally substituted with a fluorine atom. $X_1$, $X_2$, $X_3$, $X_4$, $X_6$ and $X_7$ each independently represent any of a single bond, a phenylene group, a naphthylene group, an ether group, an ester group, and an amide group. $X_5$ represents any of a single bond, an ether group, and an ester group. Y represents any of an oxygen atom and a $-NR^{19}-$ group. $R^{19}$ represents any of a hydrogen atom, a linear, branched, or cyclic alkyl group having 2 to 12 carbon atoms, and a phenyl group, and optionally have one or more selected from the group consisting of an ether group, a carbonyl group, an ester group, and an amide group. Y optionally forms a ring together with $R^4$. $Rf_1$' and $Rf_5$' each represent a fluorine atom, a trifluoromethyl group, or a linear or branched alkyl group having 1 to 4 carbon atoms, and have at least one fluorine atom. "m" represents an integer of 1 to 4. a1, a2, a3, a4, a5, a6, and a7 are $0 \leq a1 \leq 1.0$, $0 \leq a2 \leq 1.0$, $0 \leq a3 \leq 1.0$, $0 \leq a4 \leq 1.0$, $0 \leq a5 \leq 1.0$, $0 \leq a6 \leq 1.0$, $0 \leq a7 \leq 1.0$, and $0 < a1+a2+a3+a4+a5+a6+a7 \leq 1.0$. $M^+$ represents an ion selected from the group consisting of an ammonium ion, a sodium ion, a potassium ion, and a silver ion.)

In the above-mentioned general formula (2), a1 to a7 are ratios of the repeating units A1 to A7, respectively.

Repeating Unit A

Specific examples of monomers of fluorosulfonic acid salts to obtain the repeating units A1 to A5, among the repeating units A1 to A7 shown by the above-mentioned general formula (2), can include the following.

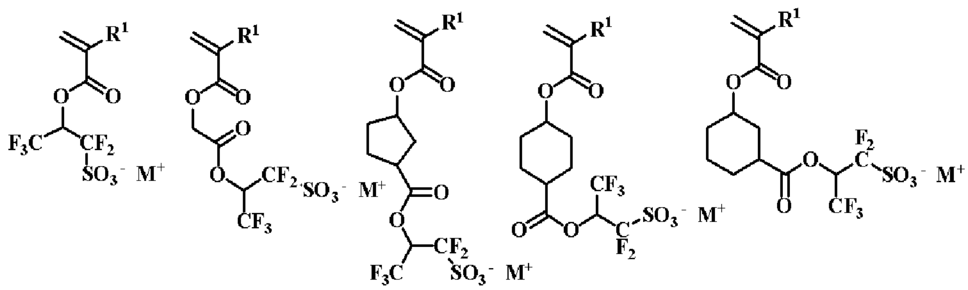

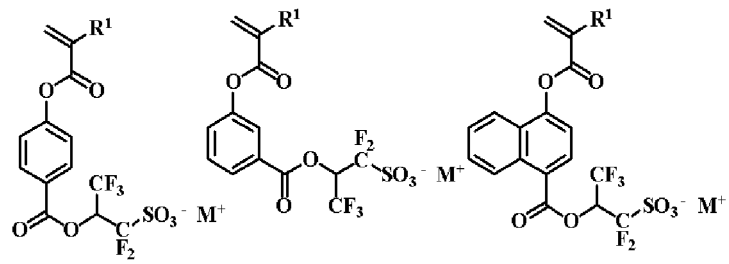

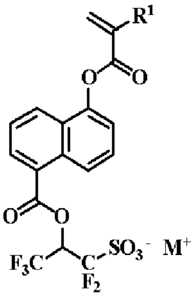

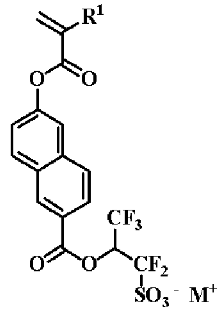

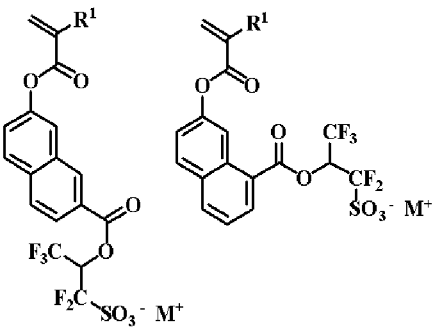

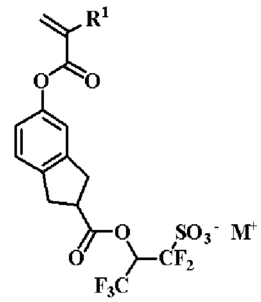

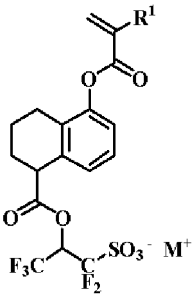

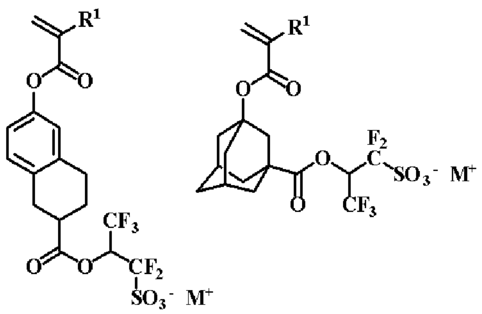

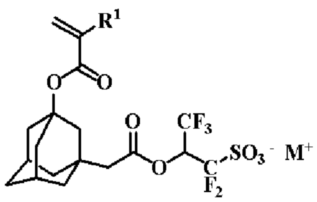

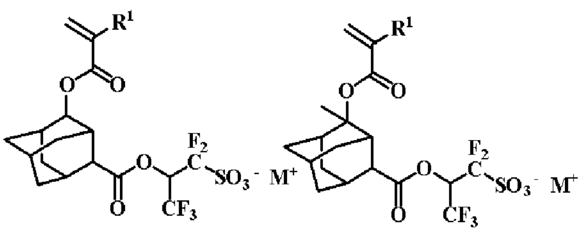

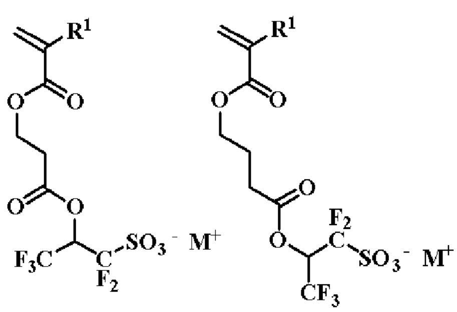

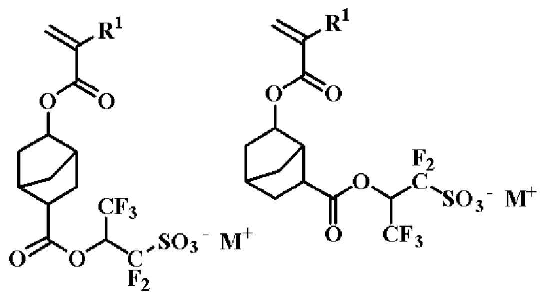

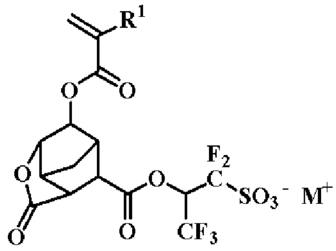

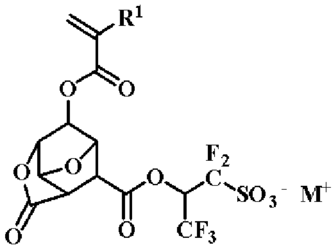

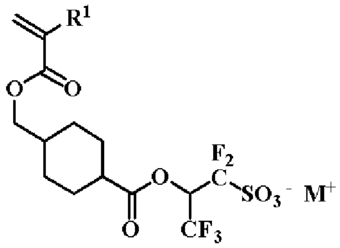

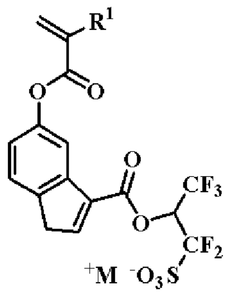

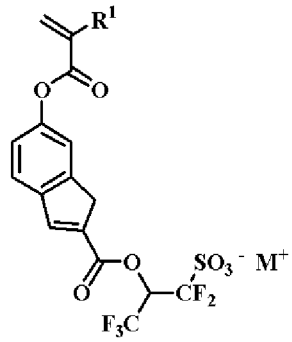

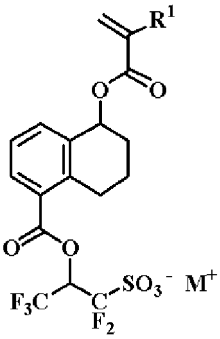

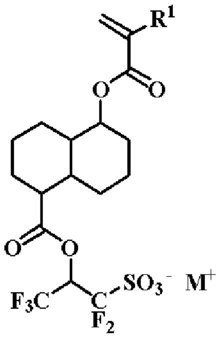

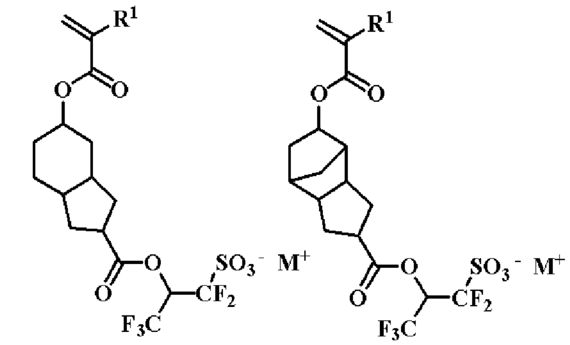

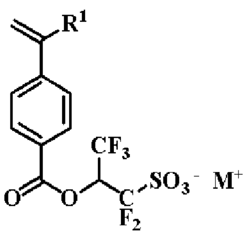

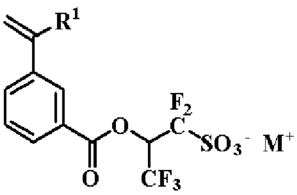

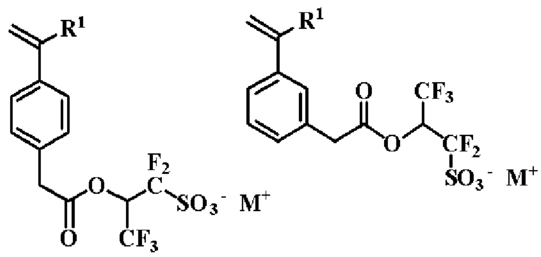

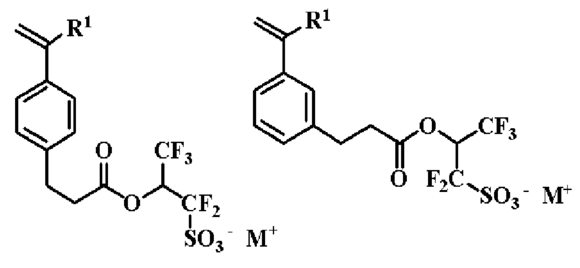

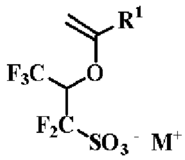

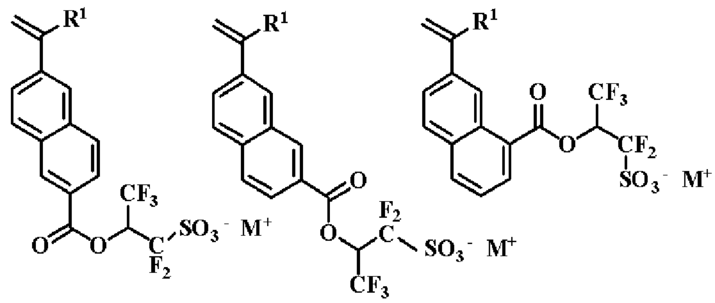

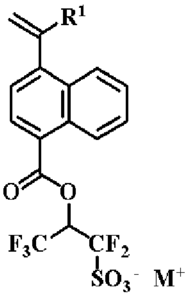

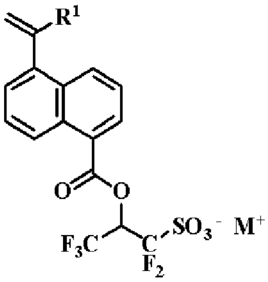

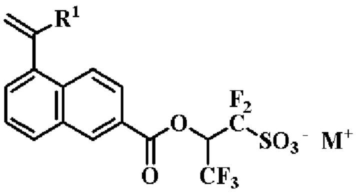

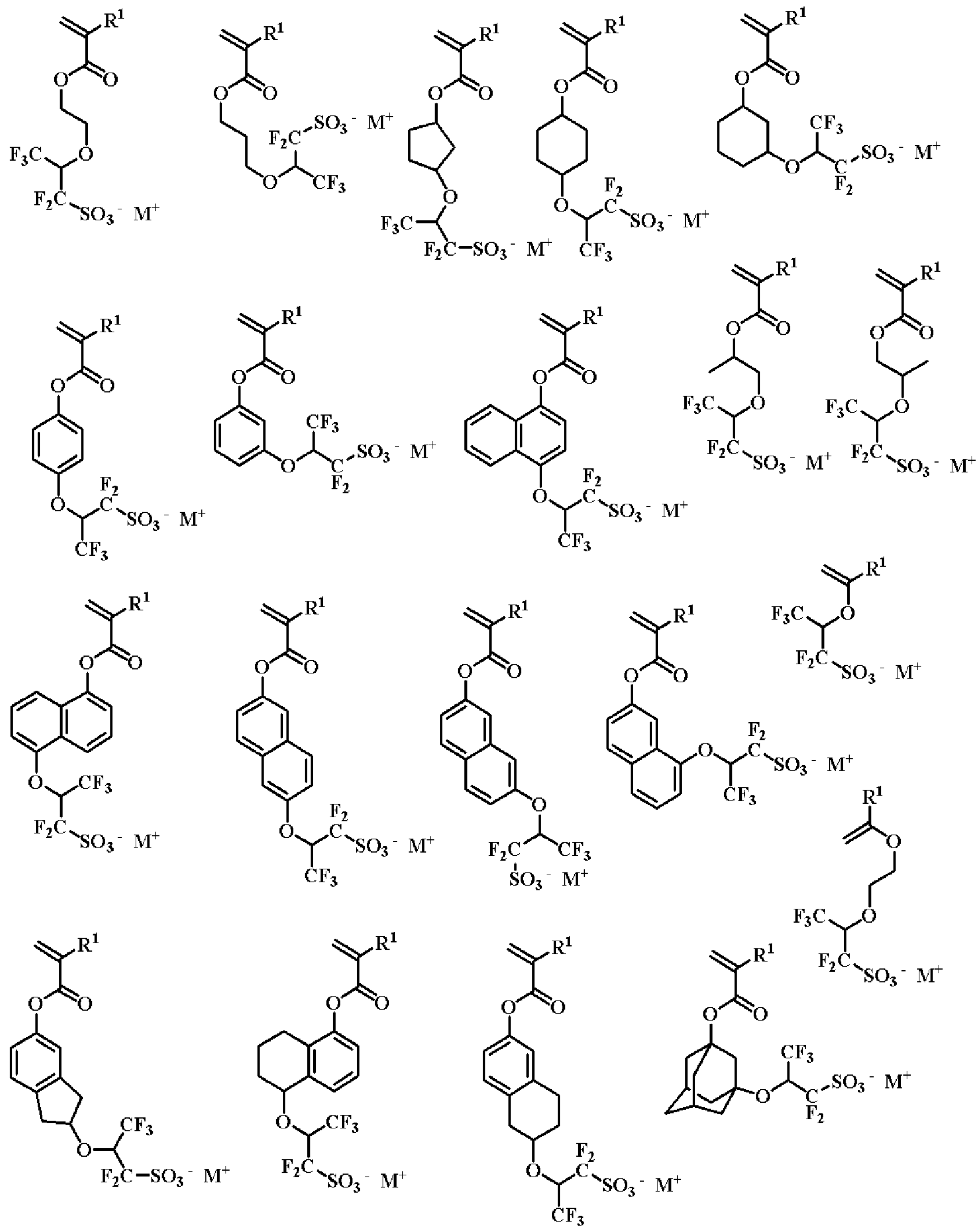

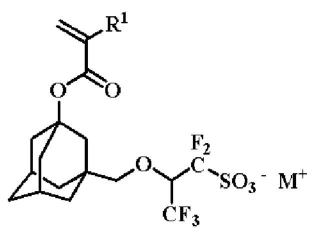

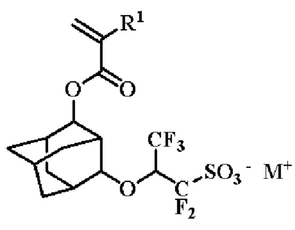

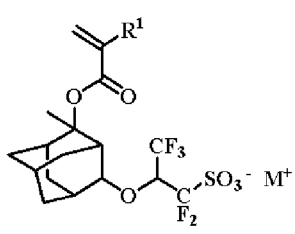

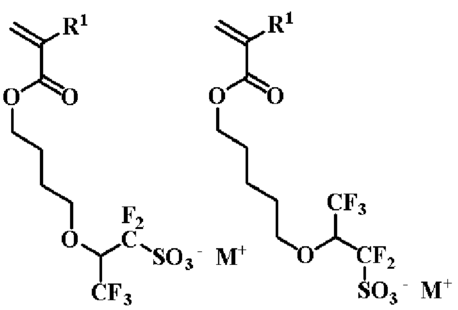
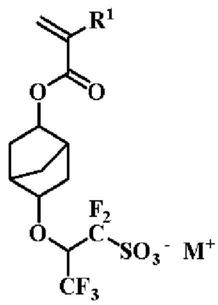
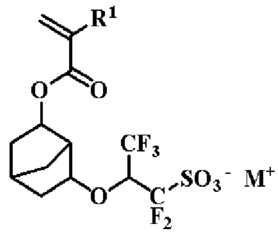

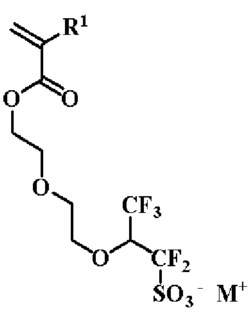
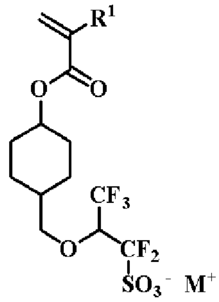
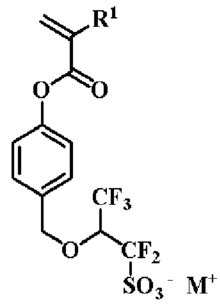
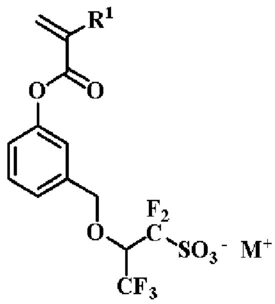

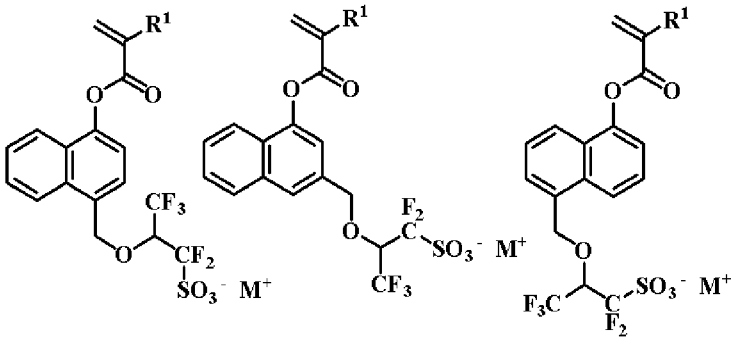
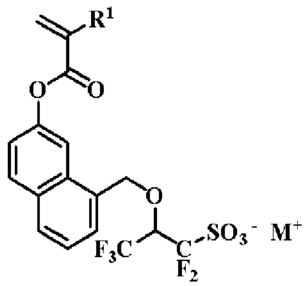

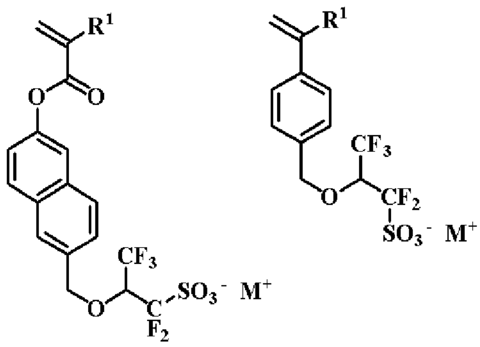
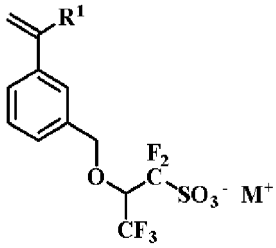
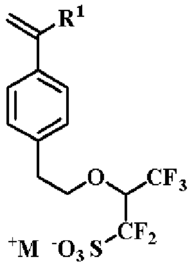

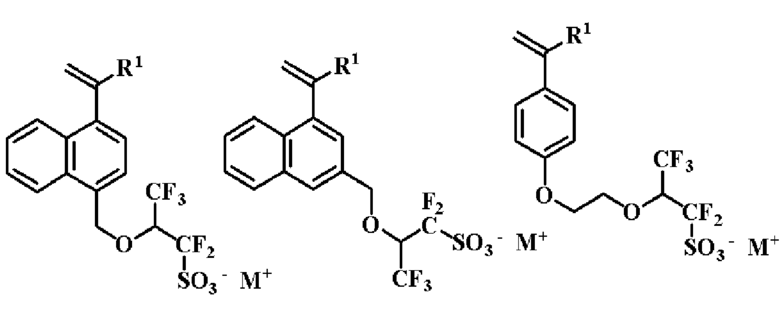
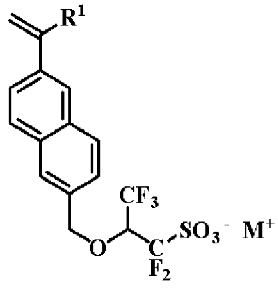

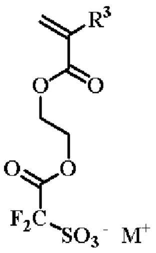
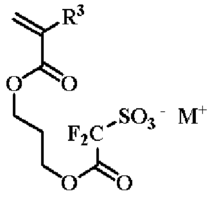
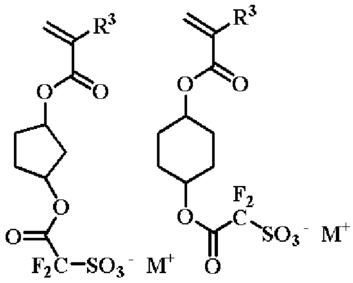
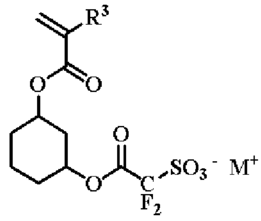

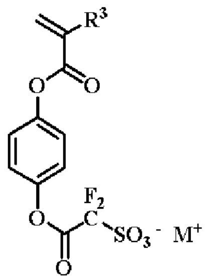
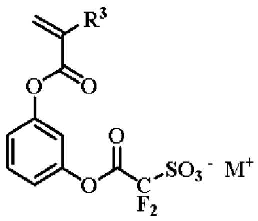
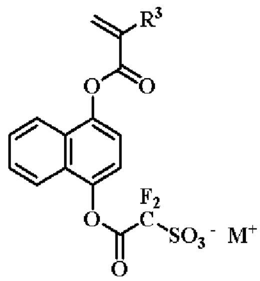
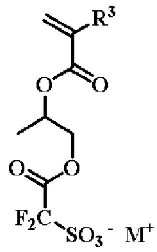
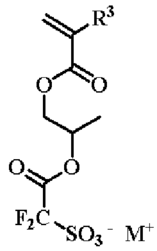

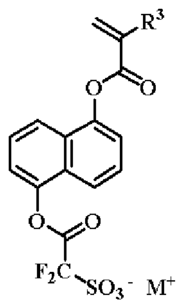
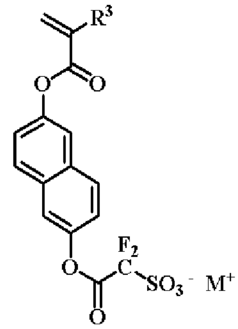
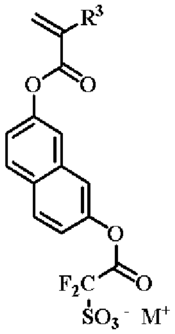
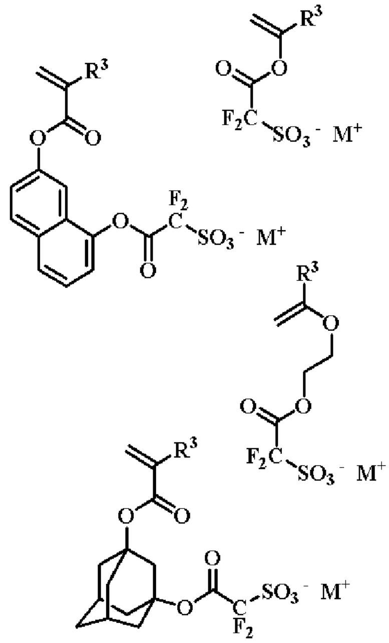

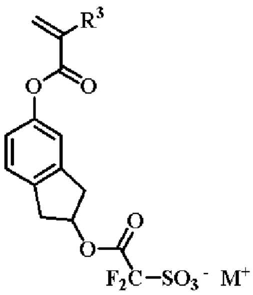
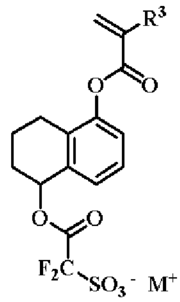
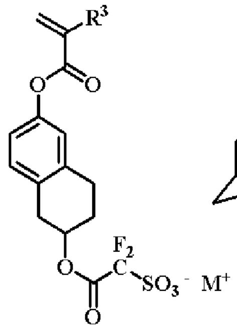

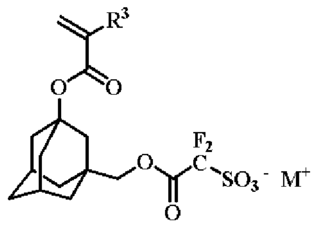
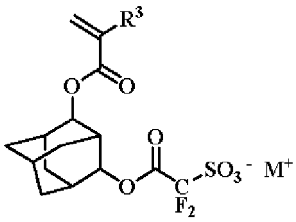
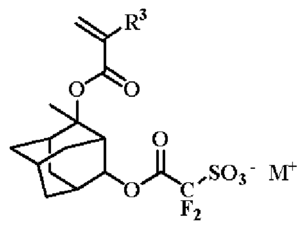

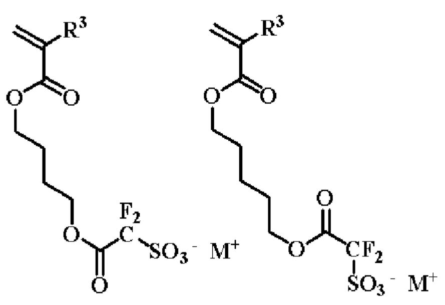
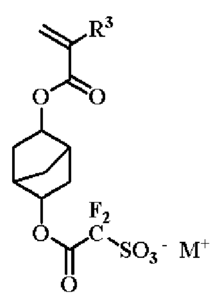
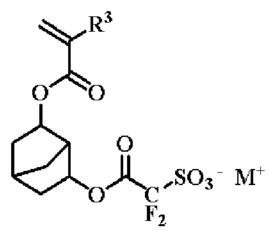

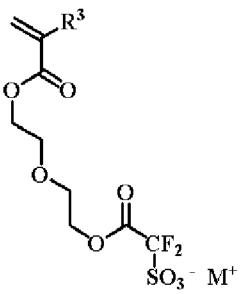
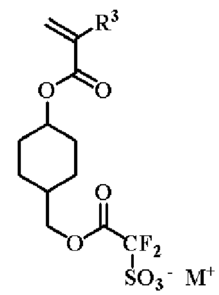
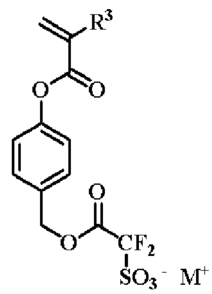
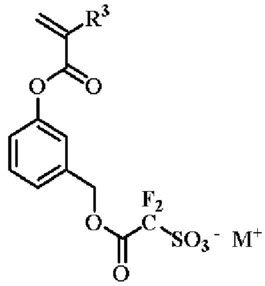

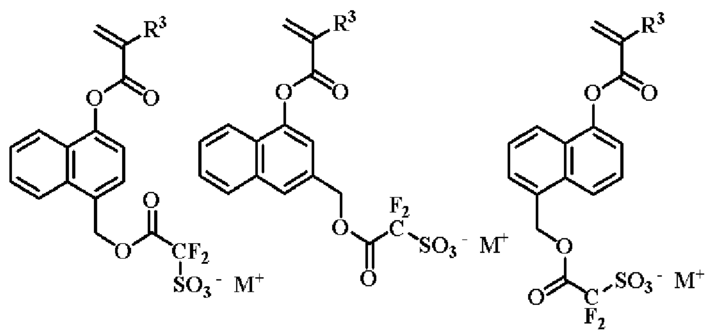
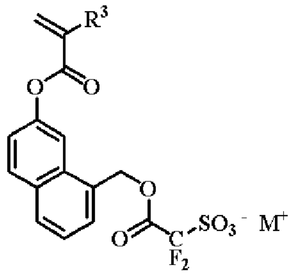

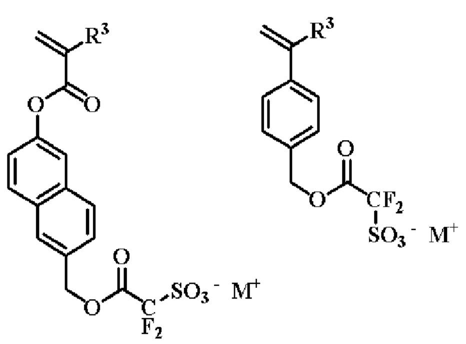
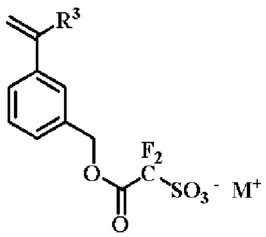
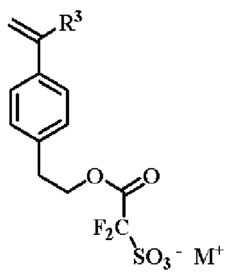

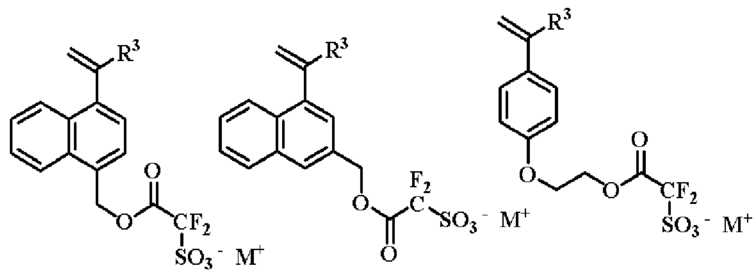
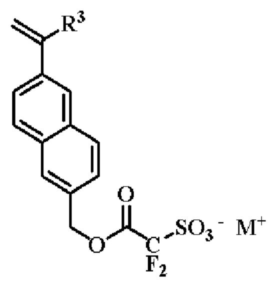

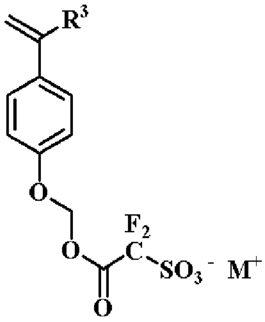

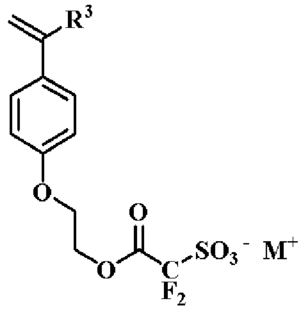

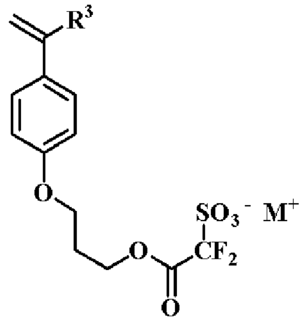

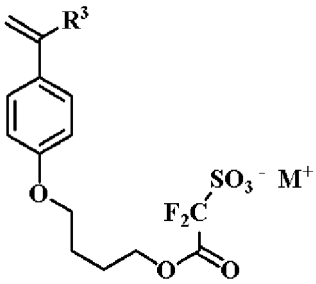

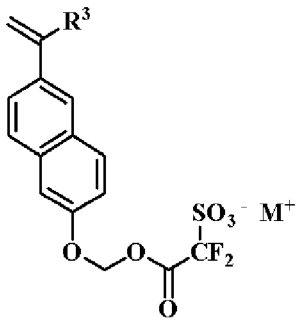

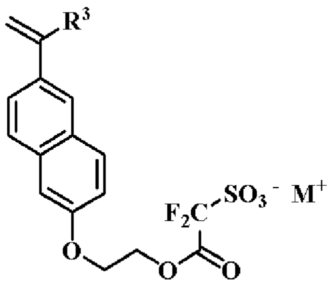

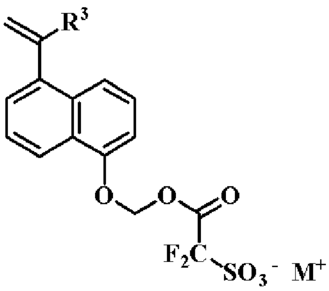

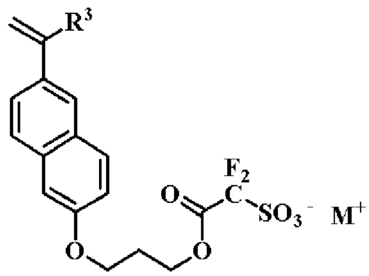

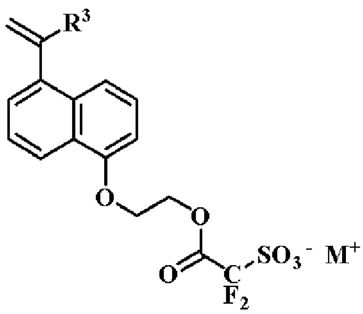

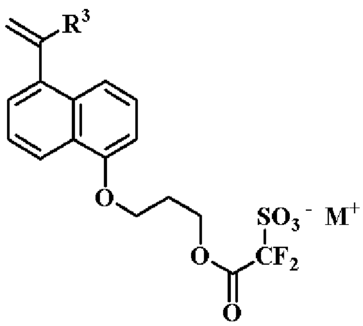

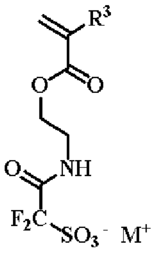
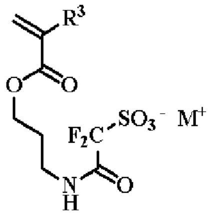
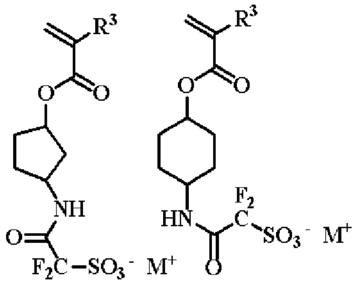
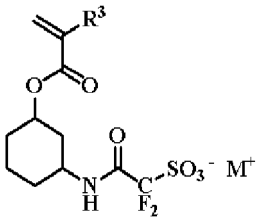

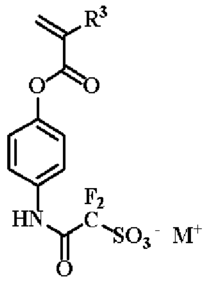
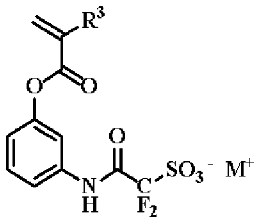
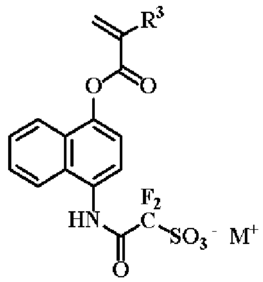
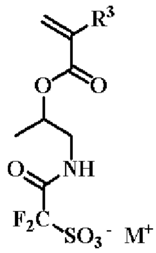
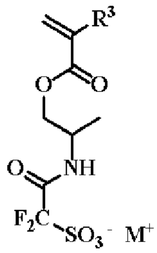

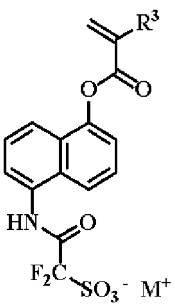
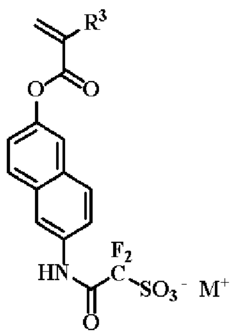
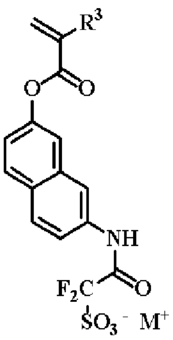
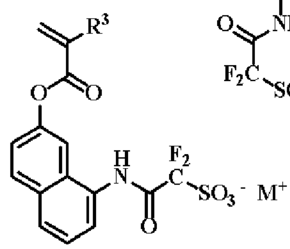
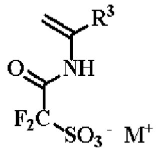
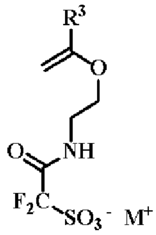

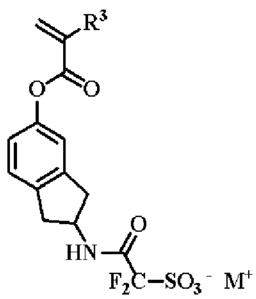
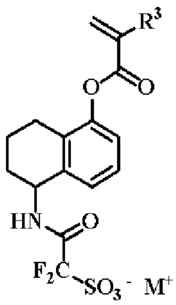
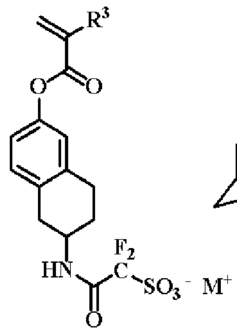
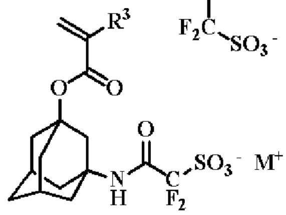

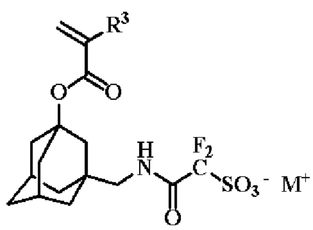
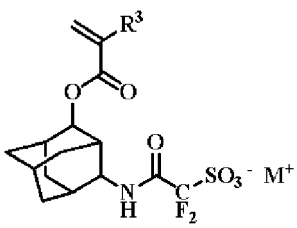
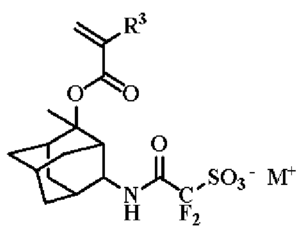

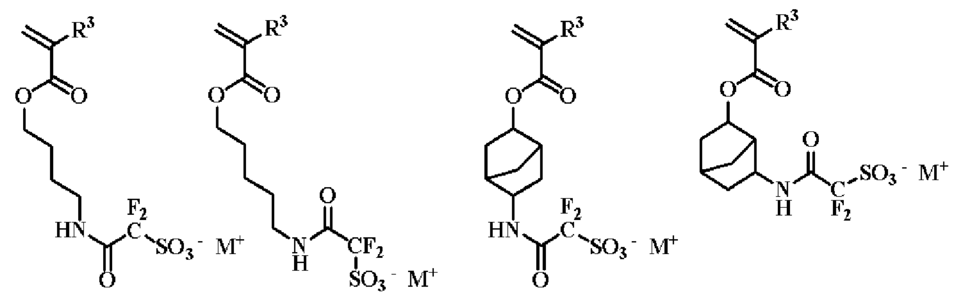

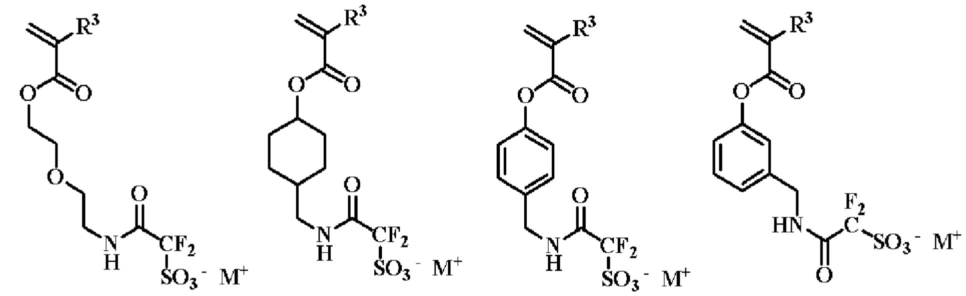

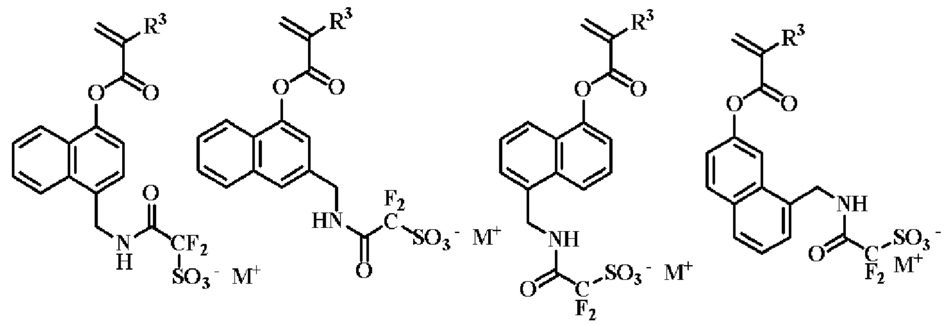

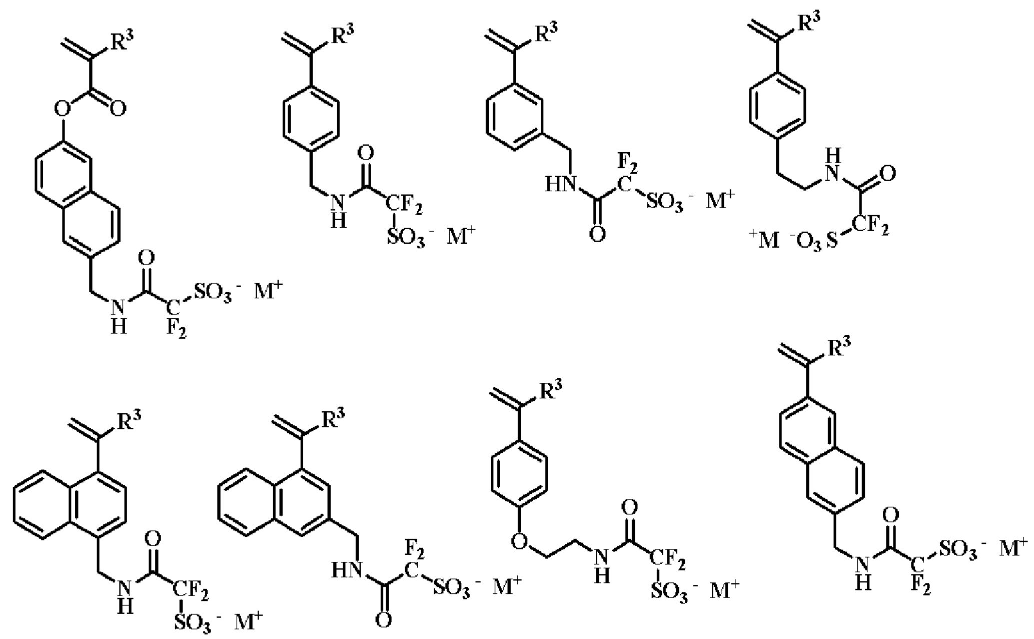

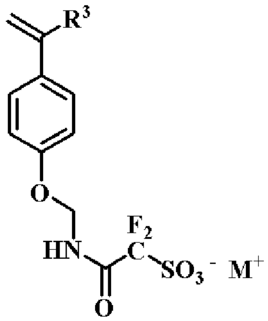
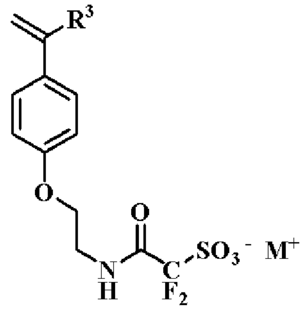
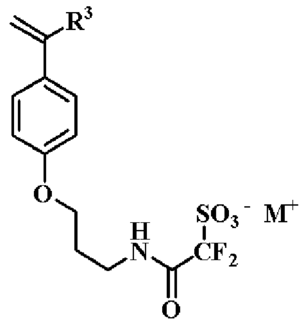

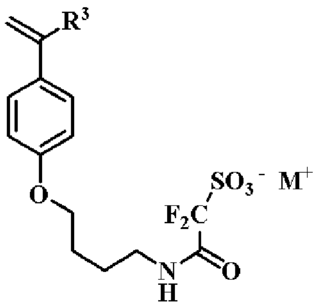
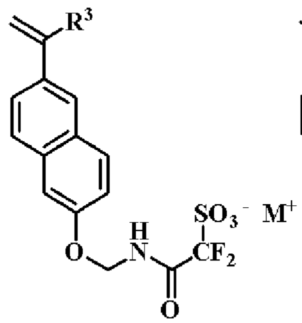
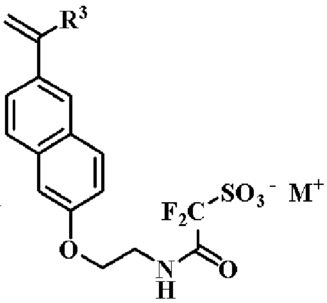

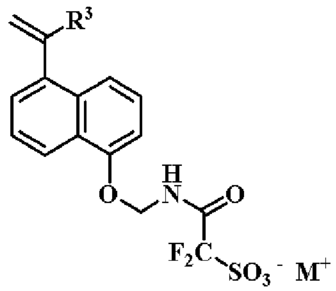
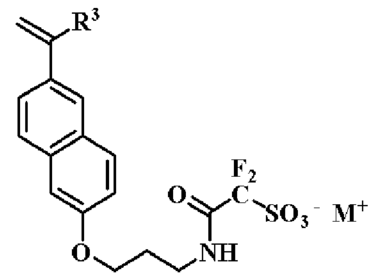

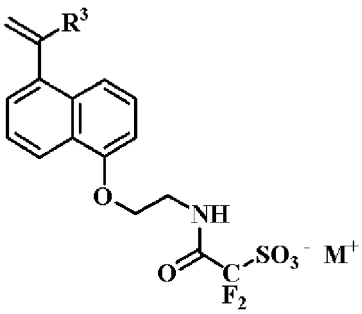
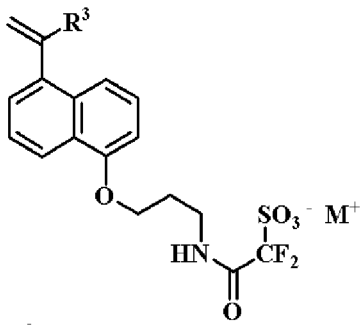

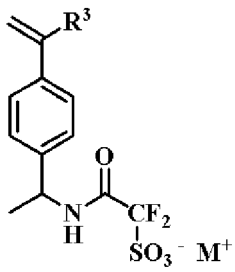
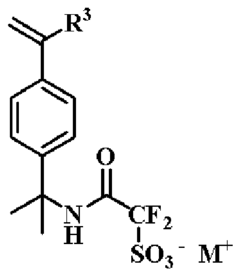

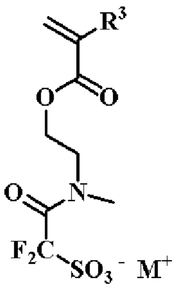
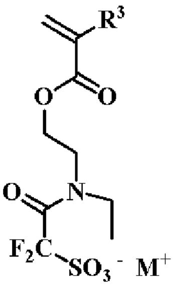
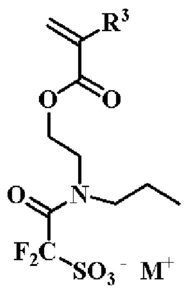
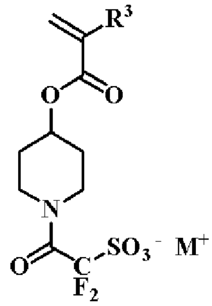
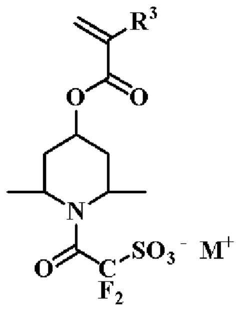

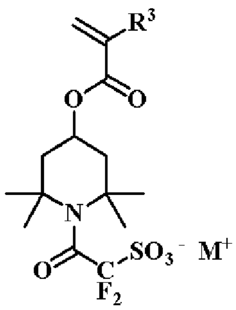
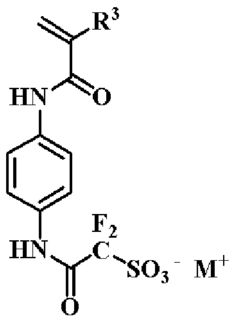
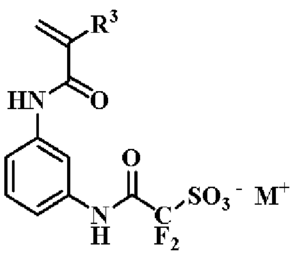
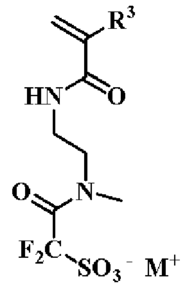

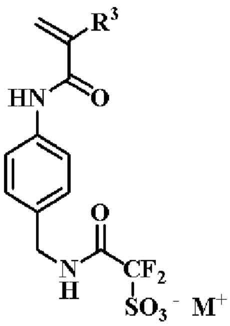
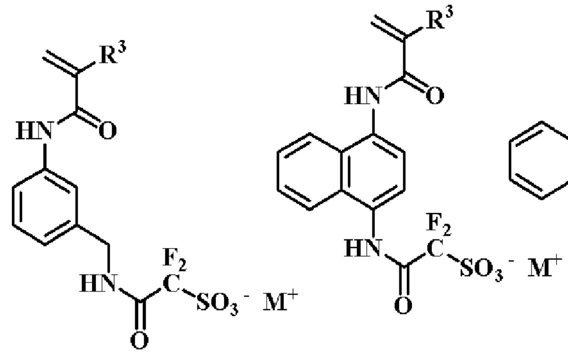
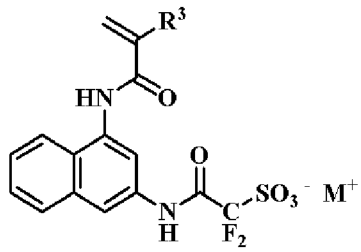

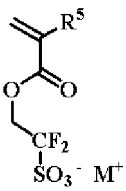
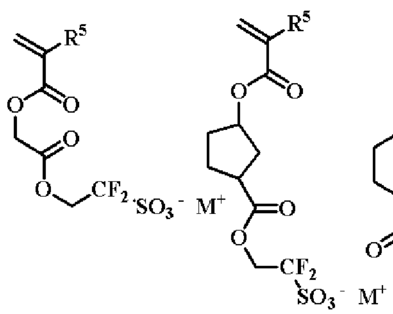
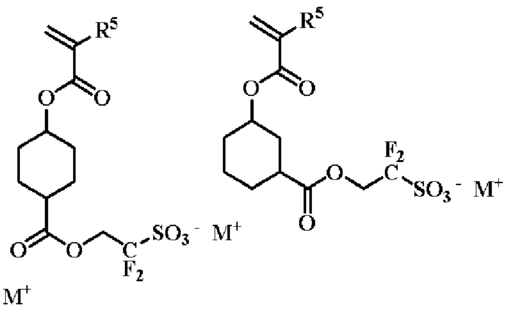

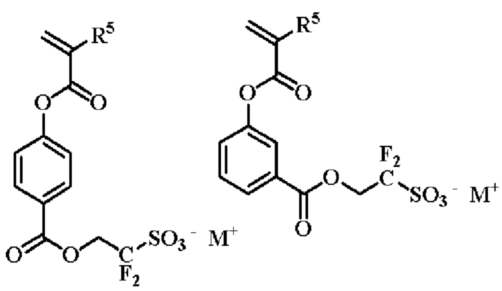
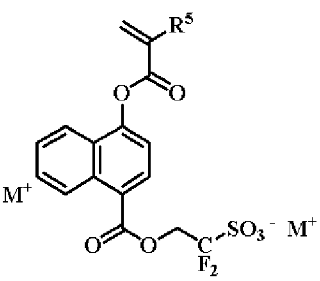

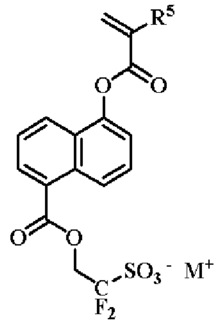
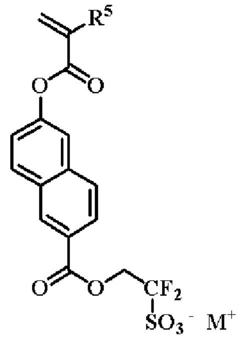
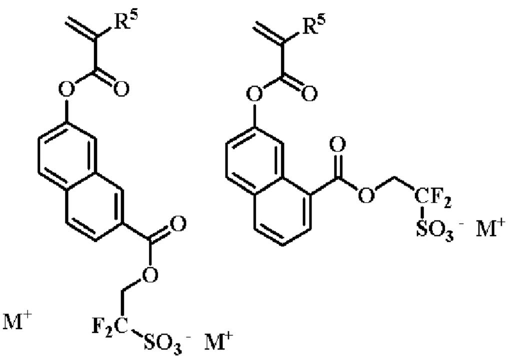

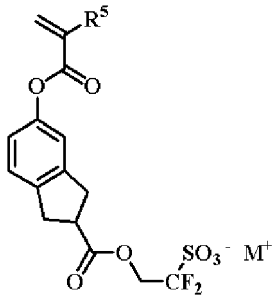
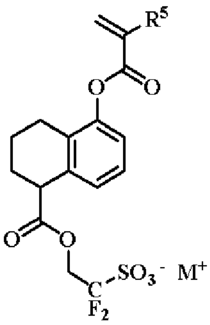
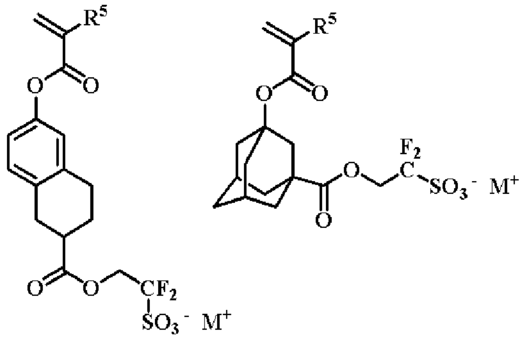

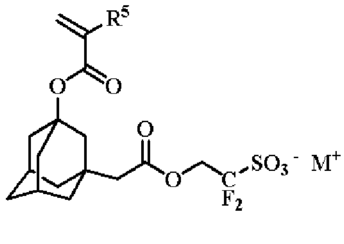
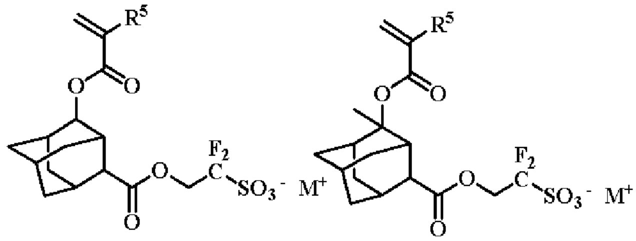

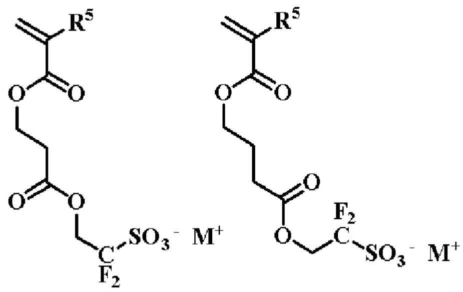
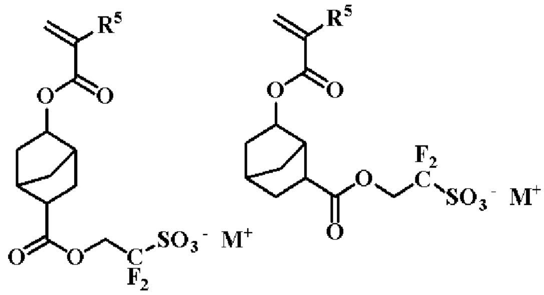

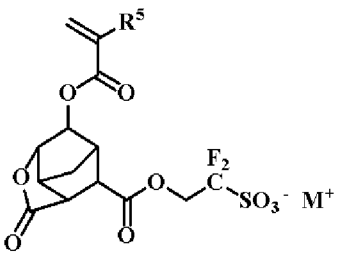
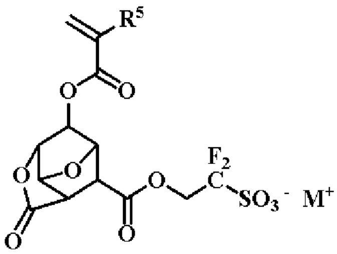
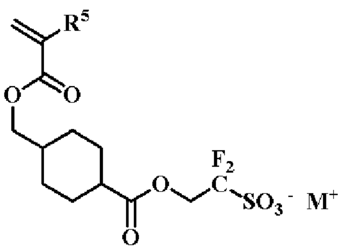

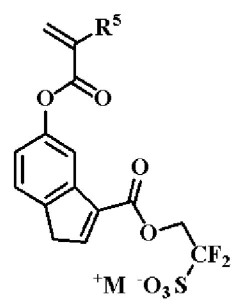

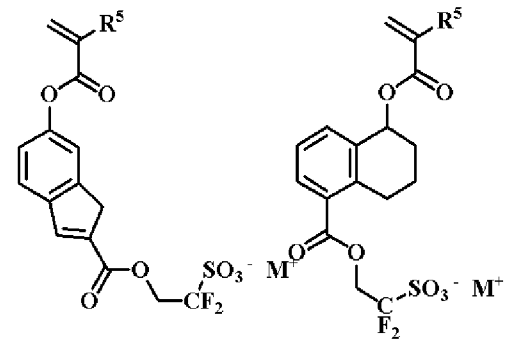

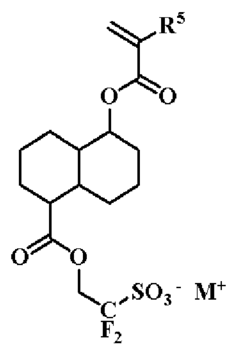

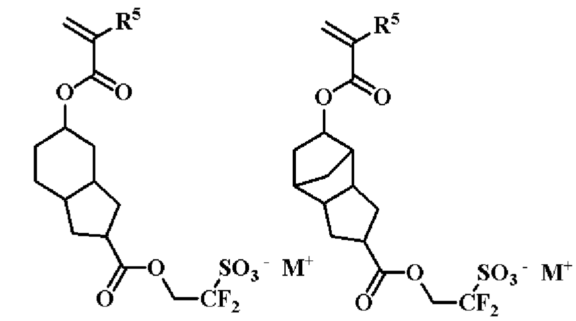

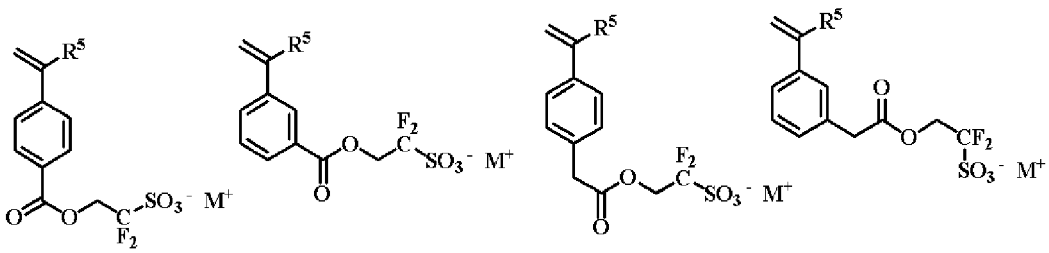

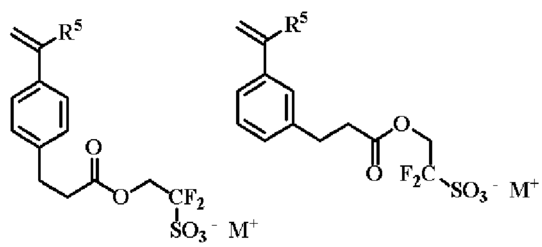

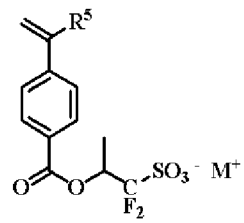

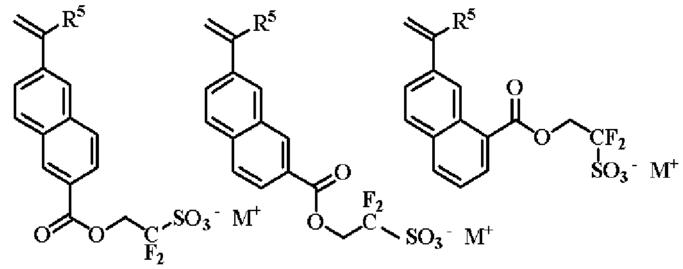

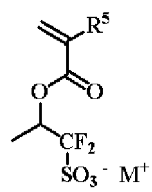

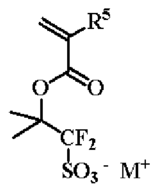

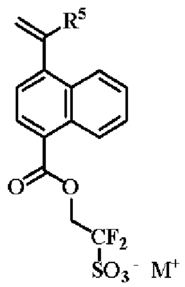
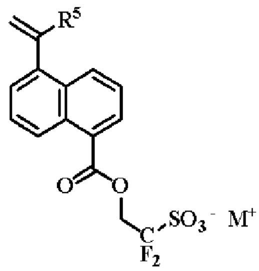
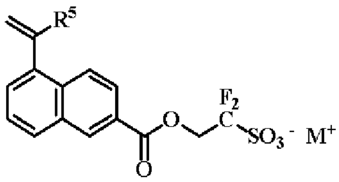

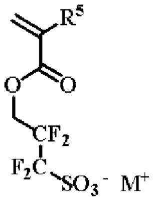
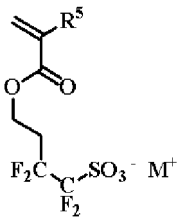
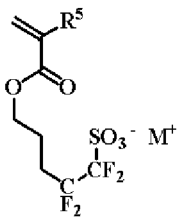
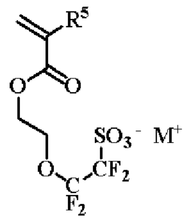
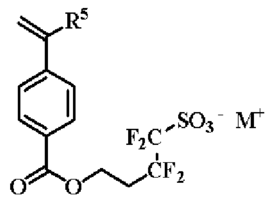

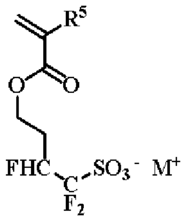

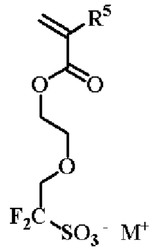
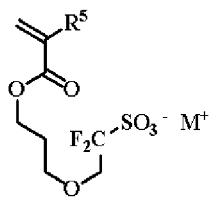
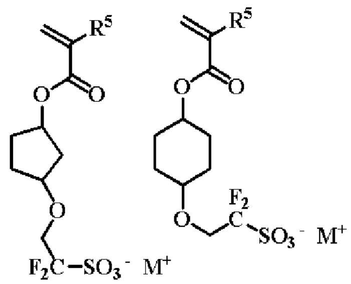
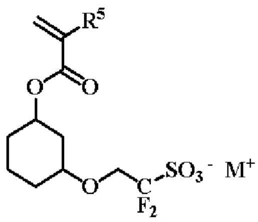

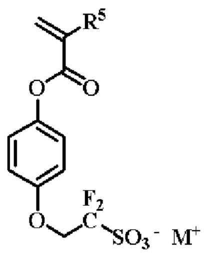
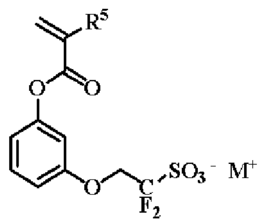
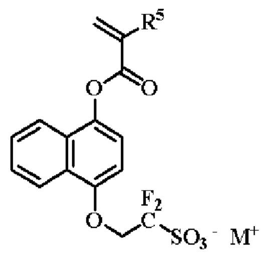
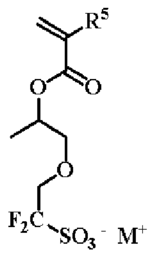
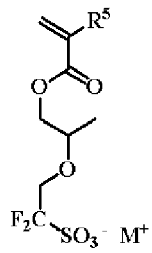

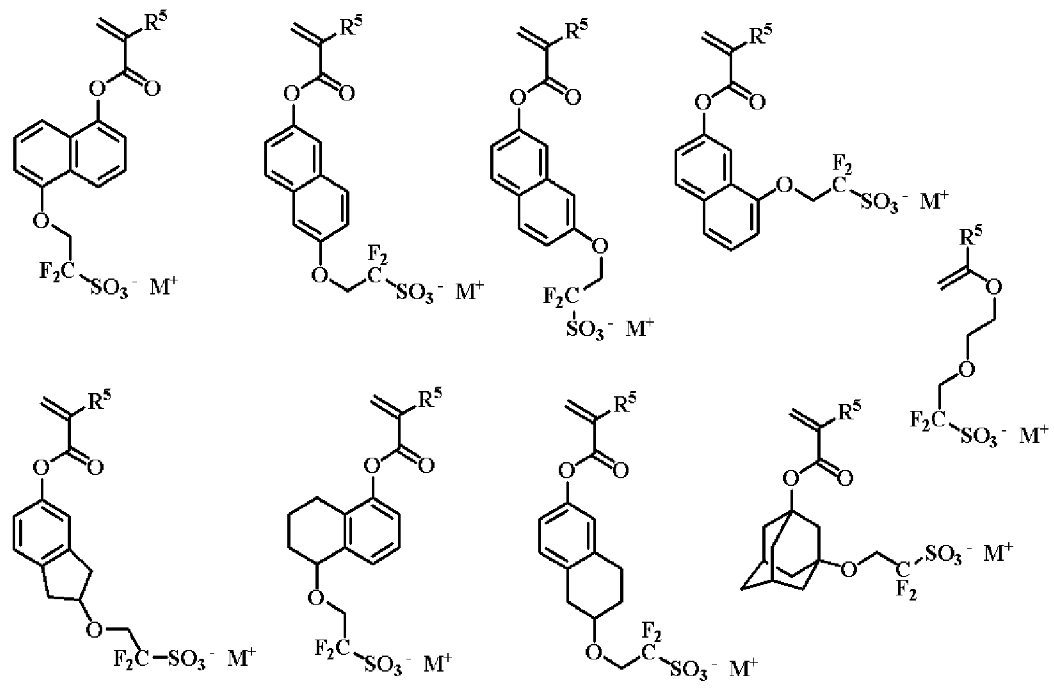

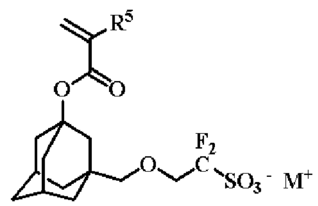

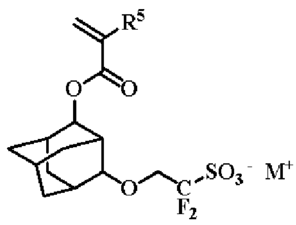

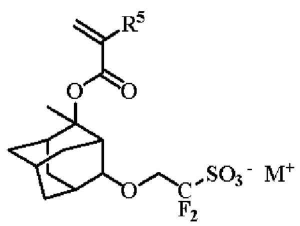

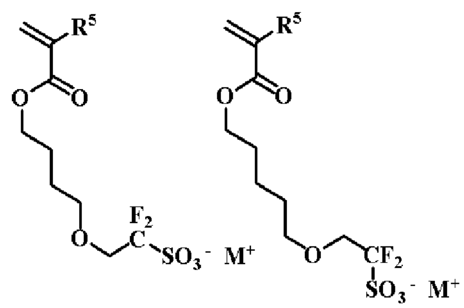

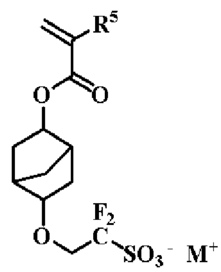

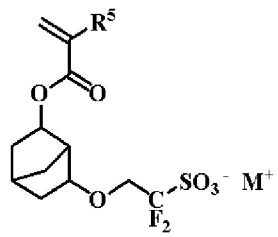

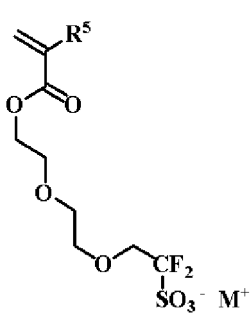

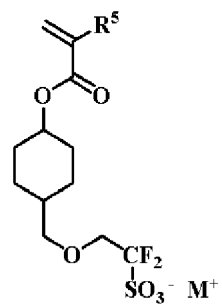

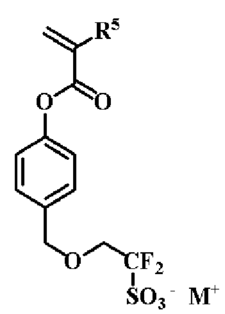

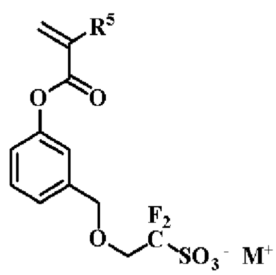

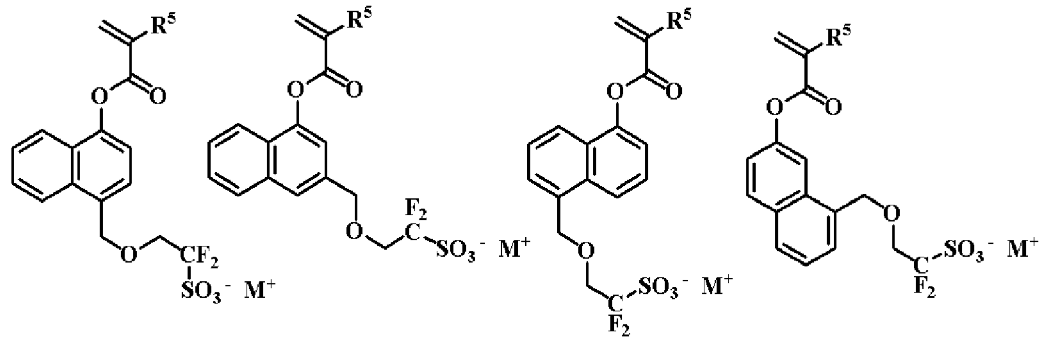

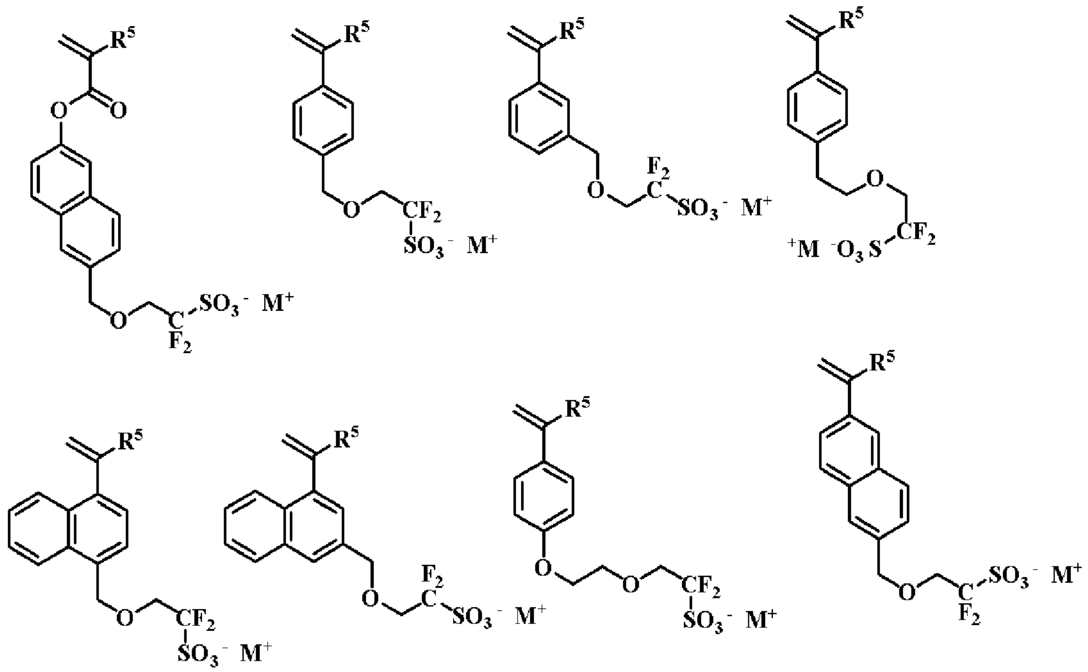

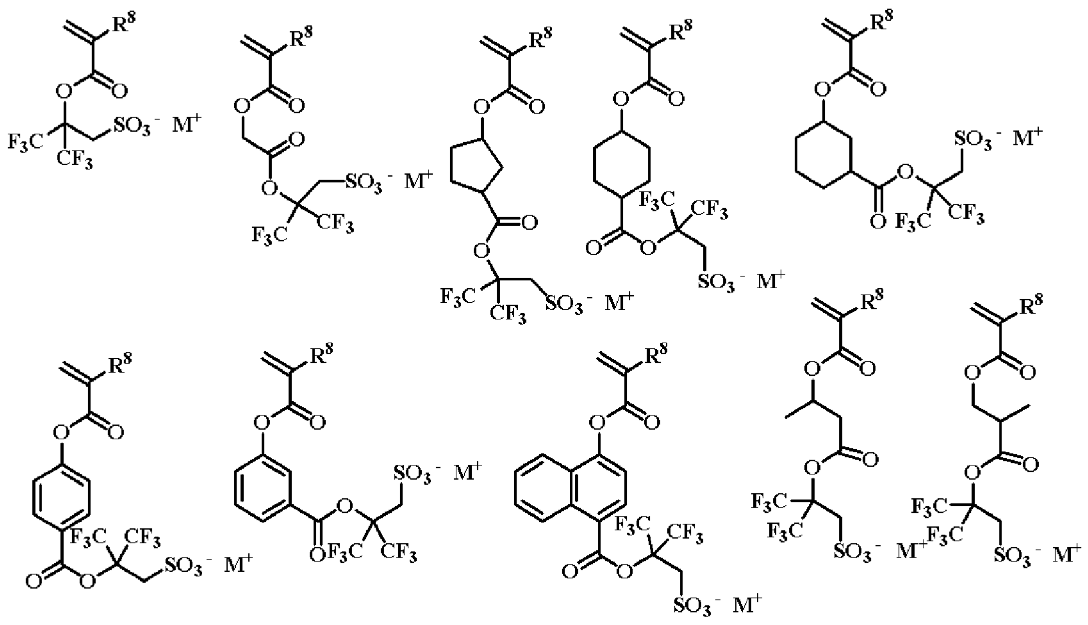

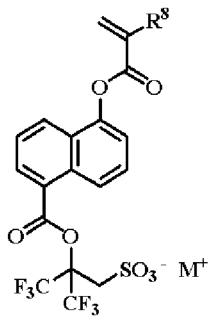
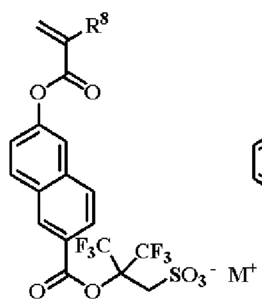
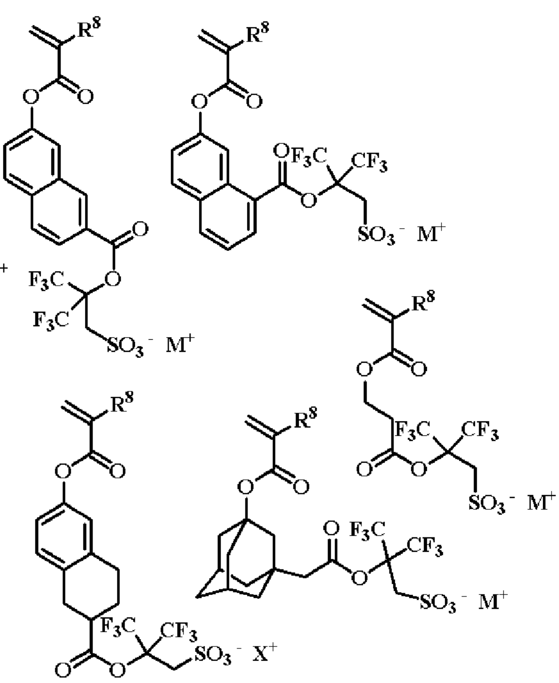
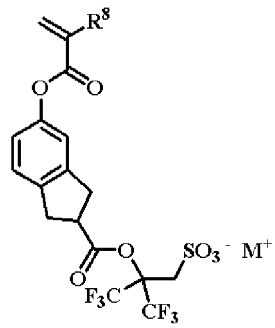
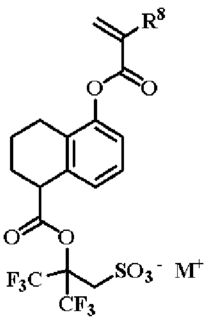
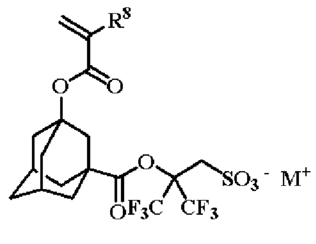
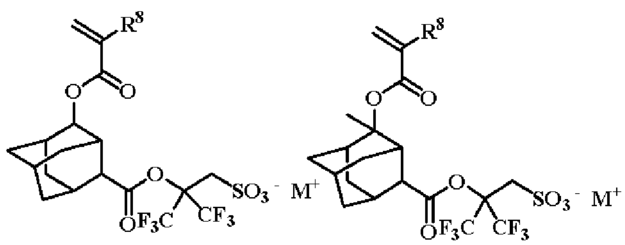
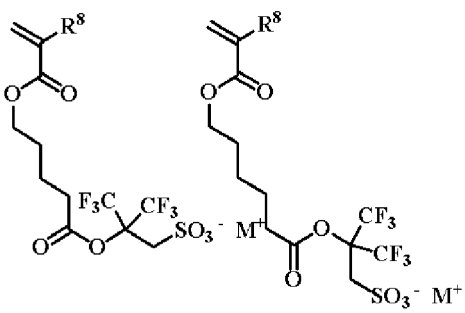
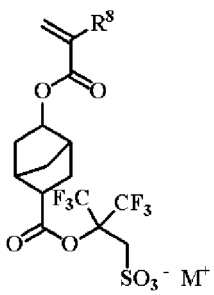
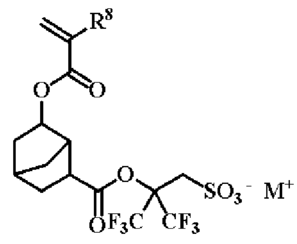
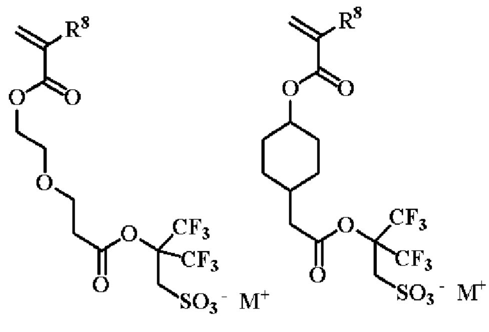
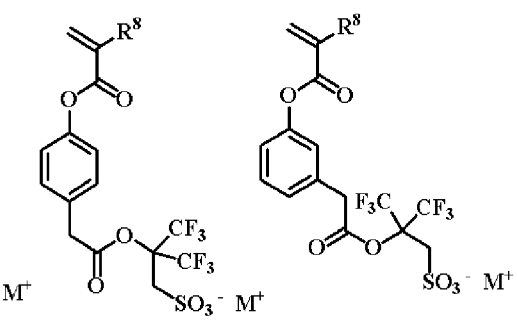

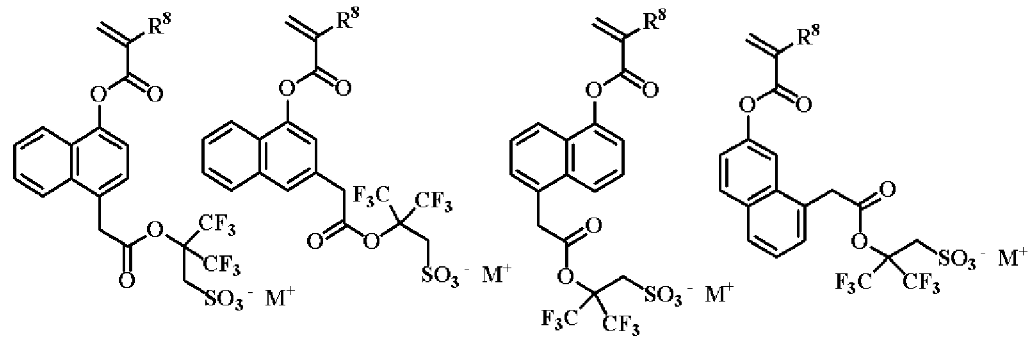

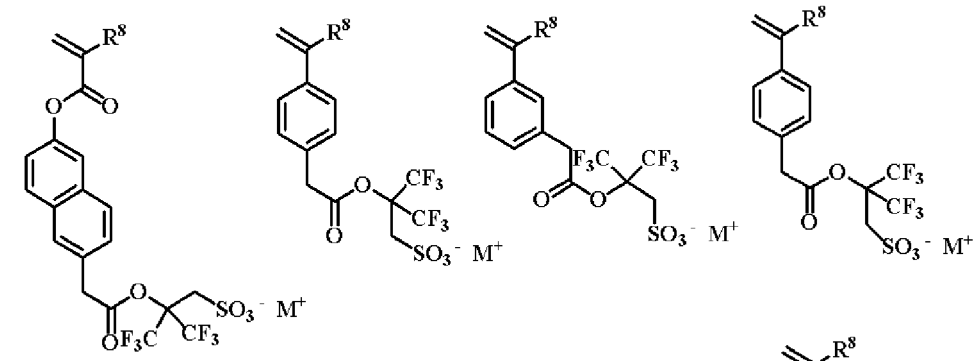

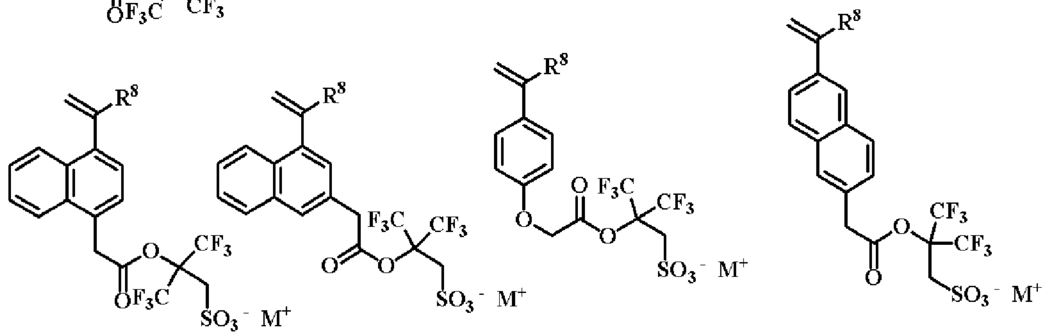

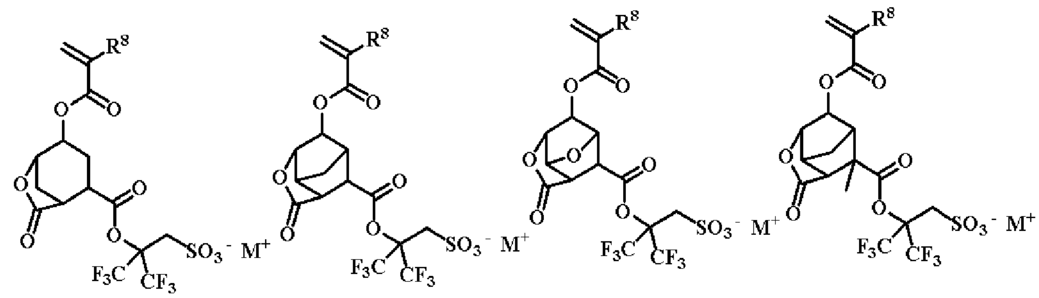

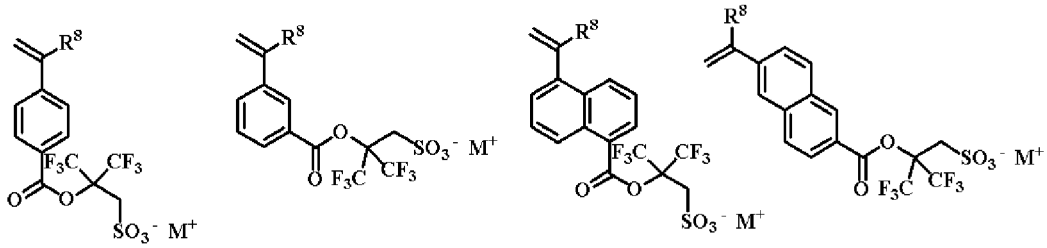

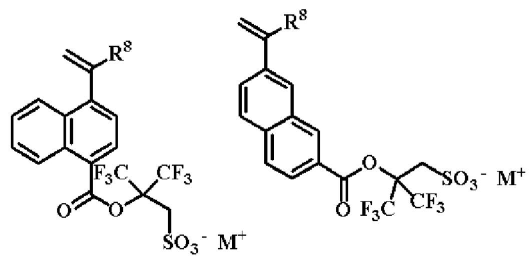

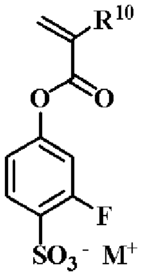

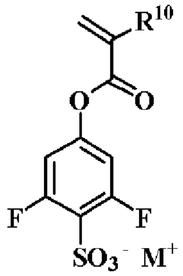

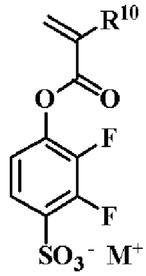

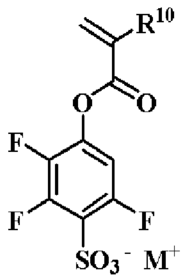

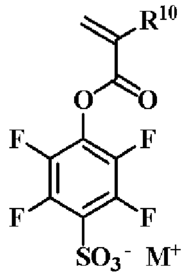

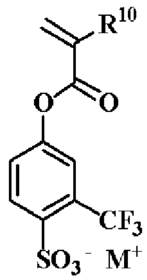

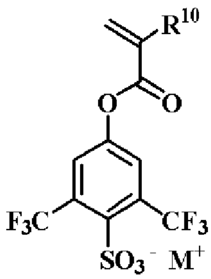

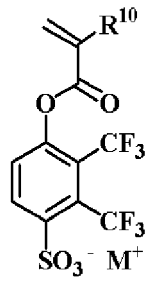

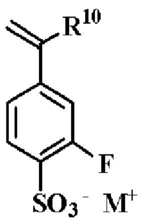

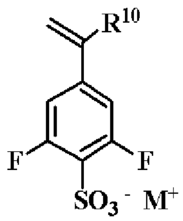

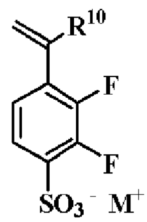

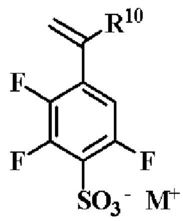

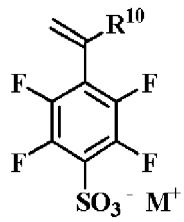

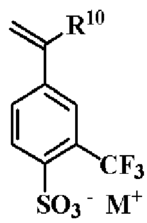

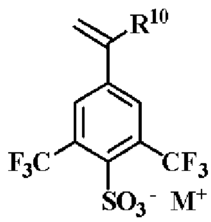

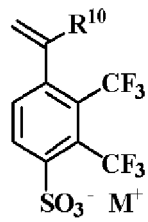

Specific examples of monomers of sulfonimide salts to obtain the repeating unit A6 shown by the above-mentioned general formula can include the following.

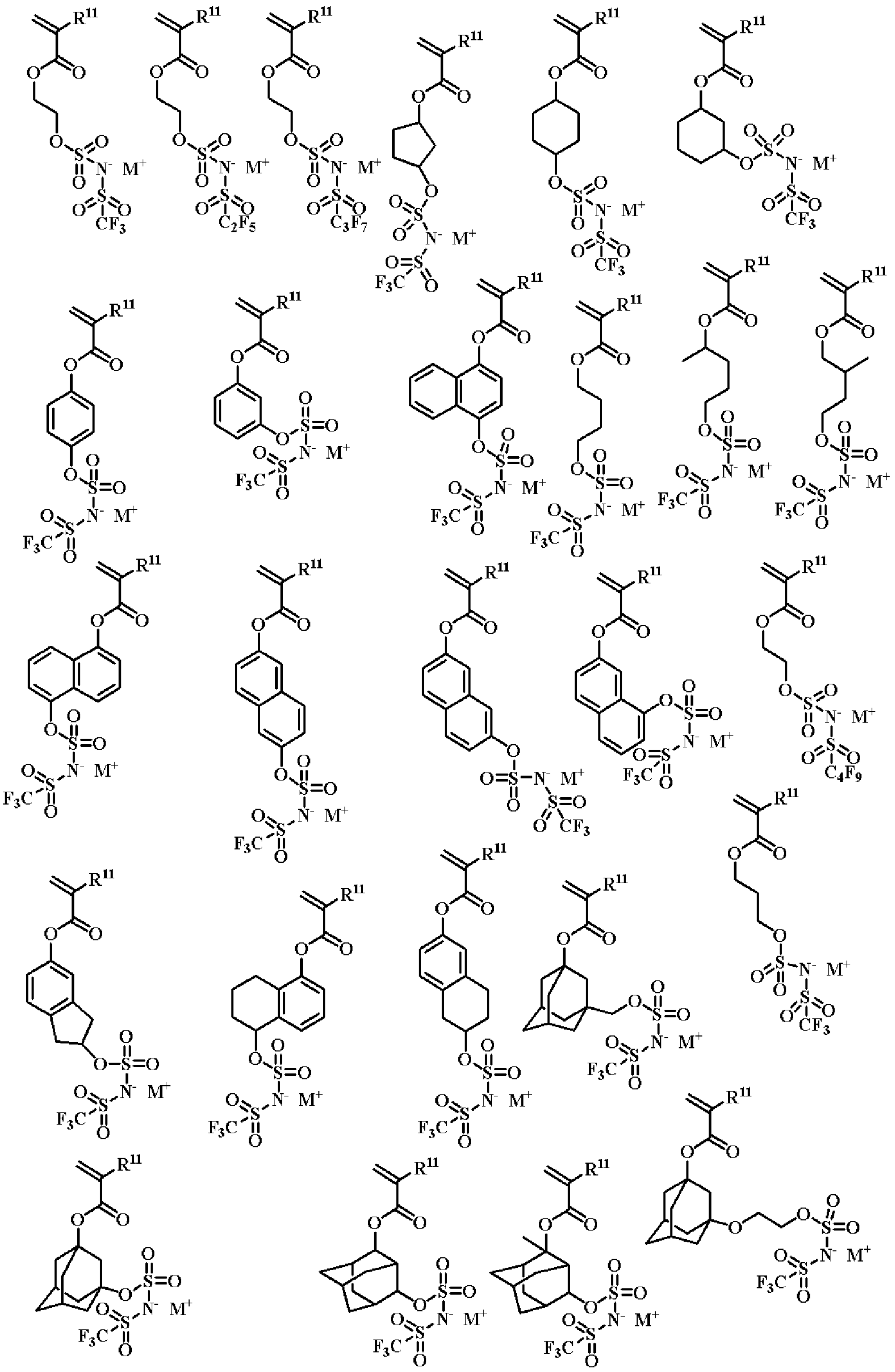

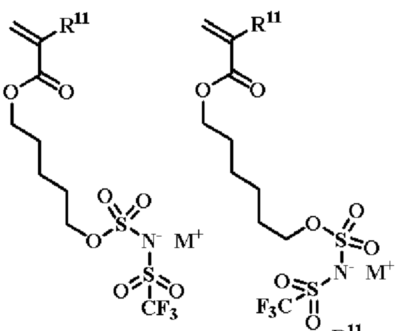
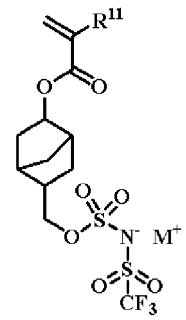
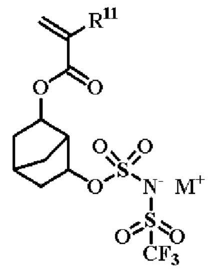

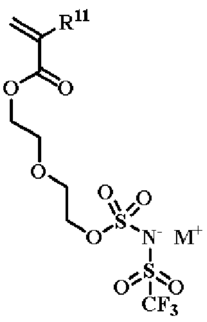
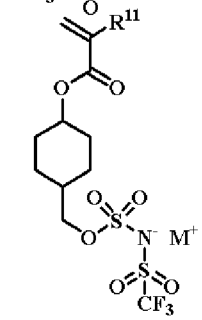
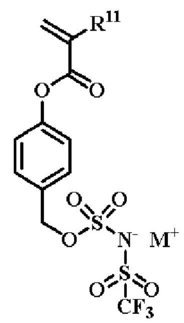
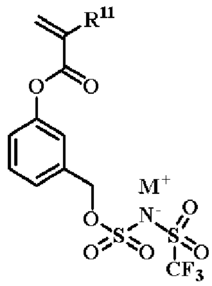

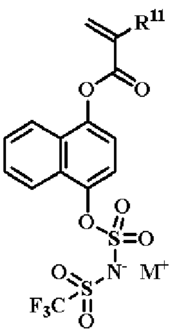
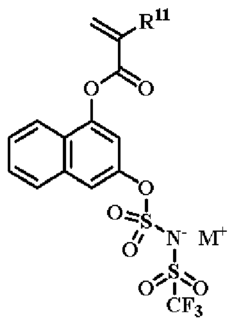
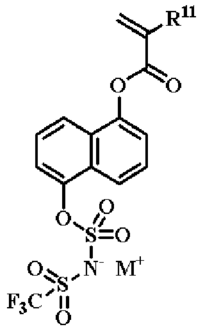
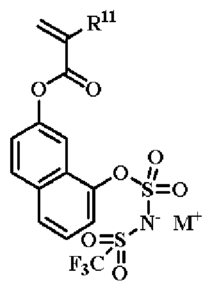

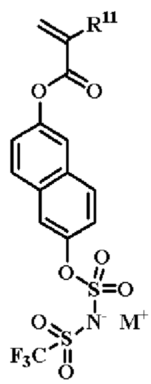
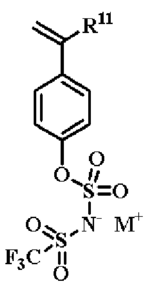
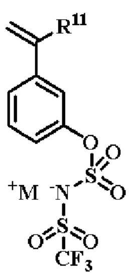
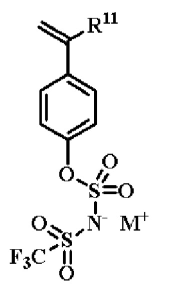

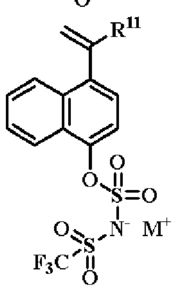
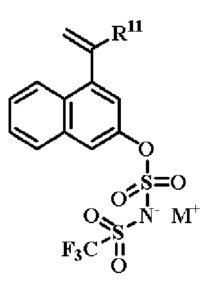
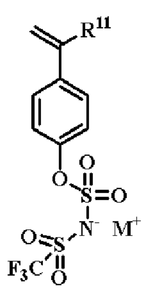
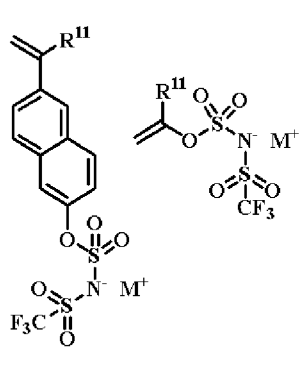

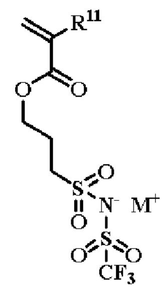
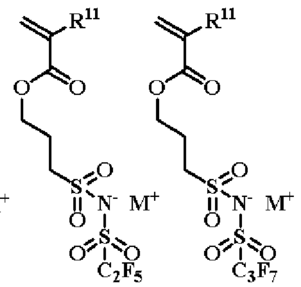
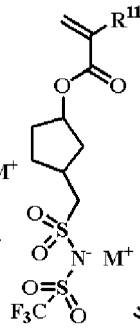
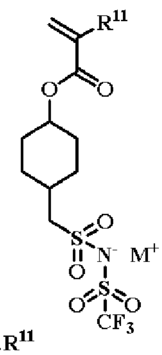
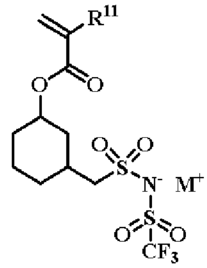
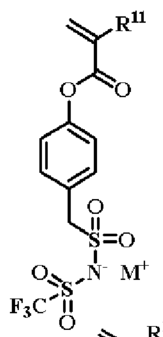
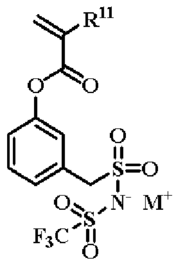
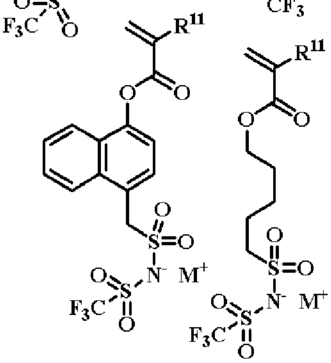
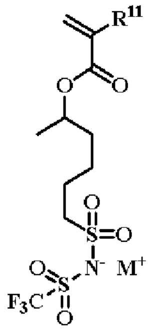
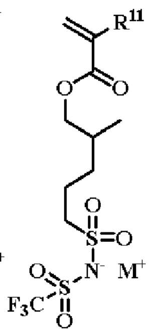
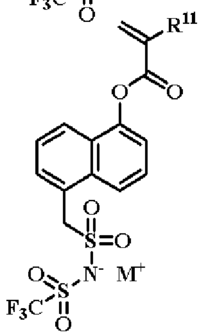
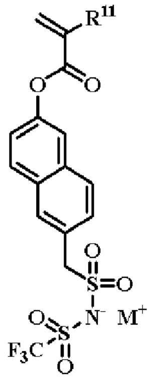
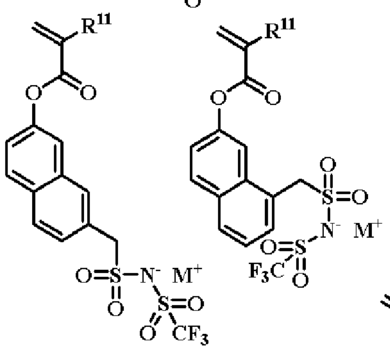
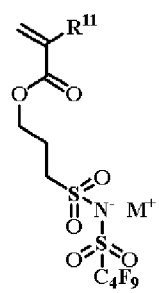
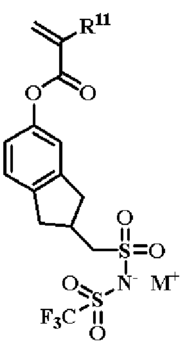
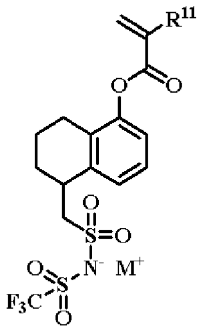
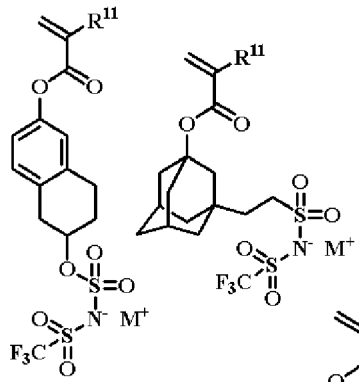
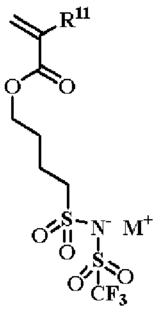
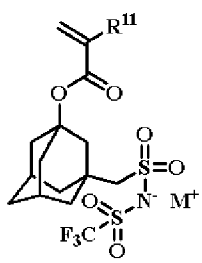
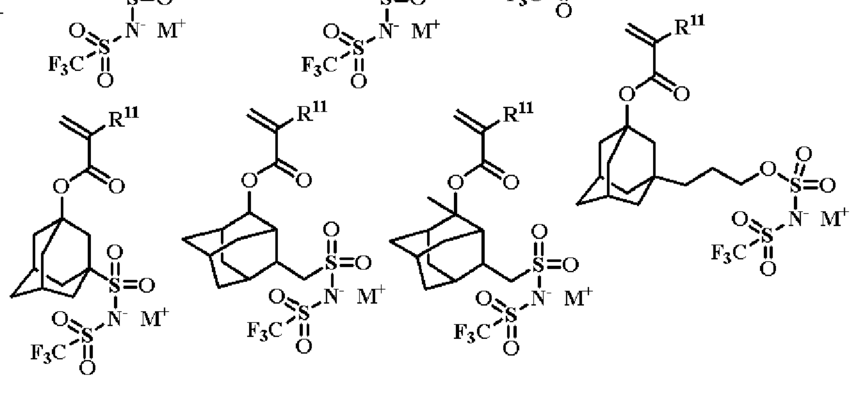

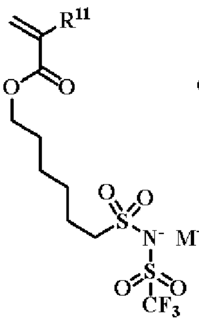
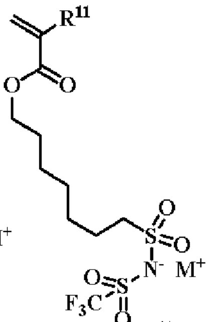
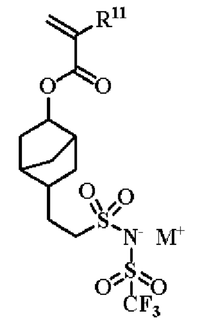
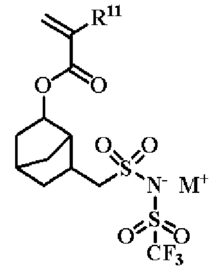
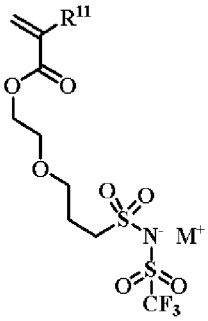
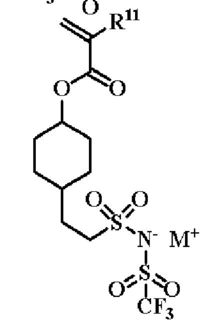
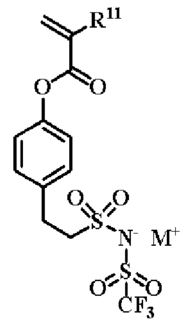
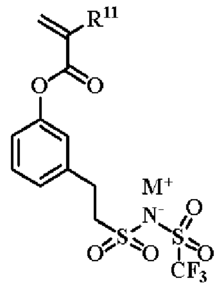
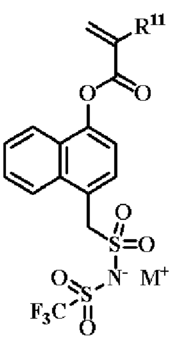
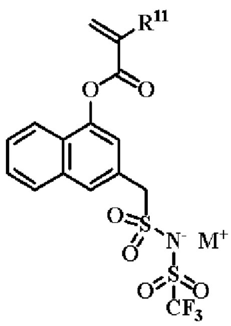
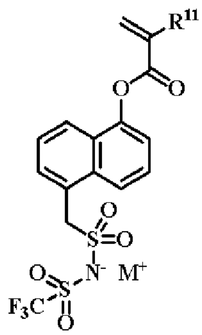
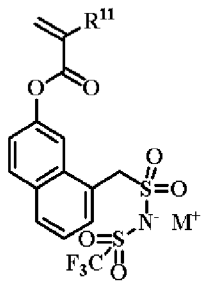
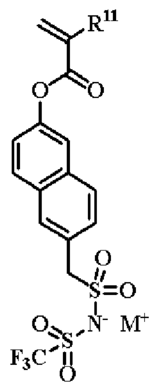
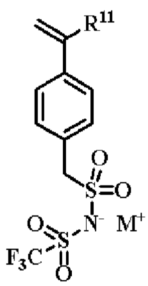
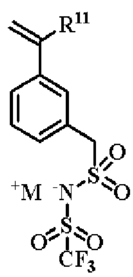
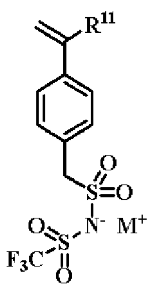
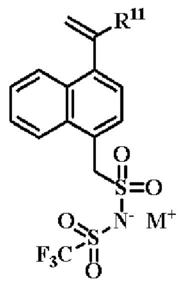
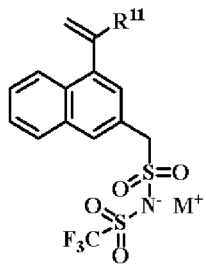
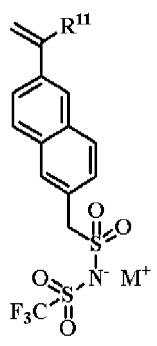

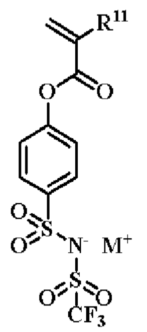
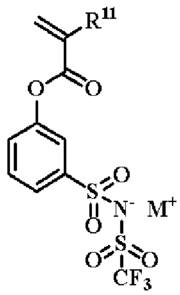
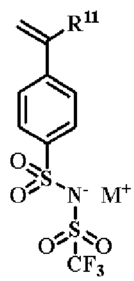
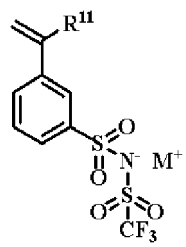

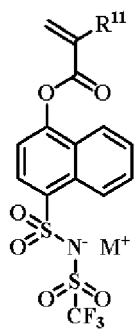
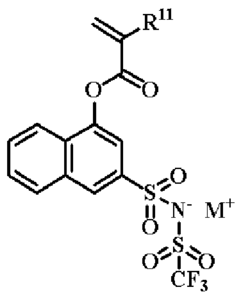
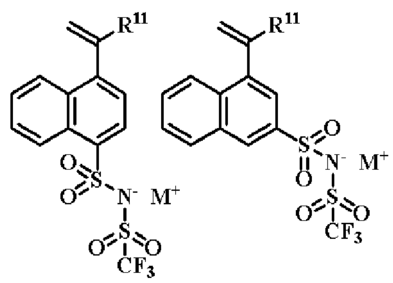

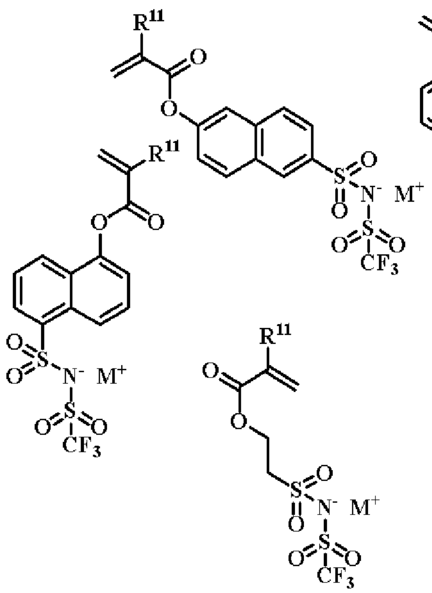
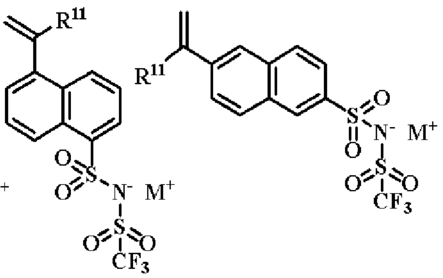

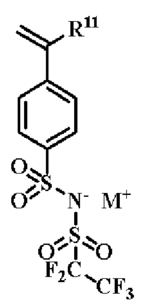
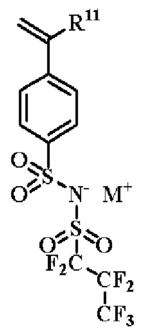

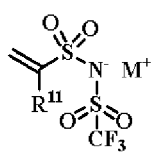
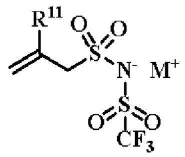

Specific examples of monomers of N-carbonyl sulfonamide salts to obtain the repeating unit A7 shown by the above-mentioned general formula can include the following.

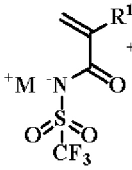 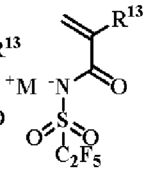 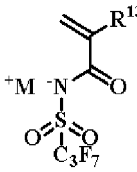 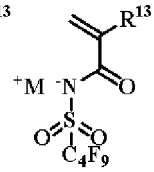 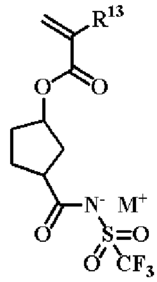 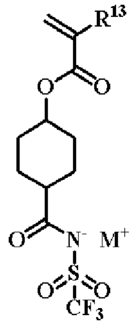 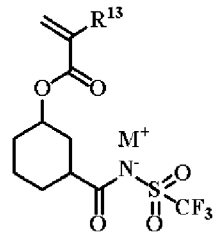

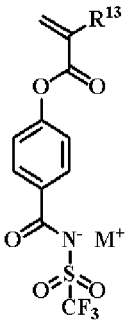 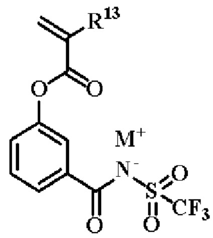 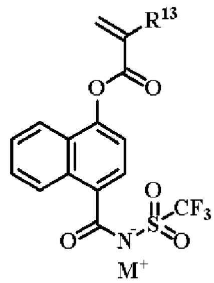 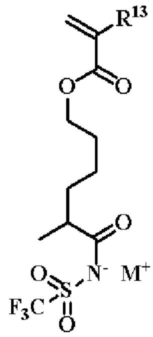 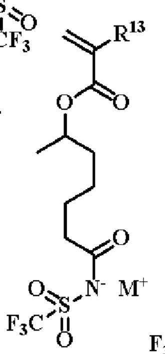 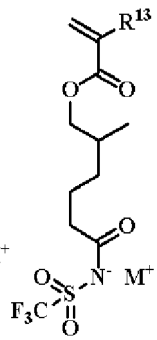

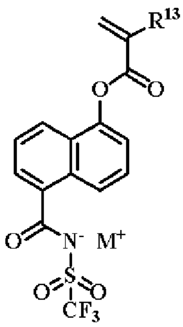 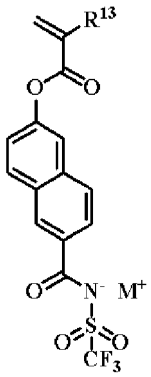 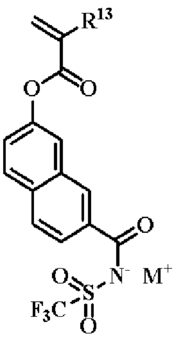 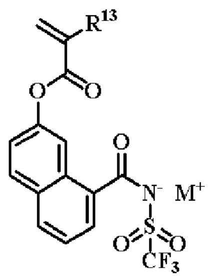 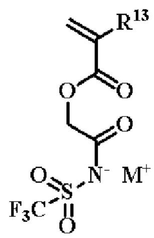

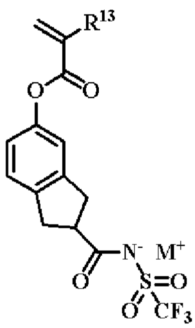 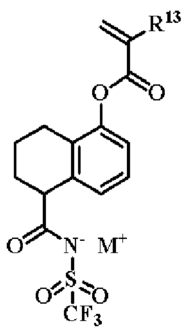 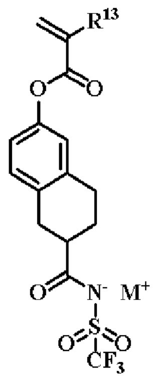 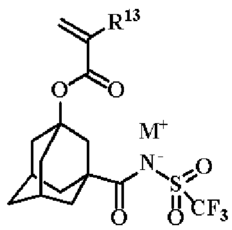 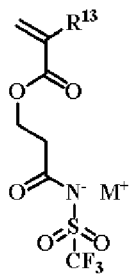

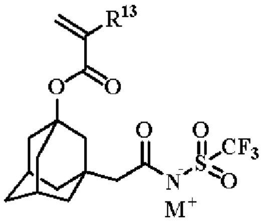 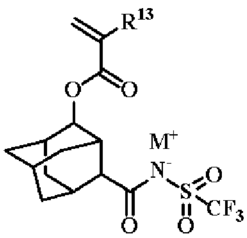 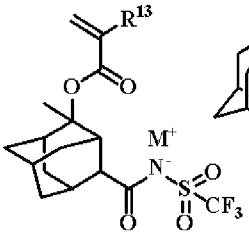 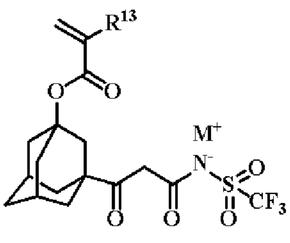

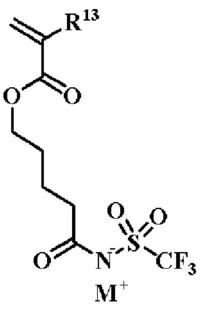
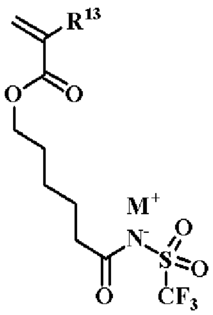
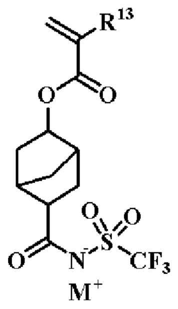
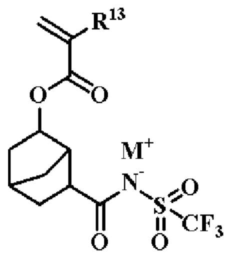

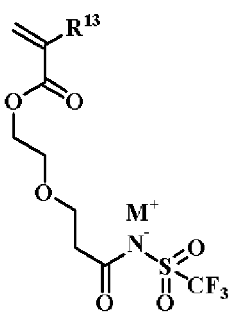
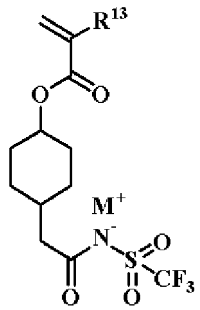
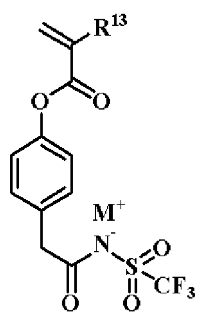
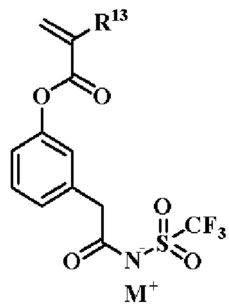

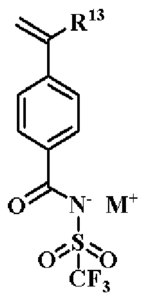
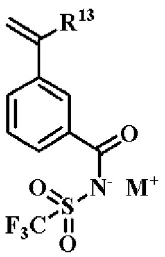

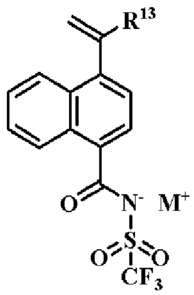
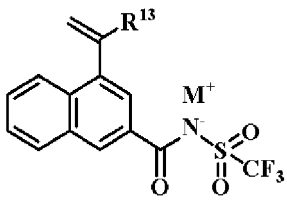
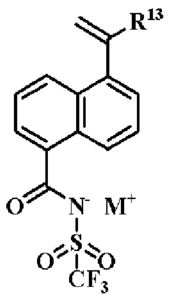
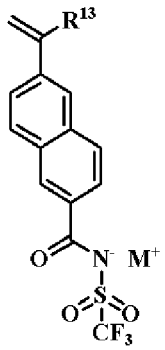

(In the formulae, $R^1$, $R^3$, $R^5$, $R^8$, $R^{10}$, $R^{11}$, and $R^{13}$ are as described above.)

The above-described ionic material (A) preferably contains an ammonium ion (ammonium cation) shown by the following general formula (3) as an ammonium ion for constituting the ammonium salts.

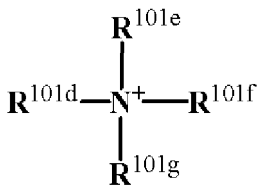
(3)

(In the general formula (3), $R^{101d}$, $R^{101e}$, $R^{101f}$, and $R^{101g}$ each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, and optionally have one or more selected from the group consisting of an ether group, a carbonyl group, an ester group, a hydroxy group, an amino group, a nitro group, a sulfonyl group, a sulfinyl group, a halogen atom, and a sulfur atom; and $R^{101d}$ and $R^{101e}$, or $R^{101d}$, $R^{101e}$, and $R^{101f}$, are optionally bonded to each other together with a nitrogen atom bonded therewith to form a ring in which $R^{101d}$ and $R^{101e}$, or $R^{101d}$, $R^{101e}$, and $R^{101f}$, represent an alkylene group having 3 to 10 carbon atoms, or to form a heteroaromatic ring having the nitrogen atom in the general formula (3) within the ring.)

Specific examples of the ammonium ion shown by the above-mentioned general formula (3) can include the following.

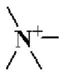 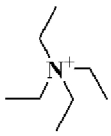 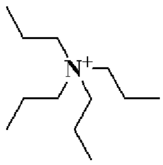 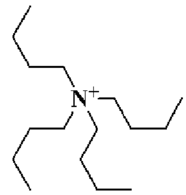 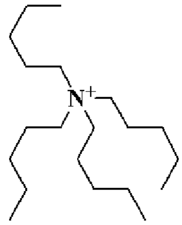

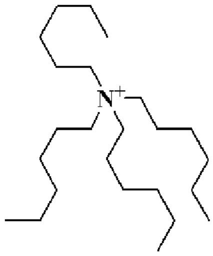 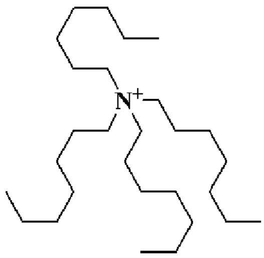 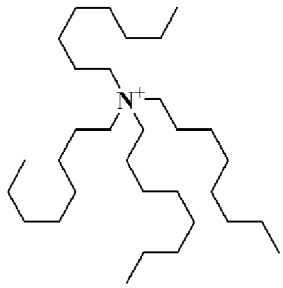

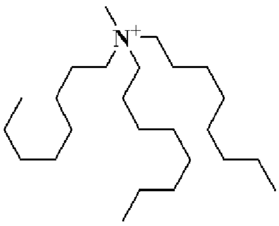 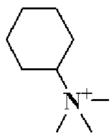 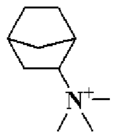 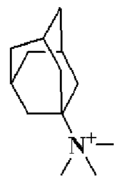 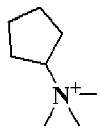 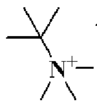 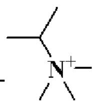

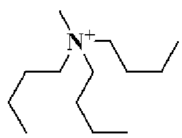 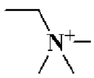 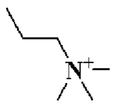 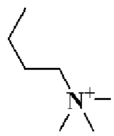 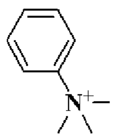

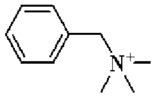 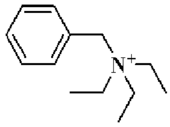 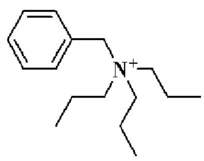 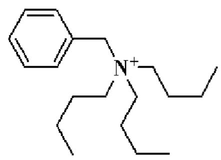

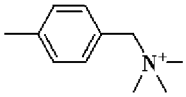 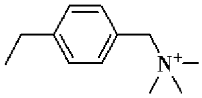 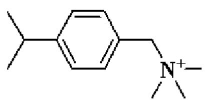 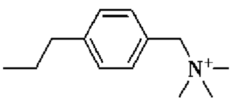

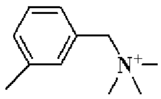 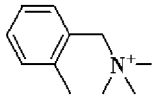 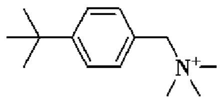 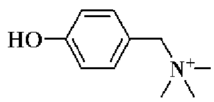

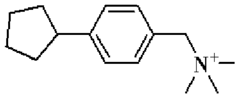 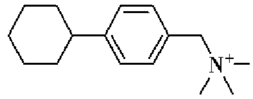 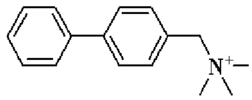

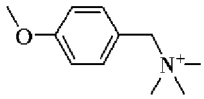 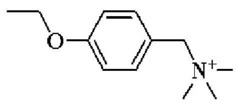 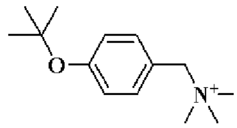

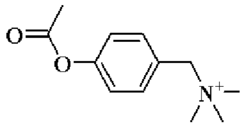 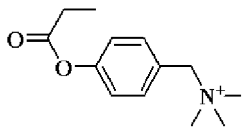 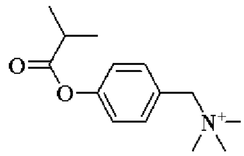

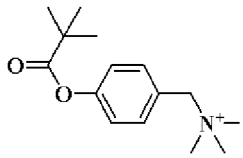 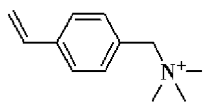 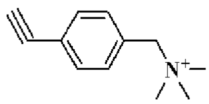

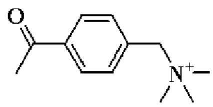 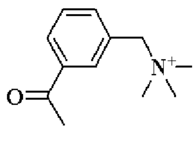 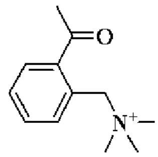

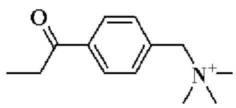 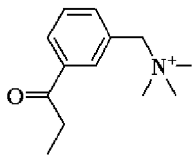 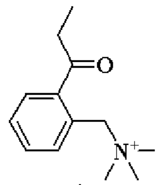

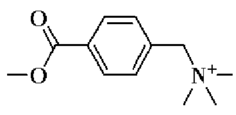 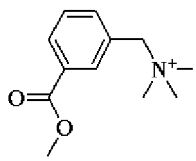 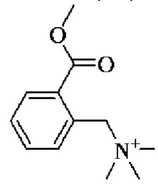

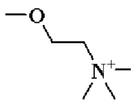 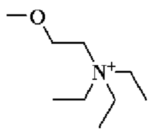 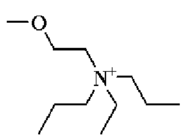 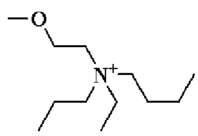

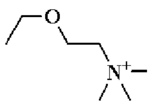 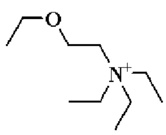 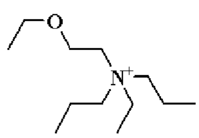 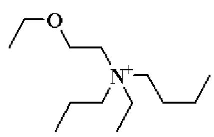

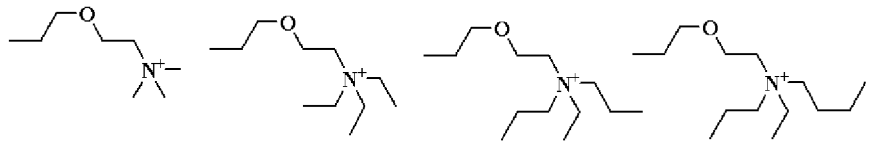

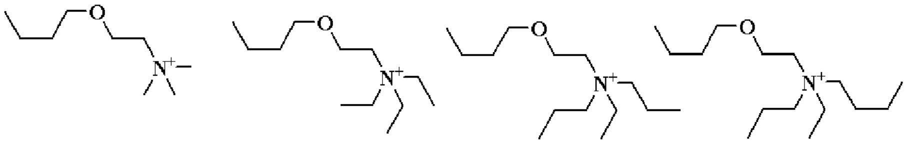

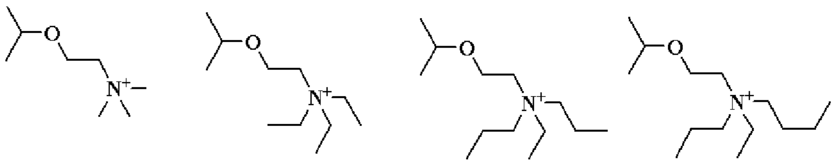

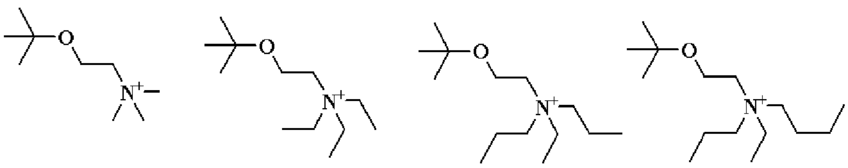

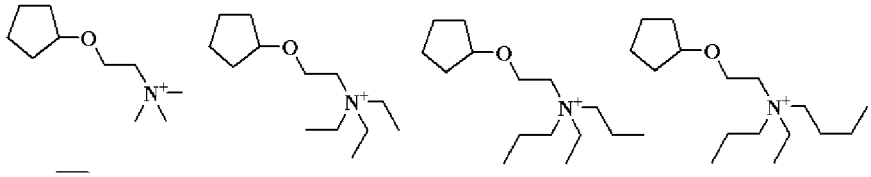

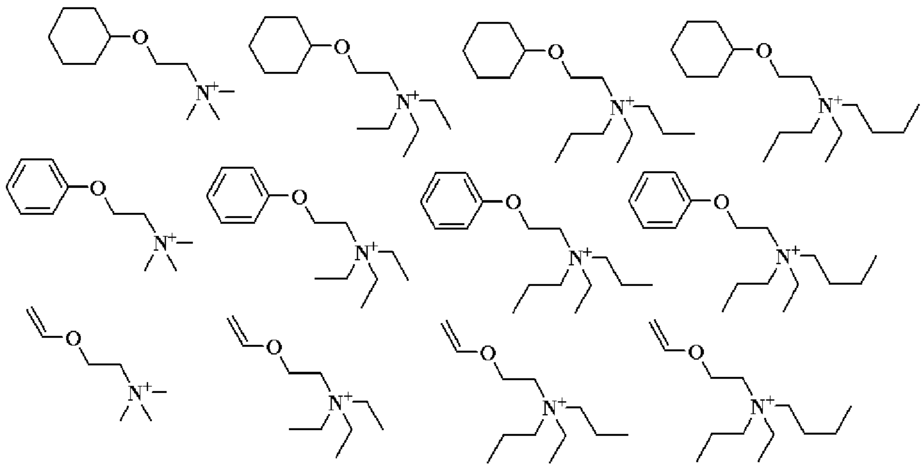

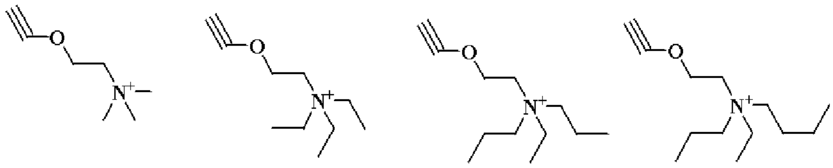

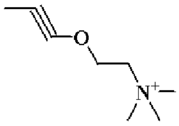
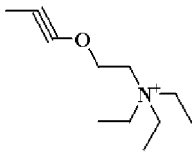
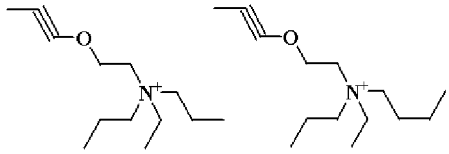

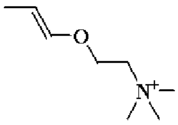
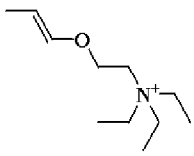
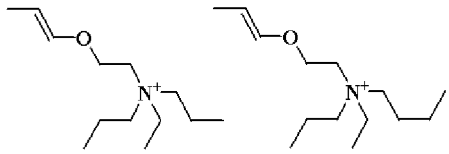

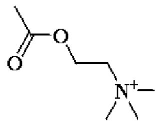
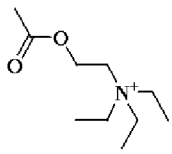
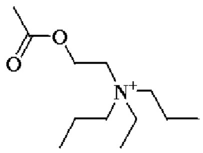
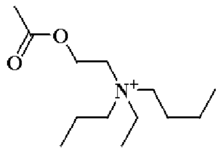

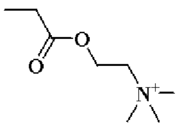
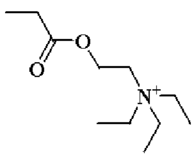
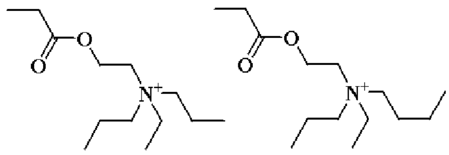

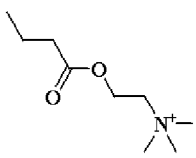
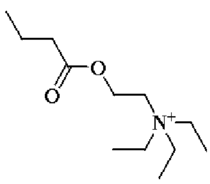
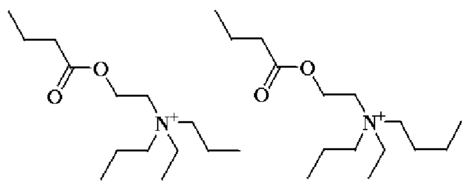

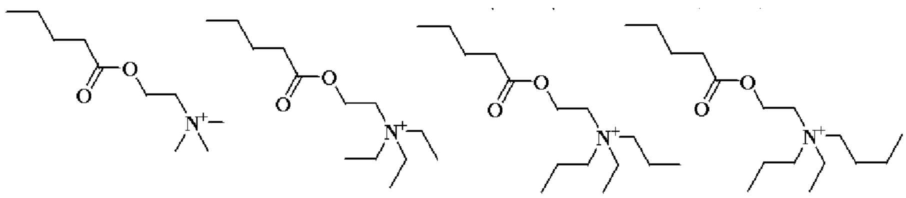

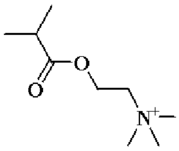
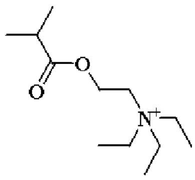
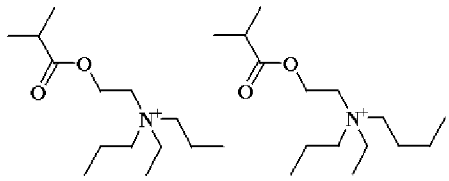

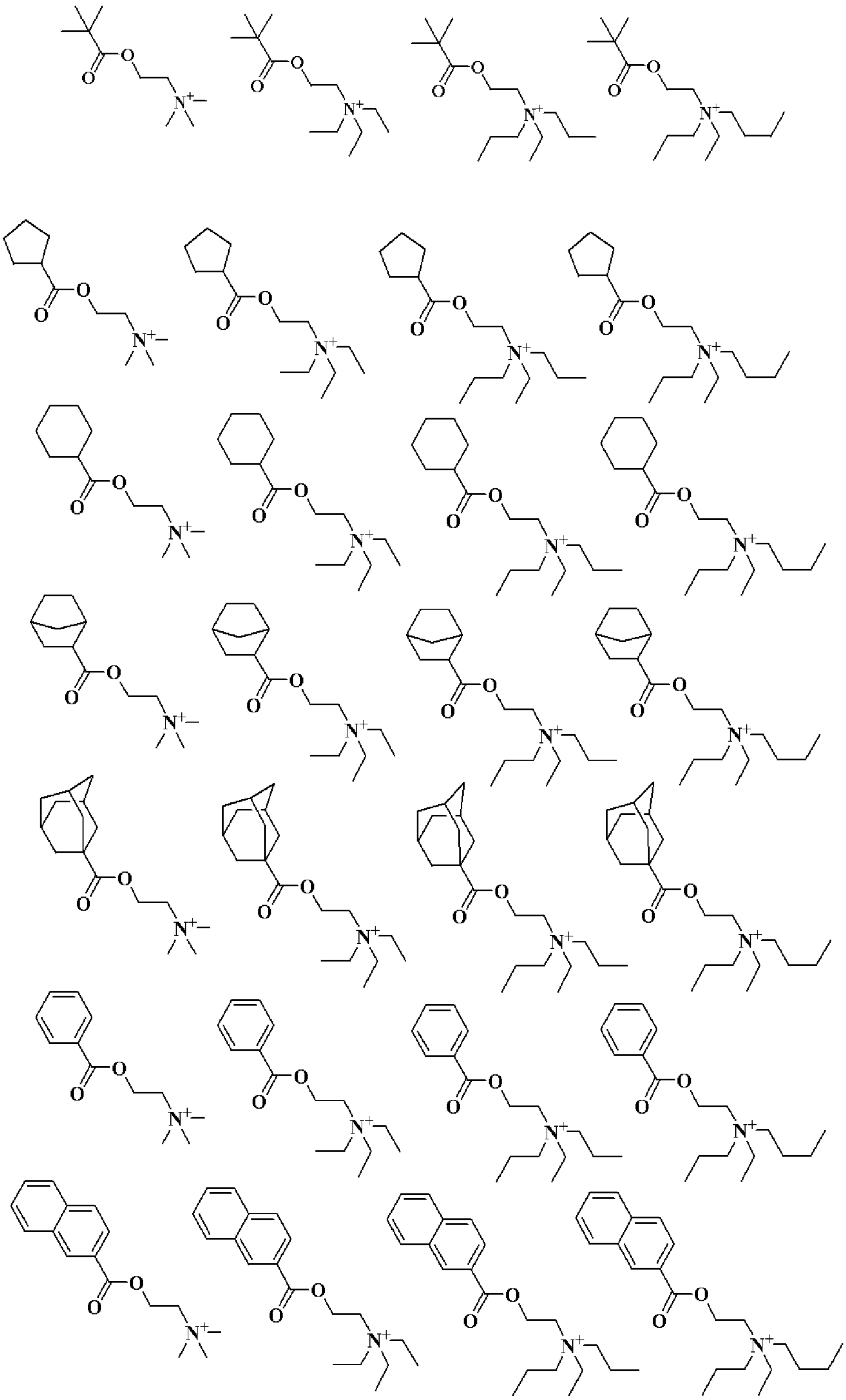

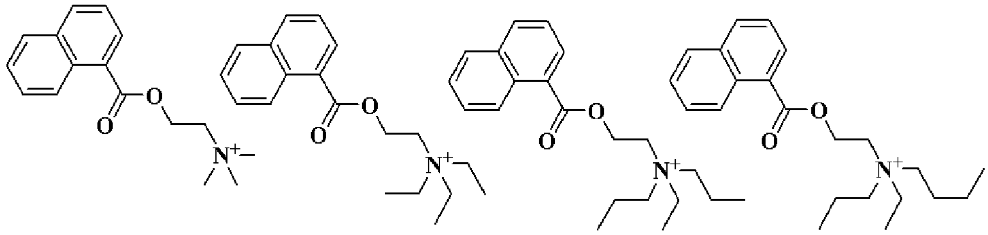

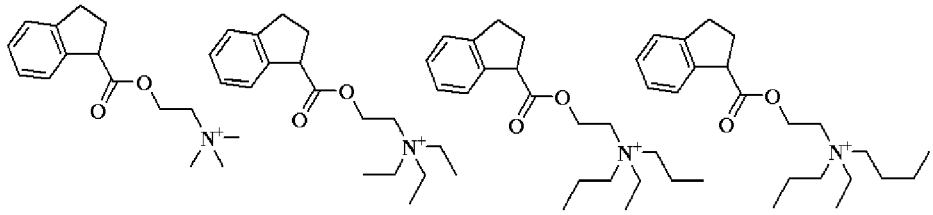

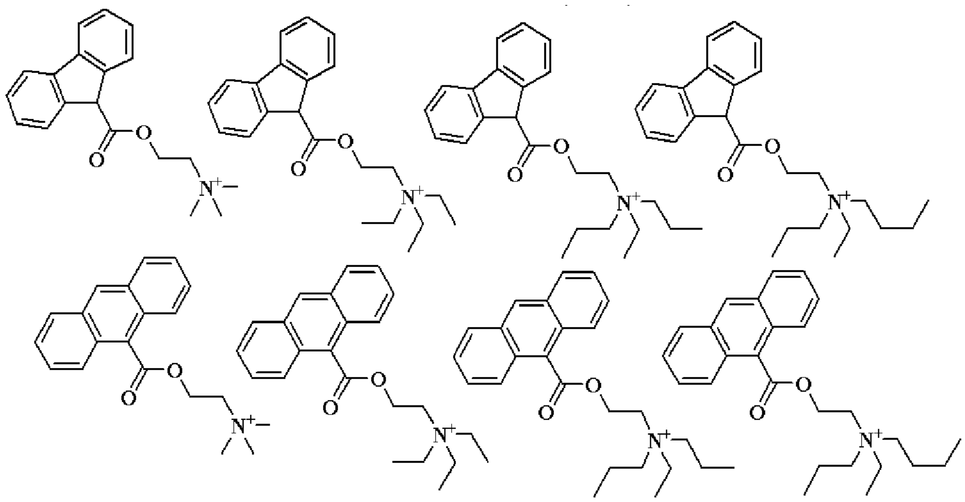

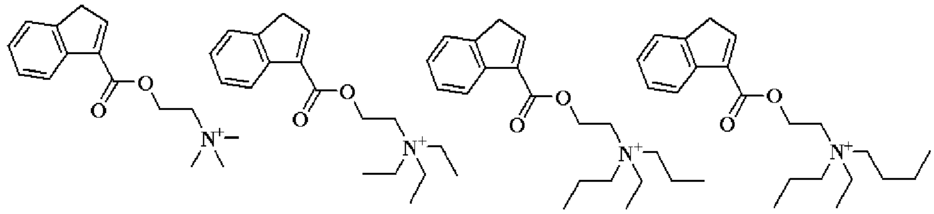

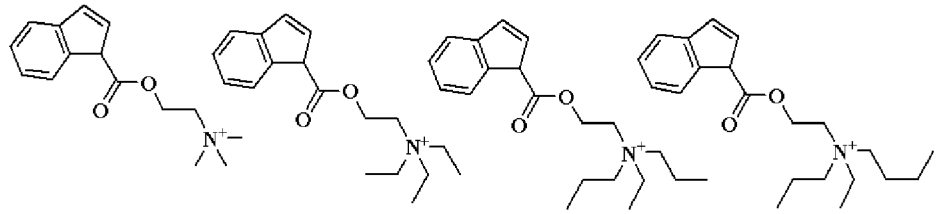

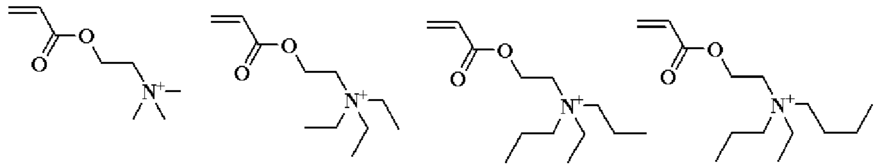

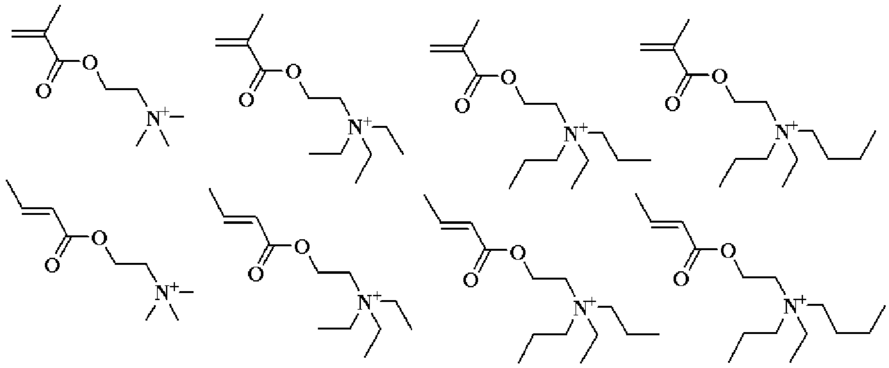

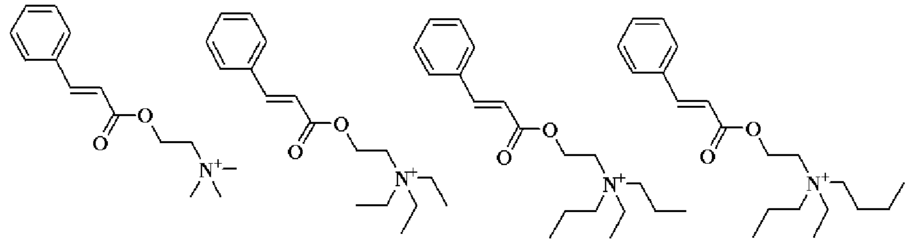

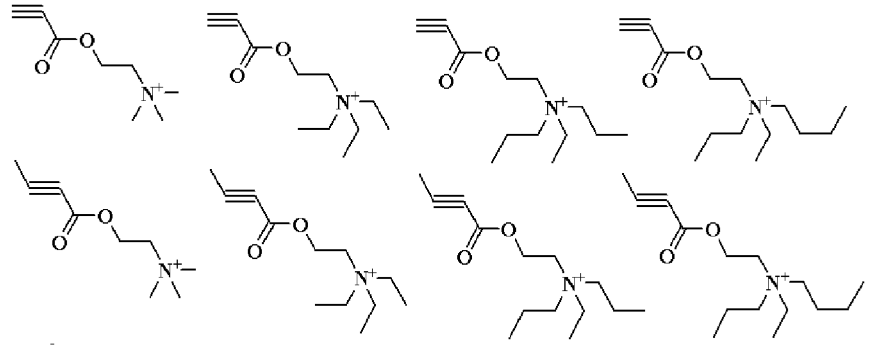

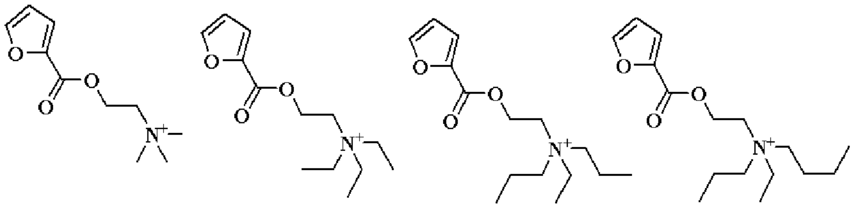

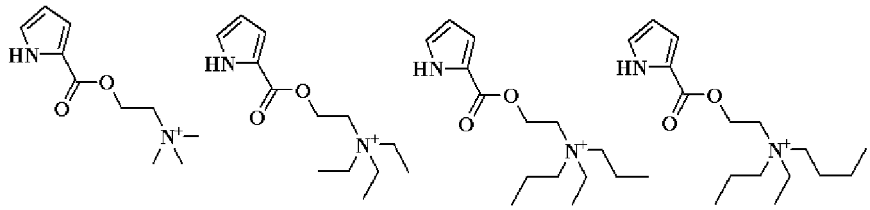

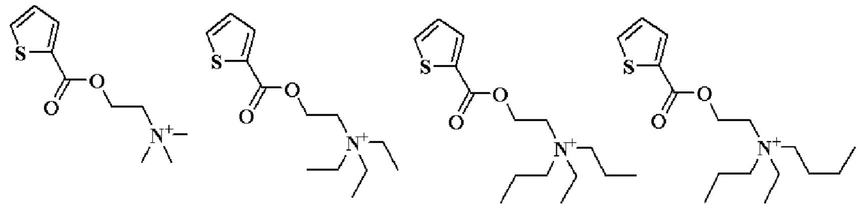

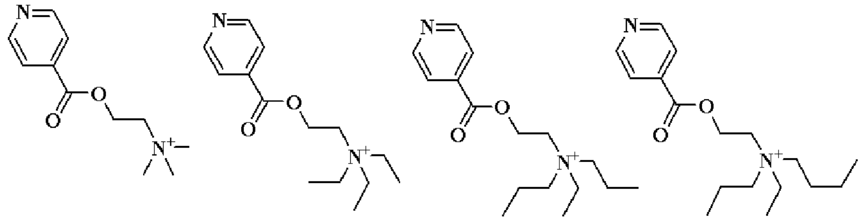

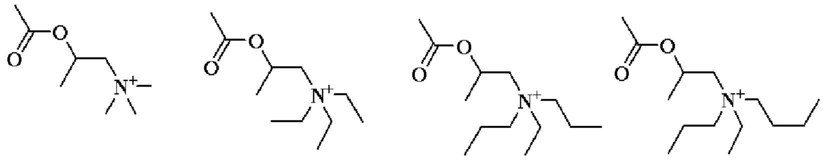

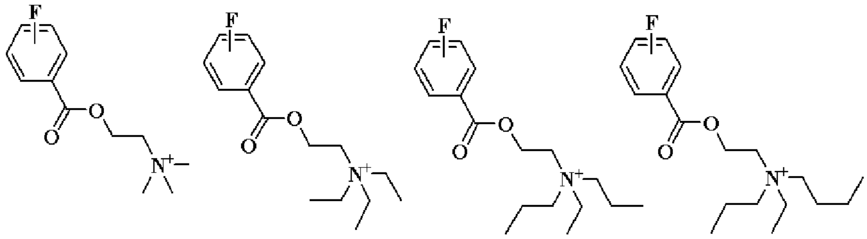

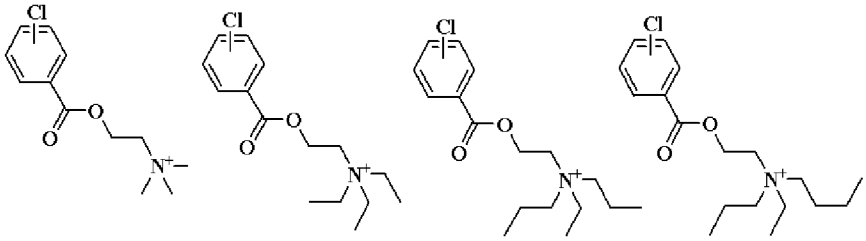

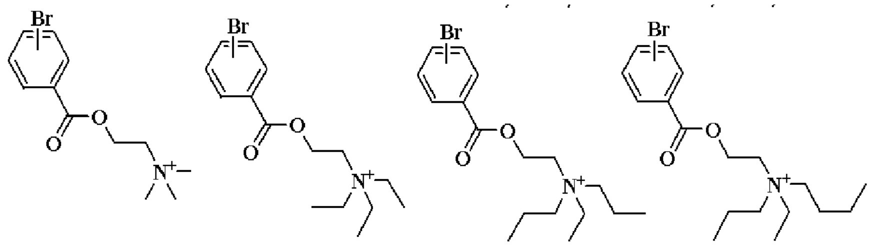

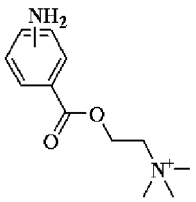
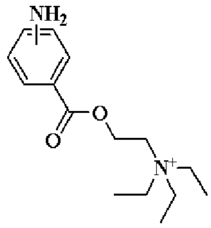
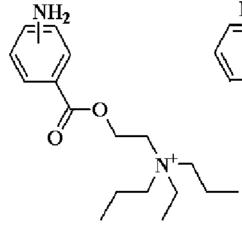
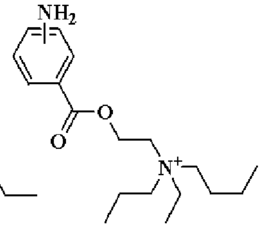

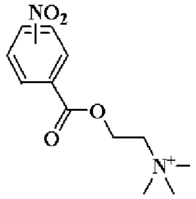
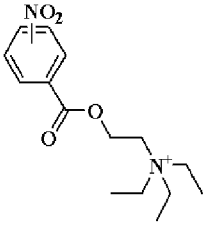
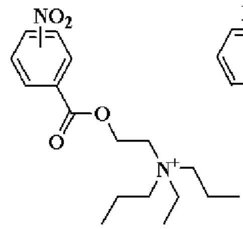
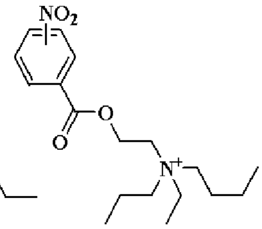

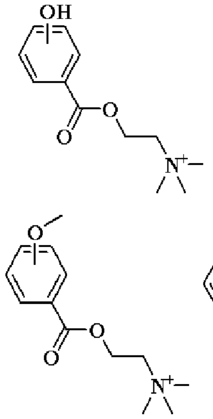
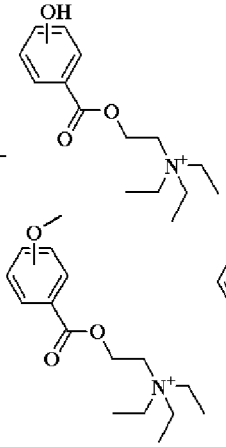
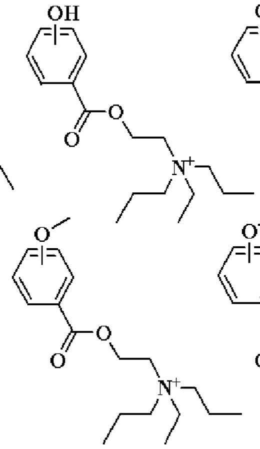
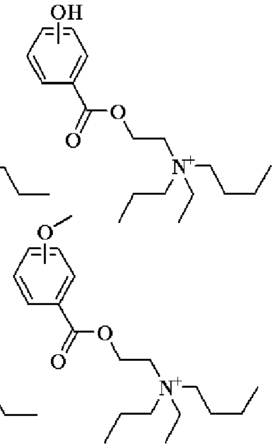

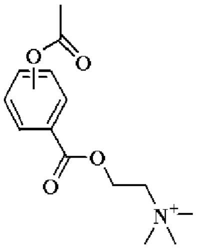
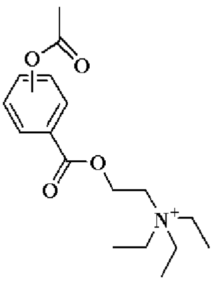
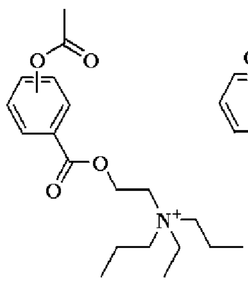
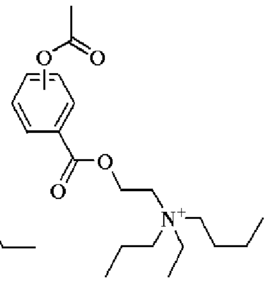

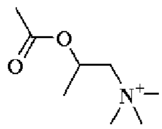
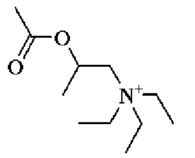
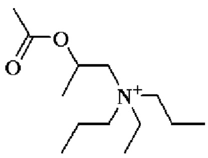
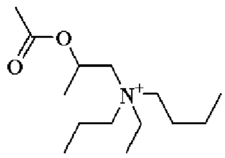

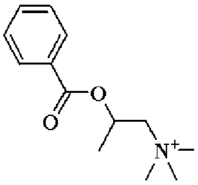 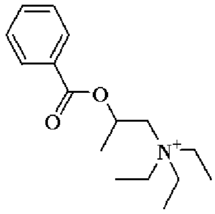 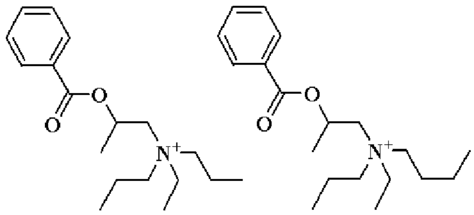

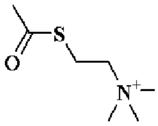 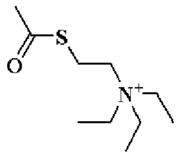 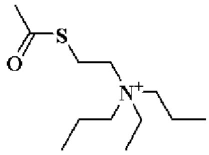 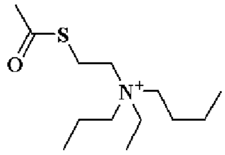

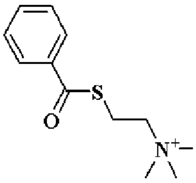 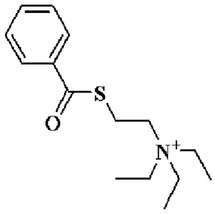 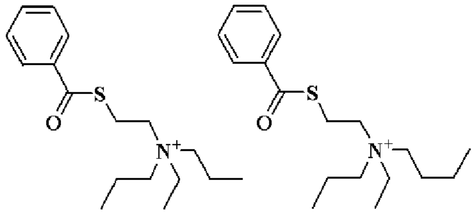

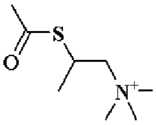 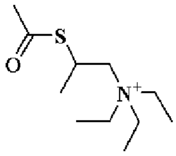 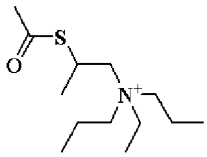 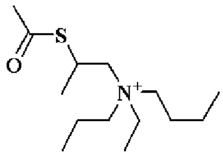

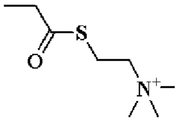 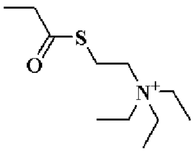 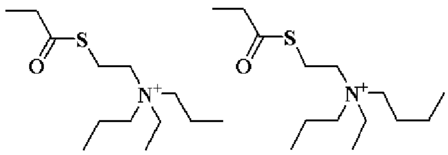

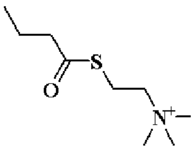 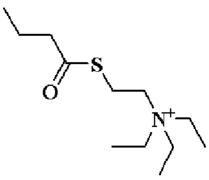 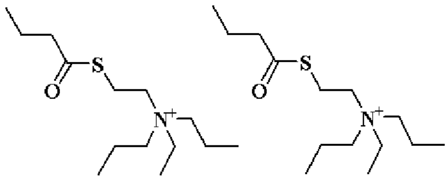

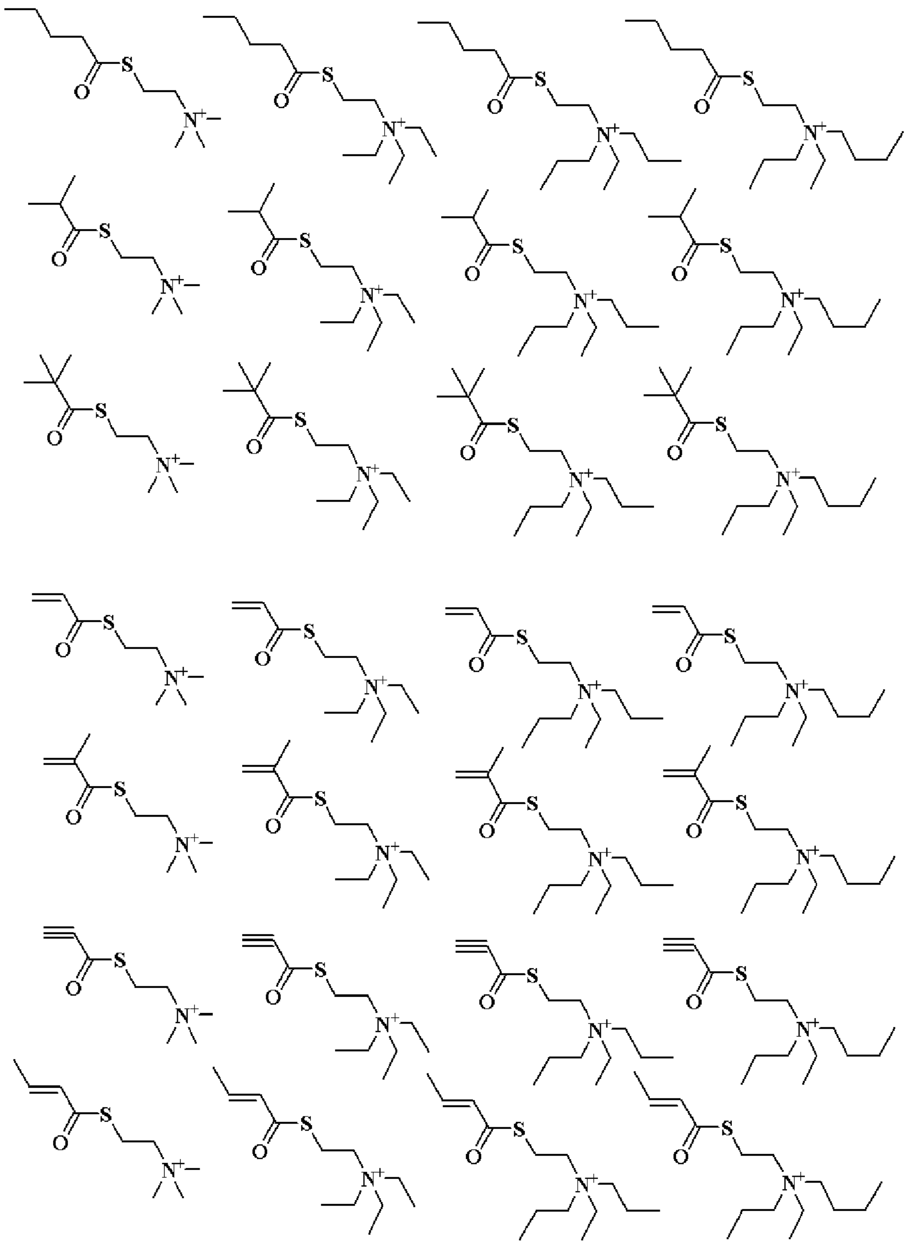

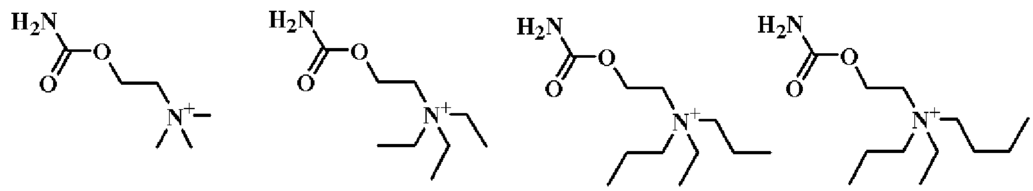

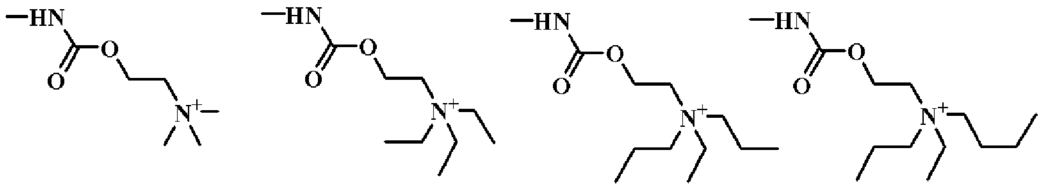

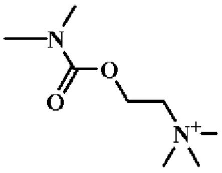
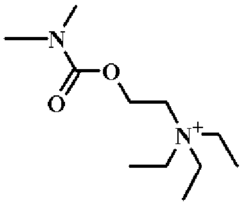
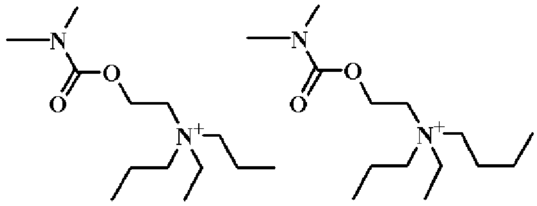

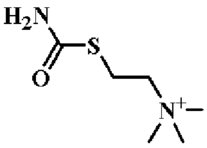
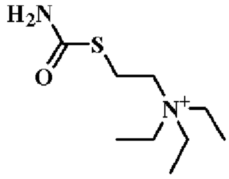
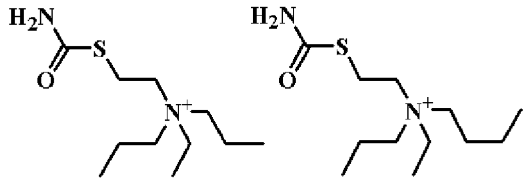

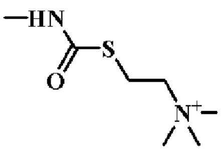
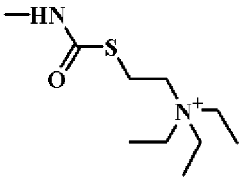
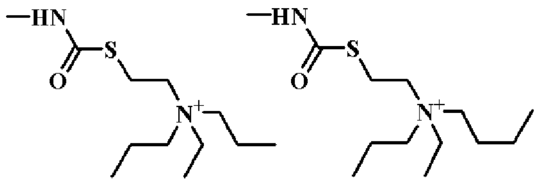

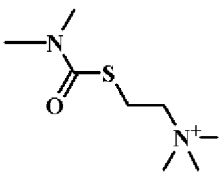
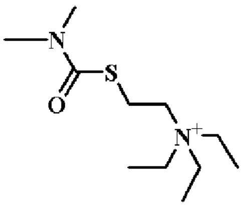
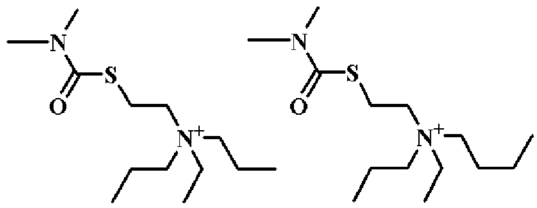

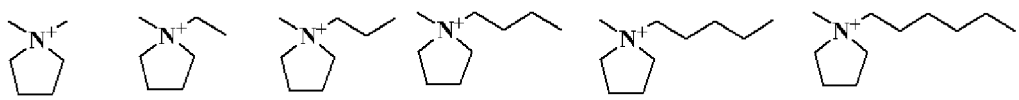

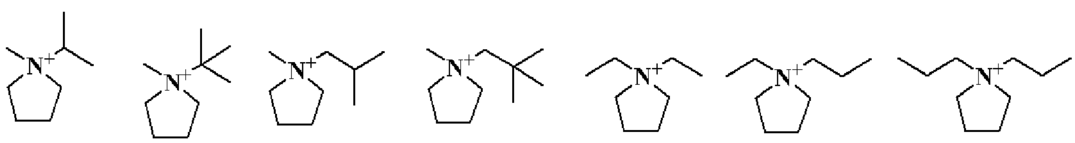

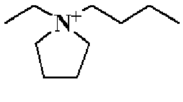
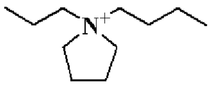
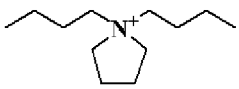
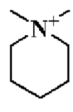
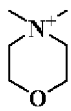
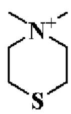

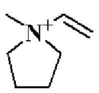
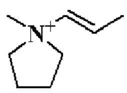
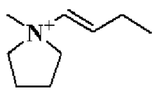
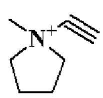
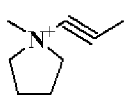
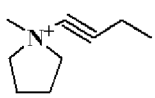
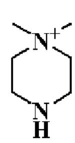

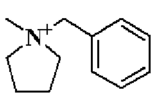
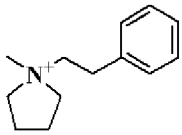
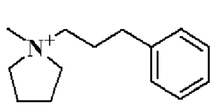

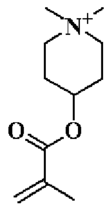
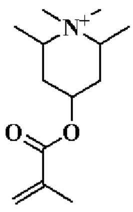
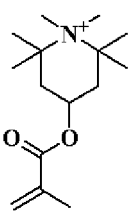

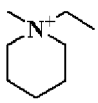
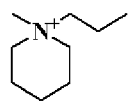
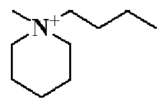
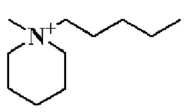
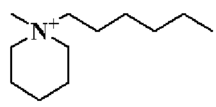
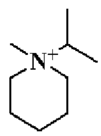

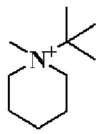
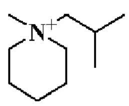
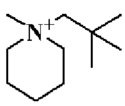
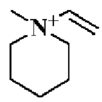
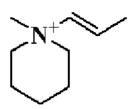
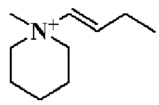

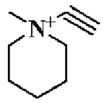
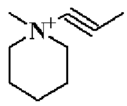
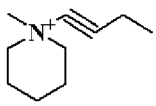
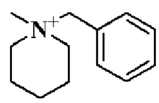
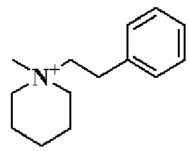
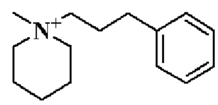

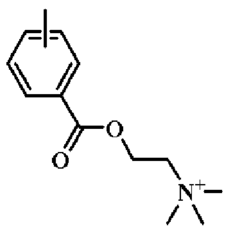
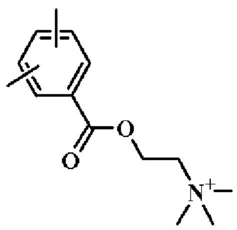
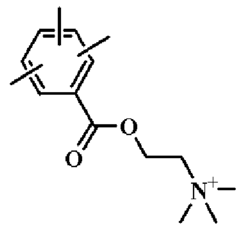
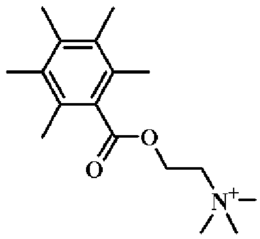

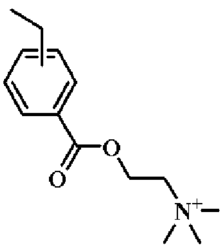
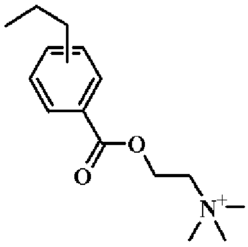
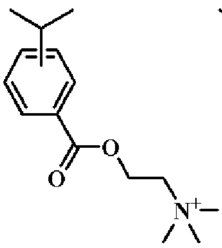
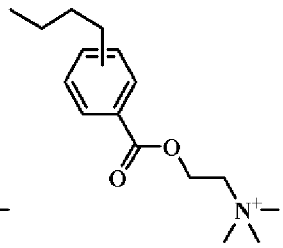

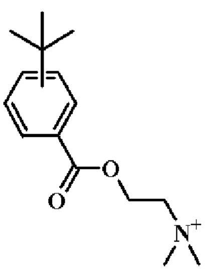
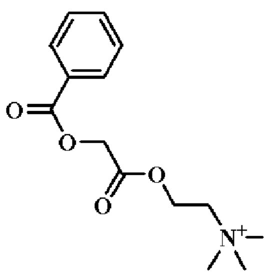
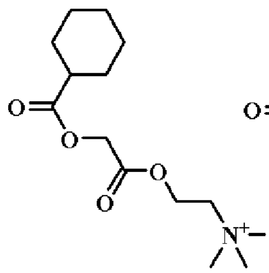
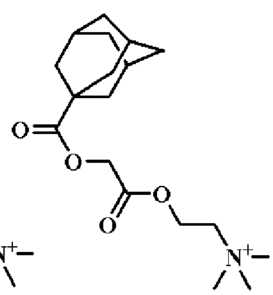

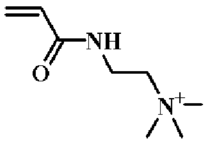

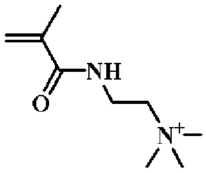

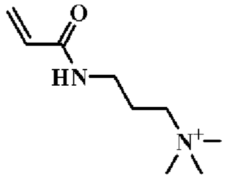

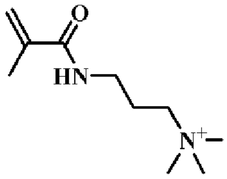

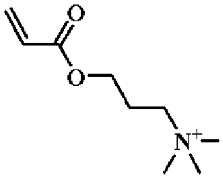

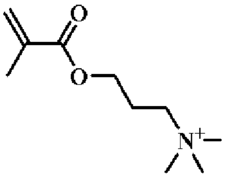

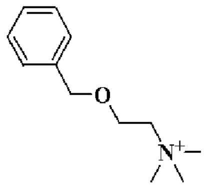

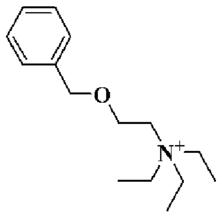

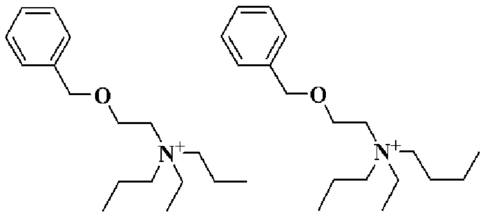

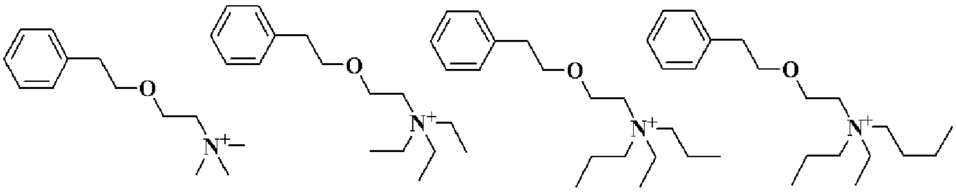

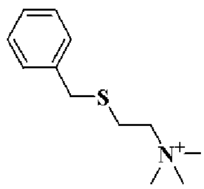

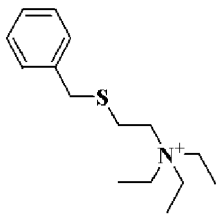

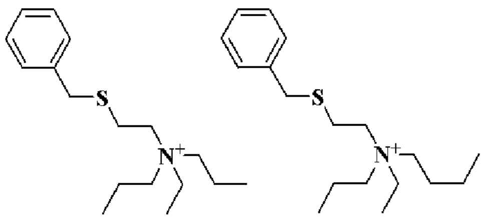

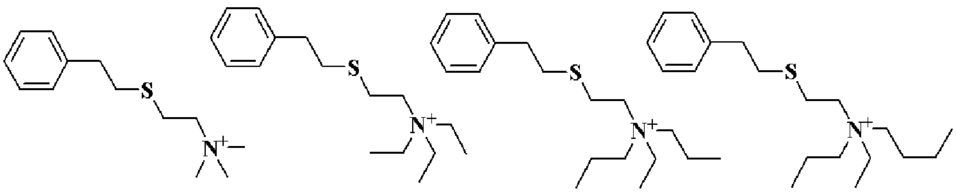

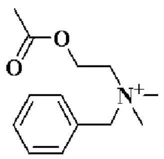

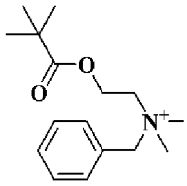

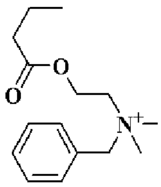

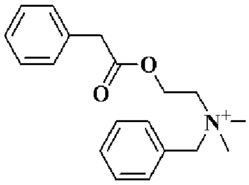

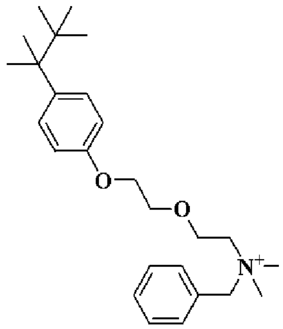

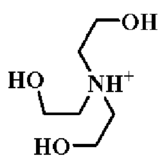

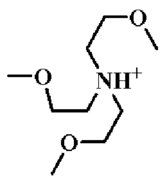

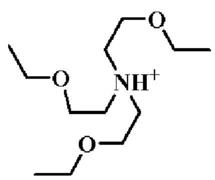

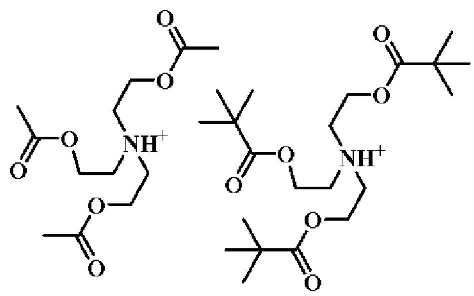

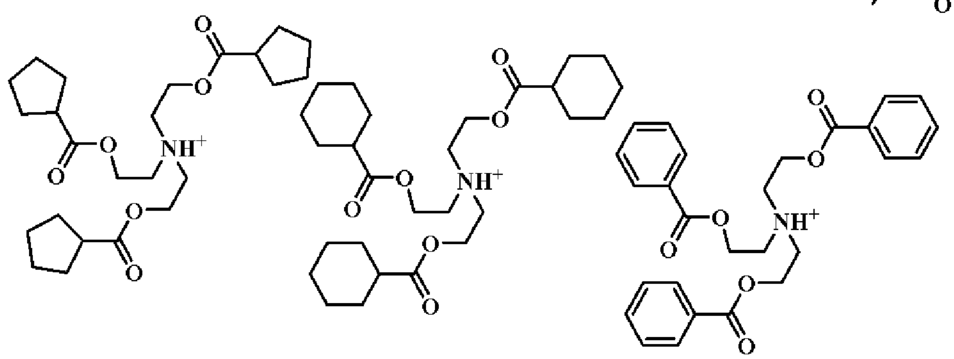

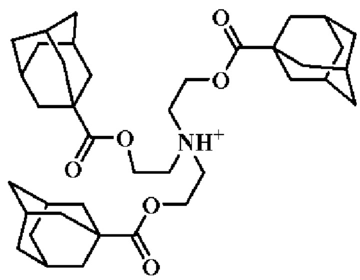

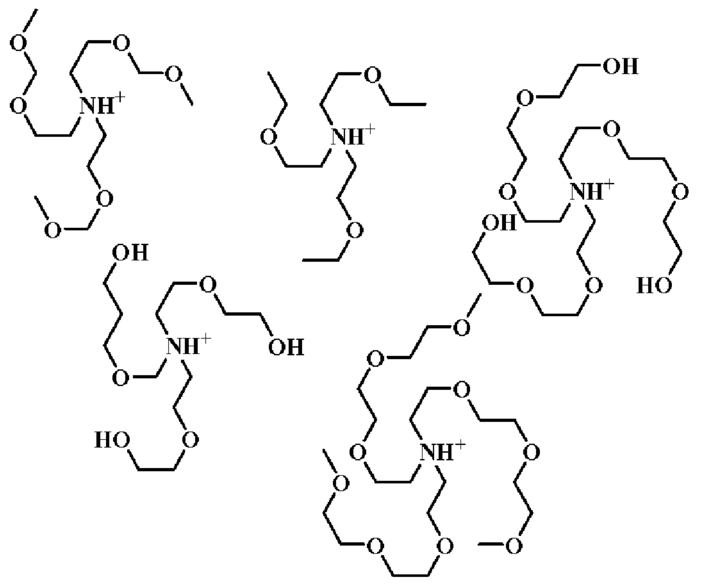
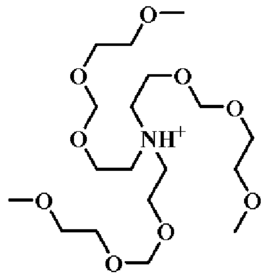

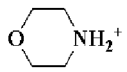
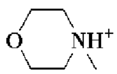
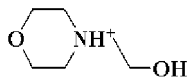
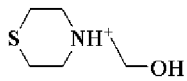
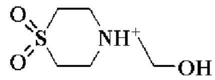

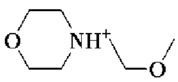
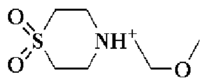
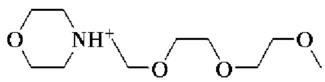

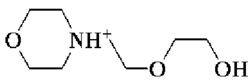
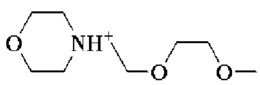
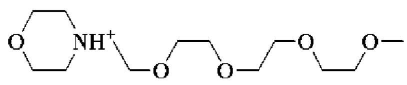

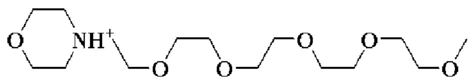
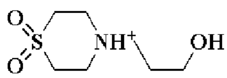
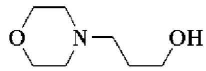

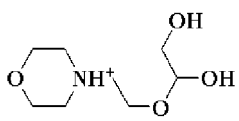
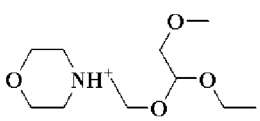
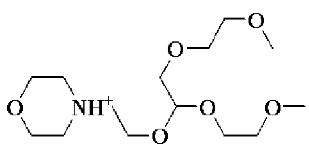

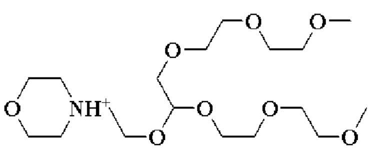
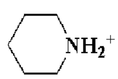
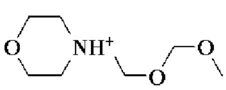
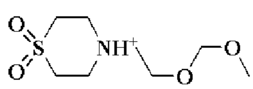

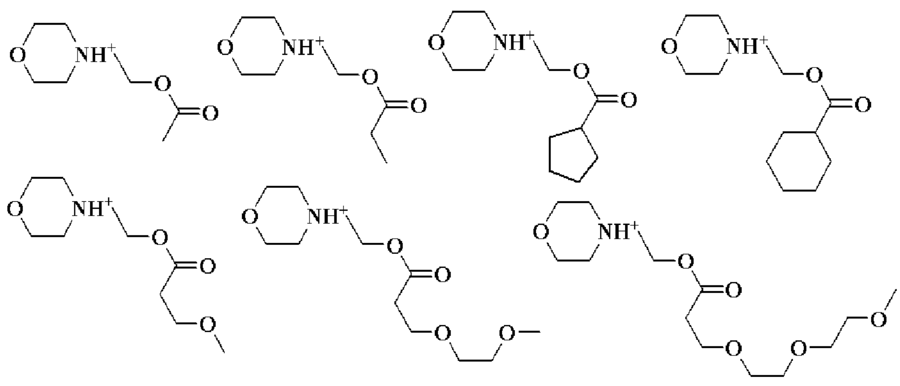

As the ammonium ion shown by the above-mentioned general formula (3), a tertiary ammonium ion or a quaternary ammonium ion is especially preferable.

Repeating Unit B

In the component (A) of the material for forming the conductive film used for the bio-electrode according to the present invention, a repeating unit B having a glyme chain can be copolymerized, in addition to the above-mentioned repeating units A1 to A7, to improve conductivity. Specific examples of monomers for obtaining the repeating unit B having the glyme chain can include the following. Copolymerizing the repeating unit having the glyme chain can promote the movement of ions emitted from the skin within a dry electrode film, and increase sensitivity of the dry electrode.

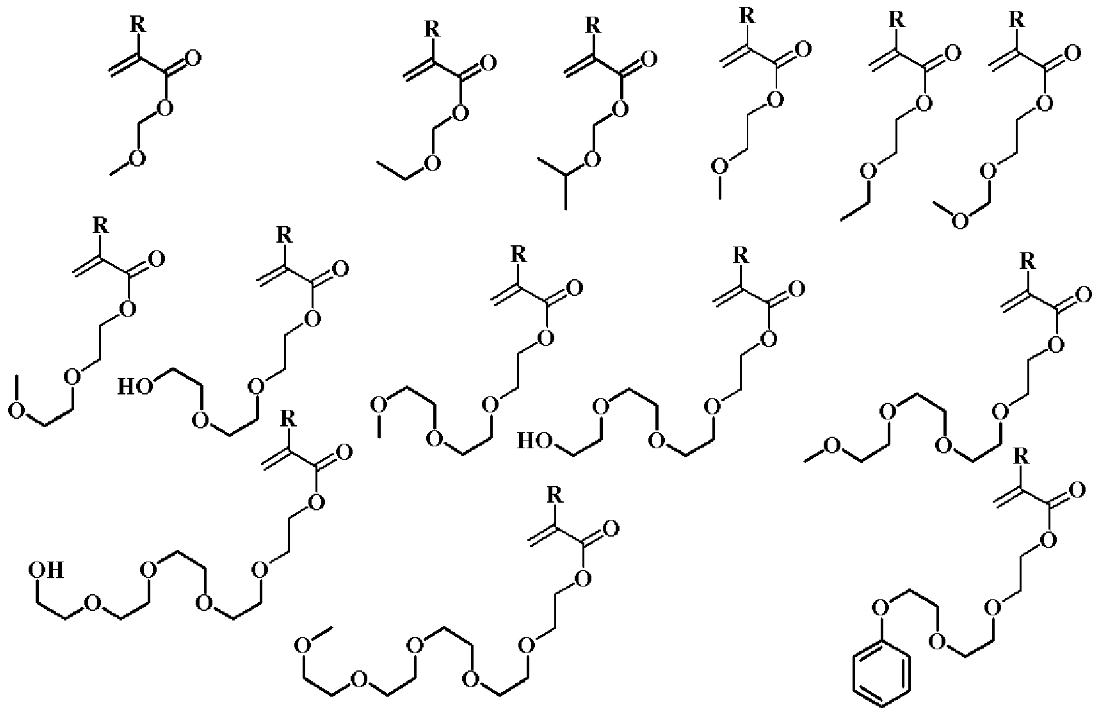

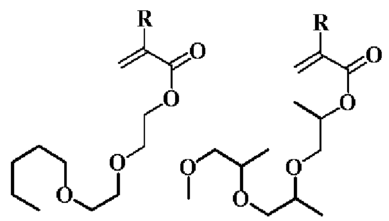

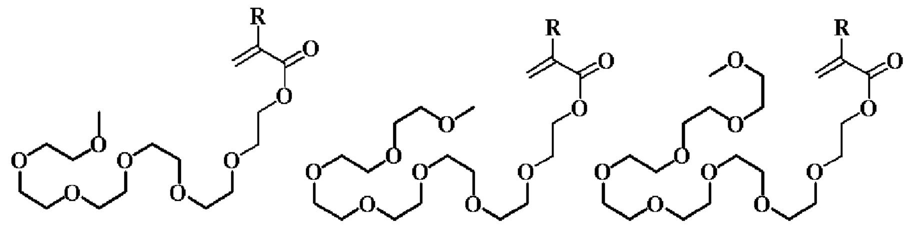

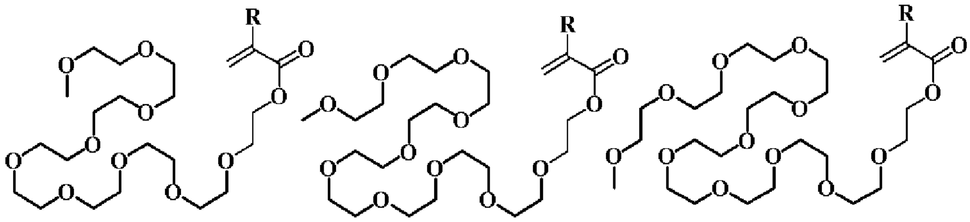

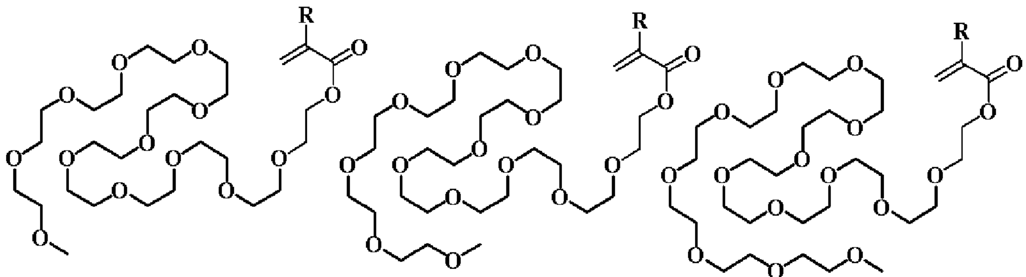

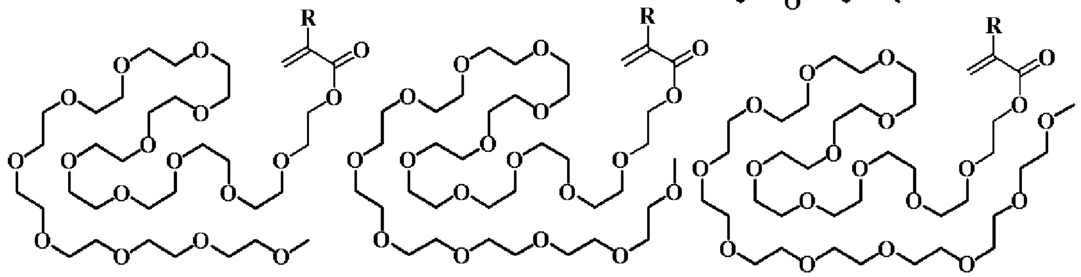

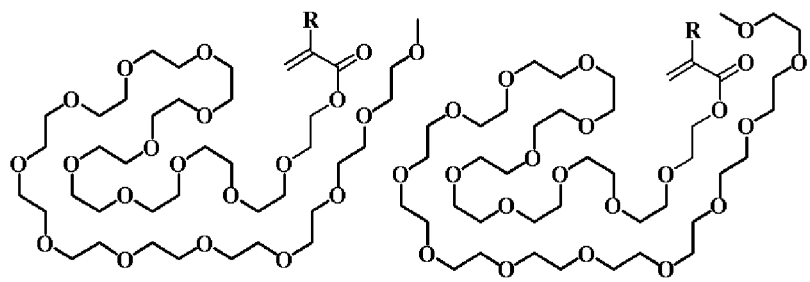

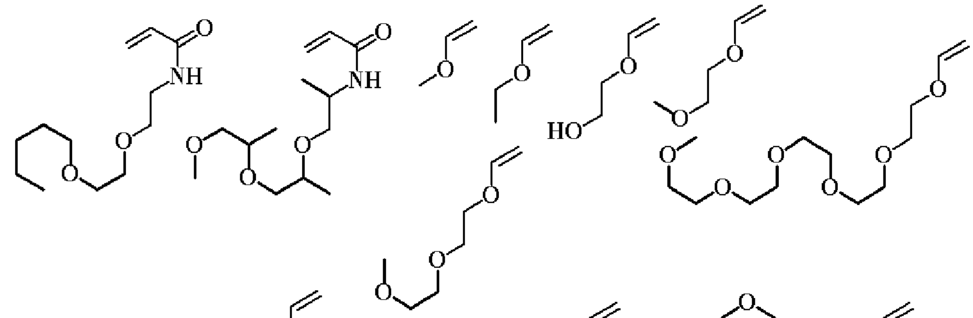

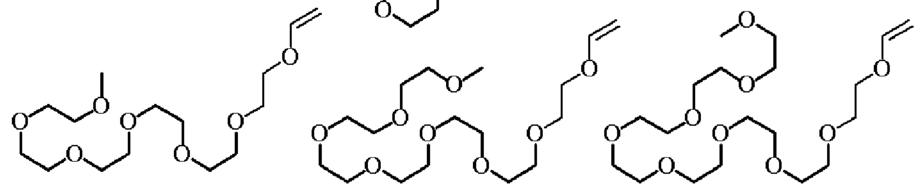

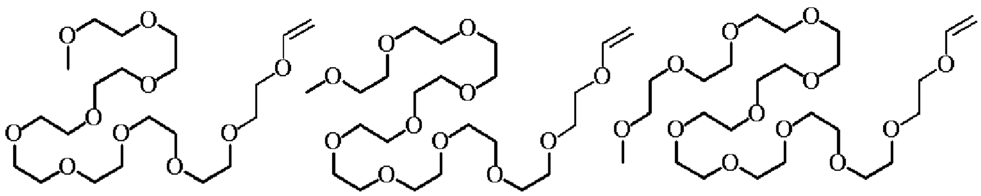

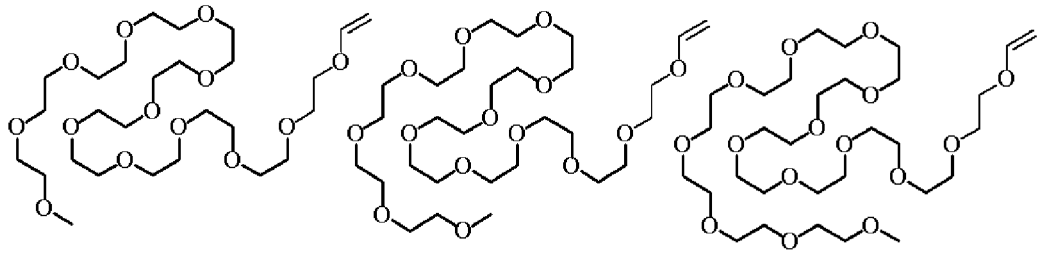

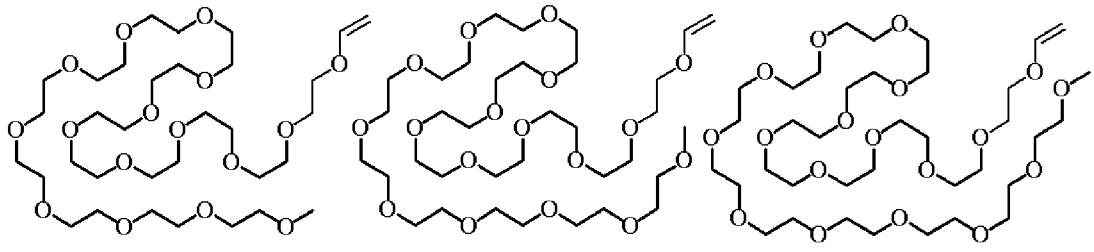

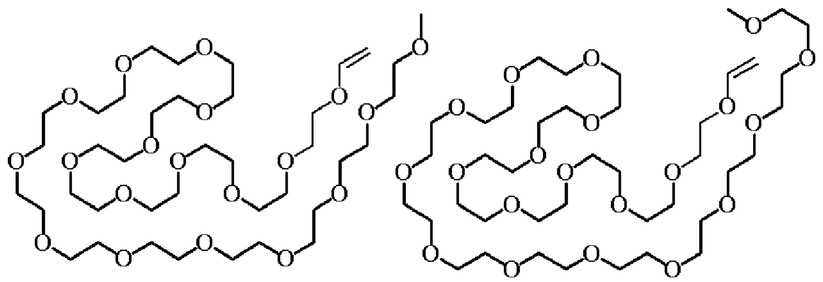

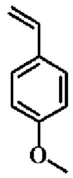 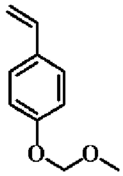 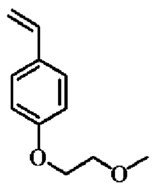 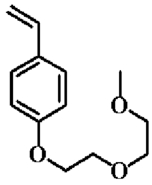 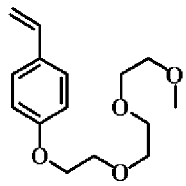 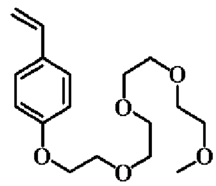

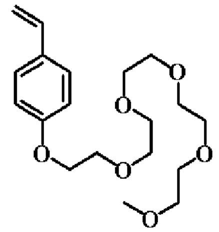 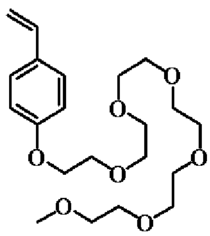 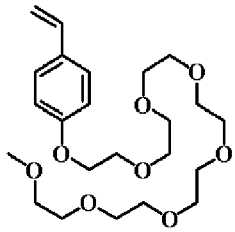 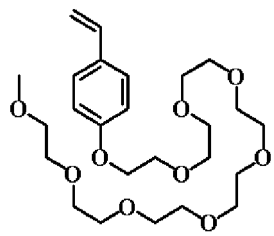

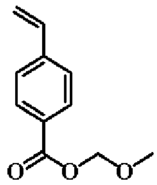 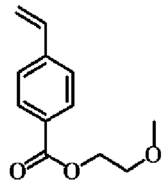 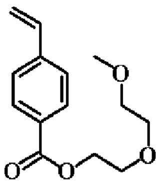 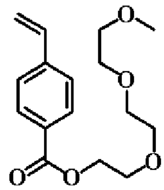 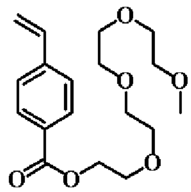

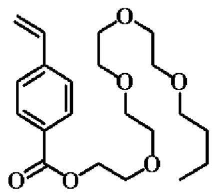 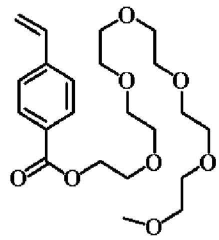 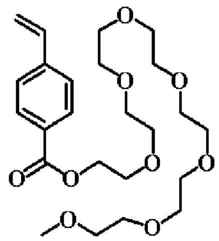 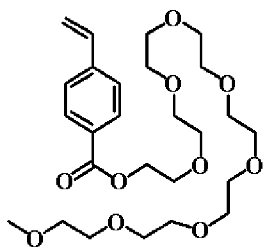

R represents a hydrogen atom or a methyl group.

Repeating Unit C

In the component (A) of the material for forming the conductive film used for the bio-electrode according to the present invention, a hydrophilic repeating unit C having a hydroxy group, a carboxyl group, ammonium salts, betaine, an amide group, pyrolidone, a lactone ring, a lactam ring, a sultone ring, sodium salts of sulfonic acid, or potassium salts of sulfonic acid can be copolymerized, in addition to the above-mentioned repeating units A1 to A7, to improve conductivity. Specific examples of monomers for obtaining the hydrophilic repeating unit C can include the following. Copolymerizing the repeating unit containing these hydrophilic groups can increase sensitivity to ions emitted from the skin and increase sensitivity of the dry electrode.

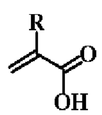 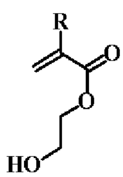 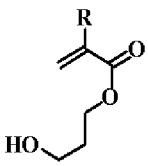 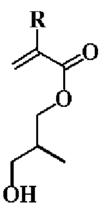 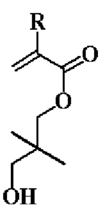 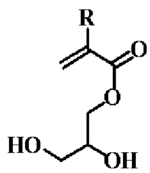 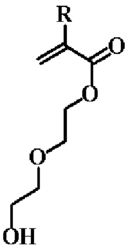

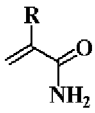
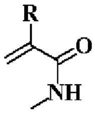
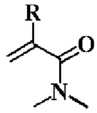
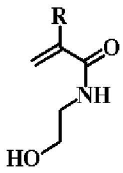
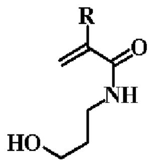
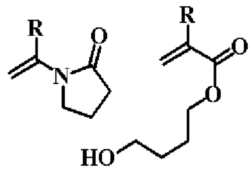

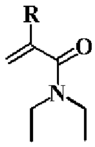
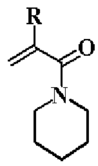
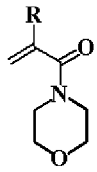
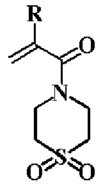
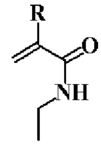
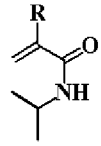
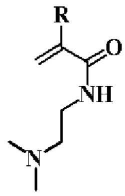

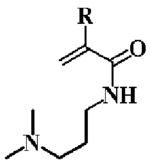
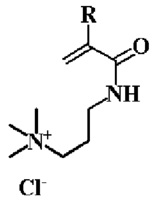
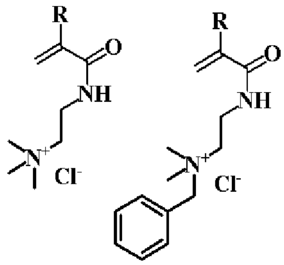
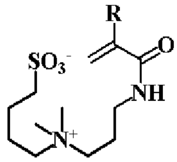
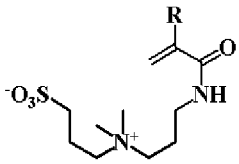

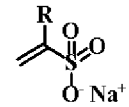
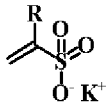
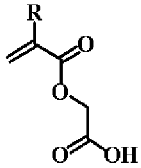
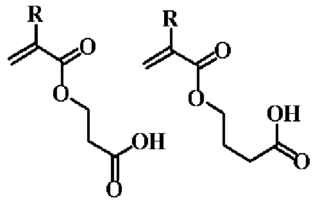

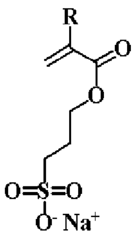
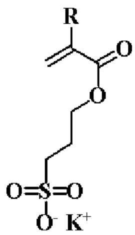
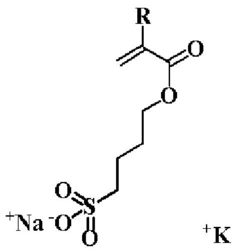
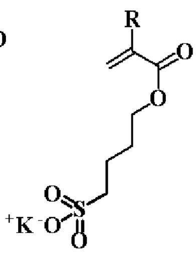
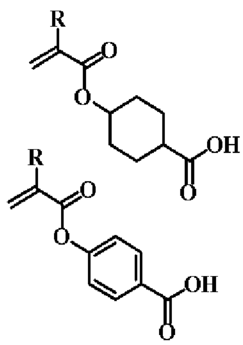

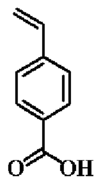
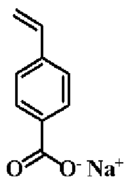
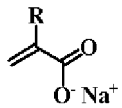
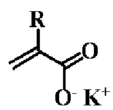

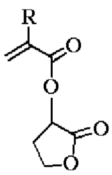
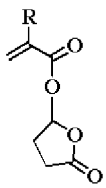
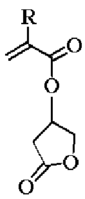
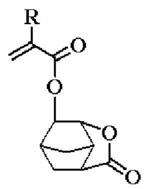
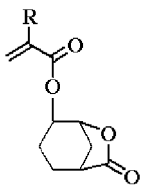

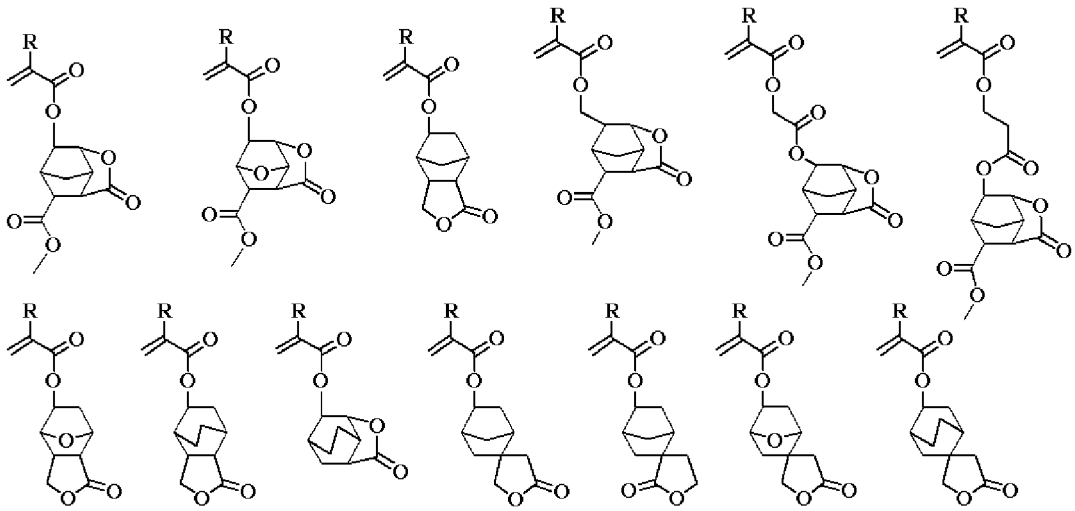

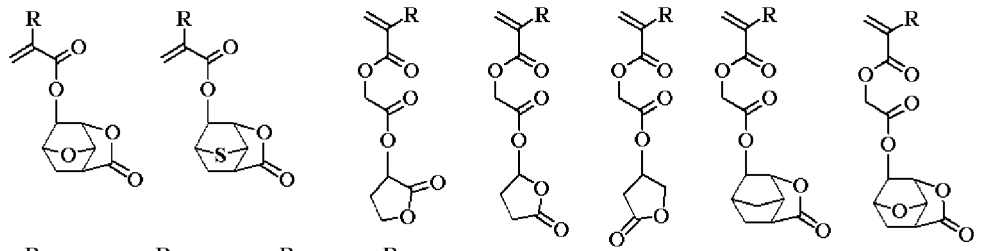

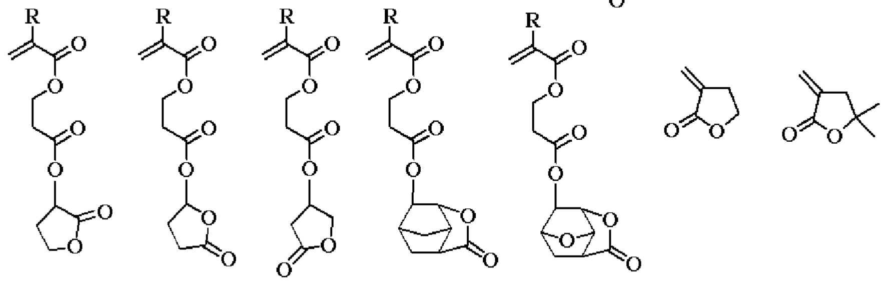

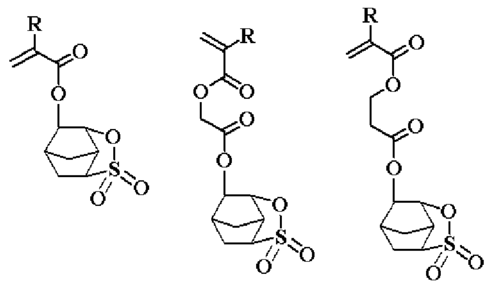

R represents a hydrogen atom or a methyl group.

Repeating Unit D

In the component (A) of the material for forming the conductive film used for the bio-electrode according to the present invention, a repeating unit D that adds adhesive power, in addition to the above-mentioned repeating units A1 to A7, B, and C. Specific examples of monomers for obtaining the repeating unit D can include the following.

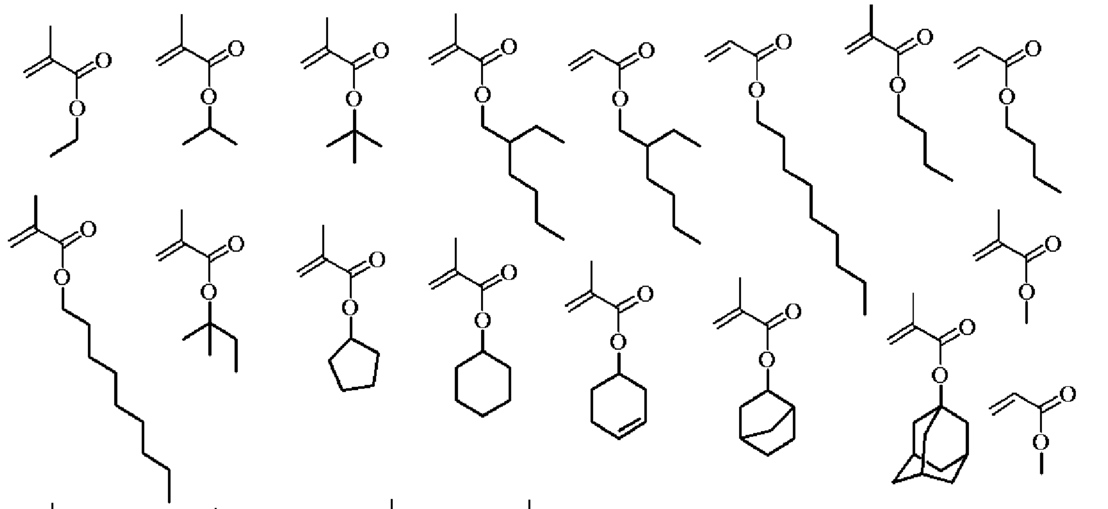

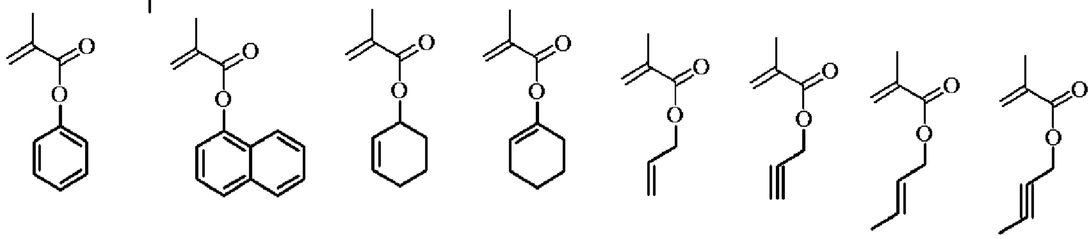

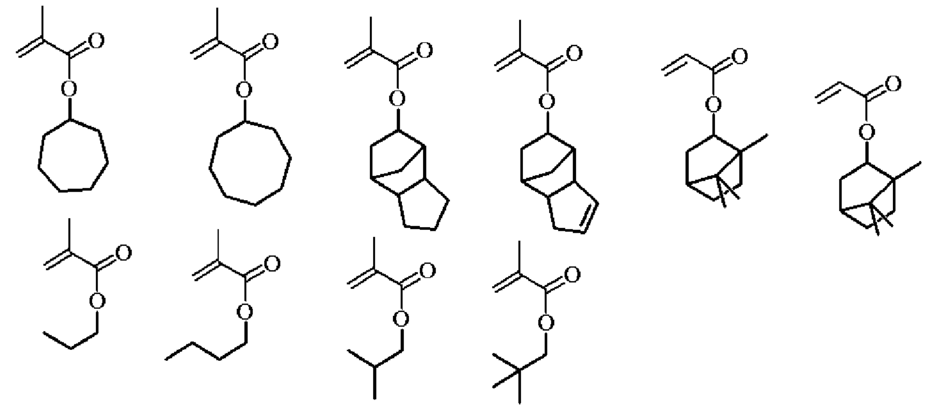

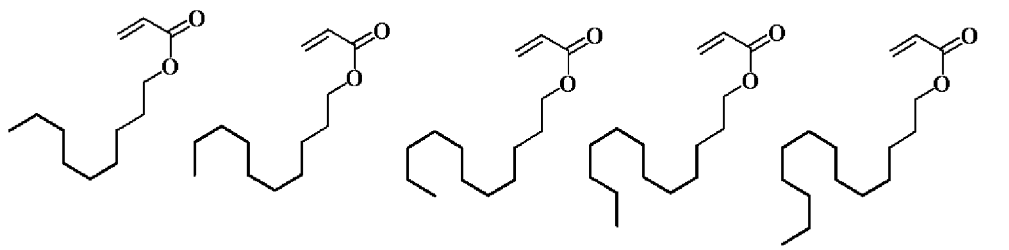

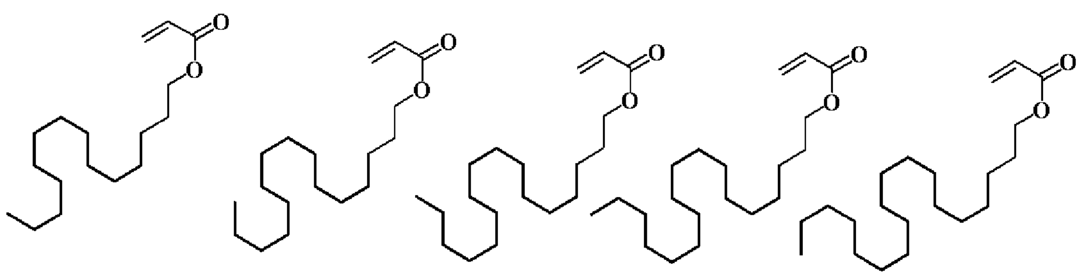

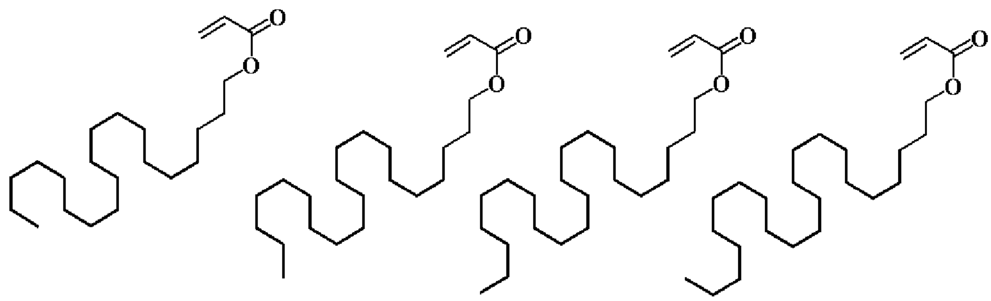

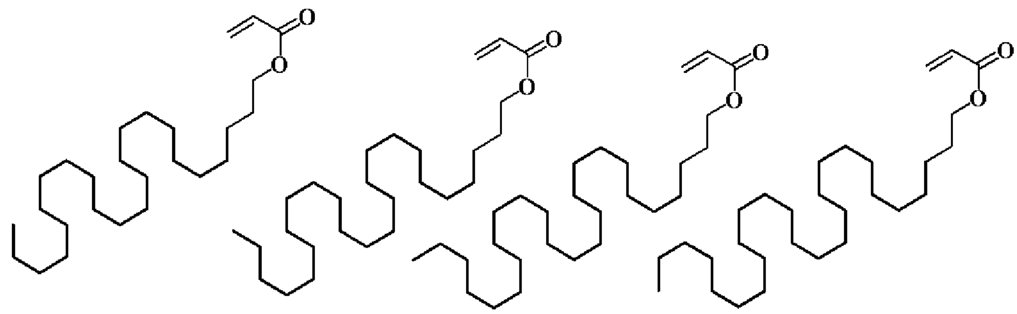

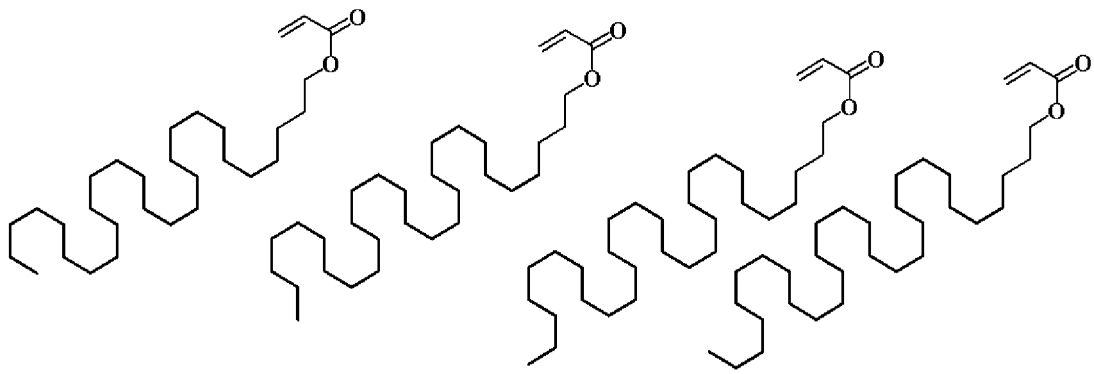

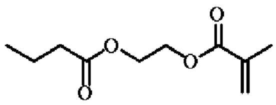
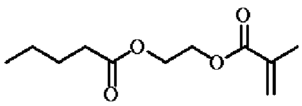
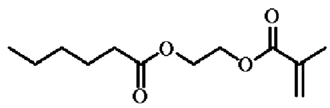

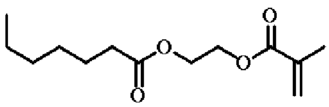
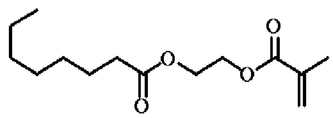
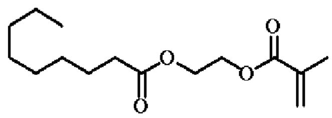

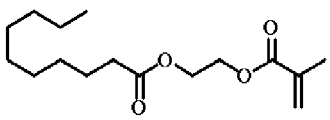
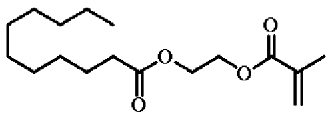
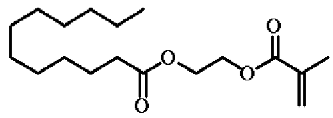

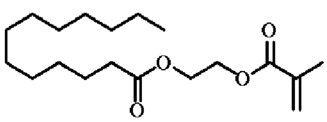
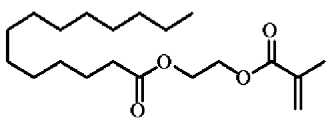
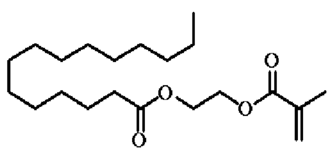

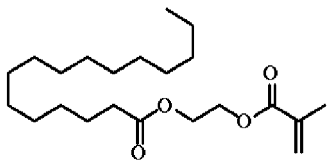
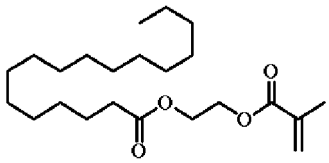
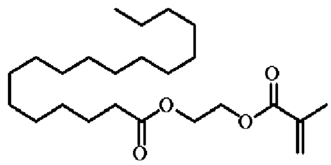

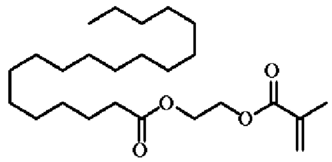
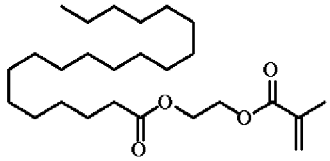
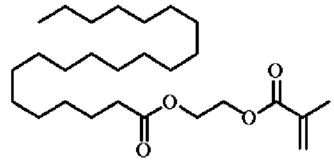

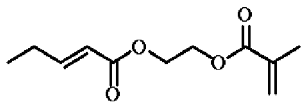
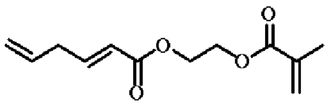
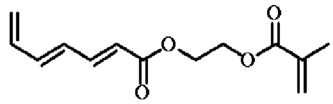

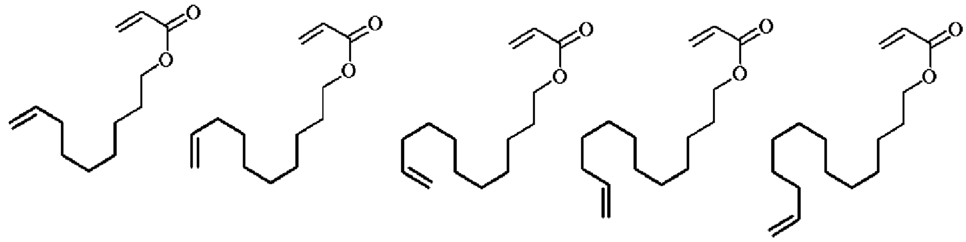

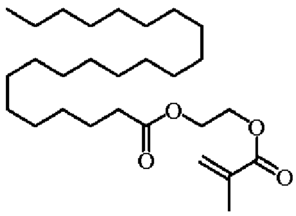
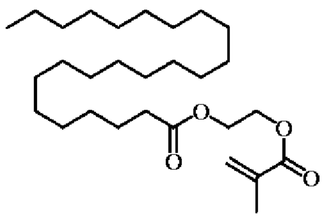
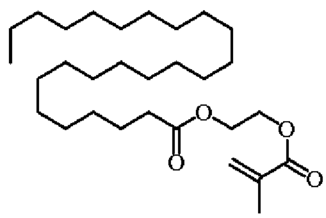

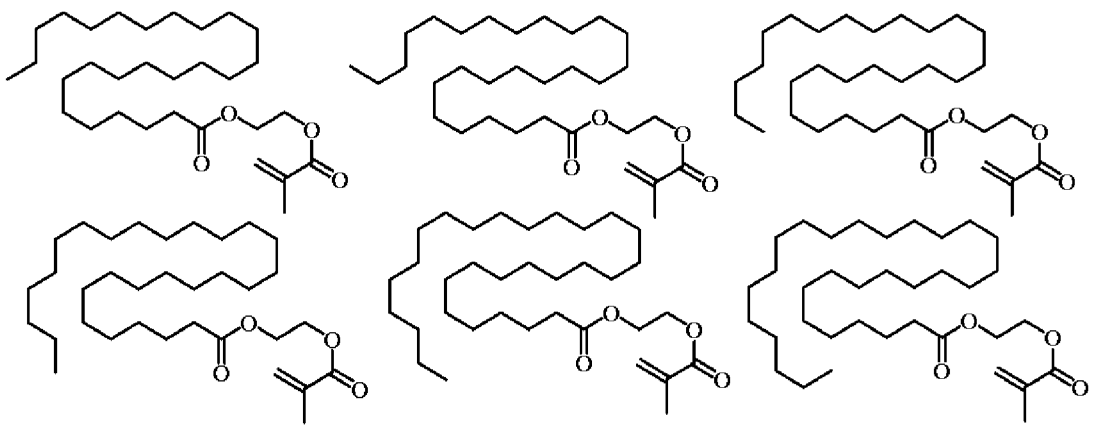

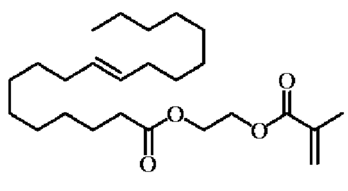
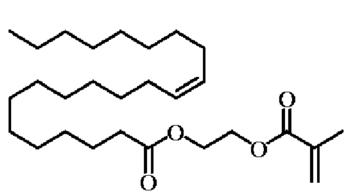
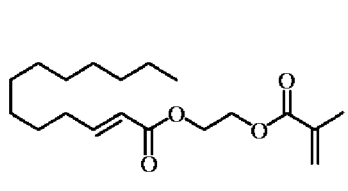

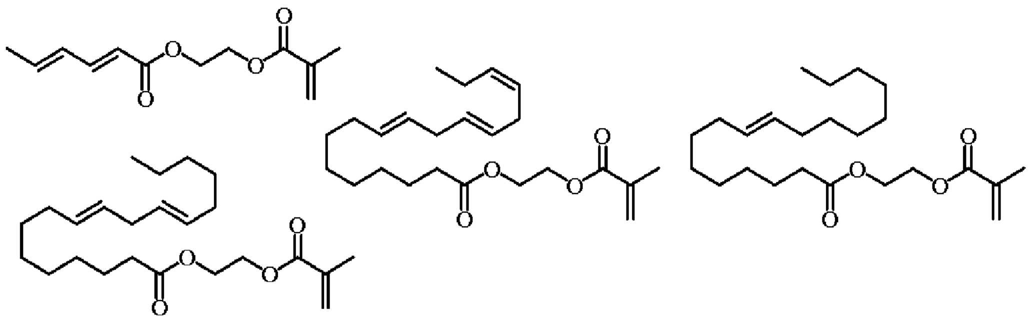

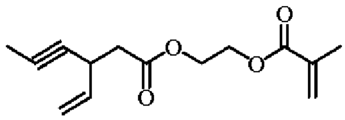 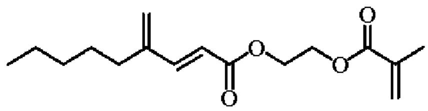

Repeating Unit E

In the component (A) of the material for forming the conductive film used for the bio-electrode according to the present invention, a cross-linking repeating unit E can be copolymerized in addition to the above-mentioned repeating units A1 to A7, B, C, and D. Examples of the cross-linking repeating unit E can include a repeating unit having an oxirane ring or an oxetane ring.

Specific examples of monomers for obtaining the repeating unit E having the oxirane ring or the oxetane ring can include the following.

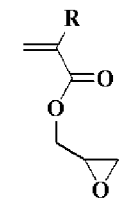 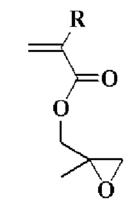 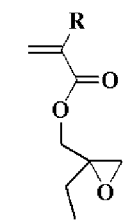 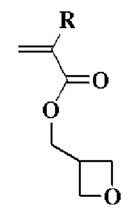 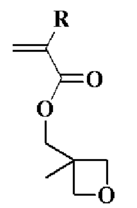 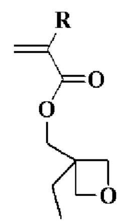

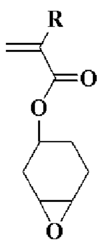 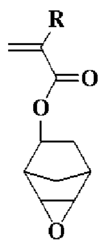 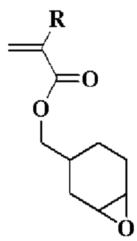 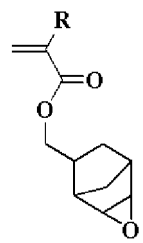 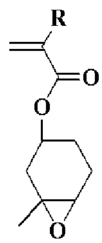 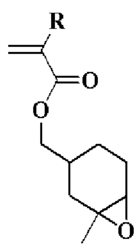

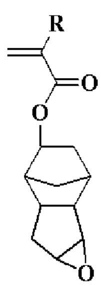 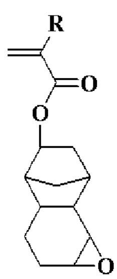 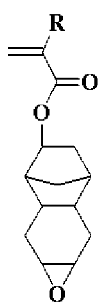 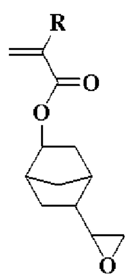 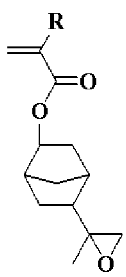 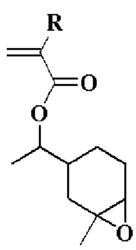

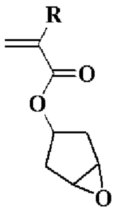 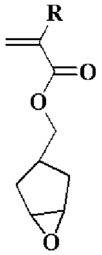 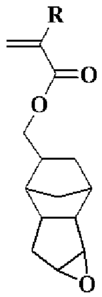 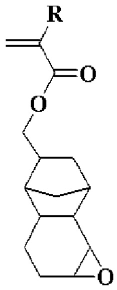 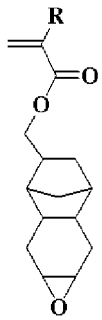 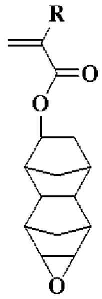 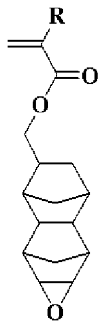

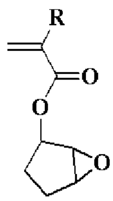 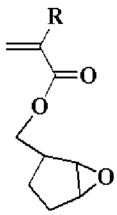 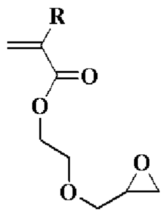 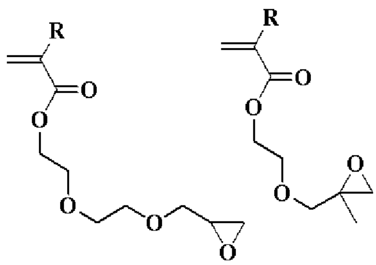 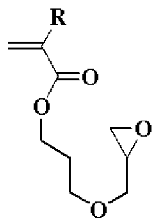

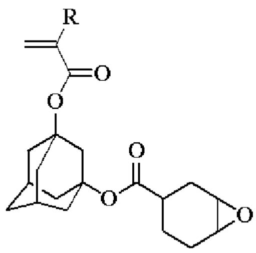 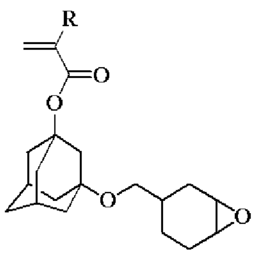 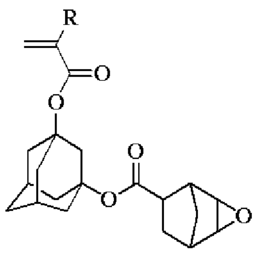 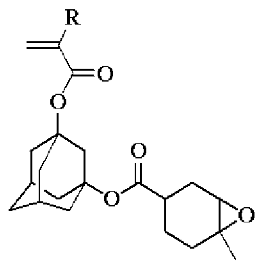

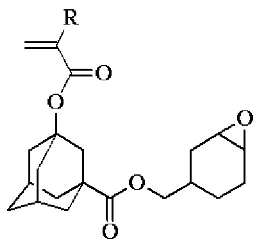 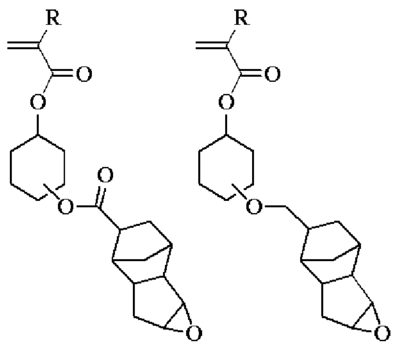 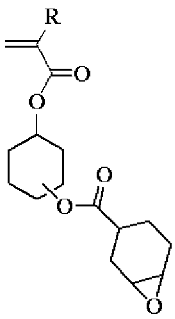 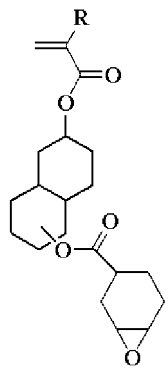

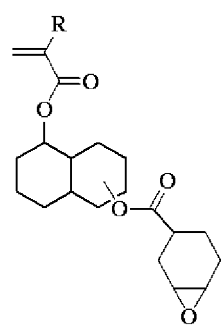 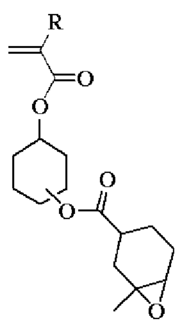 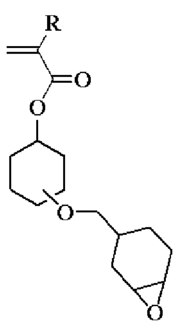 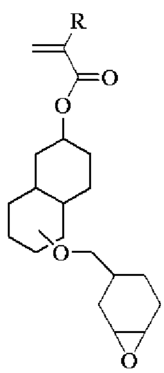 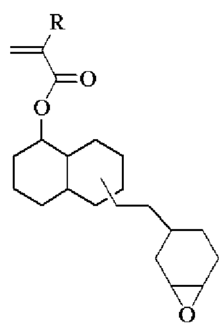

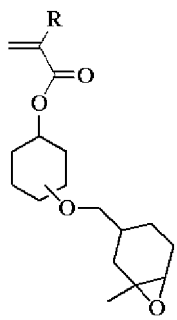 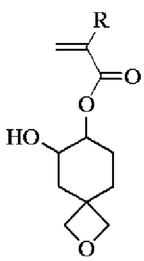 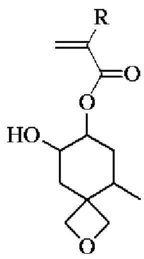 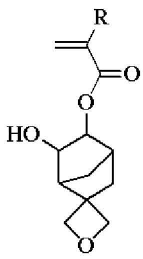 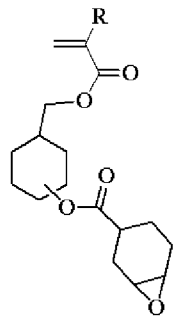

Herein, R represents a methyl group or a hydrogen atom.

Repeating Unit F

The component (A) of the material for forming the conductive film used for the bio-electrode according to the present invention can have a repeating unit F having silicon, in addition to the above-mentioned repeating units A1 to A7, B, C, D, and E. Specific examples of monomers for obtaining the repeating unit F can include the following.

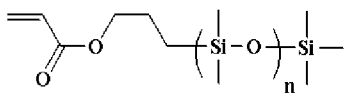 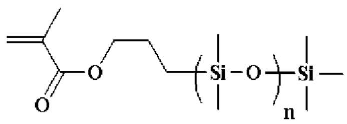 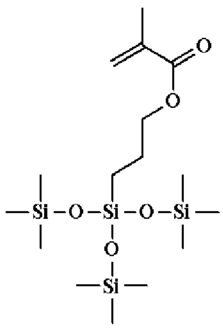 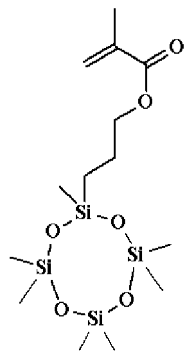

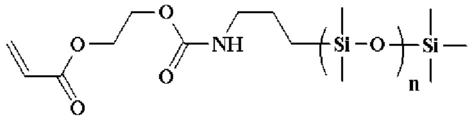

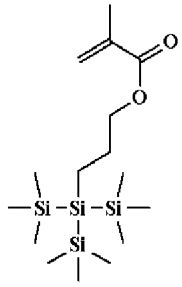

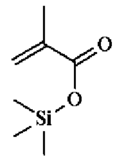

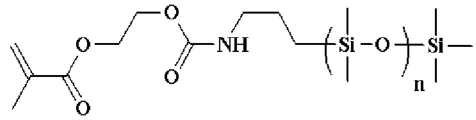

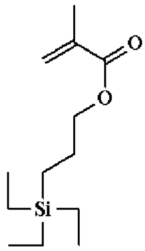

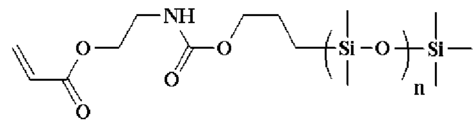

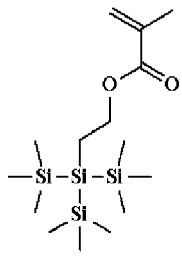

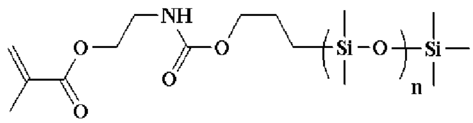

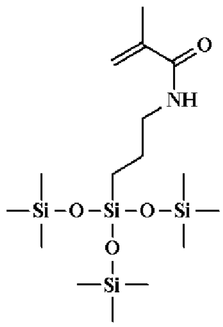

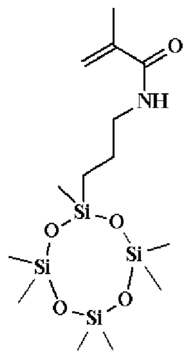

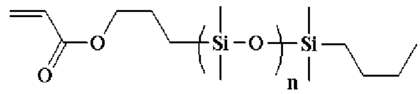

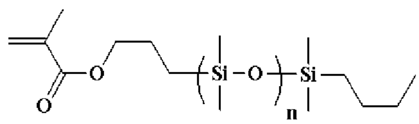

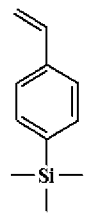

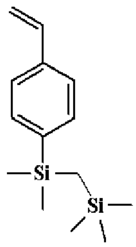

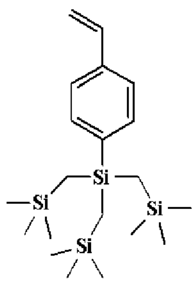

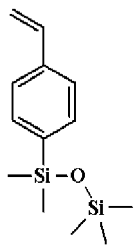

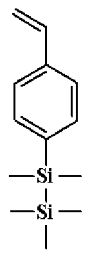

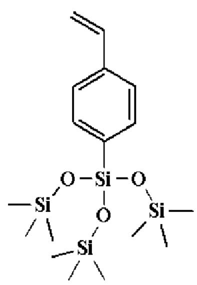

n represents an integer of 0 to 100.

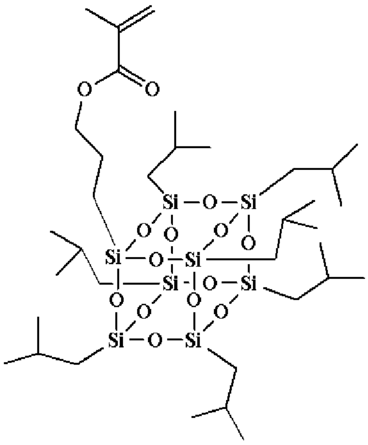 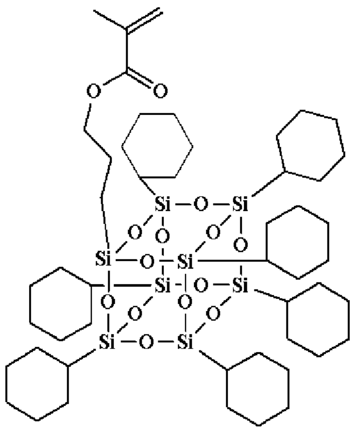 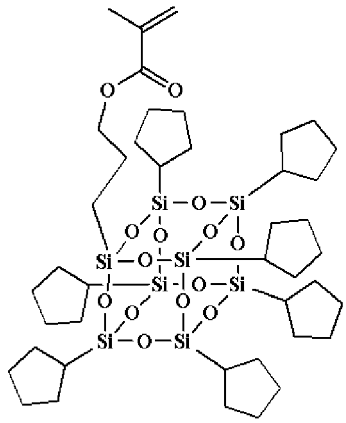

Repeating Unit G

The component (A) of the material for forming the conductive film used for the bio-electrode according to the present invention can have a repeating unit G having fluorine, in addition to the above-mentioned repeating units A1 to A7, B, C, D, E, and F.

Specific examples of monomers for obtaining the repeating unit G having fluorine can include the following.

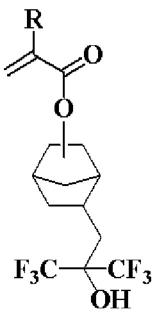 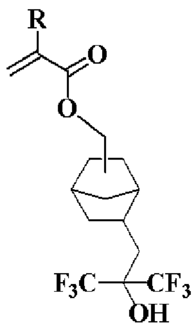 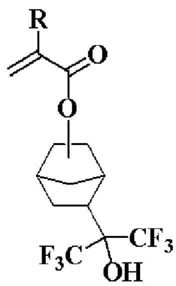 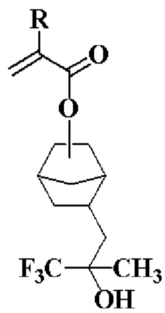 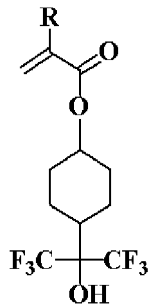 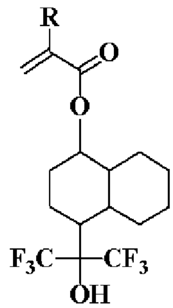

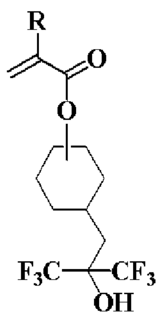 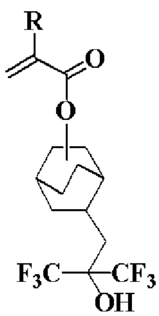 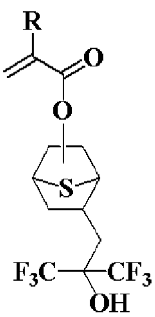 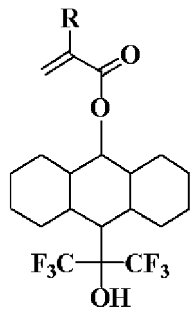

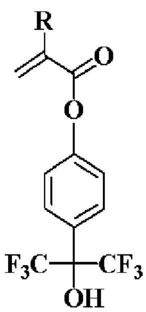 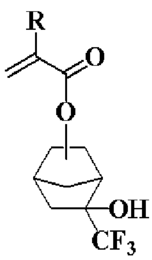 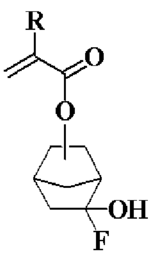 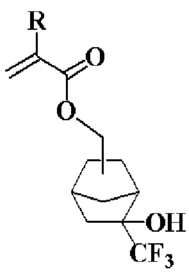

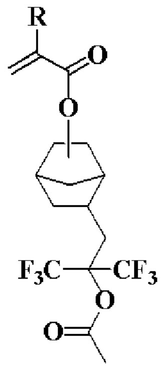
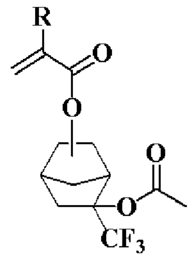
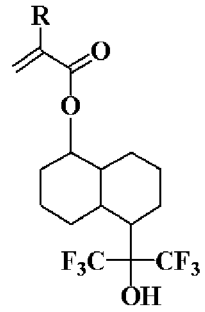
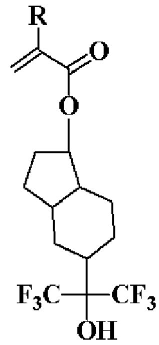

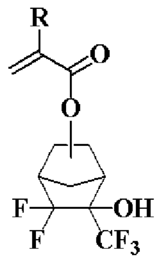
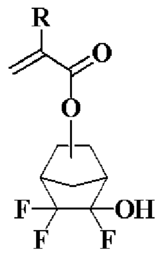
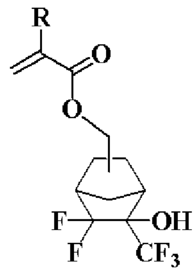
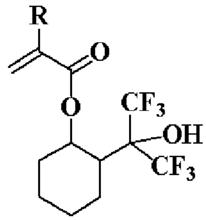

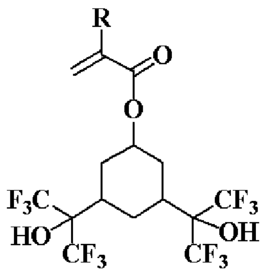
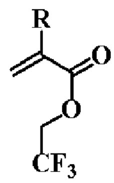
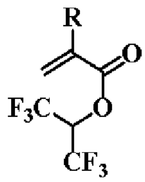
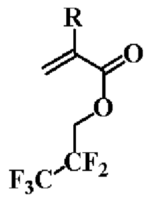
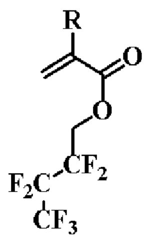

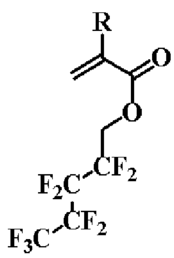
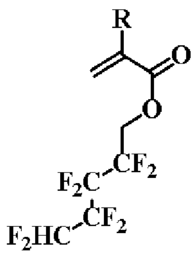
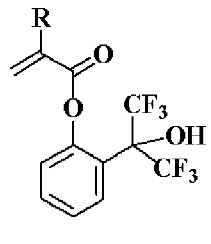
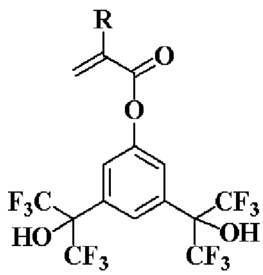

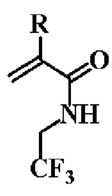
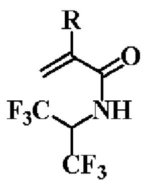
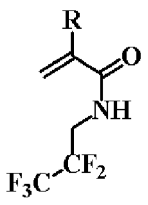
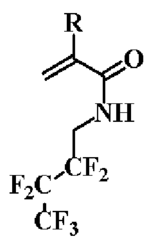
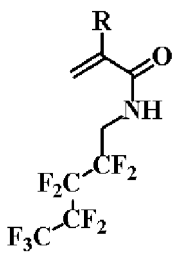
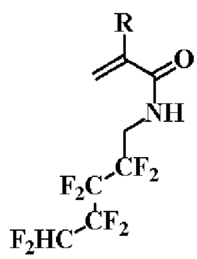

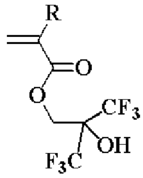 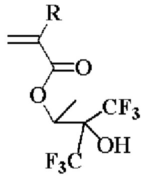 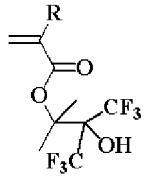 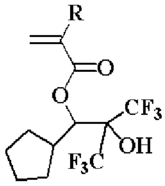 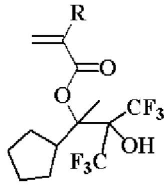 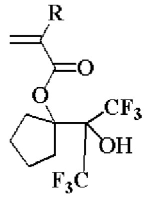

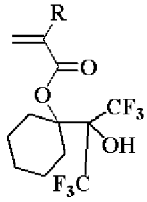 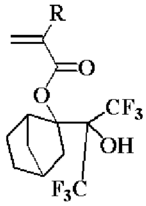 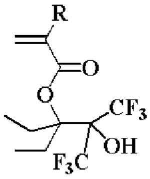 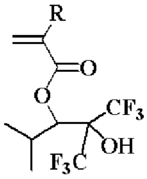

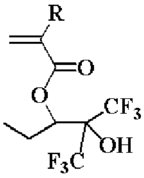 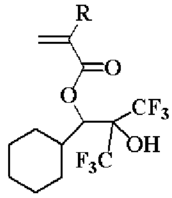 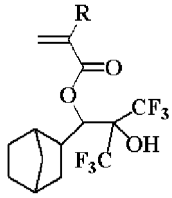 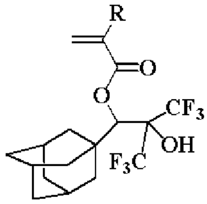 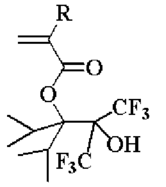

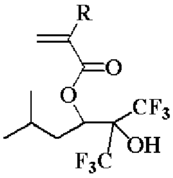 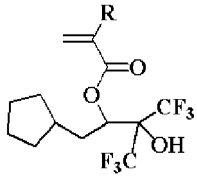 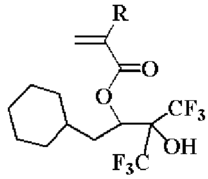 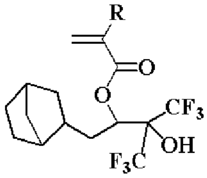 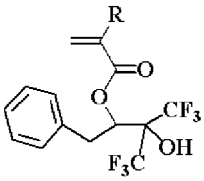

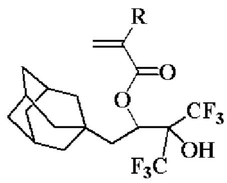 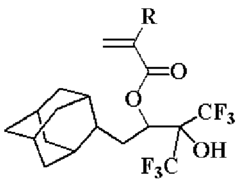 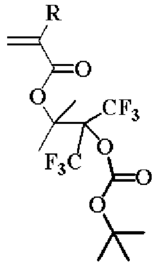 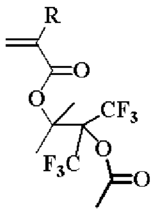

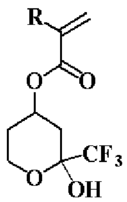 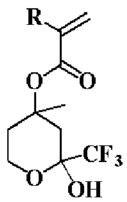 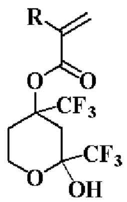 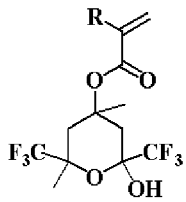 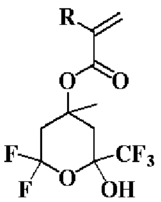 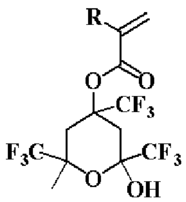

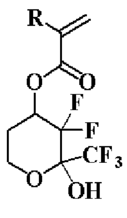 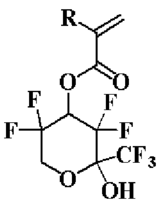 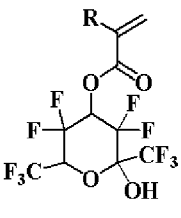 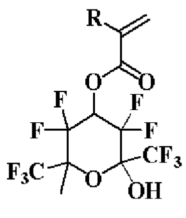 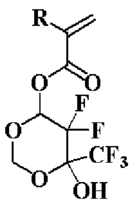 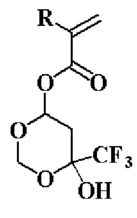

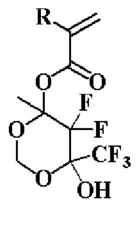 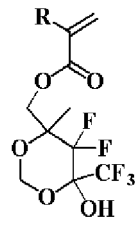 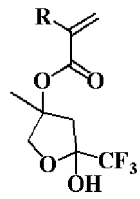 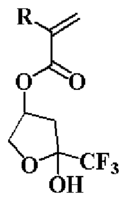 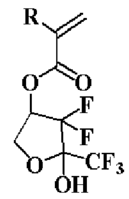 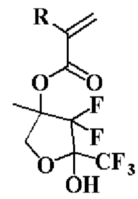 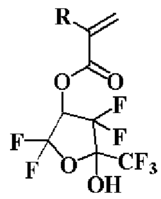

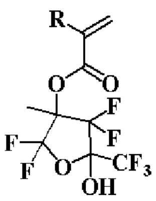 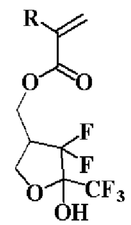 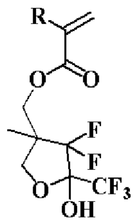 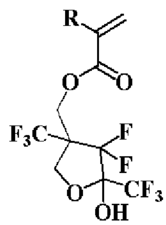 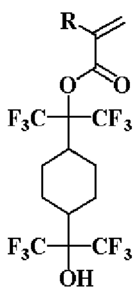 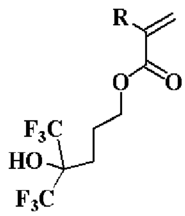

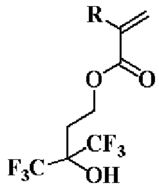 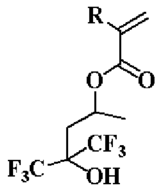 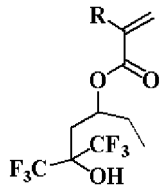 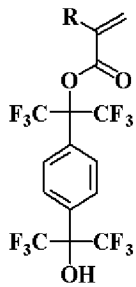 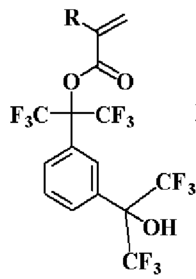 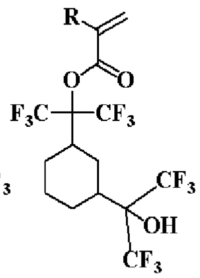

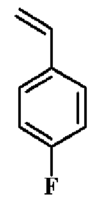 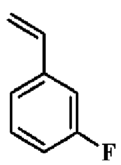 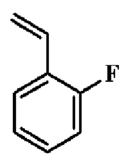 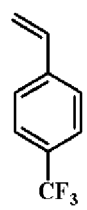 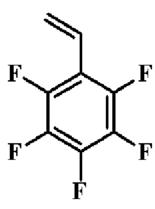 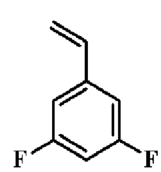

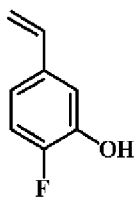 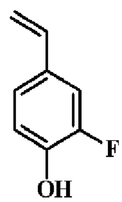 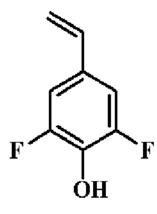 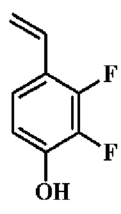 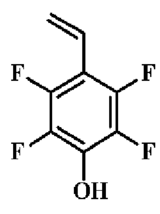

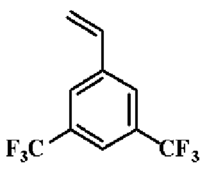 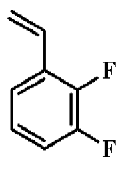 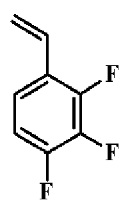 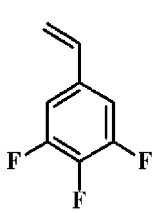

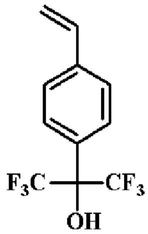 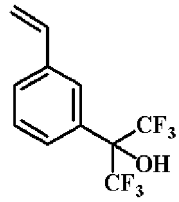 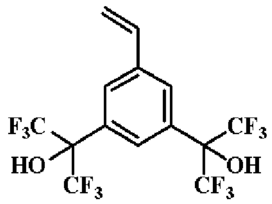

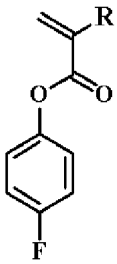 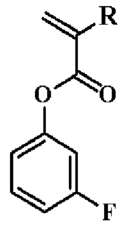 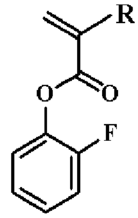 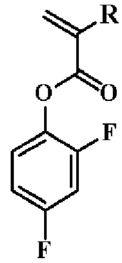 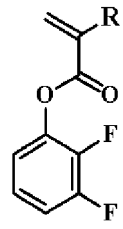 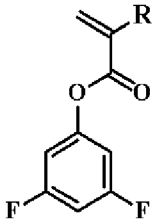 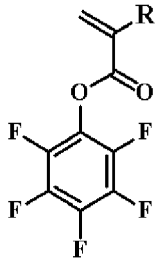

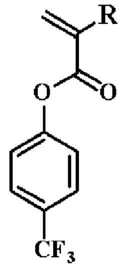 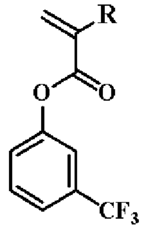 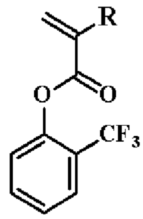 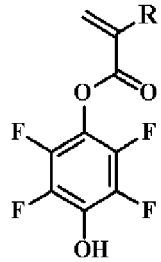 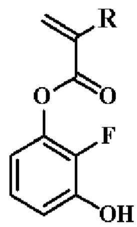

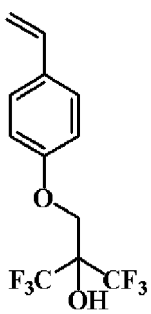 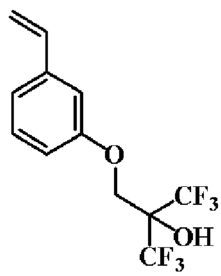 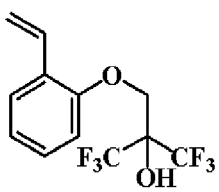

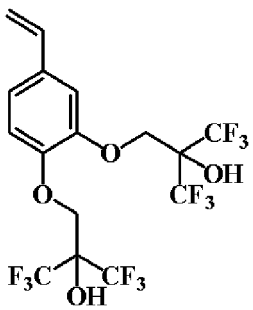 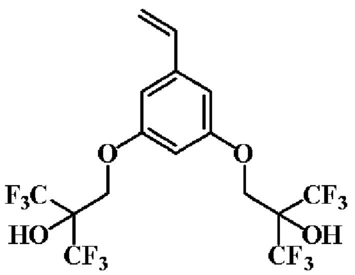

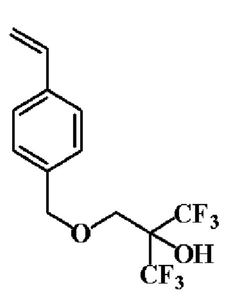
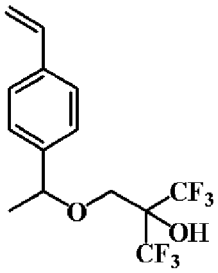
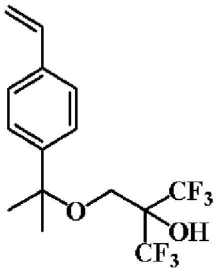

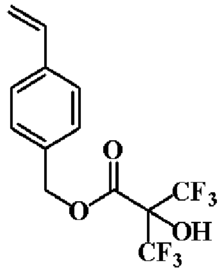
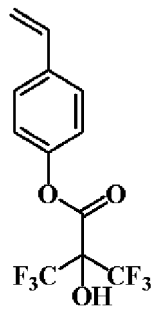

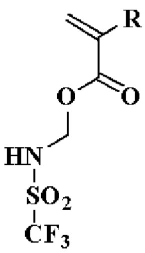
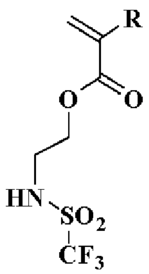
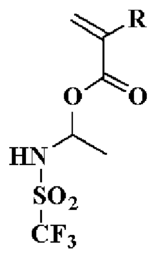
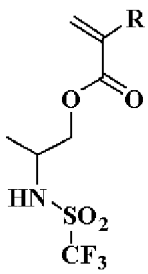
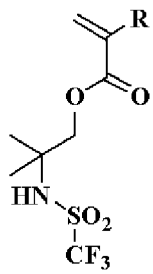
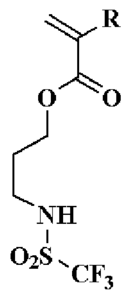

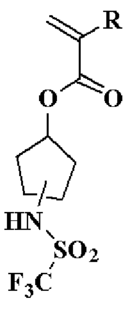
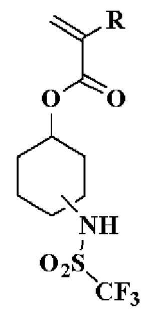
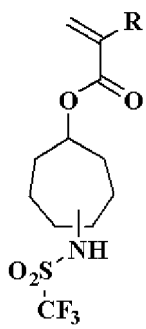
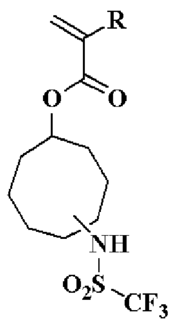

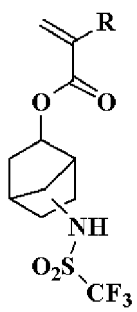
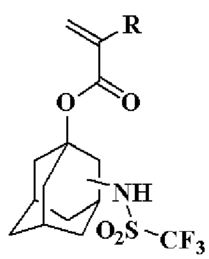
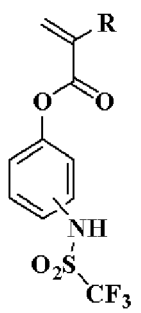
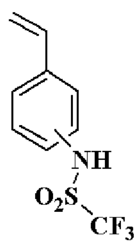

Herein, R represents a hydrogen atom or a methyl group.

Examples of a method of synthesizing the ionic material of the component (A) can include a method of adding a radical polymerization initiator to a desired monomer, among the monomers for providing the repeating units A1 to A7, B, C, D, E, F, and G, in an organic solvent and heat-polymerizing the desired monomer to obtain a polymer compound as a copolymer.

Examples of the organic solvent used at the time of polymerization can include toluene, benzene, tetrahydrofuran, diethyl ether, and dioxane. Examples of the polymerization initiator can include 2,2'-azobisisobutyronitrile (AIBN), 2,2'-azobis(2,4-dimethylvaleronitrile), dimethyl 2,2-azobis(2-methylpropionate), benzoyl peroxide, and lauroyl peroxide. A heating temperature is preferably 50 to 80°C, and reaction time is preferably 2 to 100 hours, more preferably 5 to 20 hours.

Ratios of the repeating units A1 to A7, B, C, D, E, F, and G in the ionic material (A) satisfy relations of $0 \le a1 < 1.0$, $0 \le a2 < 1.0$, $0 \le a3 < 1.0$, $0 \le a4 < 1.0$, $0 \le a5 < 1.0$, $0 \le a6 < 1.0$, $0 \le a7 < 1.0$, $0 < a1+a2+a3+a4+a5+a6+a7 \le 1.0$, $0 \le b < 1.0$, $0 \le c < 1.0$, $0 \le d < 1.0$, $0 \le e < 0.9$, $0 \le f < 0.9$, and $0 \le g < 0.9$, preferably satisfy relations of $0 \le a1 \le 0.9$, $0 \le a2 \le 0.9$, $0 \le a3 \le 0.9$, $0 \le a4 \le 0.9$, $0 \le a5 \le 0.9$, $0 \le a6 \le 0.9$, $0 \le a7 \le 0.9$, $0.01 \le a1+a2+a3+a4+a5+a6+a7 \le 0.9$, $0.03 \le b \le 0.9$, $0 \le c \le 0.8$, $0 \le d \le 0.8$, $0 \le e < 0.8$, $0 \le f < 0.8$, and $0 \le g < 0.8$, and more preferably satisfy relations of $0 \le a1 \le 0.8$, $0 \le a2 \le 0.8$, $0 \le a3 \le 0.8$, $0 \le a4 \le 0.8$, $0 \le a5 \le 0.8$, $0 \le a6 \le 0.8$, $0 \le a7 \le 0.8$, $0.02 \le a1+a2+a3+a4+a5+a6+a7 \le 0.8$, $0.05 \le b \le 0.9$, $0 \le c \le 0.7$, $0 \le d \le 0.5$, $0 \le e \le 0.3$, $0 \le f < 0.7$, and $0 \le g < 0.7$. b to g are ratios of the repeating units B to G, respectively.

Note that, for example, $a1+a2+a3+a4+a5+a6+a7+b+c+d+e+f+g=1$ represents that, in the polymer compound having the repeating units A1, A2, A3, A4, A5, A6, A7, B, C, D, E, F, and G, a sum of the repeating units A1, A2, A3, A4, A5, A6, A7, B, C, D, E, F, and G is 100 mol% with respect to a sum of the entire repeating units, and $a1+a2+a3+a4+a5+a6+a7+b+c+d+e+f+g<1$ represents that a sum of the repeating units A1, A2, A3, A4, A5, A6, A7, B, C, D, E, F, and G is less than 100 mol% with respect to the sum of the entire repeating units and the polymer compound has another repeating unit other than the repeating units A1, A2, A3, A4, A5, A6, A7, B, C, D, E, F, and G.

A molecular weight of the component (A), as a weight-average molecular weight of each monomer, is preferably 500 or more, more preferably 1,000 or more and 1,000,000 or less, more preferably 2,000 or more and 500,000 or less. In addition, when an amount of an ionic monomer (remaining monomer) that is not incorporated in the component (A) after polymerization is small, there is no possibility that the remaining monomer soaks into the skin and causes allergy in a biocompatibility test. Thus, the amount of the remaining monomer is preferably reduced. The amount of the remaining monomer is preferably 10 parts by mass or less relative to 100 parts by mass of the entire component (A). One kind of the component (A) may be used alone, or two or more kinds thereof having different molecular weights, different degrees of dispersion, and different polymerized monomers may be used in combination. The weight-average molecular weight (Mw) and degree of dispersion (Mw/Mn) of the polymer were checked by gel permeation chromatography (GPC) using tetrahydrofuran (THF) as a solvent.

Resin (B)

The resin (B) that is a component blended with the material for forming the conductive film used in the bio-electrode according to the present invention is compatibilized with the above-mentioned ionic material (salts) (A) and prevents elution of salts, that retains a conductivity improver such as a metal powder, a carbon powder, a silicon powder, and a lithium titanate powder, that contains silicone for imparting water repellency, and that manifests adhesiveness. In a case where the ionic material (A) contains silicone and has adhesiveness, the resin (B) is not necessarily required and may not contain silicone. Note that the resin is only required to be a resin other than the above-described component (A), and is preferably either or both of a thermosetting resin and a light curing resin, more preferably one or more selected from the group consisting of a silicone-based resin, an acrylic-based resin, and a urethane-based resin, and especially preferably one or more selected from the group consisting of the silicone-based resin, the acrylic-based resin containing silicone, and the urethane-based resin containing silicone. Among these resins, a silicone-based adhesive is the most preferable in terms of water repellency, allowing the skin to breathe, and hardly giving a feeling of discomfort when the bio-electrode is attached to the skin.

Examples of the adhesive, silicone-based resin can include an addition reaction curing type resin or a radical cross-linking reaction curing type resin. As the addition reaction curing type resin, for example, a resin that is described in JP 2015-193803 A, and that contains a diorganosiloxane having an alkenyl group, an MQ resin having an $R_3SiO_{0.5}$ unit and a $SiO_2$ unit, an organohydrogenpolysiloxane having a plurality of SiH groups, a platinum catalyst, an addition reaction control agent, and an organic solvent can be used. As the radical cross-linking reaction curing type resin, for example, a resin that is described in JP 2015-193803 A, and that contains a diorganosiloxane that may not have an alkenyl group, an MQ resin having an $R_3SiO_{0.5}$ unit and a $SiO_2$ unit, an organic peroxide, and an organic solvent can be used. Herein, R represents a substituted or unsubstituted monovalent hydrocarbon group having 1 to 10 carbon atoms.

Alternatively, a polysiloxane-resin integrated compound formed by a condensation reaction of an MQ resin and a polysiloxane having silanol at a polymer end or in a side chain can be used. Since the MQ resin contains a large amount of the silanol, addition of the MQ resin increases an adhesive force. However, the MQ resin does not have a

cross-linking property, and thus is not molecularly bonded to the polysiloxane. As described above, the integration of the polysiloxane and the resin can increase the adhesive force.

In addition, modified siloxane having a group selected from the group consisting of an amino group, an oxirane group, an oxetane group, a polyether group, a hydroxy group, a carboxyl group, a mercapto group, a methacryl group, an acrylic group, a phenolic group, a silanol group, a carboxylic acid anhydride group, an aryl group, an aralkyl group, an amide group, an ester group, and a lactone ring can also be added to the silicone-based resin. The addition of the modified siloxane increases dispersibility of the component (A) in the silicone resin. The modified siloxane may be modified siloxane that is modified at one end or both ends or in a side chain.

As the adhesive, acrylic-based resin, for example, a resin that is described in JP 2016-011338 A, and that has a hydrophilic (meth)acrylic acid ester or a long chain hydrophobic (meth)acrylic acid ester as repeating units can be used. In a case where the ion polymer does not contain silicone, a (meth)acrylic acid ester having a siloxane bond needs to be copolymerized to improve water repellency.

As the adhesive, urethane-based resin, for example, a reactant of a polyurethane-polyol compound which is described in JP 2000-256640 A and a polyfunctional isocyanate compound, and a resin that is described in JP 2019-076695 A and JP 2019-076696 A and that has a urethane bond, a polyether or polyester bond, a polycarbonate bond, and a siloxane bond in a side chain can be used.

To prevent a decrease of conductivity due to elution of the component (A) from the conductive film, in the composition of the conductive film of the bio-electrode according to the present invention, the resin (B) preferably has high compatibility with the above-described component (A). To prevent the conductive film from being peeled off the porous film or the conducting path, in the composition of the conductive film of the bio-electrode according to the present invention, the resin (B) preferably has adhesiveness with respect to the porous film and the conducting path. To increase compatibility of the resin (B) with the porous film, the conducting path, and the ion polymer, it is effective to use a resin with a high polarity or a resin with adhesiveness. Examples of such a resin can include an polyacrylic resin, a polyurethane resin, and an adhesive silicone resin, or the like that has one or more selected from the group consisting of an ether bond, an ester bond, an amide bond, an imide bond, a urethane bond, a thiourethane bond, and a thiol group. Meanwhile, the living body contact layer including the conductive film is in contact with the living body, and thus is susceptible to the influence of sweat from the living body. Hence, in the composition of the conductive film of the bio-electrode according to the present invention, the resin (B) preferably has high water repellency and is less susceptible to hydrolysis. To cause the resin to have high water repellency and be less susceptible to hydrolysis, it is effective to use the resin containing silicone.

In the composition of the conductive film of the bio-electrode according to the present invention, a blending amount of the component (B) is preferably 0 to 2000 parts by mass, more preferably 10 to 1000 parts by mass relative to 100 parts by mass of the ion polymer (A). One kind of the component (A) may be used alone, or two or more kinds thereof may be used in combination.

Note that the conductive film is a cured product of the composition of the conductive film for obtaining a bio-signal as described later. By curing the composition, the living body contact layer becomes to have preferable adhesiveness with respect to both the skin and the conductive base material. Note that a curing means is not specifically limited, and a general means can be used. For example, a cross-linking reaction using either or both of heat and light, acid, a base catalyst, or the like can be used.

In a case where a diorganosiloxane having an alkenyl group and an organohydrogenpolysiloxane having a plurality of SiH groups is used as the component (B), the diorganosiloxane and the organohydrogenpolysiloxane can be cross-linked by an addition reaction using a platinum catalyst.

Examples of the platinum catalyst include: a platinum-based catalyst such as chloroplatinic acid, an alcohol solution of chloroplatinic acid, a reactant of chloroplatinic acid and alcohol, a reactant of chloroplatinic acid and an olefin compound, a reactant of chloroplatinic acid and siloxane containing a vinyl group, and a platinum-olefin complex, and a platinum-siloxane containing a vinyl group complex; and a platinum-group metal-based catalyst such as a rhodium complex and a ruthenium complex. Alternatively, an alcohol-based solvent, a hydrocarbon-based solvent, a siloxane-based solvent in which any of these catalysts is dissolved and dispersed may be used.

Note that an additive amount of the platinum catalyst is preferably in a range from 5 to 2,000 ppm, especially from 10 to 500 ppm, relative to 100 parts by mass of the resin that combines (A) and (B).

In a case where an addition curing-type silicone resin is used, an addition reaction control agent may be added. This addition reaction control agent is added as a quencher for preventing the platinum catalyst from acting in a solution under a low-temperature environment after coating film formation and before heat-curing. Specific examples of the addition reaction control agent include 3-methyl-1-butyne-3-ol, 3-methyl-1-pentyne-3-ol, 3,5-dimethyl-1-hexyne-3-ol, 1-ethynylcyclohexanol, 3-methyl-3-trimethylsiloxy-1-butyne, 3-methyl-3-trimethylsiloxy-1-pentyne, 3,5-dimethyl-3-trimethylsiloxy-1-hexyne, 1-ethynyl-1-trimethylsiloxy-cyclohexane, bis(2,2-dimethyl-3-butynoxy)dimethylsilane, 1,3,5,7-tetramethyl-1,3,5,7-tetravinylcyclotetrasiloxane, and 1,1,3,3-tetramethyl-1,3-divinyldisiloxane.

An additive amount of the addition reaction control agent is preferably in a range from 0 to 10 parts by mass,

especially from 0.05 to 3 parts by mass relative to 100 parts by mass of the resin.

Examples of a photocuring method include a method of using a resin having a (meth)acrylate terminal or an olefin terminal, or adding a cross-linker having (meth)acrylate, olefin, or a thiol group at an end and also adding a photo-radical generator that generates a radical with light, and a method of using a resin or a cross-linker having an oxirane group, an oxetane group, and a vinyl ether group, and adding a photo-acid generator that generates acid with light.

Examples of the photo-radical generator can include acetophenone, 4,4'-dimethoxybenzyl, benzil, benzoin, benzophenone, 2-benzoylbenzoic acid, 4,4'-bis(dimethylamino)benzophenone, 4,4'-bis(diethylamino)benzophenone, benzoin methyl ether, benzoin ethyl ether, benzoin isopropyl ether, benzoin butyl ether, benzoin isobutyl ether, 4-benzoylbenzoic acid, 2,2'-bis(2-chlorophenyl)-4,4',5,5'-tetraphenyl-1,2'-biimidazole, 2-benzoylbenzoic acid methyl, 2-(1,3-benzodioxol-5-yl)-4,6-bis(trichloromethyl)-1,3,5-triazine, 2-benzyl-2-(dimethylamino)-4'-morpholinobutyrophenone, 4,4'-dichlorobenzophenone, 2,2-diethoxyacetophenone, 2,2-dimethoxy-2-phenylacetophenone, 2,4-diethylthioxanthen-9-one, diphenyl(2,4,6-trimethylbenzoyl)phosphine oxide(BAPO), 1,4-dibenzoylbenzene, 2-ethylanthraquinone, 1-hydroxycyclohexyl phenyl ketone, 2-hydroxy-2-methylpropiophenone, 2-hydroxy-4'-(2-hydroxyethoxy)-2-methylpropiophenone, 2-isonitrosopropiophenone, and 2-phenyl-2-(p-toluenesulfonyloxy)acetophenone.

The resin can also be cured by addition of a pyrolysis-type radical generator. Examples of the pyrolysis-type radical generator can include 2,2'-azobis(isobutyronitrile), 2,2'-azobis(2,4-dimethylvaleronitrile), 2,2'-azobis(2-methylbutyronitrile), 2,2'-azobis(methylpropionamidine)hydrochloric acid, 2,2'-azobis[2-(2-imidazolin-2-yl)propane]hydrochloric acid, 2,2'-azobis(4-methoxy-2,4-dimethylvaleronitrile), 2,2'-azobis(2-methylbutyronitrile), 2,2'-azobis(cyclohexane-1-carbonitrile), 1-[(1-cyano-1-methylethyl)azo]formamide, 2,2'-azobis[2-methyl-N-(2-hydroxyethyl)propionamide], 2,2'-azobis[N-(2-propenyl)-2-methylpropionamide], 2,2'-azobis(N-butyl-2-methylpropionamide), dimethyl-2,2'-azobis(isobutyrate), 4,4'-azobis(4-cyanopentanoic acid), dimethyl-2,2'-azobis(2-methylpropionate), benzoyl peroxide, tert-butyl hydroperoxide, cumene hydroperoxide, di-tert-butyl peroxide, di-tert-amyl peroxide, di-n-butyl peroxide, and dicumyl peroxide.

Examples of the photo-acid generator include a sulfonium salt-based acid generator, iodonium salt-based acid generator, sulfonyldiazomethane-based acid generator, N-sulfonyloxyimide-based acid generator, and an oxime-O-sulfonate-based acid generator. Specific examples of the photo-acid generator include a photo-acid generator described in paragraphs [0122] to [0142] of JP 2008-111103 A, and a photo-acid generator described in JP 2009-080474 A.

Note that an additive amount of the radical generator or the photo-acid generator is preferably in a range from 0.1 to 50 parts by mass with respect to 100 parts by mass of the resin.

Component (C)

The composition of the conductive film of the bio-electrode according to the present invention can further contain, as a component (C), one or more selected from the group consisting of a carbon powder, a metal powder, a silicon powder, and a lithium titanate powder. The carbon powder and the metal powder among the component (C) are added to increase electronic conductivity, and the silicon powder and the lithium titanate powder are added to increase ion reception sensitivity.

Metal Powder

To increase electronic conductivity, a metal powder selected from the group consisting of gold, silver, platinum, copper, tin, titanium, nickel, aluminum, tungsten, molybdenum, ruthenium, chrome, and indium can be added to the composition of the conductive film of the bio-electrode according to the present invention. Note that an additive amount of the metal powder is preferably in a range from 1 to 50 parts by mass relative to 100 parts by mass of the resin.

As a kind of the metal powder, gold, silver, or platinum is preferable in terms of conductivity, and silver, copper, tin, titanium, nickel, aluminum, tungsten, molybdenum, ruthenium, or chrome is preferable in terms of price. A precious metal is preferable in terms of bio-compatibility. From these viewpoints, silver is the most preferable on the whole.

Examples of the shape of the metal powder can include a spherical shape, a disk shape, a flake shape, and a needle shape, but a powder in the flake shape exhibits the highest conductivity when being added, and preferable. The metal powder preferably has such a flake shape with a relatively low density and a large specific surface area as that a size thereof is 100 um or less, a tap density is 5 $g/cm^3$ or less, and a specific surface area is 0.5 $m^2/g$ or more.

Carbon Material

As the conductivity improver, a carbon material can be added. Examples of the carbon material can include carbon black, black lead, carbon nanotube, and carbon fiber. The carbon nanotube may be either single-wall carbon nanotube or multiwall carbon nanotube, and a surface thereof may be modified by an organic group. Especially, the carbon material is preferably either or both of the carbon black and the carbon nanotube. An additive amount of the

carbon material is preferably in a range from 1 to 50 parts by mass relative to 100 parts by mass of the resin.

Silicon Powder

The silicon powder can be added to the composition of the conductive film of the bio-electrode according to the present invention to increase ion reception sensitivity. Examples of the silicon powder can include powders composed of silicon, silicon monoxide, and silicon carbide. A particle diameter of the powders is preferably 100 um or less, more preferably 1 um or less. Finer particles are capable of receiving more ions because a surface area thereof becomes larger, and constitute a bio-electrode with high sensitivity. An additive amount of the silicon powder is preferably in a range from 1 to 50 parts by mass relative to 100 parts by mass of the resin.

Lithium Titanate Power

The lithium titanate powder can be added to the composition of the conductive film of the bio-electrode according to the present invention to increase ion reception sensitivity. Examples of the lithium titanate power can include a powder shown by molecular formulae of $Li_2TiO_3$, $LiTiO_2$, and $Li_4Ti_5O_{12}$ having a spinel structure, and a spinel structure product is preferable. Alternatively, a lithium titanate particle complexed with carbon can be used. A particle diameter of the powders is preferably 100 um or less, more preferably 1 um or less. Finer particles are capable of receiving more ions because a surface area thereof becomes larger, and constitute a bio-electrode with a high sensitivity. These lithium titanate powers may be a powder complexed with carbon. An additive amount of the lithium titanate power is preferably in a range from 1 to 50 parts by mass relative to 100 parts by mass of the resin.

Optional Component

The composition of the conductive film of the bio-electrode according to the present invention can contain an optional component such as a tackifier, a cross-linker, a cross-linking catalyst, an ionic additive, and an organic solvent.

Tackifier

The tackifier may be added to the composition of the conductive film of the bio-electrode according to the present invention to add adhesiveness with respect to the living body. Examples of the tackifier can include a silicone resin, non-cross-linked siloxane, non-cross-linked poly(meth)acrylate, and non-cross-linked polyether.

Cross-linker

An epoxy-based cross-linker can be added to the composition of the conductive film of the bio-electrode according to the present invention. The cross-linker in this case is a compound having a plurality of epoxy groups or oxetane groups within one molecule. An additive amount of the cross-linker is 1 to 30 parts by mass relative to the 100 parts by mass of the resin.

Cross-linking Catalyst

A catalyst for cross-linking the epoxy groups or the oxetane groups can be added to the composition of the conductive film of the bio-electrode according to the present invention. As the catalyst in this case, a catalyst described in paragraphs [0027] to [0029] of JP 2019-503406 A can be used. An additive amount of the catalyst is 0.01 to 10 parts by mass relative to 100 parts by mass of the resin.

Ionic Additive

An ionic additive for increasing ion conductivity can be added to the composition of the conductive film of the bio-electrode according to the present invention. In consideration of bio-compatibility, examples of the ionic additive can include sodium chloride, potassium chloride, calcium chloride, saccharin, acesulfame potassium, and salts described in JP 2018-044147 A, JP 2018-059050 A, JP 2018-059052 A, and JP 2018-130534 A.

The composition of the conductive film of the bio-electrode according to the present invention can contain a silicone compound having a polyglycerol group shown by the following general formula (4). A blending amount of the silicone compound having the polyglycerol group is preferably 0.01 to 100 parts by mass, more preferably 0.5 to 60 parts by mass relative to 100 parts by mass of the component (A). One kind of the silicone compound having the polyglycerol group may be used alone, or two or more kinds thereof may be used in combination.

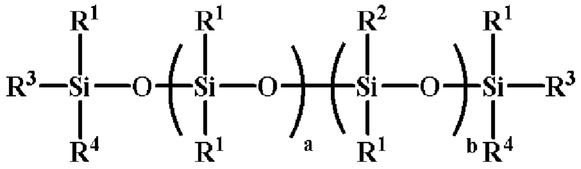

(4)

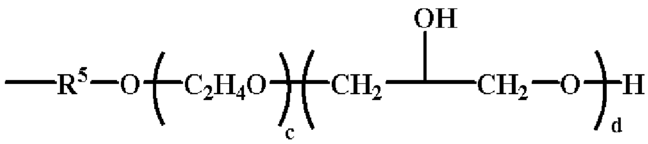

(4)-1

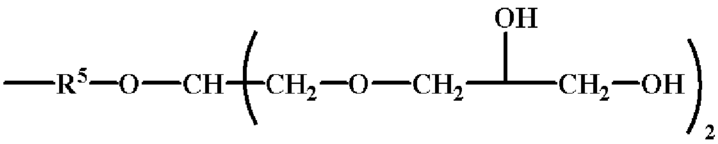

(4)-2

(In the formula, $R^1$ represents identical or non-identical a linear or branched alkyl group having 1 to 10 carbon atoms or a phenyl group, $R^2$ represents a group having a polyglycerol group structure shown by the formula (4)-1 or (4)-2, $R^3$ and $R^4$ are identical or non-identical $R^1$ or $R^2$. $R^4$ and $R^4$ may be coupled to each other to be an ether group and form a ring. "a" represents 0 to 6, "b" represents 0 to 4, and "a + b" represents 0 to 10. Note that at least one $R^3$ represents $R^2$ when b is 0. $R^5$ represents an alkylene group having 2 to 10 carbon atoms or an aralkylene group having 7 to 10 carbon atoms, "c" represents 0 to 10, and "d" represents 2 to 6.)

Examples of the silicone compound having the polyglycerol group can include the following.

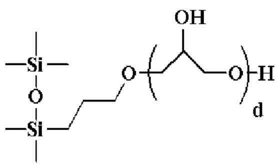

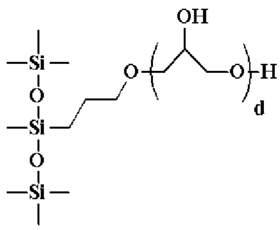

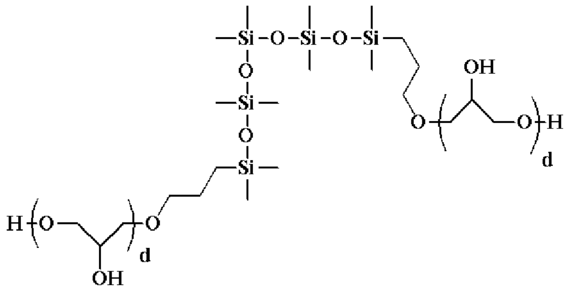

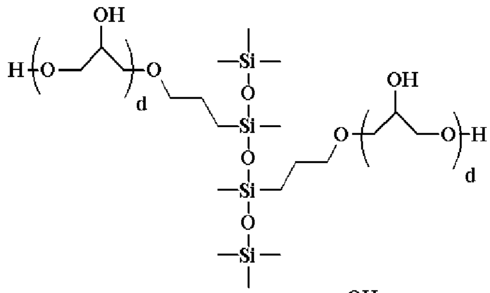

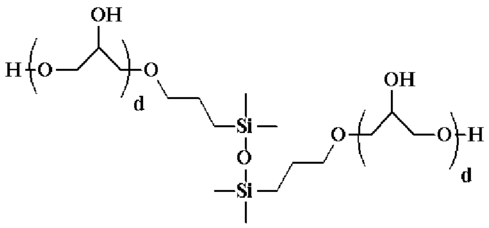

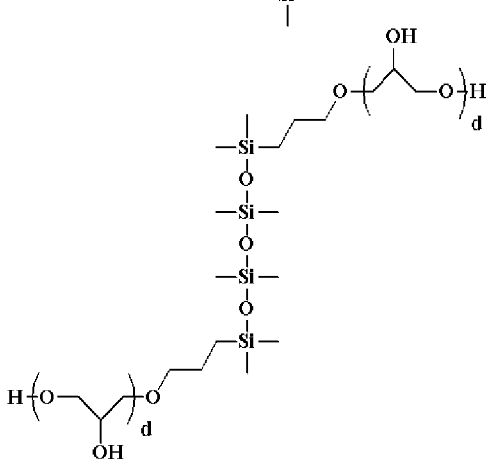

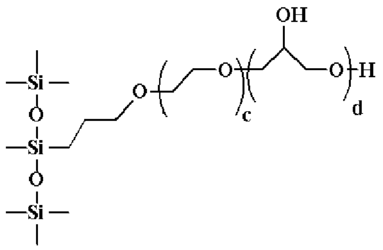

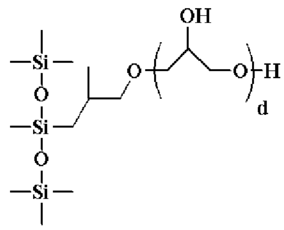

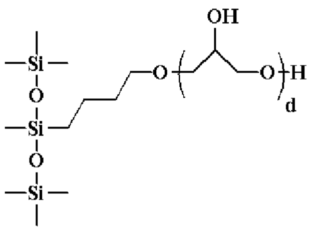

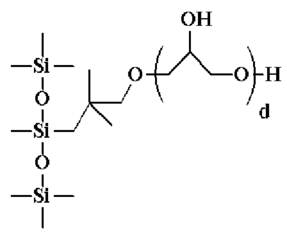

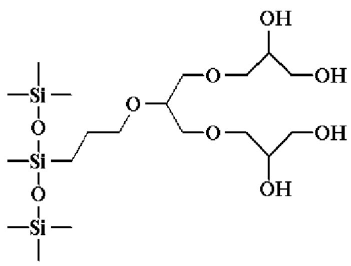

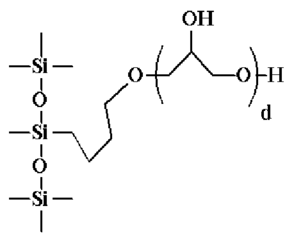

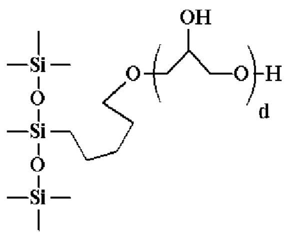

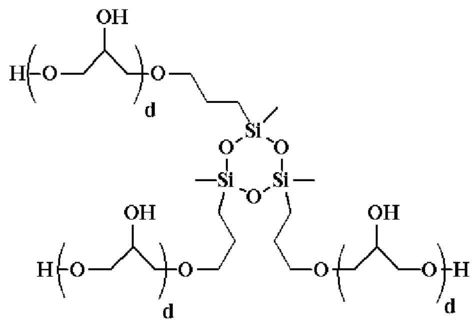

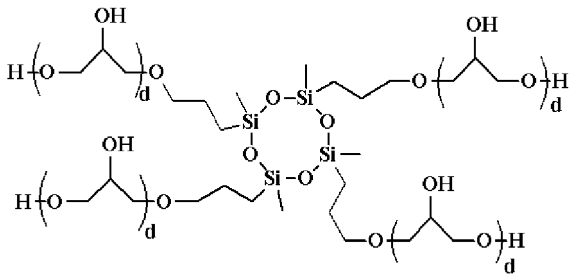

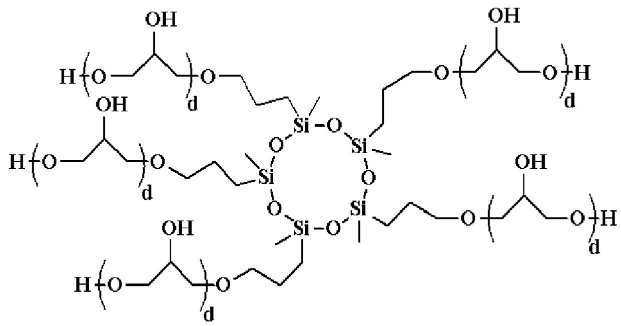

Organic Solvent

The organic solvent can be added to the composition of the conductive film of the bio-electrode according to the present invention. Specific examples of the organic solvent can include an aromatic hydrocarbon-based solvent such as toluene, xylene, cumene, 1,2,3-trimethylbenzene, 1,2,4-trimethylbenzene, 1,3,5-trimethylbenzene, styrene, alpha-methyl styrene, butylbenzene, sec-butylbenzene, isobutylbenezene, cymene, diethylbenzene, 2-ethyl-p-xylene, 2-propyltoluene, 3-propyltoluene, 4-propyltoluene, 1,2,3,5-tetramethyltoluene, 1,2,4,5-tetramethyltoluene, tetrahydronaphthalene, 4-phenyl-1-butene, tert-amylbenzene, amylbenzene, 2-tert-butyltoluene, 3-tert-butyltoluene, 4-tert-butyltoluene, 5-isopropyl-m-xylene, 3-methylethylbenzene, tert-butyl-3-ethylbenzene, 4-tert-butyl-o-xylene, 5-tert-butyl-m-xylene, tert-butyl-p-xylene, 1,2-diisopropylbenzene, 1,3-diisopropylbenzene, 1,4-diisopropylbenzene, dipropylbenzene, pentamethylbenzene, hexamethylbenzene, hexylbenzene, and 1,3,5-triethylbenzene; an aliphatic hydrocarbon-based solvent such as n-heptane, isoheptane, 3-methylhexane, 2,3-dimethylpentane, 3-ethylpentane, 1,6-heptadiene, 5-methyl-1-hexyne, norbornane, norbornene, dicyclopentadiene, 1-methyl-1,4-cyclohexadiene, 1-heptyne, 2-heptyne, cycloheptane, cycloheptene, 1,3-dimethylcyclopentane, ethylcyclopentane, methylcyclohexane, 1-methyl-1-cyclohexene, 3-methyl-1-cyclohexene, methylenecyclohexane, 4-methyl-1-cyclohexene, 2-methyl-1-hexene, 2-methyl-2-hexene, 1-heptene, 2-heptene, 3-heptene, n-octane, 2,2-dimethylhexane, 2,3-dimethylhexane, 2,4-dimethylhexane, 2,5-dimethylhexane, 3,3-dimethylhexane, 3,4-dimethylhexane, 3-ethyl-2-methylpentane, 3-ethyl-3-methylpentane, 2-methylheptane, 3-methylheptane, 4-methylheptane, 2,2,3-trimethylpentane, 2,2,4-trimethylpentane, cyclooctane, cyclooctene, 1,2-dimethylcyclohexane, 1,3-dimethylcyclohexane, 1,4-dimethylcyclohexane, ethylcyclohexane, vinylcyclohexane, isopropylcyclopentane, 2,2-dimethyl-3-hexene, 2,4-dimethyl-1-hexene, 2,5-dimethyl-1-hexene, 2,5-dimethyl-2-hexene, 3,3-dimethyl-1-hexene, 3,4-dimethyl-1-hexene, 4,4-dimethyl-1-hexene, 2-ethyl-1-hexene, 2-methyl-1-heptene, 1-octene, 2-octene, 3-octene, 4-octene, 1,7-octadiene, 1-octyne, 2-octyne, 3-octyne, 4-octyne, n-nonane, 2,3-dimethylheptane, 2,4-dimethylheptane, 2,5-dimethylheptane, 3,3-dimethylheptane, 3,4-dimethylheptane, 3,5-dimethylheptane, 4-ethylheptane, 2-methyloctane, 3-methyloctane, 4-methyloctane, 2,2,4,4-tetramethylpentane, 2,2,4-trimethylhexane, 2,2,5-trimethylhexane, 2,2-dimethyl-3-heptene, 2,3-dimethyl-3-heptene, 2,4-dimethyl-1-heptene, 2,6-dimethyl-1-heptene, 2,6-dimethyl-3-heptene, 3,5-dimethyl-3-heptene, 2,4,4-trimethyl-1-hexene, 3,5,5-trimethyl-1-hexene, 1-ethyl-2-methylcyclohexane, 1-ethyl-3-methylcyclohexane, 1-ethyl-4-methylcyclohexane, propylcyclohexane, isopropylcyclohexane, 1,1,3-trimethylcyclohexane, 1,1,4-trimethylcyclohexane, 1,2,3-trimethylcyclohexane, 1,2,4-trimethylcyclohexane, 1,3,5-trimethylcyclohexane, allylcyclohexane, hydrindane, 1,8-nonadiene, 1-nonine, 2-nonine, 3-nonine, 4-nonine, 1-nonene, 2-nonene, 3-nonene, 4-nonene, n-decane, 3,3-dimethyloctane, 3,5-dimethyloctane, 4,4-dimethyloctane, 3-ethyl-3-methylheptane, 2-methylnonane, 3-methylnonane, 4-methylnonane, tert-butylcyclohexane, butylcyclohexane, isobutylcyclohexane, 4-isopropyl-1-methylcyclohexane, pentylcyclopentane, 1,1,3,5-tetramethylcyclohexane, cyclododecane, 1-decene, 2-decene, 3-decene, 4-decene, 5-decene, 1,9-decadiene, decahydronaphthalene, 1-decyne, 2-decyne, 3-decyne, 4-decyne, 5-decyne, 1,5,9-decatriene, 2,6-dimethyl-2,4,6-octatriene, limonene, myrcene, 1,2,3,4,5-pentamethylcyclopentadiene, alpha-phellandrene, pinene, terpinene, tetrahydrodicyclopentadiene, 5,6-dihydrodicyclopentadiene, dicyclopentadiene, 1,4-decadiyne, 1,5-decadiyne, 1,9-decadiyne, 2,8-decadiyne, 4,6-decadiyne, n-undecane, amylcyclohexane, 1-undecene, 1,10-undecadiene, 1-undecyne, 3-undecyne, 5-undecyne, tricyclo[6.2.1.0$^{2,7}$]undeca-4-ene, n-dodecane, n-tridecane, 2-methylundecane, 3-methylundecane, 4-methylundecane, 5-methylundecane, 2,2,4,6,6-pentamethylheptane, 1,3-dimethyladamantane, 1-ethyladamantane, 1,5,9-cyclododecatriene, 1,2,4-trivinylcyclohexane, and isoparaffin; a ketone-based solvent such as cyclohexanone, cyclopentanone, 2-octanone, 2-nonanone, 2-heptanone, 3-heptanone, 4-heptanone, 2-hexanon, 3-hexanon, diisobutyl ketone, methylcyclohexanone, and methyl n-pentyl ketone; an alcohol-based solvent such as 3-methoxybutanol, 3-methoxy-3-methylbutanol, 1-methoxy-2-propanol, and 1-ethoxy-2-propanol; an ether-based solvent such as propylene glycol monomethyl ether, ethylene glycol monomethyl ether, propylene glycol monoethyl ether, ethylene glycol monoethyl ether, propylene glycol dimethyl ether, diethylene glycol monomethyl ether, diethylene glycol monoethyl ether, diethylene glycol monopropyl ether, diethylene glycol monobutyl ether, diethylene glycol monopentyl ether, diethylene glycol monoheptyl ether, diethylene glycol diethyl ether, diethylene glycol dipropyl ether, diethylene glycol dibutyl ether, diisopropylether, diisobutyl ether, diisopentyl ether, di-n-pentylether, methyl cyclopentyl ether, methyl cyclohexyl ether, di-n-butyl ether, di-sec-butyl ether, diisopentyl ether, di-sec-pentyl ether, di-tert-amyl ether, di-n-hexyl ether, and anisole; an ester-based solvent such as propylene glycol monomethyl ether acetate, propylene glycol monoethyl ether acetate, ethyl lactate, ethyl pyruvate, butyl acetate, 3-methoxypropionic acid methyl ester, 3-ethoxypropionic acid ethyl ester, tert-butyl acetate, tert-butyl propionate, and propylene glycol mono-tert-butyl ether acetate; a lactone-based solvent such as gamma-butyrolactone; and water.

Note that an additive amount of the organic solvent is preferably in a range from 10 to 50,000 parts by mass relative to 100 parts by mass of the resin.

Other Additives

Silica particles can be mixed with the composition of the conductive film of the bio-electrode according to the present invention. The silica particles have hydrophilic surfaces, are well compatible with a hydrophilic ion polymer and polyglycerol silicone, and are capable of enhancing dispersibility of the ion polymer and the polyglycerol silicone with respect to a hydrophobic silicone adhesive. Either dry silica particles or wet silica particles can be preferably used. Silica particles modified by fluorosulfonamide salts or fluorosulfonimide salts can also be added.

The composition of the conductive film described above is mixed, and filtered as necessary, and a solution of the conductive film is thereby produced.

Bio-electrode

The bio-electrode according to the present invention will be described in detail below with reference to the drawings, but the present invention is not limited thereto.

FIG. 1 shows a cross section of the bio-electrode 10 according to the present invention. The bio-electrode 10 includes the porous, stretchy base material 1, the conducting path 2, the conductive film 3, and the release liner 4. The porous, stretchy base material 1 includes the non-woven fabric 1-1, the adhesive layer 1-2, and the reinforcing film 1-3. The conducting path 2 includes the upper snap 2-1 and the lower snap 2-2. The bio-electrode 10 has such a shape as to protrude by a thickness of the conductive film 3 and has a configuration that the conductive film 3 more easily adheres to the skin.

FIG. 2 is a schematic cross-sectional exploded illustration showing components of the bio-electrode according to the present invention. FIG. 3 is a schematic bird's-eye illustration showing the components of the bio-electrode according to the present invention. As the conducting path penetrating the non-woven fabric 1-1 serving as the porous, stretchy base material (porous film), the upper snap 2-1 and the lower snap 2-2 that sandwich the top and bottom of the non-woven fabric 1-1 are shown. The reinforcing film 1-3 may be located to be in contact with the conductive film 3 as shown in FIG. 1, may be located on the surface of the non-woven fabric 1-1 on the opposite side of the skin, or may be located on both sides. Alternatively, there may be no reinforcing film 1-3. The reinforcing film 1-3 may have electric

conductivity. To have electric conductivity, a plastic film surface may be covered with a conductive film of silver, copper, iron, stainless used steel (SUS), silver chloride, or the like, the metal film may be used, or a fiber cloth coated with a metal may be used.

The porous film is used for allowing the skin to breathe, and has stretchiness. The porous film has at least the stretchiness of 5%, preferably 10% or more. The porous film may have a fibrous form, or may be a membrane film having continuous holes. As the fibrous film, a non-woven fabric is preferable. Examples of a material of the porous film can include polyethylene, polypropylene, polystyrene, nylon, polycarbonate, polyethylene terephthalate (PET), polyethylene naphthalate (PEN), polyurethane, polyimide, and tetrafluoroethylene. Fibers used for the porous film such as the non-woven fabric and the membrane film may be subjected to antimicrobial treatment. The total film thickness of the porous, stretchy base material and the conductive film in combination is 1 mm or less, preferably 800 um or less, more preferably 500 um or less. When the film thickness is small, the bio-electrode is comfortable to be worn with a less feeling of wearing. When the film thickness is too small, however, the bio-electrode gets wrinkled when the release linear is peeled off and the bio-electrode is attached, and becomes easy to come off due to decreased film strength. Thus, an optimum film thickness needs to be selected, and can be, for example, 5 um or more.

The adhesive layer 1-2 made of an adhesive can be attached to the porous film. In a case where the adhesiveness of the conductive film alone is insufficient, the adhesive layer around the conductive film is effective to prevent the bio-electrode from peeling away. As the adhesive for the porous film, the above-described silicone-based adhesive, acrylic-based adhesive, and urethane-based adhesive can be used. However, in a case where the adhesiveness of the conductive film is sufficiently high, the adhesive layer of the porous film is not necessarily required.

The conducting path that penetrates the porous film is preferably a snap-type conducting path, but may not have a sandwiching form, and the snaps may be substituted by wiring. A material of the snaps may be a metal or conductive carbon. The conducting path preferably contains one or more selected from the group consisting of gold, silver, silver chloride, platinum, aluminum, magnesium, tin, tungsten, iron, copper, nickel, stainless-steel, chrome, titanium, carbon, and a conductive polymer. Each snap does not need to be conductive to the inside. In this case, only a surface of a resin snap is coated with a metal or carbon. In a case of a gel electrode, a surface of the gel electrode in contact with a gel on a lower surface of the lower snap needs to be made of silver chloride. However, since the ion polymer according to the present invention has high polarization, the bio-electrode is capable of transmitting an electric signal without using a reduction reaction of silver chloride, and thus there may be or may not be silver chloride.

In a case where the inside of each snap is made of a resin and the snap is coated with carbon, the bio-electrode passes X rays when an x-ray radiograph is taken with the bio-electrode being attached to the skin, and thus does not become a shadow. In a case where each snap is coated with a precious metal such as gold, platinum, and silver, in addition to carbon, the bio-electrode is favorable even under an intense magnetic field environment such as magnetic resonance imaging (MRI) without generating heat.

The reinforcing film 1-3 shown in FIGs. 2 and 3 is inserted to relax stress on an interface between the soft, stretchy non-woven fabric 1-1 and the hard lower snap 2-2. A PET film, a PEN film, a thermoplastic polyurethane (TPU) film, or the like can be applied to the reinforcing film 1-3. Since the reinforcing film 1-3 is used only for the purpose of stress relaxation, there may be or may not be the reinforcing film 1-3.

Production Method for Bio-electrode

FIG. 4 shows an example of the production method for the bio-electrode according to the present invention. A non-woven fabric sheet with the adhesive layer is perforated and a top and bottom thereof is sandwiched by the snaps. In a case where the reinforcing film is used, the reinforcing film is preferably sandwiched between the non-woven fabric sheet with the adhesive layer and the lower snap. The adhesive conductive film containing silicone is printed on the release liner. Examples of a printing method can include screen printing, stencil printing, flexographic printing, gravure printing, and ink-jet printing. After printing, baking is performed for drying of the solvent and curing. Finally, the conductive film is crimped onto the snap with the release liner being attached thereto, and the bio-electrode is thereby produced.

The bio-electrode according to the present invention is preferably a bio-electrode in which top of the conductive film is covered with the release liner. Alternatively, it is possible to produce the bio-electrode by applying the adhesive conductive film containing silicone onto the entire surface of the release liner, evaporating the solvent, curing the film by baking or the like, thereafter covering the other surface of the film with the release liner, cutting into a predetermined shape with laser or a sharp blade, peeling off the release liner on the one side, and crimping the conductive film onto the snap with the one side of the release liner being attached thereto.

In a case where the adhesive conductive film containing silicone is applied to the entire surface of the release liner, the conductive film can be applied to the release liner on a plate using a bar coater or a roll coater, or can be continuously applied to the release liner on a roll by a roll-to-roll process. The film thickness of the adhesive conductive film is preferably in a range from 1 to 1000 $\mu$m, more preferably in a range from 5 to 500 $\mu$m.

The above-mentioned release liner is selected from the group consisting of a fluorine resin, polyethylene,

polypropylene, cyclic polyolefin, polyethylene terephthalate, polybutylene terephthalate, polyethylene naphthalate, polybutylene naphthalate, polyvinyl chloride, polymethylmethacrylate, polyvinyl acetate, polystyrene, polymethylpentene, polyimide, polyphenylene sulfide, polysulfone, polyethersulfone, polyetherketone, polyamide-imide, polyetherimide, polyacetal, polycarbonate, polyphenylene ether, polyacrylonitrile, cellophane, and paper, and a fluorine-based peeling agent or silicone/fluorine-based peeling agent is preferably applied to the release liner other than the release liner consisting of the fluorine resin. Each of these materials for the base material may be used alone, but may be a base material obtained by laminating a plurality of materials. For example, when a silicone adhesive solution is applied onto fibers such as paper, the solution soaks into the fibers, and the release liner becomes unable to be peeled off after being cured. Hence, another plastic film is pasted onto the paper, and a top of the plastic film is coated with a fluorine-based peeling agent.

Since the release liner needs to be peeled off the conductive film, the base material preferably does not have stretchiness for smooth peel-off.

To peel off the silicone adhesive-based conductive film, a fluorine-based peeling agent needs to be applied onto the base material of the release liner. Specifically, the release liner is coated with, for example, a fluorosilicone peeling agent described in JP 2011-201033 A and JP 2020-100764 A as the peeling agent.

In a case where the adhesive conductive film is sandwiched by two release liners, each release liner is cut into a specific shape, and thereafter one of the release liners is peeled off and an adhesive surface thereof is attached to the lower snap. However, there is a case where differential peeling does not occur when one release liner is peeled off. In a case where peel forces of the release liners are equal, the differential peeling becomes difficult. In this case, the peeling force needs to be changed by changing of a kind of the peeling agent of either one of the release liners or a thickness of the base material. The thicker the base material is, the peeling force tends to be larger. Thus, in a case where the identical peeling agent is used, two sheets of release liner base materials having different film thicknesses are used.

Alternatively, the adhesive conductive film is applied onto a release liner, and another release liner having a fibrous form, fine irregularities formed thereon, or holes formed therein can be used as the other release liner. The release liner having the fibrous form, the fine irregularities formed thereon, or the holes formed therein is capable of mitigating a peeling force because of its small contact area with respect to the adhesive conductive film, and the issue of differential peeling is unlikely to occur.

The adhesive conductive film does not contain a volatile component such as water unlike the gel electrode, and thus does not degrade in performance due to evaporation of the component. Hence, the adhesive conductive film is covered with the release liner having the fibrous form or the holes formed therein, and can be thereby stored for a long period of time.

FIG. 5 shows a case where the conductive film is applied to the entire surface of the release liner. Examples of an application method can include roll coating, bar coating, slit coating, spray coating, and spin coating. After application and baking, the release liner is cut into a predetermined size, and crimped and transferred onto the lower snap. Since the transferred release liner is present only in a region of the conductive film, the release liner is replaced with a release liner having a large area to cover a surrounding adhesive portion.

FIG. 5 shows a case where one surface of the conductive film is covered with the release liner. FIG. 6 shows a case where both surfaces of the conductive film are covered with the respective release liners.

In FIG. 7, the non-woven fabric whose top and bottom are sandwiched by the snaps is flipped vertically, and the conductive film is directly formed on the lower snap. In the formation of the conductive film, since there is the adhesive layer on the periphery, a non-contact printing method is preferable to prevent the conductive film from being in contact with the adhesive layer. Examples of the non-contact printing can include ink-jet printing and nozzle-jet printing.

By making a comparison of processes in FIGs. 4, 5, and 6, the process in FIG. 4 is preferable because of its simplicity and high throughput. The simplest process among processes in FIGs. 4 to 7 is a process in FIG. 7.

In FIG. 8, a wiring line is extended from the lower snap in substitution for the upper snap, and a female connector is attached to a terminal of the wiring line. FIG. 9 shows a male connector. Only the lower snap can be arranged without the upper snap.

FIG. 10 shows a photograph of a bio-electrode that has been actually produced. A conductive film side in black, which is to be attached to the skin, is shown as the front side on the left, and an upper snap side is shown as the front side on the right.

FIGs. 11 and 12 each show a thickness of the produced bio-electrode shown in FIG. 10. The bio-electrode has such a thin film configuration as that the film thickness of the conductive film is 20 um, and the total film thickness of the porous film and the conductive film in combination is 390 $\mu$m.

FIG. 13 shows a size of each component of the produced bio-electrode shown in FIG. 10.

The snap for extracting a bio-signal can also be arranged outside the conductive film. When the snap for the bio-electrode attached to the skin is connected to a measurement apparatus, the center snap type shown in FIG. 1 applies slight stress, which causes the conductive film 3 to come off the skin. Thus, there is a case where sensitivity to

the bio-signal decreases. To reduce stress applied to the conductive film 3 when the snap for the bio-electrode is connected to the measurement apparatus, the bio-electrode preferably has a configuration in which the snap is arranged outside the conductive film as shown in FIG. 14. In this case, the conductive film 3 and the lower snap 2-2 need to be connected to each other by a conductive wiring line 1-5 as the conducting path. The conductive wiring line 1-5 preferably has stretchiness. FIG. 14 shows the conductive wiring line 1-5 formed on a film 1-4. Each of the film 1-4 and the conductive wiring line 1-5 preferably has stretchiness. The conductive wiring line having an accordion shape has stretchiness. The stretchy, conductive wiring line is preferable because a linear wiring layout can be used and an area thereof is small. In this case, for example, a polyurethane sheet is preferable as the stretchy film. A conductive paste in which a conductive filler and a solvent are mixed in a stretchy resin is printed to form the conductive wiring line.

FIG. 15 is a schematic cross-sectional illustration showing an example of a component configuration before the bio-electrode according to the present invention is assembled. The upper snap 2-1 and the lower snap 2-2 are arranged outside the conductive film 3.

FIG. 16 is a schematic bird's-eye illustration showing an example of a component configuration before the bio-electrode according to the present invention is assembled. The upper snap 2-1 and the lower snap 2-2 are arranged outside the conductive film 3.

As shown in FIG. 17, the upper snap 2-1 can also be made flat. In this case, the upper snap 2-1 and a code terminal of the measurement apparatus can be coupled to each other using a magnetic force.

Alternatively, as shown in FIG. 18, the upper snap 2-1 and the lower snap 2-2 can be arranged outside the conductive film 3, and the lower snap 2-2 can be covered with the conductive film 3. In this case, since the conductive film 3 is in contact with both the conductive wiring line 1-5 and the conductive lower snap 2-2, the bio-electrode can manifest high conductivity.

FIG. 19 is a schematic cross-sectional illustration showing an example of a component configuration before the bio-electrode according to the present invention is assembled. The upper snap 2-1 and the lower snap 2-2 are arranged outside the conductive film 3, and the lower snap 2-2 is covered with the conductive film 3.

FIG. 20 is a schematic bird's-eye illustration showing an example of a component configuration before the bio-electrode according to the present invention is assembled. The upper snap 2-1 and the lower snap 2-2 are arranged outside the conductive film 3, and the lower snap 2-2 is covered with the conductive film 3.

FIG. 21 shows a photograph of the bio-electrode produced according to an embodiment of the present invention. The upper snap 2-1 and the lower snap 2-2 are arranged outside the conductive film 3, and the lower snap 2-2 is covered with the conductive film 3.

Alternatively, as shown in FIG. 22, the upper snap 2-1 and the lower snap 2-2 sandwiching the conductive wiring line 1-5 formed on the stretchy film can be arranged on the opposite side of the non-woven fabric 1-1.

FIG. 23 shows a photograph of the bio-electrode that has been actually produced. The conductive film side in black, which is to be attached to the skin, is shown as the front side on the left, and the upper snap side is shown as the front side on the right.

As shown in FIG. 24, the snap may not be attached. FIG. 25 shows a photograph of a bio-electrode that has been actually produced. The conductive film side in black, which is to be attached to the skin, is shown as the front side on the left, and the conductive wiring line side is shown as the front side on the right. In this case, as shown in FIG. 26, the bio-electrode is sandwiched by conductive clips or the like to be conducted with a device.

As shown in FIG. 27, a conductive wiring line printed on the stretchy film can be substituted by conductive fibers 1-6 as the conducting path. The conductive fibers, in a configuration in which the surface of the fibers is coated with a metal or a conductive polymer, has both stretchiness and conductivity.

The present invention relates to the bio-electrode including the adhesive, conductive film formed on the non-woven fabric that allows the skin to breathe, and may have holes for escaping sweat to the outside. This is because, when sweat is accumulated between the skin and the conductive film, the conductive film comes off the skin and the sensitivity to a bio-signal decreases. FIG. 28 shows a case where the conductive film and the conductive wiring line are perforated. FIG. 29 shows a case where the non-woven fabric is additionally perforated. FIG. 30 shows a case where the adhesive layer and the non-woven fabric outside the conductive film are also perforated.

Each hole in FIGs. 28 to 30 preferably has a size of 3 mm or less in diameter, preferably 2 mm or less in diameter, and preferably has such an area as that a void ratio is 20% or less. When the area of a perforated portion is 20% or more, the bio-electrode comes off the skin due to decreased strength, and the area of a portion of the bio-electrode is reduced, whereby the sensitivity to a bio-signal decreases.

As shown in FIG. 31, the conductive wiring line 1-5 can also be arranged on the conductive film 3 side. In this case, since the conductive wiring line 1-5 does not penetrate the non-woven fabric 1-1, the bio-electrode can be more easily produced.

As shown in FIG. 32, the conductive wiring line and the film can be replaced with the conductive fibers 1-6.

Each component of the bio-electrode according to the present invention will be described in a more detailed manner hereinbelow.

Living Body Contact Layer

The bio-electrode according to the present invention includes the adhesive, conductive film that contains silicone and that serves as the living body contact layer formed on the conducting path that penetrates the porous film. This conductive film is a portion that is actually in contact with the living body when the bio-electrode is used, and has conductivity and adhesiveness. The conductive film is a cured product of the composition of the conductive film of the bio-electrode according to the present invention described above, that is, an adhesive resin layer containing silicone and additives such as the ionic material (salts) (A) and the resin (B) described above.

Note that as an adhesive force of the conductive film is preferably in a range of 0.5 N/25 mm or more and 20 N/25 mm or less. As a method of measuring the adhesive force, a method shown in JIS Z 0237 is generally used. As the base material, a metal substrate such a SUS (stainless-steel) substrate and a PET substrate can be used, but the adhesive force can also be measured with the human skin. Surface energy of the human skin is lower than that of metals and various plastics and close to that of Teflon (registered trademark), and the human skin has less adhesiveness.

The thickness of the conductive film of the bio-electrode is preferably 1 um or more and 5 mm or less, more preferably 2 um or more and 3 mm or less. As the conductive film becomes thinner, the adhesive force becomes lower, but the conductive film becomes more flexible, lighter, and more compatible to the skin. The thickness of the conductive film can be selected in view of adhesiveness and a texture with the skin.

In the bio-electrode according to the present invention, another adhesive film aside from the conductive film may be arranged to prevent the conductive film from coming off the living body when used similarly to a conventional bio-electrode (for example, the bio-electrode described in JP 2004-033468 A). In a case where another adhesive film is arranged, the adhesive film is only required to be formed using an acrylic-based adhesive film material, a urethane-based adhesive film material, a silicone-based adhesive film material, or the like. Especially, the silicone-based adhesive film material has high oxygen permeability, and thereby allows the skin to breathe while the bio-electrode remains to be attached to the skin. The adhesive film material of the silicone type has high water repellency, and thus is less susceptible to a decrease in adhesiveness due to sweat. Furthermore, the adhesive film material of the silicone type is preferable because of its property of being less irritating to the skin. Note that in the bio-electrode according to the present invention, since the addition of the tackifier to the composition of the conductive film or the use of the resin having preferable adhesiveness to the living body can prevent the bio-electrode from coming off as described above, the above-mentioned adhesive film is not necessarily arranged separately.

The wiring line between the bio-electrode according to the present invention and the sensor device and other members when the bio-electrode is used as a wearable device are not specifically limited. For example, a joining part to be joined with a hook described in JP H5-082405 A and JP H5-009505 A may be used. In this case, the hook protruding from the bio-electrode is connected to a conductive wiring line, whereby the bio-electrode is connected to the device. Alternatively, a stretchy base material on which wiring formed by printing described in JP 2004-033468 A and a device are mounted can be attached to the skin. In this case, electrocardiogram measurement during bathing or showering becomes possible with use of a water-proof device.

Production Method for Bio-electrode

The present invention provides a production method for a bio-electrode that includes preparing a conducting path that penetrates a porous, stretchy base material, or preparing a conducting path on the stretchy base material so that the conducting path is exposed on a side surface of the stretchy base material and forming an adhesive, conductive film containing silicon so as to be connected to one side of the conducting path, the one side being attached to the skin.

At this time, it is preferable to form the adhesive, conductive film containing silicon on one side of the conducting path that penetrates the porous, stretchy base material, or the conducting path that is on the stretchy base material and exposed on the side surface of the stretchy base material, the one side being attached to a skin, by transferring the formed, adhesive, conductive film containing silicon onto a release linear or by directly printing the formed, adhesive, conductive film containing silicon onto the conducting path.

Furthermore, it is preferable to form the bio-electrode by applying an adhesive, conductive film material containing silicon onto the release linear and curing the adhesive, conductive film material.

Note that the porous, stretchy base material, the conductive film, and the like used in the production method for the bio-electrode according to the present invention may be similar to those described above.

While a method of applying the composition of the conductive film onto the conducting path is not specifically limited, examples of the method include a method of directly applying the composition and a method of applying the composition onto another substrate and thereafter transferring the composition. In either method, for example, methods such as dip coating, spray coating, spin coating, roll coating, flow coating, doctor coating, screen printing, flexographic printing, gravure printing, and ink-jet printing are preferable.

In addition, the composition of the conductive film can be applied onto the stretchy base material. A method of

applying the composition of the conductive film onto the stretchy base material is not specifically limited, and the above-mentioned method is preferable.

A method of curing the composition of the conductive film is not specifically limited, and may be selected as appropriate depending on kinds of the components (A) and (B) used in the composition of the conductive film. For example, it is preferable to cure the composition of the conductive film using either or both of heat and light. Alternatively, it is possible to cure the composition of the conductive film by adding a catalyst that generates an acid or a base to the above-mentioned composition of the conductive film and thereby causing a cross-linking reaction.

Note that a temperature for heating is not particularly limited, and may be selected as appropriate depending on kinds of the components (A) and (B) used in the composition of the conductive film, and is, for example, preferably about 50 to 250°C.

Additionally, in a case of combining heating and light irradiation, heating and light irradiation may be simultaneously performed, heating may be performed after light irradiation, or light irradiation may be performed after heating. Alternatively, air drying may be performed for the purpose of evaporating the solvent before heating after film coating.

Putting water droplets onto the surface of the cured conductive film or spraying water vapor or mist onto the surface of the cured conductive film increases compatibility with the skin, and thereby enables quick obtaining of a bio-signal. It is also possible to use water mixed with alcohol to fine a size of droplet of water vapor or mist. It is also possible to bring the surface of the film into contact with absorbent cotton or a cloth containing water and moisten the surface of the film.

Water with which the surface of the cured conductive film is moistened may contain a salt. Water-soluble salts to be mixed with water are preferably selected from the group consisting of sodium salts, potassium salts, calcium salts, magnesium salts, and betaine.

The water-soluble salts can be, specifically, salts selected from the group consisting of sodium chloride, potassium chloride, calcium chloride, magnesium chloride, saccharin sodium salts, acesulfame potassium, sodium carboxylate, potassium carboxylate, calcium carboxylate, sodium sulfonate, potassium sulfonate, calcium sulfonate, sodium phosphate, potassium phosphate, calcium phosphate, magnesium phosphate, and betaine. Note that the above-mentioned polymer compound (A) is not included in the above-mentioned water-soluble salts.

More specific examples of the water-soluble salts can include, other than the above-mentioned salts, sodium acetate, sodium propionate, sodium pivalate, sodium glycolate, sodium butyrate, sodium valerate, sodium caproate, sodium enanthate, sodium caprylate, sodium pelargonate, sodium caprate, sodium undecylate, sodium laurate, sodium tridecanoate, sodium myristate, sodium pentadecylate, sodium palmitate, sodium margarate, sodium stearate, sodium benzoate, disodium adipate, disodium maleate, disodium phthalate, sodium butyrate, sodium 2-hydroxybutyrate, sodium 3-hydroxybutyrate, sodium 2-oxobutyrate, sodium stearate, sodium gluconate, sodium methanesulfonate, sodium 1-nonanesulfonate, sodium 1-decanesulfonate, sodium 1-dodecanesulfonate, sodium 1-undecanesulfonate, sodium cocoyl sethionate, sodium lauroyl methylalanine, sodium cocoyl methyltaurate, sodium cocoyl glutamate, sodium cocoyl sarcosinate, sodium methyl lauroyl taurate, laumidopropyl, potassium isobutyrate, potassium propionate, potassium pivalate, potassium glycolate, potassium gluconate, potassium methanesulfonate, calcium stearate, calcium glycolate, calcium gluconate, calcium 3-methyl-2-oxobutyrate, and calcium methanesulfonate. Betaine is a general term for intramolecular salts. Specifically, betaine is a compound in which three methyl groups are added to an amino group of amino acid. More specific examples of betaine include trimethylglycine, carnitine, trimethylglycine, and proline betaine.

The water-soluble salts can further contain monohydric alcohol or polyhydric alcohol having 1 to 4 carbon atoms. The alcohol is preferably selected from the group consisting of ethanol, isopropyl alcohol, ethylene glycol, diethylene glycol, triethylene glycol, glycerin, polyethylene glycol, polypropylene glycol, polyglycerol, diglycerine, and a silicone compound having a polyglycerol structure. The silicone compound having the polyglycerol structure is more preferably shown by the above-mentioned general formula (4).

As the pre-processing method using the solution containing salts, it is possible to moisten a bio-electrode film by an atomizing method or a water droplet dispense method performed on the composition of the cured conductive film (bio-electrode film). It is also possible to moisten the bio-electrode film in a high-temperature and humidity state such as in a sauna. It is also possible to cover the moistened bio-electrode film with a sheet to prevent the bio-electrode film from being dried. The sheet needs to be peeled off immediately before the bio-electrode film is attached to the skin. Thus, the sheet is coated with a peeling agent, or a Teflon film having a peeling property is used. A dry electrode covered with a peeling sheet is sealed with a bag covered with aluminum for storage for a long period of time. To prevent drying in the bag covered with aluminum, it is preferable to seal moisture within the bag.

A pre-processing method of spraying the solution containing salts is the most effective in a dry electrode that contains an ionic polymer having the repeating unit shown by the general formula (2), but is also effective in a dry electrode made of conductive fibers containing PEDOT-PSS, silver chloride, carbon, or a metal.

Wiping the skin where the bio-electrode is attached with a cloth containing water or alcohol such as ethanol containing water and glycerin or spraying water or alcohol onto the skin immediately before the bio-electrode is attached is effective in moistening the surface of the skin to obtain a bio-signal with high sensitivity and high accuracy in a short

period of time. Wiping the skin with the cloth containing water is effective not only in moistening the skin, but also in removing oils and fats on the surface of the skin, and thereby enhances the sensitivity to the bio-signal.

As described above, the production method for the bio-electrode according to the present invention allows the bio-electrode according to the present invention that has excellent conductivity and bio-compatibility, that is lightweight, and that does not significantly decrease in conductivity to be produced easily at low cost.

Measurement Method for Bio-signal

The present invention provides a measurement method for a bio-signal including attaching the bio-electrode according to the present invention to the skin, and measuring the bio-signal after bathing or showering, or during bathing or showering.

The bio-electrode according to the present invention has water repellency, and thereby enables execution of such a measurement method for a bio-signal.

The measurement method is not specifically limited except measurement is performed after bathing or showering or during bathing or showering, and a conventional measurement method can be used.

EXAMPLE

Hereinafter, the present invention will be specifically described using Examples and Comparative Examples. However, the present invention is not limited thereto.

The ionic polymer 1 blended with the conductive film solution was synthesized as follows. Each monomer in an amount of 20 mass% in a cyclopentanone solution was put into a reaction container and mixed, the reaction container was cooled to -70°C in a nitrogen atmosphere, and each of decompression/deaeration and nitrogen blowing was repeated three times. After a temperature was increased to a room temperature, azobisisobutyronitrile (AIBN) as a polymerization initiator was added in an amount of 0.01 moles relative to 1 mole of the entire monomers, the temperature was increased to 60°C, thereafter the solution was reacted for 15 hours, and cyclopentanone was evaporated by an evaporator. After a part of a polymer solution was dried, the composition of the obtained polymer was checked by $^1$H-NMR. A molecular weight (Mw) and degree of dispersion (Mw/Mn) of the obtained polymer were checked by gel permeation chromatography (GPC) using tetrahydrofuran (THF) as a solvent. The ionic polymer 1 is as follows.

The ionic polymer 1

Mw = 38,100

Mw/Mn = 1.91

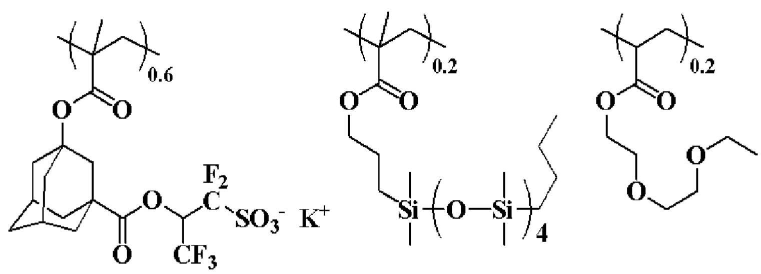

(The number of repetitions in the formula represents an average value)

The following ionic polymers 2 to 14 were polymerized by a similar method.

The ionic polymer 2

Mw = 36,100

Mw/Mn = 1.93

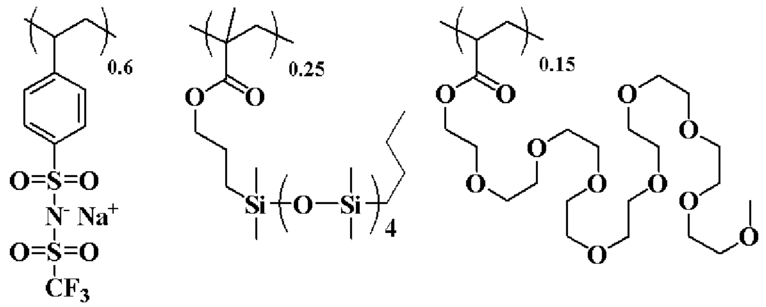

(The number of repetitions in the formula represents an average value)

The ionic polymer 3

Mw = 150,600

Mw/Mn = 1.85

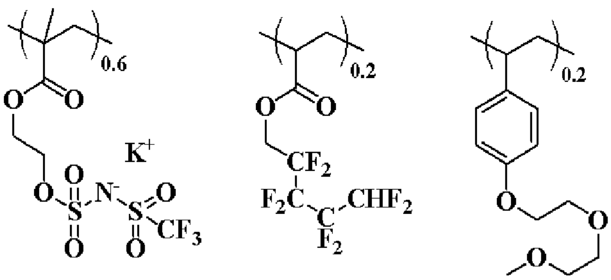

The ionic polymer 4

Mw = 44,400

Mw/Mn = 1.94

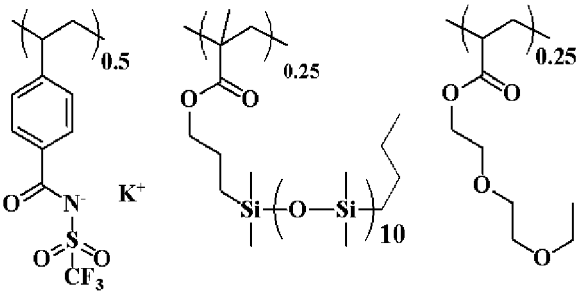

(The number of repetitions in the formula represents an average value)

The ionic polymer 5

Mw = 43,100

Mw/Mn = 1.88

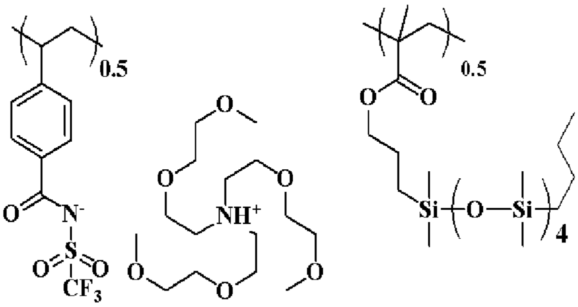

(The number of repetitions in the formula represents an average value)

The ionic polymer 6

Mw = 41,200

Mw/Mn = 1.72

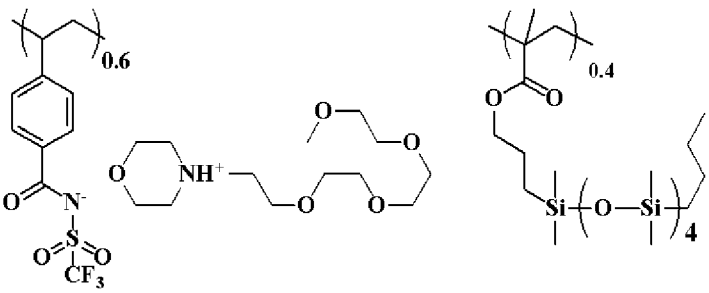

The ionic polymer 7

Mw = 43,600

Mw/Mn = 1.93

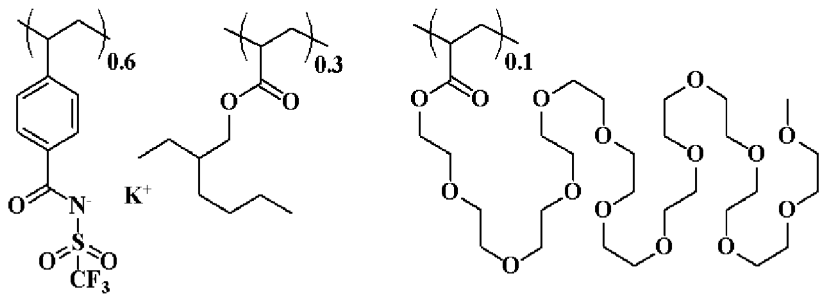

The ionic polymer 8

Mw = 31,600

Mw/Mn = 2.10

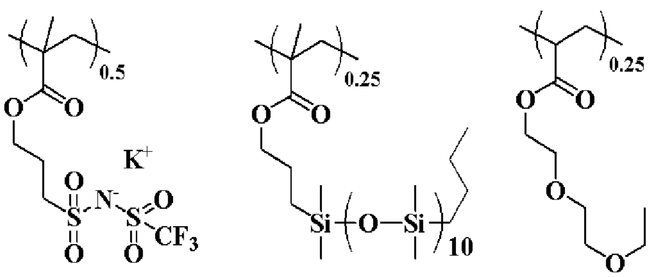

(The number of repetitions in the formula represents an average value)

The ionic polymer 9

Mw = 55,100

Mw/Mn = 2.02

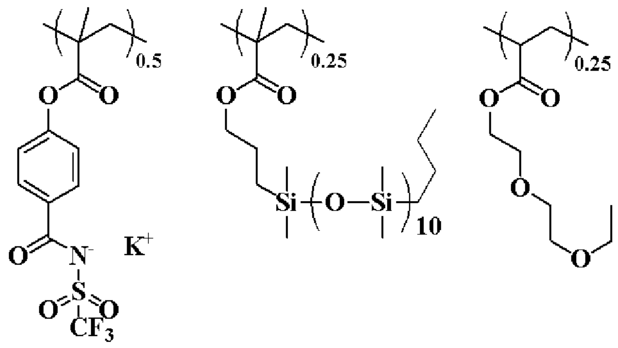

(The number of repetitions in the formula represents an average value)

The ionic polymer 10

Mw = 87,500

Mw/Mn = 2.01

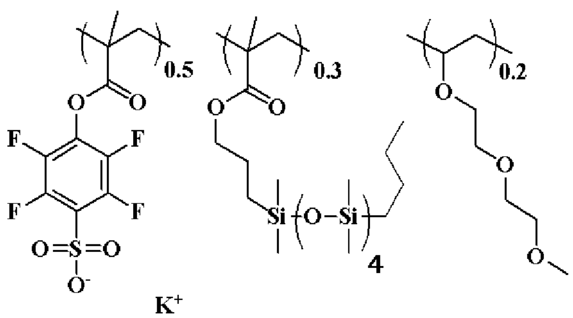

(The number of repetitions in the formula represents an average value)

The ionic polymer 11

Mw = 43,600

Mw/Mn = 1.91

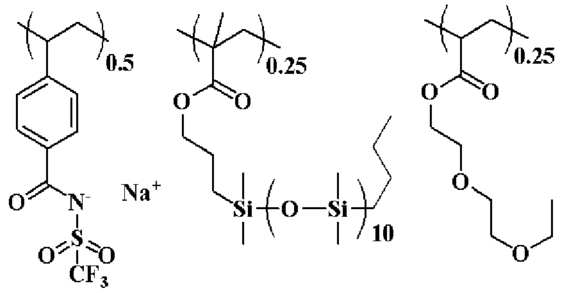

(The number of repetitions in the formula represents an average value)

The ionic polymer 12

$$Mw = 97,100$$

$$Mw/Mn = 2.20$$

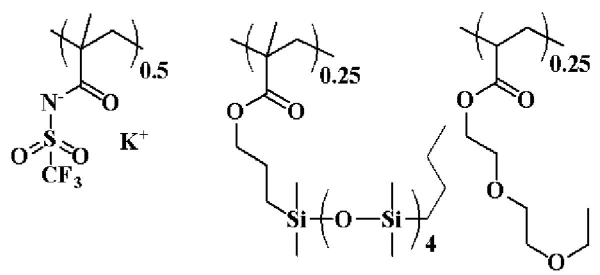

(The number of repetitions in the formula represents an average value)

The ionic polymer 13

$$Mw = 68,900$$

$$Mw/Mn = 2.26$$

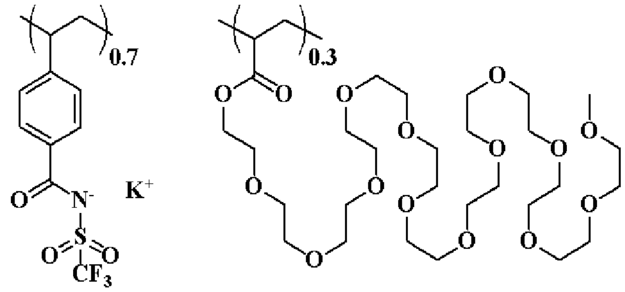

The ionic polymer 14

Mw = 67,300

Mw/Mn = 2.00

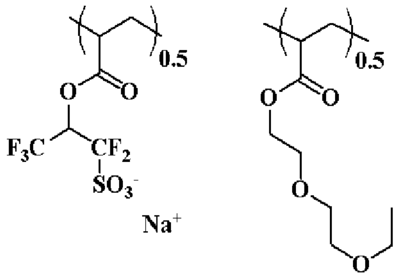

Siloxane compounds 1 to 4 blended as the silicone-based resin in the conductive film solution are shown below.

Siloxane Compound 1

A vinyl group-containing polydimethylsiloxane having a viscosity of 27,000 mPa in a 30% toluene solution, and having an alkenyl group content of 0.007 mols/100 g, in which a terminal of the molecular chain is blocked with a $SiMe_2Vi$ group was used as the siloxane compound 1.

Siloxane Compound 2

A 60% toluene solution containing polysiloxane of an MQ resin composed of an $Me_3SiO_{0.5}$ unit and a $SiO_2$ unit ($Me_3SiO_{0.5}$ unit/$SiO_2$ unit = 0.8) was used as the siloxane compound 2.

Siloxane Compound 3

A solution composed of 40 parts by mass of vinyl group-containing polydimethylsiloxane having a viscosity of 42,000 mPa in a 30% toluene solution, and having an alkenyl group content of 0.007 mols/100 g, in which a terminal of the molecular chain is blocked with OH, 100 parts by mass of a 60% toluene solution containing polysiloxane of an MQ resin composed of an $Me_3SiO_{0.5}$ unit and a $SiO_2$ unit ($Me_3SiO_{0.5}$ unit/$SiO_2$ unit = 0.8), and 26.7 parts by mass of toluene was heated for four hours while being refluxed, cooled, a polydimethylsiloxane was bonded to the MQ resin, and the resultant was used as the siloxane compound 3.

(Siloxane Compound 4)

KF-99 from Shin-Etsu Chemical Co., Ltd. was used as methyl hydrogen silicone oil.

Acrylic adhesive polymers 1 and 2 blended as acrylic-based adhesive resins in the conductive film solution are shown below.

Acrylic adhesive polymer 1

Mw = 560,000

Mw/Mn = 2.69

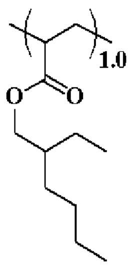

Acrylic adhesive polymer 2

Mw = 410,000

Mw/Mn = 2.87

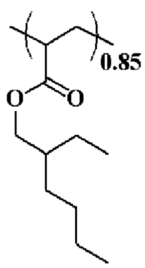

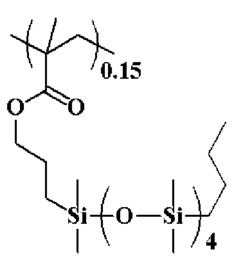

As the urethane adhesive, a mixture of Polythick UPS-1A and Polythick UPS-1B from Sanyo Chemical Industries, Ltd. was used. These urethane adhesives do not contain silicone.

An organic solvent blended in the conductive film solution is shown below.

Isoper G: isoparaffin-based solvent from Standard Oil Company
Isoper M: isoparaffin-based solvent from Standard Oil Company
DGDE: diethylene glycol diethyl ether

The lithium titanate powder, the platinum catalyst, and the conductivity improver (carbon black, carbon nanotube) blended as additives in the conductive film solution, silver flakes blended as the metal powder, 1-ethynylcyclohexanol blended as the addition reaction control agent are shown below.

Lithium titanate powder: from Sigma-Aldrich Co. LLC in a size of 200 nm or less
Platinum catalyst: CAT-PL 50T from Shin-Etsu Chemical Co., Ltd.
Carbon black: DENKA BLACK Li-400 from Denka Company Limited
Multiwall carbon nanotube: from Sigma-Aldrich Co. LLC in a diameter of 110 to 170 nm and a length of 5 to 9 μm.
Silver flakes: from Sigma-Aldrich Co. LLC in an average size of 10 μm.
Addition reaction control agent: 1-ethynylcyclohexanol

Conductive Film Solutions 1 to 13, Comparative Conductive Film Solution 1

In the compositions described in Tables 1 and 2, the ionic polymer, the adhesive resin, the organic solvent, and the additives (the lithium titanate powder, the platinum catalyst, and the conductivity improver), the metal powder, and the addition reaction control agent were blended and filtrated with a stainless-steel filter having 300 meshes, whereby conductive film solutions (conductive film solutions 1 to 13 and comparative conductive film solution 1) were prepared.

[Table 1]

| Conductive film solution | Ionic polymer (parts by mass) | Silicone-based resin and adhesive resin (parts bv mass) | Organic solvent (parts by mass) | Additive (parts by mass) |
|---|---|---|---|---|
| Conductive film solution 1 | Ionic polymer 1 (20) | Siloxane compound 1 (40)<br>Siloxane compound 2 (100)<br>Siloxane compound 4 (3) | Isoper G (100) | CAT-PL-50T (0.7)<br>Lithium titanate power (12)<br>Carbon black (8)<br>1-ethynylcyclohexanol (2) |
| Conductive film solution 2 | Ionic polymer 2 (20) | Siloxane compound 3 (126)<br>Siloxane compound 4 (3) | n-octane (40)<br>n-decane (20)<br>Cyclopentanone (70) | CAT-PL-50T (1.5)<br>Carbon black (10)<br>Silver flakes (20)<br>1-ethynylcyclohexanol (2) |
| Conductive film solution 3 | Ionic polymer 3 (20) | Siloxane compound 3 (126)<br>Siloxane compound 4 (3) | n-nonane (60)<br>Cyclopentanone (70) | CAT-PL-50T (1.5)<br>Carbon black (5)<br>1-ethynylcyclohexanol (2) |
| Conductive film solution 4 | Ionic polymer 4 (20) | Siloxane compound 3 (126)<br>Siloxane compound 4 (3) | Isoper G (60)<br>Cyclopentanone (70) | CAT-PL-50T (1.5)<br>Carbon black (12)<br>Multiwall carbon nanotube (4)<br>1-ethynylcyclohexanol (2) |
| Conductive film solution 5 | Ionic polymer 5 (20) | Poly thick UPS-1A (60)<br>Poly thick UPS-1B (40) | DGDE (60)<br>Cyclopentanone (40) | Carbon black (20) |
| Conductive film solution 6 | Ionic polymer 6 (20) | Poly thick UPS-1A (60)<br>Poly thick UPS-1B (40) | DGDE (60)<br>Cyclopentanone (40) | Carbon black (20) |
| Conductive film solution 7 | Ionic polymer 7 (20) | Acrylic adhesive polymer 2 (100) | DGDE (60)<br>Cyclopentanone (40) | Carbon black (20) |
| Conductive film solution 8 | Ionic polymer 8 (20) | Siloxane compound 3 (126)<br>Siloxane compound 4 (3) | n-decane (30)<br>n-octane (30)<br>Cyclopentanone (70) | CAT-PL-50T (1.5)<br>Carbon black (6)<br>1-ethynylcyclohexanol (2) |
| Conductive film solution 9 | Ionic polymer 9 (20) | Siloxane compound 3 (126)<br>Siloxane compound 4 (3) | n-decane (30)<br>n-octane (30)<br>Cyclopentanone (70) | CAT-PL-50T (1.5)<br>Carbon black (18)<br>1-ethynylcyclohexanol (2) |

(continued)

| Conductive film solution | Ionic polymer (parts by mass) | Silicone-based resin and adhesive resin (parts bv mass) | Organic solvent (parts by mass) | Additive (parts by mass) |
|---|---|---|---|---|
| Conductive film solution 10 | Ionic polymer 10 (25) | Siloxane compound 3 (126)<br>Siloxane compound 4 (3) | n-decane (30)<br>n-octane (30)<br>Cyclopentanone (70) | CAT-PL-50T (1.5)<br>Carbon black (20)<br>1-ethynylcyclohexanol (2) |
| Conductive film solution 11 | Ionic polymer 11 (20) | Siloxane compound 3 (126)<br>Siloxane compound 4 (3) | n-decane (30)<br>n-octane (30)<br>Cyclopentanone (70) | CAT-PL-50T (1.5)<br>Carbon black (20)<br>1-ethynylcyclohexanol (2) |
| Conductive film solution 12 | Ionic polymer 12 (20) | Siloxane compound 3 (126)<br>Siloxane compound 4 (3) | Isoper M (30)<br>n-octane (30)<br>Cyclopentanone (70) | CAT-PL-50T (1.5)<br>Carbon black (20)<br>1-ethynylcyclohexanol (2) |
| Conductive film solution 13 | Ionic polymer 13 (20) | Siloxane compound 3 (126)<br>Siloxane compound 4 (3) | n-decane (20)<br>n-dodecane (20)<br>n-tridecane (20)<br>Cyclopentanone (70) | CAT-PL-50T (1.5)<br>Carbon black (20)<br>1-ethynylcyclohexanol (2) |

[Table 2]

| Conductive film solution | Ionic polymer (parts by mass) | Adhesive resin (parts by mass) | Organic solvent (parts by mass) | Additive (parts by mass) |
|---|---|---|---|---|
| Comparative conductive film solution 1 | Ionic polymer 14 (20) | Acrylic adhesive polymer 1 (100) | DGDE (60)<br>Cyclopentanone (40) | Carbon black (20) |

Production of Bio-electrode

As the stretchy base material, a polyethylene non-woven fabric (with a thickness of 400 μm) to which an acrylic adhesive is applied as a porous film 1 and that has stretchiness of 20%, a polyurethane non-woven fabric (with a thickness of 400 μm) to which an acrylic adhesive is applied as a porous film 2 and that has stretchiness of 50%, a polyethylene non-woven fabric (with a thickness of 380 μm) to which a urethane adhesive is applied as a porous film 3 and that has stretchiness of 20%, a polyethylene non-woven fabric (with a thickness of 330 μm) to which a silicone adhesive is applied as a porous film 4 and that has stretchiness of 20%, and a urethane membrane film (with a thickness of 150 μm) to which an acrylic adhesive is applied as a porous film 5 and that has stretchiness of 20% were used. As a comparative non-porous film, a TPU sheet that has stretchiness of 10% and a film thickness of 50 μm was used.

As the conducting path, a snap 1 including upper and lower snaps whose plastic surfaces were coated with silver, a snap 2 coated with carbon, and a snap 3 that was coated with silver and that has a lower snap whose contact portion with a conductive film was coated with silver chloride were used.

As Comparative Example 3, a commercially available, gel electrode-based bio-electrode (Kendall H135SG)

was used.

As a reinforcing film around the snaps, a PET film with a thickness of 20 μm was used.

The bio-electrode was produced in the processes shown in FIG. 4. The porous film with the adhesive or the TPU sheet, and the PET film were perforated by punching, and sandwiched by the upper and lower snaps. To print the conductive film, each conductive film solution or the comparative conductive film solution was screen-printed on a Teflon sheet and baked at 125°C for 15 minutes, whereby a conductive film pattern with a diameter of 17 mm and a thickness of 20 μm was formed. The conductive film pattern was crimped and transferred onto the lower snap, and the release liner was attached onto the conductive film pattern.

Production of Silicone Adhesive Conductive Film on Release Liner

(1) Production of the silicone adhesive conductive film on the release liner shown in FIG. 4

The silicone adhesive conductive film pattern was printed by a screen printing machine on the release liner using the conductive film solution with a perforated stencil mask, air-dried at a room temperature for ten minutes, and thereafter cured at 125°C in an oven for ten minutes, whereby the silicone adhesive conductive film pattern with a film thickness of 50 μm was formed.

(2) Production of the silicone adhesive conductive film sandwiched by the release liners shown in FIG. 6

The conductive film solution was applied onto the release linear using an automatic film applicator (slit coater) from Allgood Company, air-dried at a room temperature for ten minutes, and thereafter cured at 125°C in an oven for ten minutes. A slit width of the slit coater was set as 350 μm, 550 μm, and 740 μm, respectively, whereby the silicone adhesive conductive films with respective film thicknesses of 50 μm, 100 μm, and 150 μm after curing were formed. The silicone adhesive conductive film with the film thickness of 50 μm was applied to production of the bio-electrode in Examples 16, 19, 20, and 21 in Table 3, the silicone adhesive conductive film with the film thickness of 100 μm was applied to production of the bio-electrode in Example 17 in Table 3, and the silicone adhesive conductive film with the film thickness of 150 μm was applied to production of the bio-electrode in Example 18 in Table 3.

As the release linear, the following release liners were used.

1: PET Separator SS1A (thickness of 100 μm) from Nippa Corporation
2: PET Separator SS1A (thickness of 50 μm) from Nippa Corporation
3: PET Separator FSD5 (thickness of 100 μm) from Nippa Corporation
4: PET Separator FSD5 (thickness of 38 μm) from Nippa Corporation
5: Teflon fiber sheet (thickness of 280 μm)

A peel force of the PET Separator FSD5 is less than a peel force of the PET Separator SS1A, and is easily peeled off. A peel force of the PET Separator SS1A (thickness of 50 μm) is less than a peel force of the PET Separator SS1A (thickness of 100 μm). A peel force of the Teflon sheet is less than a peel force of the PET Separator FSD5 (thickness of 38 μm).

The release liner to be attached after the silicone adhesive conductive solution is applied and cured needs to have a peel force that is less than that of the release liner to which the silicone adhesive conductive solution is applied. This is because the two upper and lower release liners having equal peel forces may prevent differential peeling.

In a case where two release liners are described in Table 3, the release liner on the left side is the one to which the silicone adhesive conductive solution is applied and the release liner on the right side is the one that covers the cured silicone adhesive conductive film.

FIG. 33 shows a photograph of a sheet of the silicone adhesive conductive film that was used to produce the bio-electrode in Example 16, to a top and bottom of which the release liners were attached, and that had a width of 16 cm, a length of 29 cm, and a thickness of 50 μm.

FIG. 10 shows a photograph of the bio-electrode produced according to Examples and having a cross-sectional form shown in FIG. 1. FIG. 21 shows a photograph of the bio-electrode in a form shown in FIG. 18. FIG. 23 shows a photograph of the bio-electrode in a form shown in FIG. 22. FIG. 25 shows a photograph of the bio-electrode in a form shown in FIG. 24.

Measurement of Thickness of Living Body Contact Layer

In the bio-electrode produced according to the above-mentioned production of the bio-electrode, the total thickness of the porous film and the conductive film in combination was measured with a micrometer. In a case where

the base material is the TPU sheet, the total thickness of the TPU sheet and the conductive film in combination was measured. Each of these thicknesses was regarded as the thickness of the bio-electrode. Results are shown in Table 3.

Measurement of Bio-signal

The release liner of the bio-electrode was peeled off, the skin where the bio-electrode was to be attached was wiped with moistened gauze immediately before the attachment, a positive electrode of an electrocardiograph was attached to the human body in a location of LA in FIG. 34, a negative electrode of the electrocardiograph was attached in a location of LL, and a grounding terminal was attached in a location of RA.

Immediately after the attachment and at the same time every day, in a sedentary, resting state, the snap of the bio-electrode was pinched by a clip attached to the tip of a conductive wire line, and a portable electrocardiograph HCG-901 from OMRON HEALTHCARE Co., Ltd. and the conductive wire line were connected to each other, and electrocardiographic measurement was performed. When the measurement was not performed, the bio-electrode remained to be attached to the body, and only the portable electrocardiograph and the conductive wire line were removed. Bathing was performed once a day, for 15 to 30 minutes, at about 40°C. Results are shown in Table 3. Whether an electrocardiogram (ECG) signal typified by PQRSTU waves shown in FIG. 35 was appeared was checked, and the measurement was performed until this waveform did not appear.

[Table 3]

| Example | Stretch y base materia 1 | Condu cting path | Adhesive, conductive film solution | Method of attaching conductiv e film | Relea se liner | Cross section form of bio-electrode | Thickness of bio-electrode layer (micron) | Days ECG signal can be obtained after attachment | Itching on skin |
|---|---|---|---|---|---|---|---|---|---|
| Example 1 | Porous film 1 | Snap 1 | Conductive film solution 1 | FIG. 4 | 1 | FIG. 1 | 410 | 15 | None |
| Example 2 | Porous film 2 | Snap 1 | Conductive film solution 2 | FIG. 4 | 1 | FIG. 1 | 410 | 15 | None |
| Example 3 | Porous film 3 | Snap 1 | Conductive film solution 3 | FIG. 4 | 1 | FIG. 1 | 395 | 21 | None |
| Example 4 | Porous film 4 | Snap 1 | Conductive film solution 4 | FIG. 4 | 1 | FIG. 1 | 345 | 30 | None |
| Example 5 | Porous film 5 | Snap 1 | Conductive film solution 5 | FIG. 4 | 1 | FIG. 1 | 171 | 13 | None |
| Example 6 | Porous film 1 | Snap 2 | Conductive film solution 6 | FIG. 4 | 1 | FIG. 1 | 405 | 12 | None |
| Example 7 | Porous film 1 | Snap 3 | Conductive film solution 7 | FIG. 4 | 1 | FIG. 1 | 400 | 12 | None |
| Example 8 | Porous film 1 | Snap 1 | Conductive film solution 8 | FIG. 4 | 1 | FIG. 1 | 415 | 18 | None |
| Example 9 | Porous film 1 | Snap 1 | Conductive film solution 9 | FIG. 4 | 1 | FIG. 1 | 420 | 19 | None |
| Example 10 | Porous film 1 | Snap 1 | Conductive film solution 10 | FIG. 4 | 1 | FIG. 1 | 415 | 16 | None |
| Example 11 | Porous film 1 | Snap 1 | Conductive film solution 11 | FIG. 4 | 1 | FIG. 1 | 412 | 20 | None |
| Example 12 | Porous film 1 | Snap 1 | Conductive film solution 12 | FIG. 4 | 1 | FIG. 1 | 418 | 22 | None |
| Example 13 | Porous film 1 | Snap 1 | Conductive film solution 13 | FIG. 4 | 1 | FIG. 1 | 415 | 21 | None |
| Example 14 | Porous film 1 | Snap 1 | Conductive film solution 4 | FIG. 4 | 1 | FIG. 14 | 400 | 21 | None |

(continued)

| Example | Stretch y base materia 1 | Condu cting path | Adhesive, conductive film solution | Method of attaching conductiv e film | Relea se liner | Cross section form of bio-electrode | Thickness of bio-electrode layer (micron) | Days ECG signal can be obtained after attachment | Itching on skin |
|---|---|---|---|---|---|---|---|---|---|
| Example 15 | Porous film 1 | Snap 1 | Conductive film solution 9 | FIG. 4 | 1 | FIG. 22 | 410 | 26 | None |
| Example 16 | Porous film 1 | Snap 1 | Conductive film solution 9 | FIG. 6 | 12 | FIG. 22 | 410 | 24 | None |
| Example 17 | Porous film 1 | Snap 1 | Conductive film solution 9 | FIG. 6 | 12 | FIG. 22 | 460 | 26 | None |
| Example 18 | Porous film 1 | Snap 1 | Conductive film solution 9 | FIG. 6 | 12 | FIG. 22 | 510 | 28 | None |
| Example 19 | Porous film 1 | Snap 1 | Conductive film solution 9 | FIG. 6 | 2 4 | FIG. 22 | 420 | 26 | None |
| Example 20 | Porous film 1 | Snap 1 | Conductive film solution 9 | FIG. 6 | 3 4 | FIG. 22 | 400 | 27 | None |
| Example 21 | Porous film 1 | Snap 1 | Conductive film solution 9 | FIG. 6 | 4 5 | FIG. 24 | 410 | 25 | None |
| Example 22 | Porous film 1 | Snap 1 | Conductive film solution 4 | FIG. 4 | 1 | FIG. 24 | 390 | 27 | None |
| Example 23 | Porous film 1 | Snap 1 | Conductive film solution 9 | FIG. 6 | 12 | FIG. 18 | 410 | 25 | None |
| Comparati ve Example 1 | TPU sheet | Snap 1 | Conductive film solution 1 | FIG. 4 | 1 | FIG. 1 | 215 | 3 | YES |
| Comparati ve Example 2 | Porous film 1 | Snap 1 | Conductive film solution 1 | FIG. 4 | 1 | FIG. 1 | 415 | 5 | None |
| Comparati ve Example 3 | - | - | - | - | - | - | 1135 | 1 | None |

As shown in Examples 1 to 23 in Table 3, the bio-electrode according to the present invention using the porous film as the stretchy base material and including the adhesive, conductive film containing silicon and the conducting path enabled obtaining of the bio-signal for a long period of time after the attachment. In contrast, in a case of Comparative Example 1 using the non-perforated TPU sheet as the stretchy base material, itching occurred because the skin was unable to breathe, the bio-electrode was peeled off in three days after the attachment. In a case of Comparative Example 2 in which the porous film was used as the stretchy base material but the conductive film does not contain silicon, degradation by water occurred, and days when the bio-signal was obtained became shorter. In a case of Comparative Example 3 using the commercially-available gel, the thickness of the living body contact layer was greater than that of the living body contact layer of the bio-electrode according to the present invention, and furthermore, the gel swelled and the bio-electrode came off at the time of bathing on the first day. The total film thickness of the porous film and conductive film of the bio-electrode in combination in Examples was small, and the bio-electrode was comfortably worn without a feeling of wearing caused by the attachment to the skin.

It should be noted that the present invention is not limited to the above-described embodiments. The embodiments are just examples, and any examples that substantially have the same feature and demonstrate the same functions and effects as those in the technical concept disclosed in claims of the present invention are included in the technical scope of the present invention.

## Claims

1. A bio-electrode comprising:

   a porous, stretchy base material;
   an adhesive, conductive film containing silicon; and
   a conducting path,
   wherein the conductive film is formed on one surface of the porous, stretchy base material,
   the conducting path is connected to the conductive film, and penetrates the stretchy base material to be exposed on the opposite side, or is exposed on a side surface of the stretchy base material.

2. The bio-electrode according to claim 1, wherein
   the porous, stretchy base material is a non-woven fabric or a membrane film.

3. The bio-electrode according to claim 1 or 2, wherein the adhesive, conductive film containing silicon contains an ionic material (A) selected from the group consisting of salts of ammonium, lithium, sodium, potassium, and silver formed with any of fluorosulfonic acid, fluorosulfonimide, and N-carbonylfluorosulfonic amide.

4. The bio-electrode according to claim 3, wherein

   the ionic material (A) has a partial structure shown by the following general formulae (1)-1 to (1)-4,

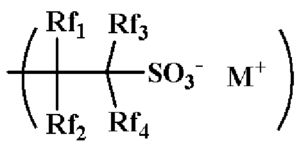

(1)-1

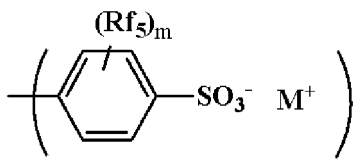

(1)-2

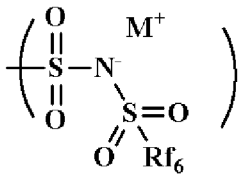

**(1)-3**

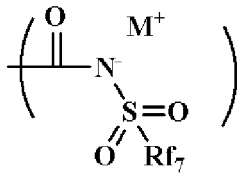

(1)-4

wherein $Rf_1$ and $Rf_2$ each represent a hydrogen atom, a fluorine atom, an oxygen atom, a methyl group, or a trifluoromethyl group, provided that when $Rf_1$ and $Rf_2$ represent an oxygen atom, the single oxygen atom represented by $Rf_1$ and $Rf_2$ bonds to a single carbon atom to form a carbonyl group; $Rf_3$ and $Rf_4$ each represent a hydrogen atom, a fluorine atom, or a trifluoromethyl group, provided that at least one of $Rf_1$ to $Rf_4$ is a fluorine atom or a trifluoromethyl group; $Rf_5$, $Rf_6$, and $Rf_7$ each represent a fluorine atom, a trifluoromethyl group, or a linear or branched alkyl group having 1 to 4 carbon atoms, and have at least one fluorine atom; $M^+$ represents an ion selected from the group consisting of an ammonium ion, a sodium ion, a potassium ion, and a silver ion; and "m" represents an integer of 1 to 4.

**5.** The bio-electrode according to claim 3 or 4, wherein the ionic material (A) comprises at least one ionic polymer selected from the group consisting of repeating units A1 to A7 shown by the following general formula (2),

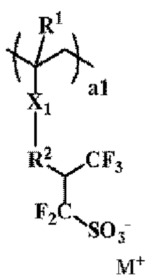

A1

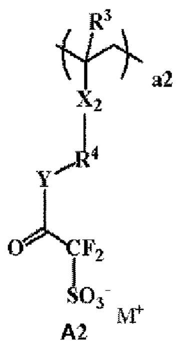

A2

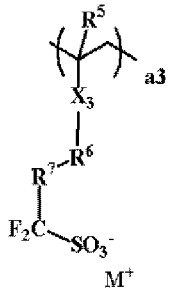

A3

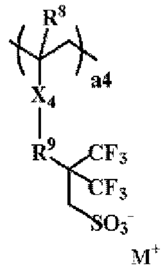

A4

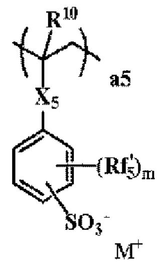

A5

(2)

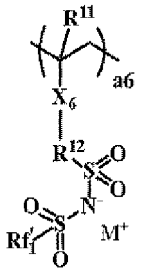

A6

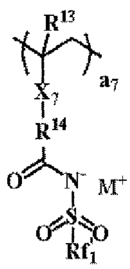

A7

wherein $R^1$, $R^3$, $R^5$, $R^8$, $R^{10}$, $R^{11}$, and $R^{13}$ each independently represent a hydrogen atom or a methyl group; $R^2$, $R^4$, $R^6$, $R^9$, $R^{12}$, and $R^{14}$ each independently represent a single bond, or a linear, branched, or cyclic hydrocarbon group having 1 to 13 carbon atoms, the hydrocarbon group optionally having either or both of an ester group and an ether group; $R^7$ represents a linear or branched alkylene group having 1 to 4 carbon atoms, and one or two hydrogen atoms in $R^7$ are optionally substituted with a fluorine atom; $X_1$, $X_2$, $X_3$, $X_4$, $X_6$ and $X_7$ each independently represent any of a single bond, a phenylene group, a naphthylene group, an ether group, an ester group, and an amide group; $X_5$ represents any of a single bond, an ether group, and an ester group; Y represents any of an oxygen atom and a $-NR^{19}-$ group; $R^{19}$ represents any of a hydrogen atom, a linear, branched, or cyclic alkyl group having 2 to 12 carbon atoms, and a phenyl group, and optionally have one or

more selected from the group consisting of an ether group, a carbonyl group, an ester group, and an amide group; Y optionally forms a ring together with $R^4$;
$Rf_1$' and $Rf_5$' each represent a fluorine atom, a trifluoromethyl group, or a linear or branched alkyl group having 1 to 4 carbon atoms, and have at least one fluorine atom; "m" represents an integer of 1 to 4; a1, a2, a3, a4, a5, a6, and a7 are $0 \leq a1 \leq 1.0$, $0 \leq a2 \leq 1.0$, $0 \leq a3 \leq 1.0$, $0 \leq a4 \leq 1.0$, $0 \leq a5 \leq 1.0$, $0 \leq a6 \leq 1.0$, $0 \leq a7 \leq 1.0$, and $0 < a1+a2+a3+a4+a5+a6+a7 \leq 1.0$; $M^+$ represents an ion selected from the group consisting of an ammonium ion, a sodium ion, a potassium ion, and a silver ion.

**6.** The bio-electrode according to any one of claims 3 to 5, wherein the ionic material (A) comprises an ammonium ion shown by the following general formula (3) as an ammonium ion for forming the ammonium salts,

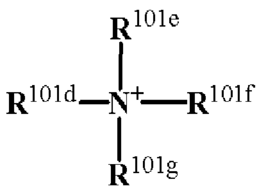
(3)

wherein $R^{101d}$, $R^{101e}$, $R^{101f}$, and $R^{101g}$ each represent a hydrogen atom, a linear, branched, or cyclic alkyl group having 1 to 15 carbon atoms, a linear, branched, or cyclic alkenyl group or alkynyl group having 2 to 12 carbon atoms, or an aromatic group having 4 to 20 carbon atoms, and optionally have one or more selected from the group consisting of an ether group, a carbonyl group, an ester group, a hydroxy group, an amino group, a nitro group, a sulfonyl group, a sulfinyl group, a halogen atom, and a sulfur atom; and $R^{101d}$ and $R^{101e}$, or $R^{101d}$, $R^{101e}$, and $R^{101f}$, are optionally bonded to each other together with a nitrogen atom bonded therewith to form a ring in which $R^{101d}$ and $R^{101e}$, or $R^{101d}$, $R^{101e}$, and $R^{101f}$, represent an alkylene group having 3 to 10 carbon atoms, or to form a heteroaromatic ring having the nitrogen atom in the general formula (3) within the ring.

**7.** The bio-electrode according to any one of claims 3 to 6, further comprising, in addition to the component (A), an adhesive resin as a component (B) that is different from the component (A), which is one or more selected from the group consisting of a silicone resin, a (meth) acrylate resin, and a urethane resin.

**8.** The bio-electrode according to claim 7, wherein the component (B) comprises a diorganosiloxane having an alkenyl group and an organohydrogenpolysiloxane having a SiH group.

**9.** The bio-electrode according to claim 7 or 8, wherein the component (B) further comprises a silicone resin having an $SiO_2$ unit and an $R_xSiO_{(4-x)/2}$ unit, wherein R represents a substituted or unsubstituted monovalent hydrocarbon group having 1 to 10 carbon atoms, and "x" represents a number in a range of 2.5 to 3.5.

**10.** The bio-electrode according to any one of claims 3 to 9, further comprising a component (C), which is one or more selected from the group consisting of a carbon powder, a metal powder, a silicon powder, and a lithium titanate powder.

**11.** The bio-electrode according to claim 10, wherein the carbon powder is one or both of carbon black and carbon nanotube.

**12.** The bio-electrode according to any one of claims 1 to 11, wherein the conducting path comprises one or more selected from the group consisting of gold, silver, silver chloride, platinum, aluminum, magnesium, tin, tungsten, iron, copper, nickel, stainless steel, chromium, titanium, carbon, and a conductive polymer.

**13.** The bio-electrode according to any one of claims 1 to 12, wherein the conducting path has a snap shape.

**14.** The bio-electrode according to any one of claims 1 to 13, wherein a total film thickness of the stretchy base material and the conductive film in combination is 1 mm or less.

**15.** The bio-electrode according to claim 14, wherein the total film thickness of the stretchy base material and the conductive film in combination is 500 μm or less.

**16.** The bio-electrode according to any one of claims 1 to 15, wherein the stretchy base material includes an adhesive layer.

**17.** The bio-electrode according to any one of claims 1 to 16, wherein a top of the conductive film is covered with a release liner.

**18.** The bio-electrode according to claim 17, wherein the release liner is selected from the group consisting of a fluorine resin, polyethylene, polypropylene, cyclic polyolefin, polyethylene terephthalate, polybutylene terephthalate, polyethylene naphthalate, polybutylene naphthalate, polyvinyl chloride, polymethylmethacrylate, polyvinyl acetate, polystyrene, polymethylpentene, polyimide, polyphenylene sulfide, polysulfone, polyethersulfone, polyetherketone, polyamide-imide, polyetherimide, polyacetal, polycarbonate, polyphenylene ether, polyacrylonitrile, cellophane, and paper, to which, excluding the fluorine resin, a fluorine-based peeling agent or a silicone/fluorine-based peeling agent is applied.

**19.** A production method for the bio-electrode according to any one of claims 1 to 18, the method comprising: preparing a conducting path that penetrates a porous, stretchy base material, or preparing a conducting path on the stretchy base material so that the conducting path is exposed on a side surface of the stretchy base material and forming an adhesive, conductive film comprising silicon so as to be connected to one side of the conducting path, the one side being attached to a skin.

**20.** The production method for the bio-electrode according to claim 19, the method comprising:

forming the adhesive, conductive film containing silicon on one side of the conducting path that penetrates the porous, stretchy base material, or the conducting path that is on the stretchy base material and exposed on a side surface of the stretchy base material, the one side being attached to a skin,
by transferring the formed, adhesive, conductive film comprising silicon onto a release linear or by directly printing the formed, adhesive, conductive film comprising silicon onto the conducting path.

**21.** The production method for the bio-electrode according to claim 19 or 20, further comprising applying an adhesive, conductive film material comprising silicon onto a release linear and curing the adhesive, conductive film material.

**22.** A measurement method for a bio-signal comprising:

attaching the bio-electrode according to any one of claims 1 to 18 to a skin; and
measuring the bio-signal after showering, or during bathing or showering.

[FIG. 1]

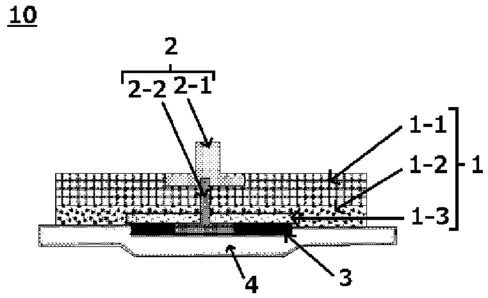


[FIG. 2]

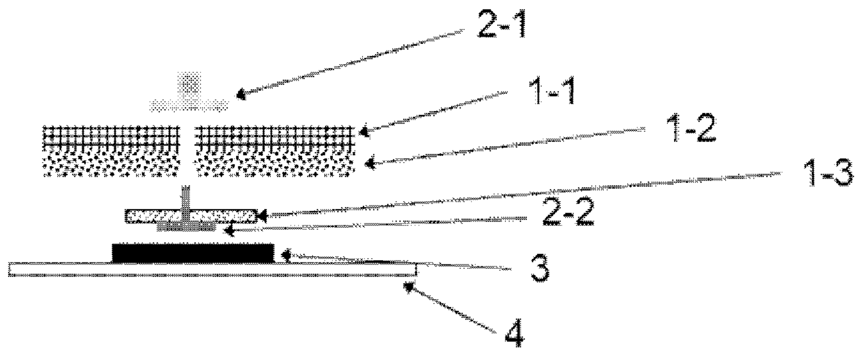


[FIG. 3]

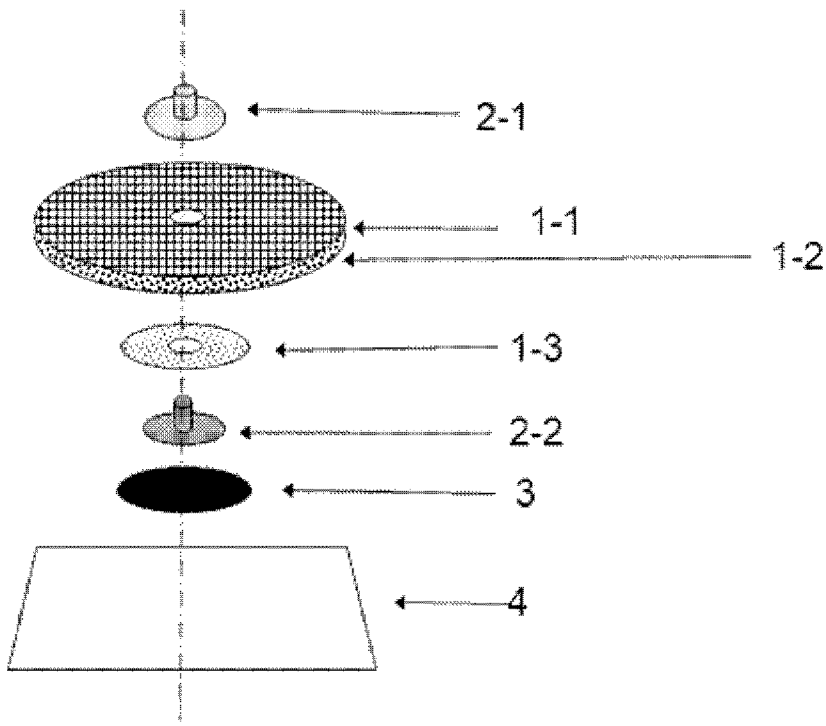

[FIG. 4]

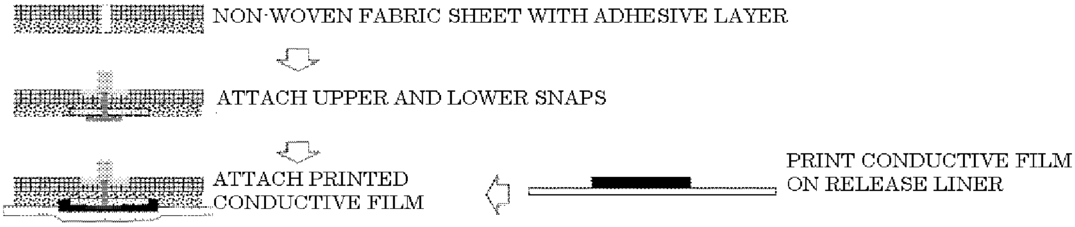


[FIG. 5]

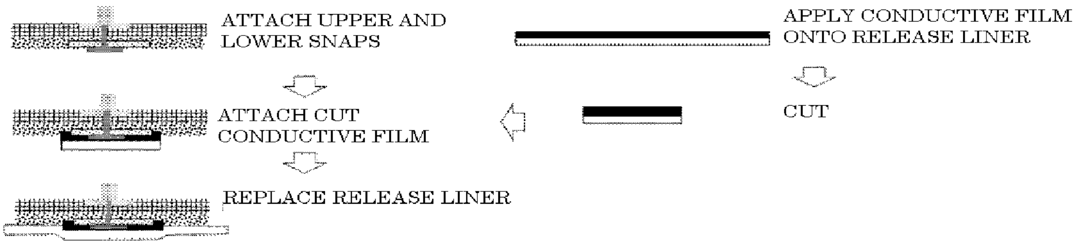


[FIG. 6]

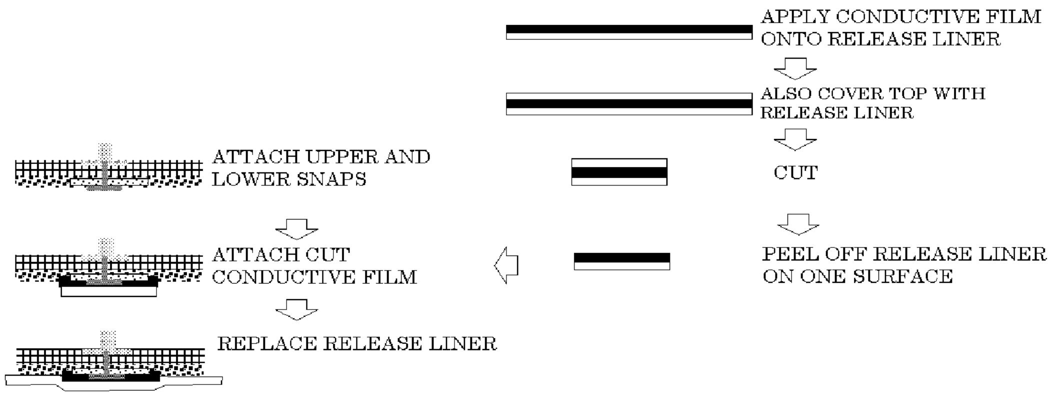


[FIG. 7]

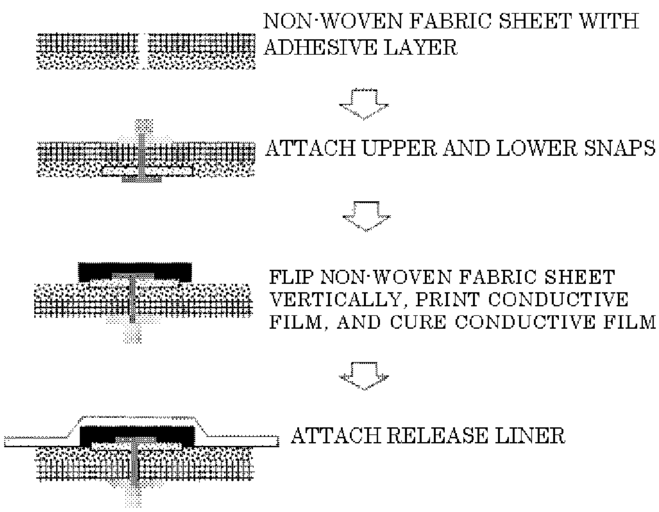

[FIG. 8]

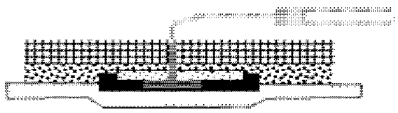

[FIG. 9]

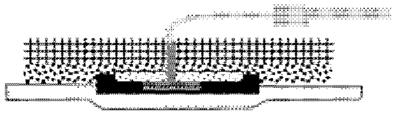

[FIG. 10]

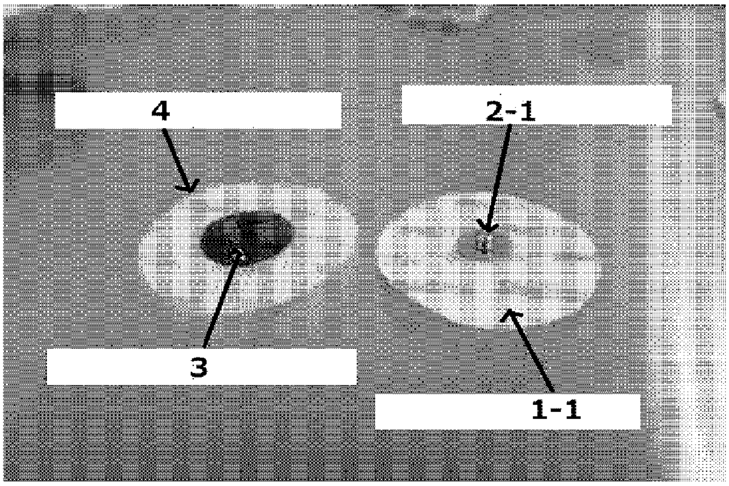


[FIG. 11]

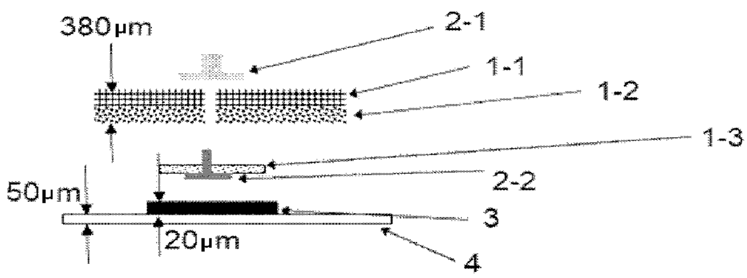


[FIG. 12]

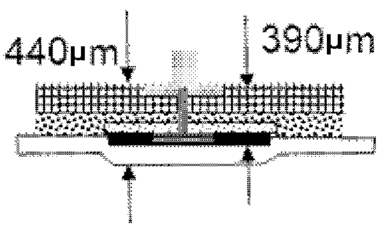

[FIG. 13]

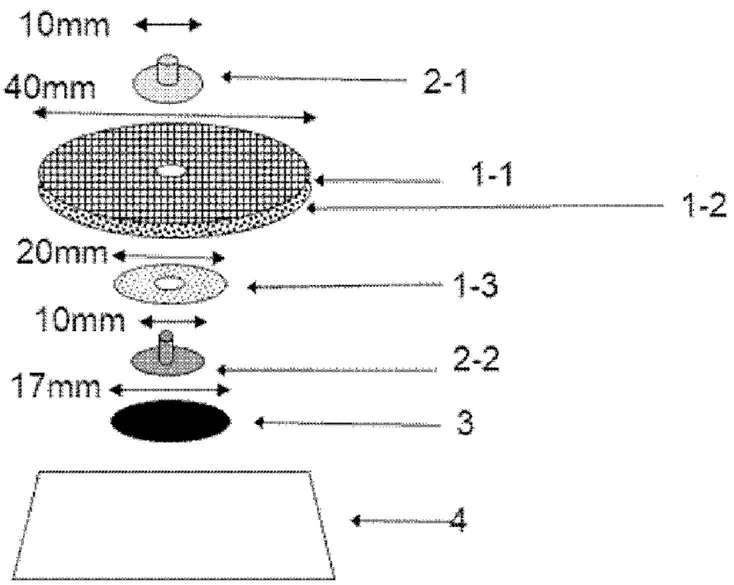


[FIG. 14]

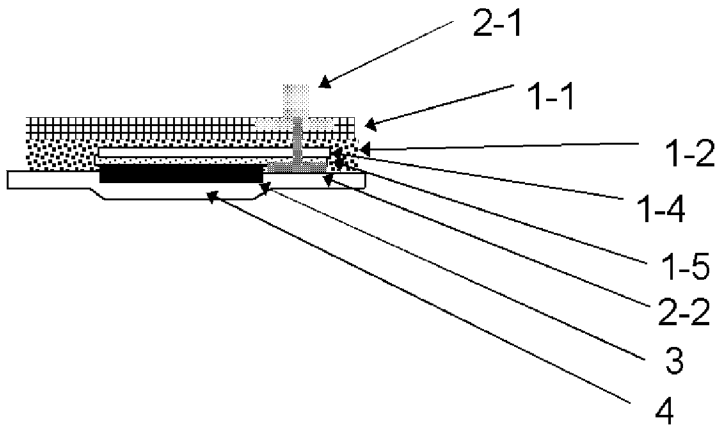


[FIG. 15]

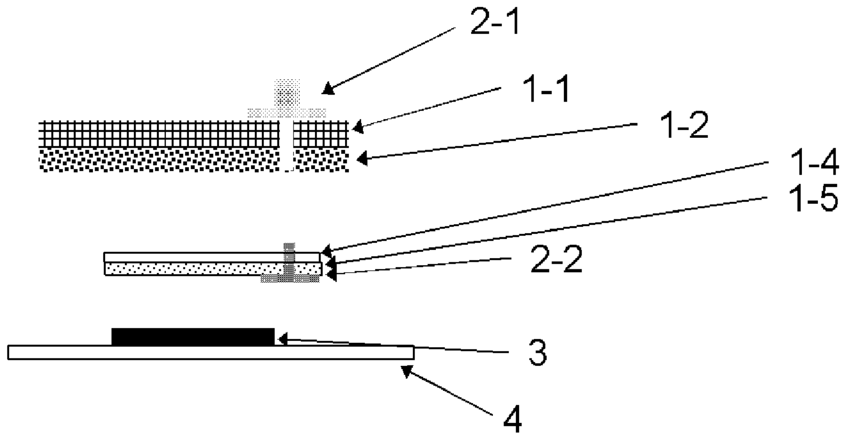

[FIG. 16]

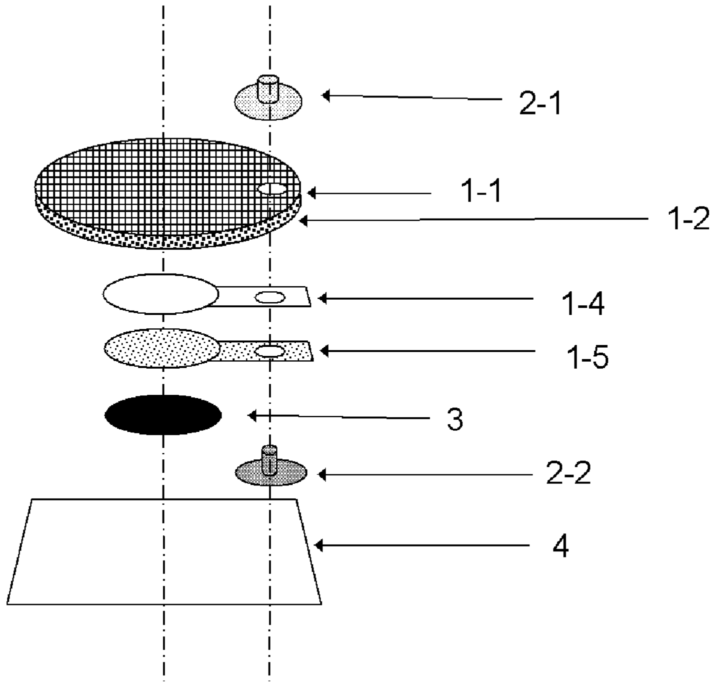


[FIG. 17]

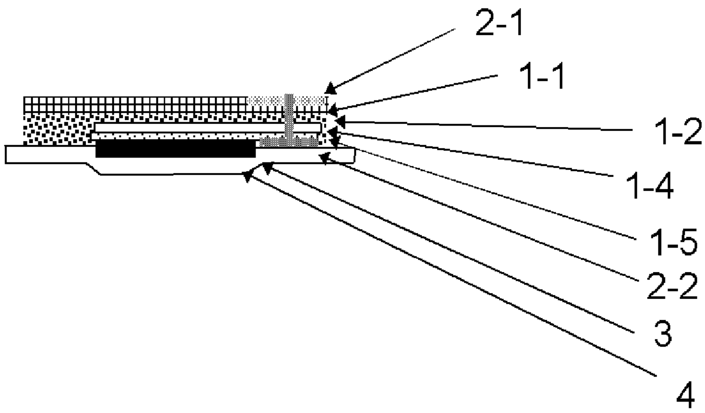


[FIG. 18]

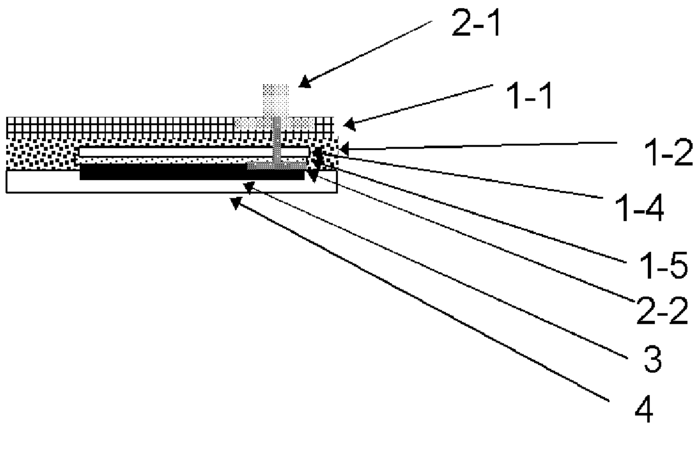

[FIG. 19]

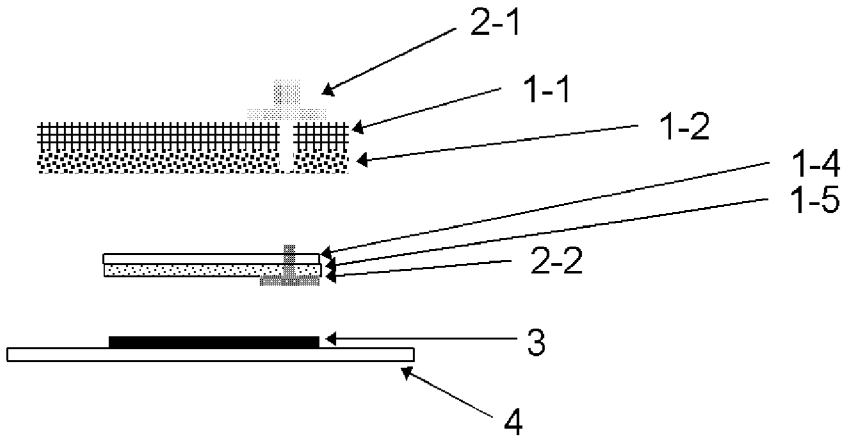


[FIG. 20]

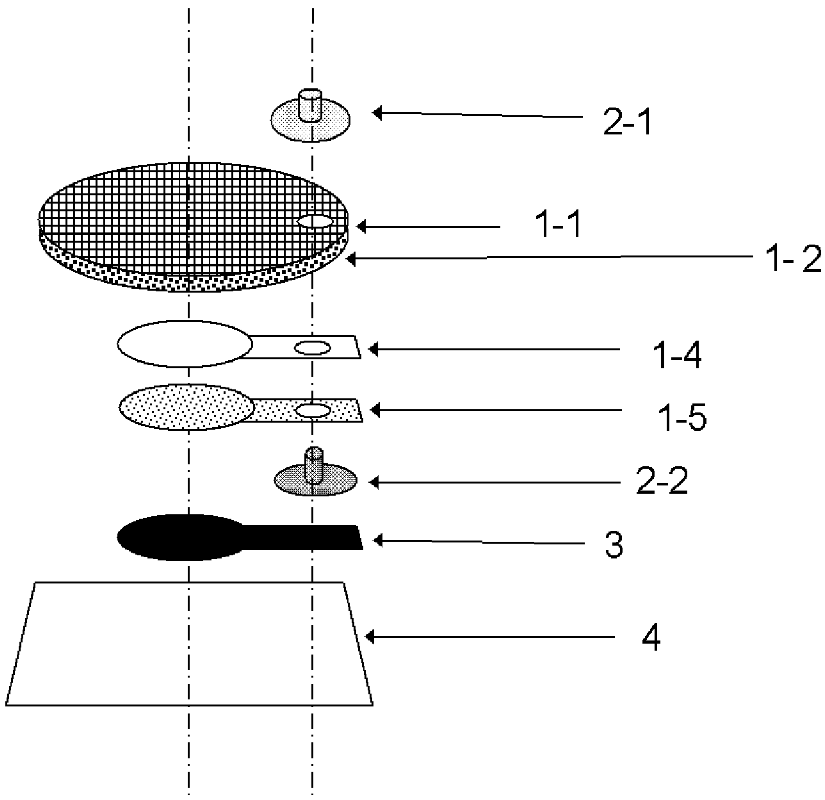

[FIG. 21]

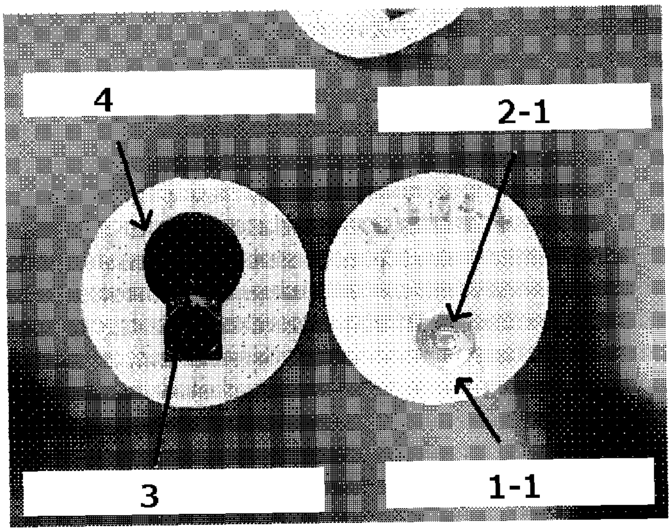


[FIG. 22]

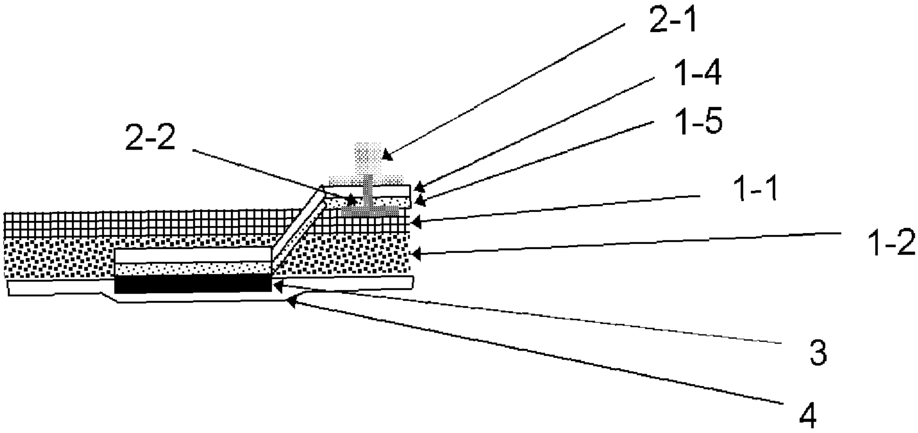


[FIG. 23]

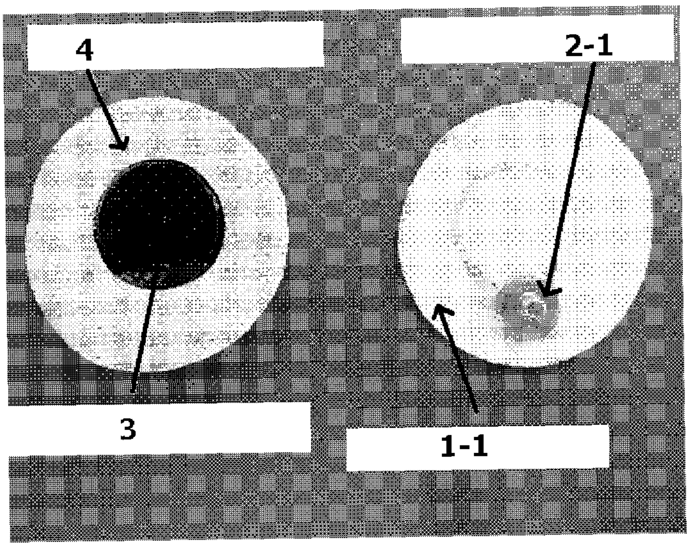

[FIG. 24]

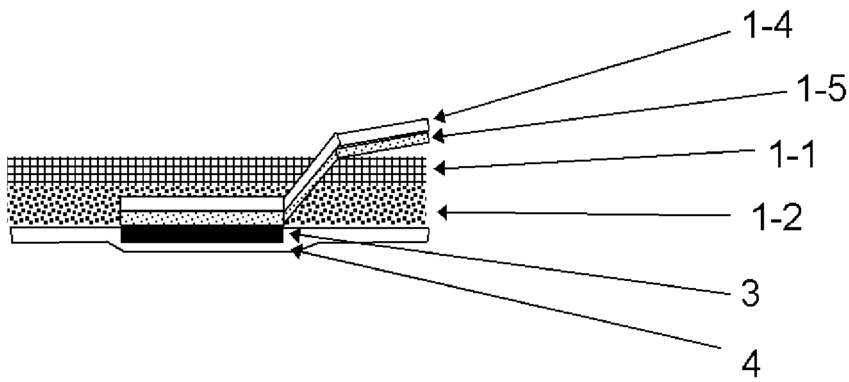


[FIG. 25]

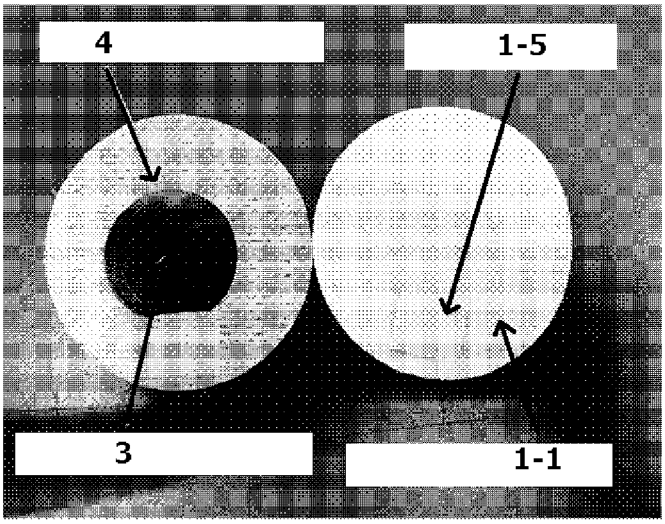


[FIG. 26]

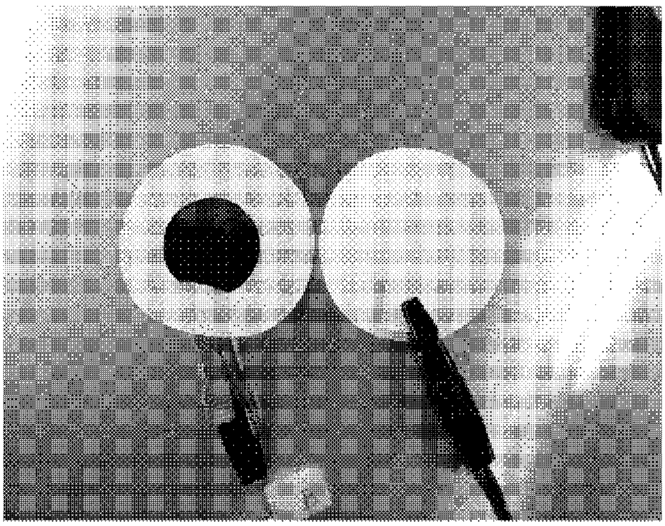

[FIG. 27]

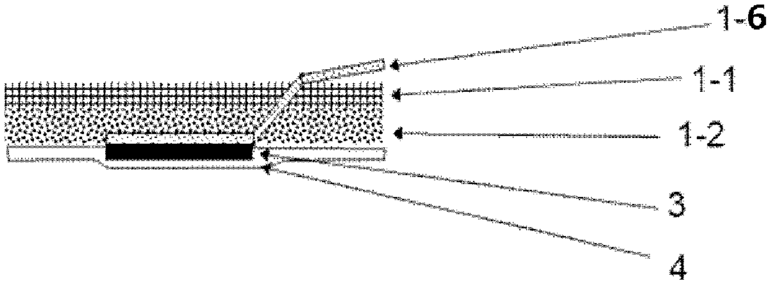


[FIG. 28]

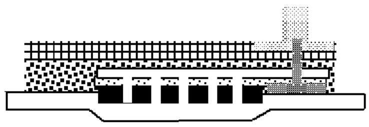

[FIG. 29]

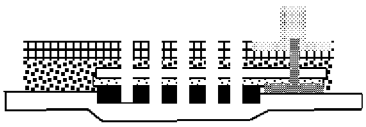

[FIG. 30]

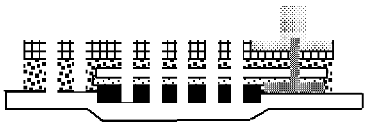

[FIG. 31]

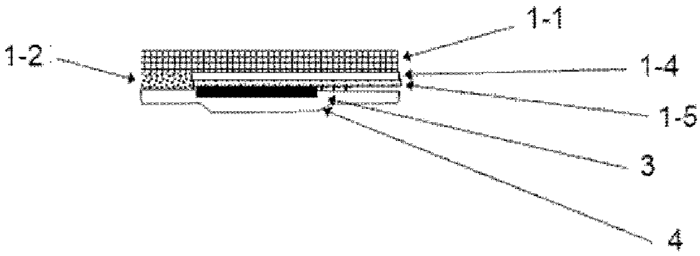


[FIG. 32]

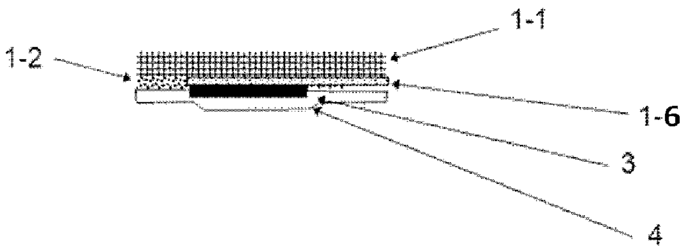

[FIG. 33]

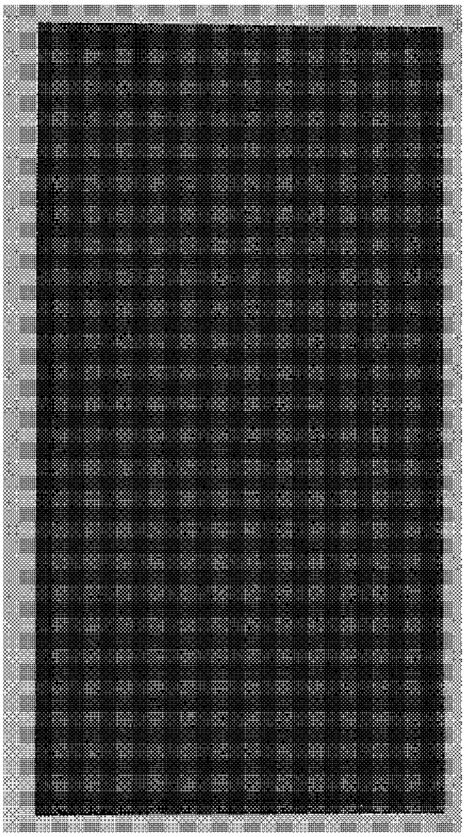

[FIG. 34]

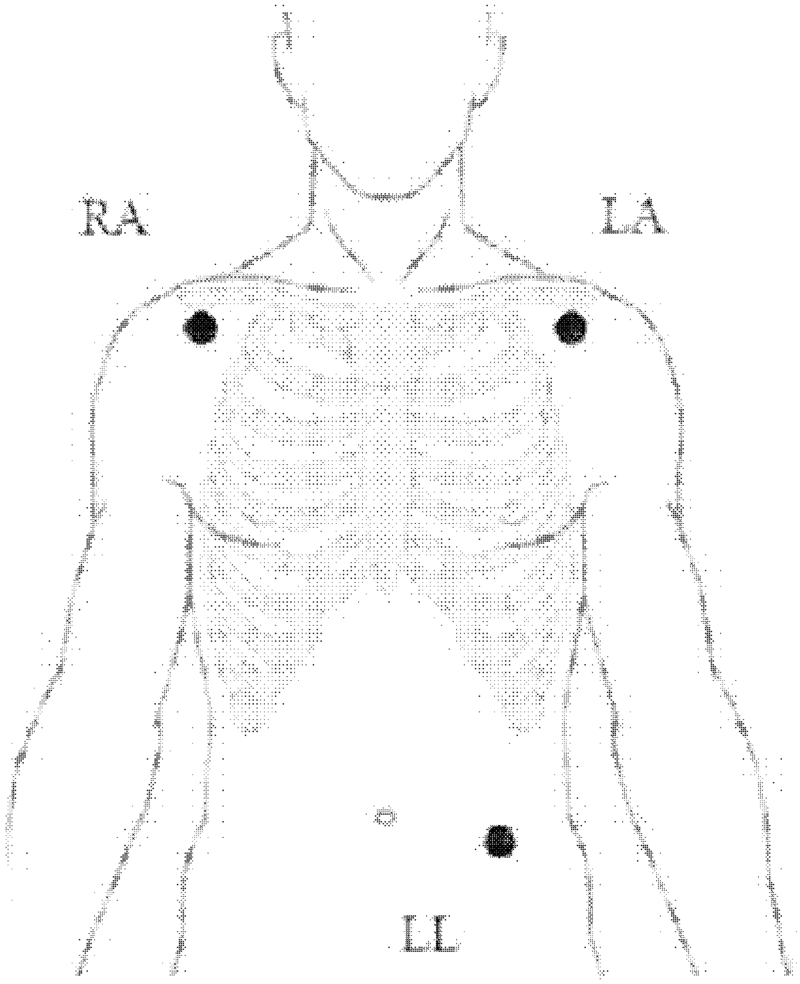


[FIG. 35]

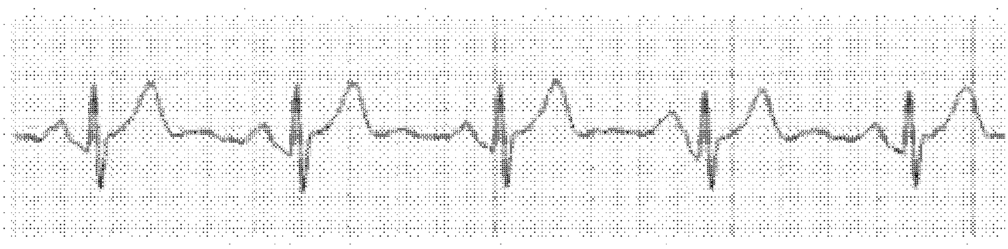

**INTERNATIONAL SEARCH REPORT**

International application No. **PCT/JP2021/048074**

**A. CLASSIFICATION OF SUBJECT MATTER**

***C08F 212/14***(2006.01)i; ***C08F 220/38***(2006.01)i; ***A61B 5/25***(2021.01)i; ***A61B 5/266***(2021.01)i
FI: A61B5/25; A61B5/266; C08F212/14; C08F220/38

According to International Patent Classification (IPC) or to both national classification and IPC

**B. FIELDS SEARCHED**

Minimum documentation searched (classification system followed by classification symbols)

A61B5/24

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2546264 Y2 (KURARAY CO., LTD.) 27 August 1997 (1997-08-27) paragraphs [0002]-[0024], fig. 1-4 | 1-22 |
| Y | JP 2021-3591 A (ASAHI KASEI CORP.) 14 January 2021 (2021-01-14) paragraphs [0017]-[0081], fig. 1-16 | 1-22 |
| Y | JP 2019-180467 A (SHIN ETSU CHEMICAL CO., LTD.) 24 October 2019 (2019-10-24) paragraphs [0014]-[0198], fig. 1-4 | 4-22 |
| Y | JP 7-500994 A (MINNESOTA MINING AND MANUFACTURING COMPANY) 02 February 1995 (1995-02-02) fig. 1-4 | 19-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 January 2022** | **22 March 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/048074**

| Patent document cited in search report | Publication date (day/month/year) | Patent family member(s) | Publication date (day/month/year) |
|---|---|---|---|
| JP 2546264 Y2 | 27 August 1997 | (Family: none) | |
| JP 2021-3591 A | 14 January 2021 | (Family: none) | |
| JP 2019-180467 A | 24 October 2019 | US 2019/0298891 A1<br>paragraphs [0018]-[0243], fig. 1-4 | |
| JP 7-500994 A | 02 February 1995 | WO 1993/009713 A1<br>fig. 1-4 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2013039151 A1
- JP 2015100673 A
- JP 2000271100 A
- JP 2018515279 A
- JP 2018126496 A
- JP 2018130533 A
- JP 2018099504 A
- JP 2019180467 A
- JP 2015193803 A
- JP 2016011338 A
- JP 2000256640 A
- JP 2019076695 A
- JP 2019076696 A
- JP 2008111103 A
- JP 2009080474 A
- JP 2019503406 A
- JP 2018044147 A
- JP 2018059050 A
- JP 2018059052 A
- JP 2018130534 A
- JP 2011201033 A
- JP 2020100764 A
- JP 2004033468 A
- JP H5082405 A
- JP H5009505 A